# EUROPEAN PATENT SPECIFICATION

(11) **EP 3 487 526 B1**
(45) Date of publication and mention of the grant of the patent: **06.07.2022**
(21) Application number: 17746213.2
(22) Date of filing: 21.07.2017
(51) Int. Cl.: A61K 39/015, A61K 39/385, A61K 39/00

(54) **BIOFUSION PROTEINS AS ANTI-MALARIA VACCINES**
BIOFUSIONSPROTEINE ALS ANTI-MALARIA-IMPFSTOFFE
PROTÉINES DE BIOFUSION COMME VACCINS ANTIPALUDIQUES

(30) Priority: 21.07.2016 EP 16180544
(43) Date of publication of application: 29.05.2019
(73) Proprietor: VAC4all Pte. Ltd., Singapore 588177 (SG)
(72) Inventor: DRUILHE, Pierre, 75015 Paris (FR)
(74) Representative: Ernest Gutmann - Yves Plasseraud S.A.S.
(86) International application number: PCT/SG2017/050369
(87) International publication number: WO 2018/017020

(56) References cited:
- EP-A1- 2 716 661
- WO-A1-00/50077
- WO-A1-2004/043488
- WO-A1-2005/040206
- WO-A2-2005/058941
- A. TOURE-BALDE ET AL: "Evidence for Multiple B- and T-Cell Epitopes in Plasmodium falciparum Liver-Stage Antigen 3", INFECTION AND IMMUNITY, vol. 77, no. 3, 12 January 2009 (2009-01-12), pages 1189-1196, XP055313334, US ISSN: 0019-9567, DOI: 10.1128/IAI.00780-07
- DRAPER SIMON J ET AL: "Recent advances in recombinant protein-based malaria vaccines", VACCINE, vol. 33, no. 52, 11 October 2015 (2015-10-11), pages 7433-7443, XP029344008, ISSN: 0264-410X, DOI: 10.1016/J.VACCINE.2015.09.093
- Sirima, S: "Protection against Malaria by MSP3 Candidate Vaccine", New England Journal of Medicine, vol. 365 15 September 2011 (2011-09-15), pages 1062-1064, XP055313330, DOI: 10.1056/NEJMc1100670 Retrieved from the Internet: URL:http://www.nejm.org/doi/pdf/10.1056/NE JMc1100670 [retrieved on 2016-10-25]
- MOORTHY V S ET AL: "Malaria vaccine developments", THE LANCET, THE LANCET PUBLISHING GROUP, GB, vol. 363, no. 9403, 10 January 2004 (2004-01-10), pages 150-156, XP004764081, ISSN: 0140-6736, DOI: 10.1016/S0140-6736(03)15267-1

## Description

The present invention relates to the protection against malaria, more particularly to a malaria vaccine which comprises a fusion protein.

### Background of the invention

The parasites responsible for malaria in human, including especially *Plasmodium falciparum,* exhibit different morphologies in the human host and express different antigens as a function of their localization in the organism of the infected host. The morphological and antigenic differences of these parasites during their life cycle in man enable at least four distinct stages of development. The very first stage of development of the parasite in human corresponds to the sporozoite form introduced into the blood of the host by bites of insect vectors of the parasite. The second stage corresponds to the passage of the parasite into the liver and to the infection of the hepatic cells in which the parasites develop to form the hepatic schizonts which, when they are mature (for example, in the case of *P. falciparum* on the 6th day after penetration of the sporozoites) release hepatic merozoites by bursting. The third stage is characterized by the infection of the blood erythrocytes by the asexual forms (blood stages schizonts) of the parasite releasing free merozoites when they mature; this erythrocytic stage of development corresponds to the pathogenic phase of the disease. The fourth stage corresponds to the formation of the forms with sexual potential (or gametocytes) which will become extracellular sexual forms or gametes in the mosquito.

There is no practical or effective vaccine that has been introduced into clinical practice. Malaria sub-unit vaccines have so far shown a major deficiency in immunogenicity. The immune responses that are observed are at low titers and of short duration. A variety of approaches have been explored to address this deficiency, including a selection of adjuvants, expression in complex vectors such as virus-like particles (VLPs), adenovirus vectors, and setting up of complex prime-boost protocols. Even the RTS,S/AS01 vaccine (Mosquirix^{®}), which was engineered using genes from the repeat and T-cell epitope in the pre-erythrocytic circumsporozoite protein (CSP) of the Plasmodium falciparum malaria parasite and a viral envelope protein of the hepatitis B virus (HBsAg), with sophisticated adjuvants such as ASO1, suffers from very short duration of malaria-specific immune responses (whereas hepatitis B responses remain high for long).

Another approach which has been used before concerns fusion proteins comprising MSP and a futher Plasmodium antigen, GLURP, as disclosed in WO2005/040206.

Chemical conjugation of Plasmodium antigens to carrier proteins has also been disclosed. For instance a vaccine comprising Plasmodium protein Pfs25H conjugated to a detoxified form of Pseudomonas aeruginosa exoprotein A (repA) is under evaluation.

However chemical conjugation has several drawbacks. It is technically demanding, hardly reproducible, and quite expensive.

There is still a great need for vaccine products which show a good immunogenicity and protection against malaria, while being easy to reproduce and affordable.

### Summary of the invention

The invention offers the advantage of an improved immunogenicity, thanks to novel constructs as defined in the appended claims.. Rather than coupling chemically the Plasmodium molecule to the carrier protein, the inventors developed a novel approach in which the parasite protein is encoded together with the carrier protein as a fusion protein, making a "bio-conjugated fusion product", also designated further herein as "bio-fusion molecule", which can be produced recombinantly, e.g. in E.coli.

The invention more particularly provides a fusion protein which comprises at least one antigenic amino acid sequence fused at the N-term and/or C-term of a carrier heterologous protein sequence, wherein the antigenic sequence comprises an epitope sequence of a Plasmodium protein and the carrier heterologous protein sequence is a sequence that is immunogenic in humans, wherein the antigenic sequence and the carrier heterologous protein sequence are as defined in the appended claims.

According to the invention, the antigenic sequence is MSP3, LSA3, or LSA5, or an epitopic fragment of MSP3 that encompasses motifs a, b, c, d, e and/or f of the C-terminal region of a Plasmodium MSP3 protein chosen among MSP3-1, MSP3-2, MSP3-3, MSP3-4, MSP3-7, MSP3-8, preferably selected from SEQ ID NO: 7 to SEQ ID NO: 54, or an epitopic fragment of LSA3 that is peptide DG729 of SEQ ID NO: 77.

A further object of the invention is a nucleic acid construct encoding the fusion protein as defined herein.

Also provided are a vector comprising the nucleic acid construct in an expression cassette as well as a host cell wherein the vector has been inserted.

It is further described an *in vitro* method for preparing a fusion protein, which method comprises allowing the host cell to reproduce under conditions which induce expression of the nucleic acid construct, and collecting the fusion protein which is so expressed.

According to the invention, a vaccine is provided, which comprises said fusion protein or a combination of at least two fusion proteins, which respectively comprise at least two different antigenic amino acid sequences, or a nucleic acid construct encoding said fusion protein(s), with a physiologically acceptable vehicle.

Such vaccine is useful in vaccinating a human subject against malaria.

### Legends to the figures

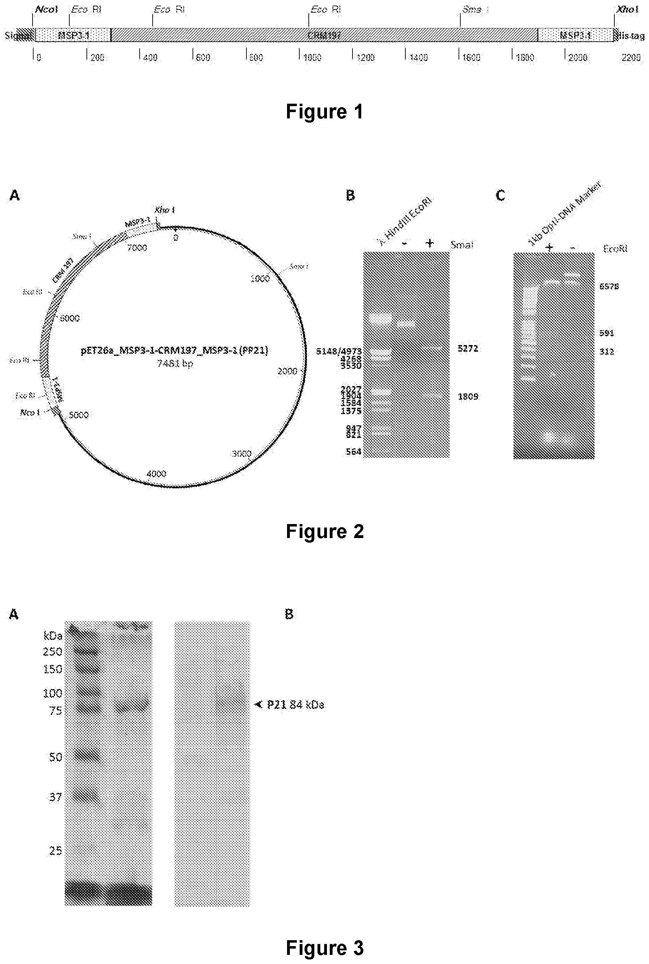

**Figure 1** is a diagramatic representation of the coding sequences for PP21 (also named "Bio-fusion MSP3-1). Scales in base pairs are shown below starting at the Nco I site.
**Figure 2** shows pET26a-PP21 plasmid map (A), mini-preparation analysis by restriction enzyme digestion with Sma I (B) or Eco R I (C).
**Figure 3****:** Recombinant protein PP21 purified by IMAC. (A) Analysis by electrophoresis under denaturing conditions and Coomassie blue staining. (B) Antigenicity assessed by Western blotting with murine anti-MSP3 immune sera at a 1:1000 dilution. The upper band observed is compatible with the theoretical molecular weight of the full length PP21 of 84 kDa. Another band at 37 kDa, less well recognized by the anti-MSP3 immune sera, could correspond to a degradation fragment of PP21.

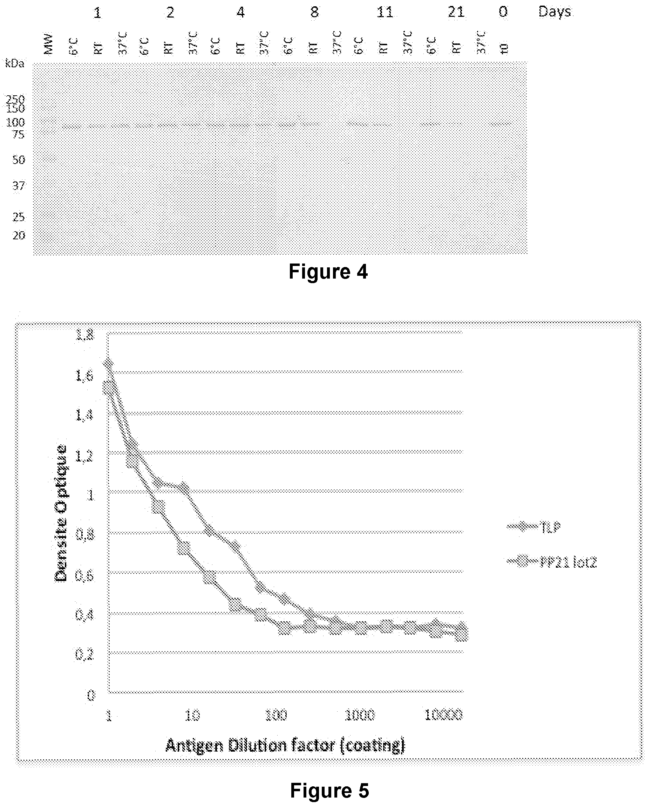

**Figure 4****:** Timescale of PP21 stability at different storage temperatures assessed by Western blotting with the anti-MSP3-1 mAb RAM1 at a 1:1000 dilution.
**Figure 5****:** Determination of the Antigen Content of PP21 as compared to the positive control MSP3-LSP. The antigenicity of pre-clinical PP21 was determined by reactivity with a large panel of human and animal anti-MSP3 sera, including a human recombinant antibody. The antigen content provides a more precise measurement of the quantity of reactive epitopes. It is performed by serial dilutions of the antigen, used for coating in an Elisa assay and revealed by a well-defined positive control (here a pool of rats immunized by the C-term region of MSP3). The reactivity of PP21 was identical to that of MSP3 synthetic and recombinant constructs

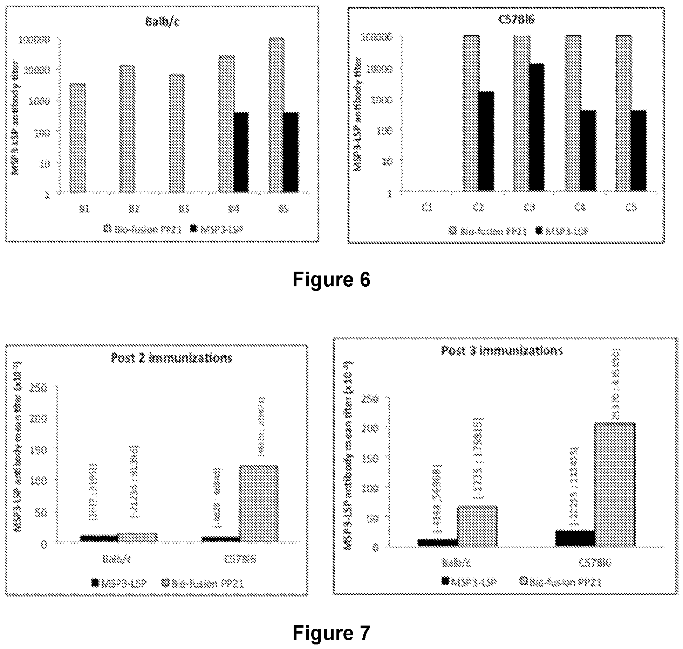

**Figure 6****:** Significant improvement in specific antibody response induced in mice following 2 immunizations by the Bio-fusion MSP3-1 conjugate as compared to the current MSP3-1-LSP. Groups of 5 (BALB/c) or 4 (C57BL/6) mice were immunized using either only 1 µg of bio-fusion MSP3-1 or 10 µg of MSP3-1 LSP in Montanide ISA720 on days 0, 14 and 28. Blood samples were collected one week after the 2^{nd} injection, on day 21 (A) and one week after the 3^{rd} immunization, on day 35. Specific MSP3-1 LSP antibody titers were determined by ELISA. The results for each mouse (1 to 5) are shown separately (here after only two immunizations).
**Figure 7****:** Comparison of antibody response induced in mice following 2 versus 3 immunizations by the Bio-fusion MSP3-1 as compared to the current MSP3-1-LSP. The third injection boosted the antibody response and confirmed the better immunogenicity of Bio-fusion MSP3-1 in C57B1/6 mice and Balb/c mice. Mean titers of MSP3-1-LSP specific antibodies induced by 3 injections of 1 µg of Bio-fusion MSP3-1 reached 3×10⁵ and 2×10⁵ in C57B1/6 and Balb/c respectively; whereas 3 injections of 10 µg of MSP3-1-LSP induced a maximum of 10⁵ and 2.5×10⁴ mean titers in C57Bl/6 and Balb/c mice respectively.

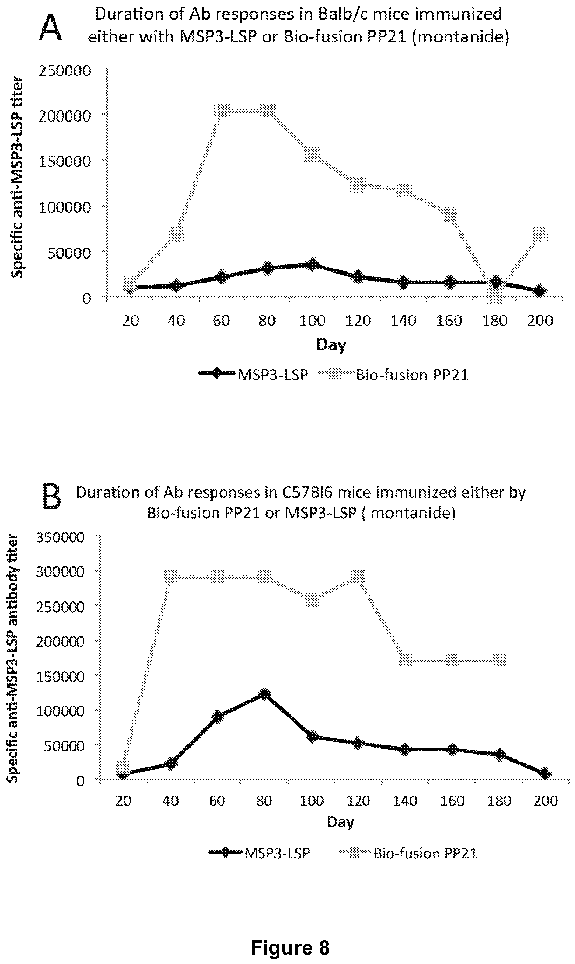

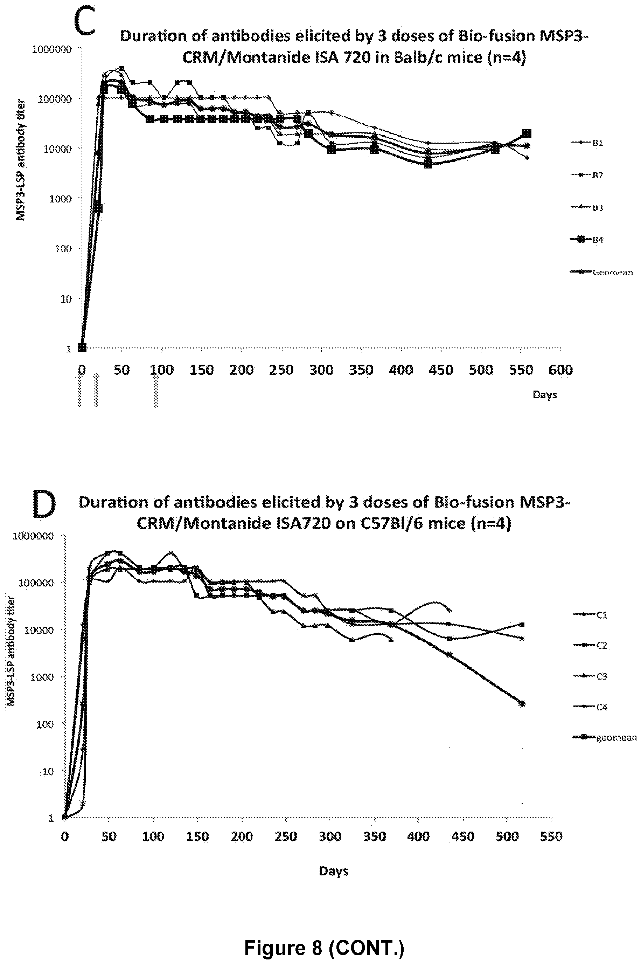

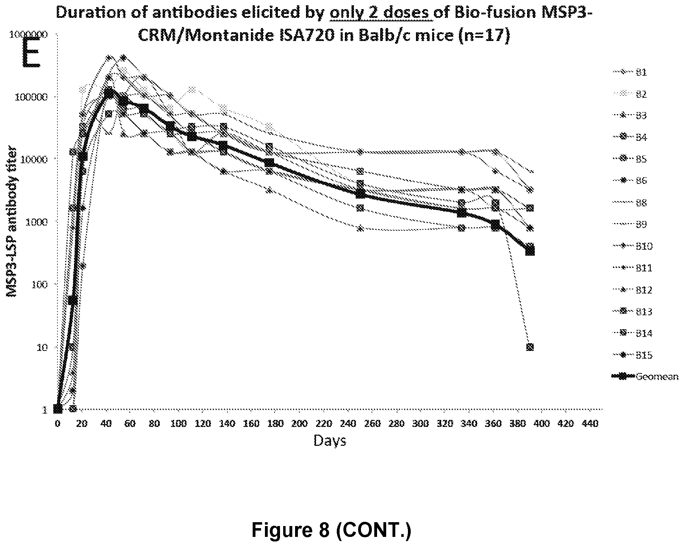

**Figure 8****:** Major improvement in duration of MSP3-1 specific antibodies induced in mice by the Bio-fusion MSP3-1 as compared to the current MSP3-1-LSP.
   A and B : comparison of Bio-Fusion MSP3-CRM with MSP3-1-LSP: BALB/c (A-upper left panel) and C57BL/6 (B-upper right panel) mice were immunized using either only 1 µg of Bio-fusion MSP3-1 or 10 µg of MSP3-1 LSP in Montanide ISA720 on days 0, 14 and 28. Blood samples were collected at different days over the follow-up. Specific MSP3-1 LSP antibody titers were determined by ELISA. The results shown correspond to the geometric mean of anti-MSP3-1 LSP antibody titers obtained in each group of mice at different days. After 160 days of follow-up the mean antibody titer in the Bio-fusion-MSP3-1 conjugate immunized mice was 1.5×10⁵ and 10⁵ in C57bl/6 and Balb/c respectively, which is at least 10 fold higher than the mean titer of antibodies induced by MSP3-1-LSP and measured at day 160 after the first immunization.
   C and D : Long-term persistence of immune responses elicited by MSP3-CRM: Groups of Balb/c and C57B16 mice (4 to 5 mice per group) were immunized by subcutaneous injection on days 0, 14 and 103 of 1µg of Bio-fusion MSP3-CRM adjuvanted in Montanide ISA 720 adjuvant (v/v). Blood samples were collected at different days. Specific MSP3 antibody titers were determined by ELISA. Results show that the titer increases remarkably after two immunizations and reaches at day 49 a maximum, whereas the third antigen injection, performed on day 103, did not induce any detectable increase in the magnitude of the antibody response. These specific antibodies decreased only slightly over time, and remained detectable at high titer even 540 days after immunization
   E: Long-term persistence of immune responses elicited by MSP3-CRM after only 2 immunisations. In view of satisfactory responses observed after only two immunisations, this immunization scheme was analyzed in further seventeen Balb/c mice immunized by subcutaneous injection on days 0 and 14 only, using 2µg of Bio-fusion MSP3-CRM adjuvanted in Montanide ISA 720 (v/v). Blood samples were collected at different days. Specific MSP3-1-LSP antibody titers were determined by ELISA. Results show that the MSP3 antibody titer increased after two antigen injections and reaches a maximum between days 42 and 72 in all mice, and show that immune responses decreased only modestly over the 380 days follow-up of the experiment.

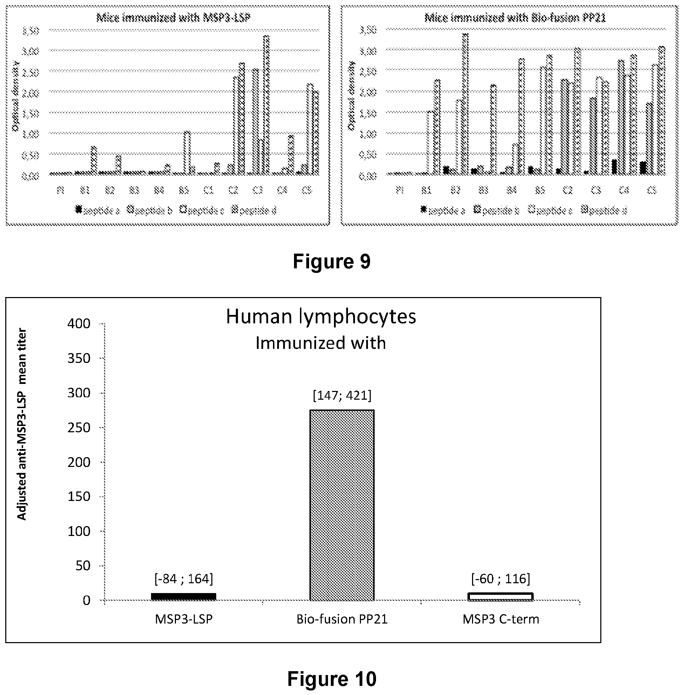

**Figure 9****:** Improved recognition of MSP3-1 ADCI (Ab-dependent cellular inhibition) target epitopes by antibodies induced in mice immunized with Bio-fusion MSP3-1 as compared to MSP3-1-LSP. In the MSP3-1-LSP, The peptides "b", "c" and "d" define distinct B-cell epitopes targeted by antibodies effective in the ADCI assay correlated to the protective effect of ant-MSP3-1 antibodies. To evaluate the ability of Bio-fusion MSP3-1 to induce antibodies to these epitopes we determined by ELISA the titers of antibodies to the peptides a, b, c and d in the sera. Hence the number of ADCI targets recognized by the novel formulation is considerably wider, and with higher titers. BALB/c and C57BL/6 mice were immunized using either only 1 µg of Bio-fusion MSP3-1 or 10 µg of MSP3-1 LSP in Montanide ISA720 on days 0, 14 and 28. Blood samples were collected one week after the 3^{rd} immunization, on day 35. The mice sera were diluted at 1/400 and the titer of specific antibodies of different MSP3-1 peptides (a, b, c and d) was determined by ELISA. The results shown correspond to the optical density obtained with the sera of each mouse tested.
**Figure 10****:** Human antibody responses in the HIMM model (Human Immunogenicicty Mouse Model) immunized either with 1 µg of Bio-fusion MSP3-1, 10 µg of the current MSP3-1 LSP or 10 µg of the MSP3-1 C-term recombinant protein. NOD.Cg - Prkdc^{scid} - IL2rg^{tm1WjI} /SzJ (NSG) mice were engrafted with human spleen cells stimulated or not in vitro with 1 µg/ml of antigen (4 mice per group). Each NSG mouse received an intraperitoneal injection (ip) of 30 × 10⁶ antigen-primed or unprimed spleen cells. At days 7 and 21, reconstituted mice were boosted with 1 µg of Bio-fusion MSP3-1, or 10 µg of MSP3-1-LSP or 10 µg of MSP3-1 C-term recombinant protein. All antigens were adjuvanted with Montanide ISA 720 (v/v) and injected intraperitoneally (ip). Mice reconstituted with unprimed spleen cells, received adjuvant alone. Blood samples were collected one week after each immunization. Specific antibodies were analyzed by total and specific IgG ELISA. The adjusted specific antibody titer was calculated using the following formula: (antigen specific antibody titer/total human IgG concentration) × 10 (10 mg/ml is considered as the mean human total IgG concentration). Geometric mean values (95 % CI) of ELISA titers are represented.

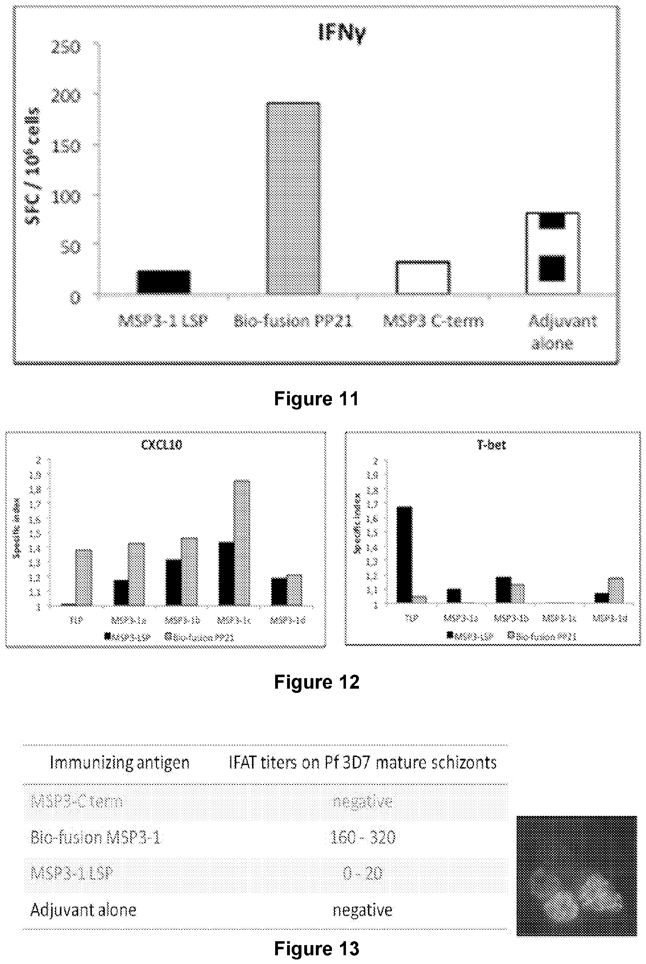

**Figure 11****:** Human IFN-γ cellular responses in the HIMM model (Hu Lymph NSG mice) immunized either with 1 µg of Bio-fusion MSP3-1, 10 µg of the current MSP3-1 LSP or 10 µg of the MSP3-1 C-term recombinant protein. Same immunizing conditions as above. Cellular responses were evaluated by IFN-γ ELISPOT. At day 28, spleen cells of each group of mice were recovered, pooled and cultured in vitro with or without antigen stimulation. The results shown correspond to the specific Spot Forming Cells (SFC) obtained with each group of mice.
**Figure 12****:** T-cell responses in HIMM: Marked improvement in CD4-Th1 responses in human lymphocytes grafted in mice immunized by either 1 µg of Bio-conjugate PP21 or 10 µg of MSP3-LSP.
**Figure 13****:** Improved recognition of parasite native proteins by human antibodies elicited in the HIMM model immunized with Bio-fusion MSP3-1. NSG mice were engrafted with human spleen cells and immunized either with 1 µg of Bio-fusion MSP3-1, 10 µg of the current MSP3-1 LSP or 10 µg of the MSP3-1 C-term recombinant protein (as described in the figure 6). The reactivity of human antibodies in day 28 mice sera, with the parasite proteins, which is a critical feature to activate the ADCI defense mechanism during a malaria attack, was evaluated by Immunofluorescence Antibody Test (IFAT) on 3D7 *Plasmodium falciparum* (Pf) infected red blood cells. The results shown correspond to the range of titers obtained with each group of mice.

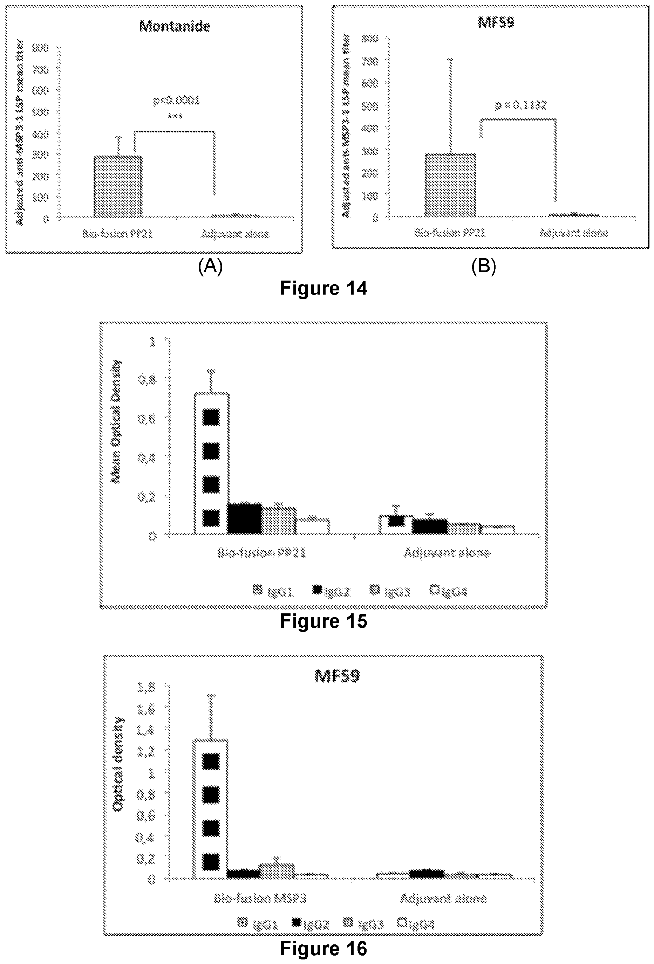

**Figure 14****:** The human antibody response elicited in the HIMM model immunized with 1 µg of Bio-fusion MSP3-1 does not depend on adjuvant used in the vaccine preparation. (A) NSG mice were engrafted with human spleen cells derived from two different donors and stimulated or not with 1 µg of Bio-fusion MSP3-1 adjuvanted with Montanide ISA720 (as described in the figure 6) (4 mice per group / donor). (B) NSG mice were engrafted with human spleen cells stimulated (9 mice) or not (8 mice) with 1 µg of Bio-fusion MSP3-1 adjuvanted with MF59 instead of Montanide ISA720 (as described in the figure 6 but with human lymphocytes from a distinct donor). In both cases, the specific MSP3-1 LSP antibody titers were determined by ELISA at day 28. The results shown correspond to the mean ± SD of the ELISA titers obtained with each group of mice.
**Figure 15****:** Antibody Subclasses of human IgG antibodies elicited in human lymphocytes in the HIMM model immunized with Bio-fusion MSP3-1 adjuvanted with Montanide ISA720. NSG mice were engrafted with human spleen cells stimulated or not with 1 µg of MSP3 Bio-fusion (as described in the figure 10). The mice sera, collected at day 28, were diluted at 1/20. The specific MSP3-1 LSP antibody titers were determined by subclass IgG ELISA. The results shown correspond to the mean ± SD of optical density obtained with immunized and non immunized mice.
**Figure 16****:** Subclasses of human IgG antibodies elicited in the HIMM model immunized with Bio-fusion MSP3-1 adjuvanted with MF59. NSG mice were engrafted with human spleen cells stimulated or not with 1 µg of Bio-fusion MSP3-1 adjuvanted with MF59 (as described in figure 14). The mice sera, collected at day 28, were diluted at 1/20. The specific MSP3-1 LSP antibody titers were determined by subclass IgG ELISA in the sera of responding mice. The results shown correspond to the mean ± SD of optical density obtained with immunized and non immunized mice. Both adjuvants led to obtain a dominance of the cytophilic subclass IgG1.

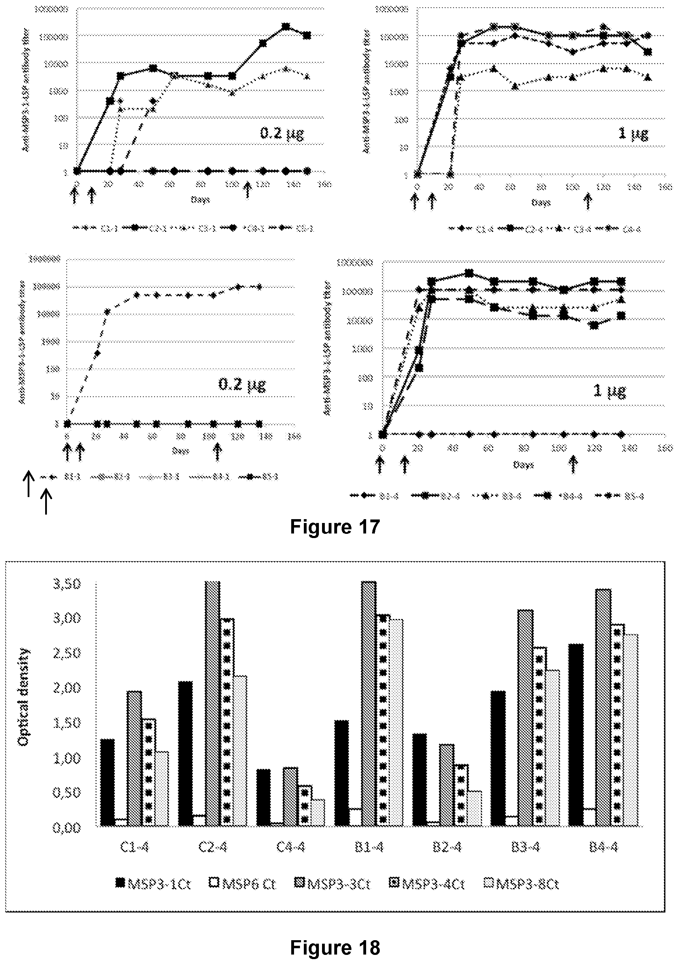

**Figure 17** **:** Immunogenicity of 0.2 or 1 µg of PP21 in Balbc mice /PP21-montanide (upper left and right), or the same doses in C57bl mice (lower left and right panels) Evolution of anti-MSP3-1-LSP antibody titers determined as in Figures 6-8
**Figure 18** : Cross-reactivity of anti-MSP3-1 Antibodies elicited by PP21 with other members of the MSP3 family of proteins (OD values by Elisa). C57B1/6 (C) and Balb/c (B) mice received 2 injections (J0, J14) of 4µg PP21. Samples were collected on day 63.

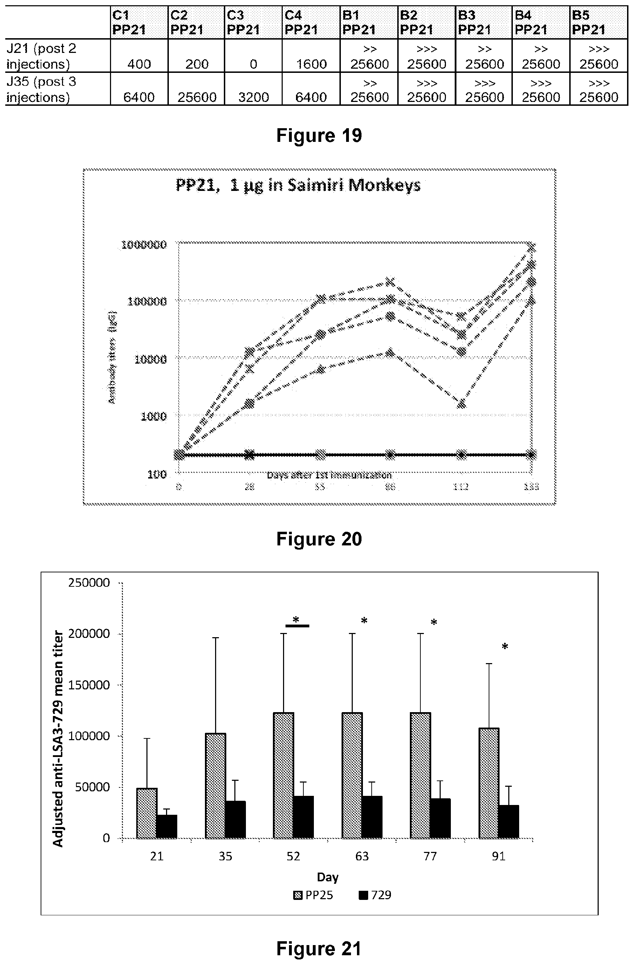

**Figure 19****:** Immunogenicity of PP21 adjuvated by Alum Hydroxyde in C57BL(C) and Balb/C mice(B), at a 1 µg dose per immunisation. Antibody Titers determined by Elisa in C57BL (C) and Balb/C mice (B), are shown following 2 immunisations or 3 immunisations. Higher dilutions than 25:600 were not tested. Note that Alum OH which is a potent adjuvant in humans yielding high Th1 responses, is in contrast usually a poor adjuvant in rodents, still induced potent responses with PP21.
**Figure 20****.** High Immunogenicity of the Bio-fusion conjugate PP21 in South-American Saimiri sciureus monkeys. Saimiri sciureus South American monkeys born in captivity in the animal house of the Primate Center of Belem, Brasil, were immunized by 1µg of PP21 adjuvated by Montanide Isa 720, administered SC on days O, 28 and 112 (dotted lines as compared to controls receiving Montanide alone, plain lines). Antibodies were determined by Elisa on the MSP3-LSP antigen, as well as by IFAT and WB (not shown). Even when using only 0.1µg antigen per immunisation, immune responses were about 5 fold lower, ie still very significant (not shown).
**Figure 21** **:** Antibody responses elicited by LSA3-CRM (PP25) versus LSA3-729 in Balb/C mice, receiving either 1µg of PP25 with Montanide ISA720 or 10µg of LSA3-729 in Montanide ISA720. Immunisations on day 0 ;7 ;28, serum collection on day 45.

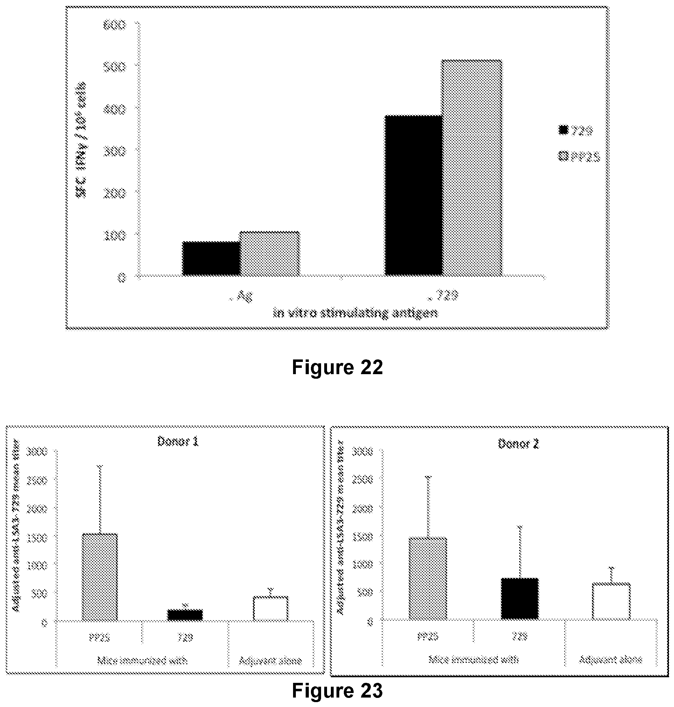

**Figure 22** : IFNγ (ELISPOT) elicited in LSA3-CRM (PP25) versus LSA3-729 immunized Balb/C mice, challenged in vitro by DG 729.
**Figure 23****:** Anti-LSA3-729 human antibody responses in HIMM and Reproducibility of the improvement of results: Anti-LSA3-729 human antibody responses in HIMM to the LSA3-CRM construct PP25 using lymphocytes from two human donors « A » and « B »

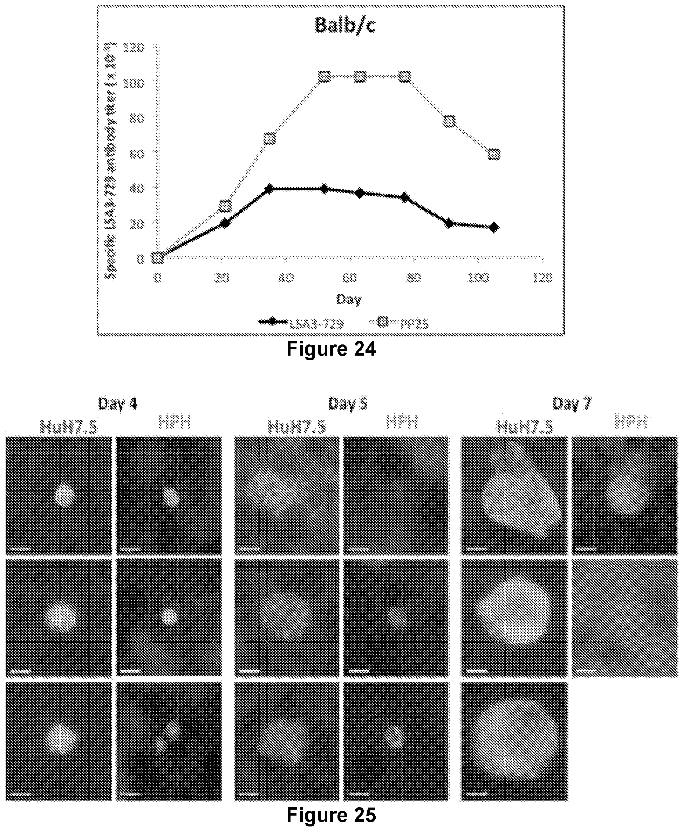

**Figure 24** **:** Improvement in the duration of immune responsees eelicited by PP25.
**Figure 25** **:** A novel host cell for P. falciparum liver stage development in HuH7.5 was developed and compared to Human Primary Hepatocytes (HPH). The figure shows representative samples of liver schizonts at 4, 5 and 7 days after infection with sporozoites, immunostained with a mouse polyclonal anti PfHsp-70. Magnification 400X, scale bar 10 µm. It was difficult to find satisfactory LS at day 7 in HPH. This hepatoma model was thereafter employed in a functional assay to measure antibodies able to inhibit the invasion of sporozoites into hepatocytes

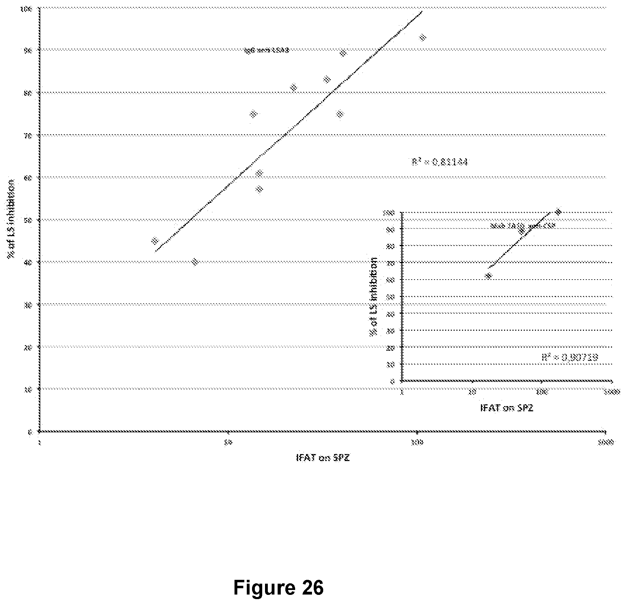

**Figure 26****:** Inhibition of sporozoite invasion in Hu7.5 hepatoma cells (ILSDA) by anti-Pf LSA3 antibodies. Shown is the correlation between anti-LSA3 reactivity on native proteins (by IFAT assays), with the functional inhibitory effect in the Inhibition of Liver Stages Development Assay (ILSDA). The smaller graph shows the same assays using the anti-Circumsporozoite mAb 2A10.

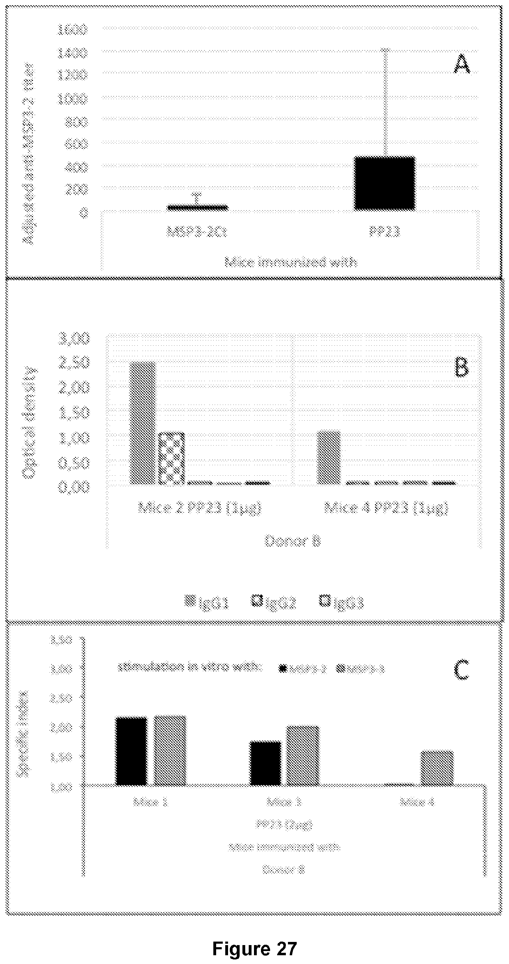

**Figure 27****:** Strong B and T cell responses were elicited in Human Lymphocytes grafted in the HIMM model, by the construct PP23 (MSP3-2-CRMP97-MSP3-3). The upper panel (A) shows the antibody responses, the middle panel (B) the isotypes distribution, which is predominantly of the IgG1 class , the main cytophilic class able to act in copperation with Monocytes in the ADCI mechanism. The lower panel (C) shows T-cells responses evaluated by secretion of IFN-γ restimulated in vitro by either MSP3-2 or MSP3-3.

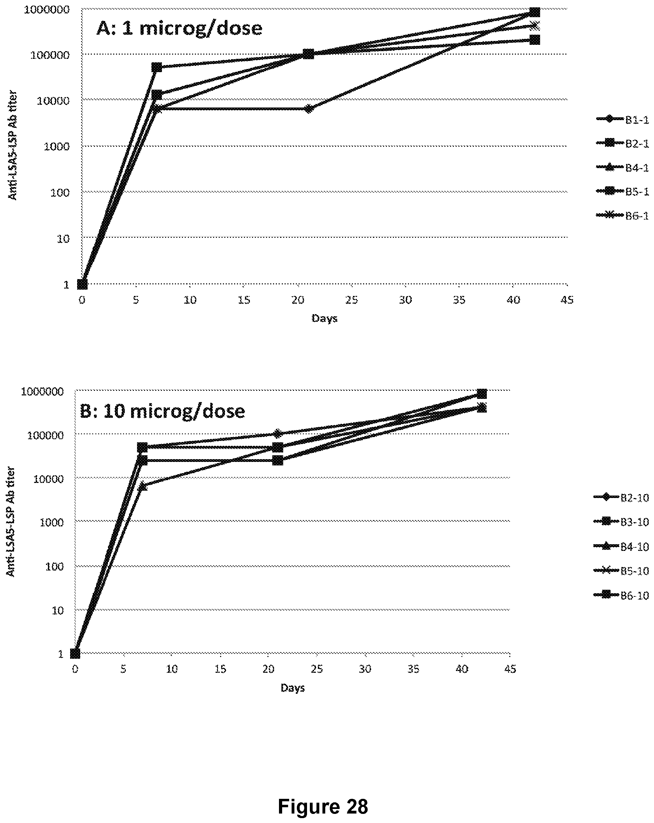

**Figure 28****:** Immunogenicity of 1 µg compared to 10 µg dose of Bio-fusion LSA5-CRM/Montanide ISA720 in BALb/c mice (n=5). Experimental conditions are similar to those described in Figure 8. Immunization by Bio-fusion LSA5-CRM /Montanide ISA720 was performed on days 0, 14 and 28 (arrows). Serum antibody detection was performed by ELISA titration on LSA5-LSP as antigen, using sera collected on days 0, 7, 21, 42.

### Detailed description of the invention

### Definitions

A "vaccine" is to be understood as meaning a composition for generating immunity for the prophylaxis and/or treatment of diseases. Accordingly, vaccines are medicaments which comprise antigens and are intended to be used in humans or animals for generating specific defense and protective substance by vaccination. A "vaccine," as used herein, means an immunogenic composition capable of eliciting partial or complete protection against malaria. A vaccine can be prophylactic for infection and/or therapeutic in an infected individual.

The term "immunogenic" means that the composition or protein to which it refers is capable of inducing an immune response upon administration. "Immune response" in a subject refers to the development of an adaptative and/or innate immune response, including a humoral immune response, a cellular immune response, or a humoral and a cellular immune response to an antigen. A "humoral immune response" refers to one that is mediated by antibodies. The term "protection" or "prevention" as used in this document refers to an absence of symptoms or to the presence of reduced symptoms of the disease after contact with the Plasmodium parasite. In particular, the term "prevention of malaria" refers to an absence of symptoms or to the presence of reduced symptoms in the treated subject after contact with a Plasmodium responsible for malaria.

The "patient" or "subject" is typically a mammal subject, preferably a human subject. The subject can be male or female, a child or an adult. A subject can be one who has been previously diagnosed or identified as having malaria. Alternatively, a subject can also be one who has not been previously diagnosed as having malaria, but who is at risk of developing such condition, e.g. due to infection or due to travel within a region in which malaria is prevalent. For example, a subject can be one who exhibits one or more symptoms for malaria. For instance the subject may be infected with Plasmodium falciparum or Plasmodium vivax. A "homologous" or "substantially homologous" sequence to an original polypeptide is typically one which has at least about 80 percent identity to the sequence of the original polypeptide or an immunogenic or epitopic fragment of the original polypeptide, and which substantially retains the desired effect on the intended target of the original polypeptide (i.e., elicits an immunogenic response, or being recognized by an antibody). In increasingly preferred embodiments, the homologous sequence has at least about 85 percent, 90 percent, 95 percent, 97 percent or 99 percent identity to the sequence of the original polypeptide or an immunogenic or epitopic fragment thereof.

An "immunogenic fragment" generally has a length of at least five amino acids. With increasing preference, the length of the immunogenic fragment is at least 7, 9, 11, 13, 15, 17 and 19 amino acids. An "epitope" generally has a length of at least five amino acids, preferably at least 7 to 14 amino acids.. An "epitopic fragment" comprises such epitope and can thus be longer.

In the context of the present invention, the term "fusion or biofusion" means that, in the recombinant protein, the carrier protein is either directly linked to the antigenic Plasmodium sequence, or is linked to the antigenic Plasmodium sequence by a peptide linker. The peptide linker may be a sequence of 1 to 35 amino acids, preferably 5 to 20 amino acids, still preferably 5 to 10 amino acids. In a preferred embodiment, the linker peptide is (Gly-Gly-Gly-Gly-Ser)n, (Gly)ₙ or (EAAAK)ₙ, wherein n is 1 to 4, preferably 1 to 3. In either embodiment, a single polypeptide is formed.

### The carrier protein

The carrier heterologous protein sequence is CRM197 or an immunogenic fragment thereof, which fragment is selected from the group consisting of Fragment A, Transmembrane Domain T, Receptor Binding domain R of CRM197, amino acid sequence 1-190 of CRM197, and amino acid sequence 1-389 of CRM197.

Fragment A of diphterin toxin extends from amino acid 1 to 201. CRM197 possesses an enzymatically inactive fragment A, showing a substitution of glycine 52 with glutamic acid. Fragment B of diphterin toxin extends from amino acid 202 to 535.

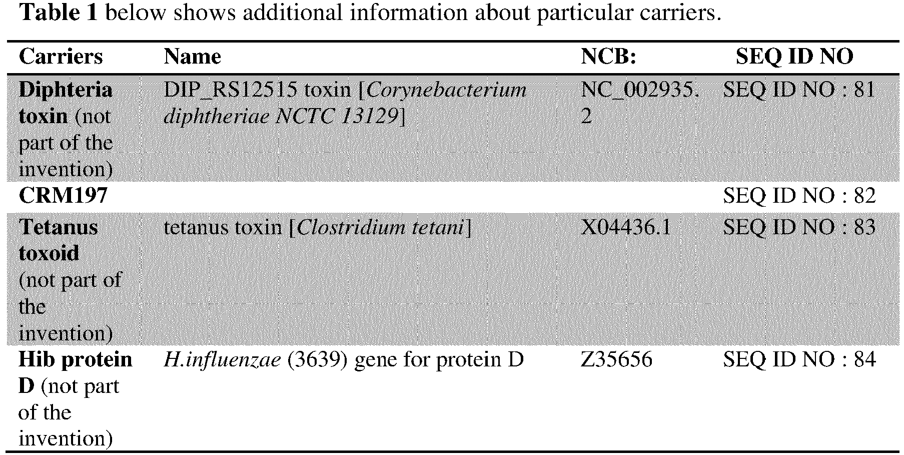

### The antigenic Plasmodium sequence

The fusion protein construct of the invention comprises at least one antigenic sequence comprising an epitope sequence of a Plasmodium protein, wherein the antigenic sequence is MSP3, LSA3, or LSA5, or an epitopic fragment of MSP3 that encompasses motifs a, b, c, d, e and/or f of the C-terminal region of a Plasmodium MSP3 protein chosen among MSP3-1, MSP3-2, MSP3-3, MSP3-4, MSP3-7, MSP3-8, preferably selected from SEQ ID NO: 7 to SEQ ID NO: 54, or an epitopic fragment of LSA3 that is peptide DG729 of SEQ ID NO: 77. The "Plasmodium" genus of parasites infecting humans include, without limitation, P. falciparum, P. vivax, P. knowles, P. ovale and P. malariae. The antigens listed below are antigens from P. falciparum.

Examples of antigenic sequences are listed in **Table 2** below.

Plasmodium falciparum merozoite surface protein 3 (MSP-3) is a known asexual blood-stage malaria vaccine candidate antigen (Sirima et al, N Engl J Med 2011, 365(11):1062-1064; Demanga et al, Infect Immun 2010, 78(1):486-494; Daher et al, Infect Immun 2010, 78(1):477-485; Rousillon et al, Roussilhon, et al, PLoS Medicine, November 2007, 4, Issue 11, e320).

In particular any of the six MSP3 proteins may be used, preferably MSP3-1, as well as peptides a,b,c,d, e,f,, the recombinants C-term, but also full length and N-term of the protein. In a preferred embodiment, the antigenic sequence is MSP3 or an epitopic fragment thereof, which fragment encompasses motifs a, b, c, d, e and/or f of the C-terminal region of a Plasmodium MSP3 protein chosen among MSP3-1, MSP3-2, MSP3-3, MSP3-4, MSP3-7, MSP3-8.

In P. falciparum, liver stage antigen-3 (LSA-3) is an antigen expressed at the pre-erythrocytic stage (Toure-Balde et al: Infect Immun 2009, 77(3): 1189-1196; Daubersies et al: Nat Med 2000, 6(11): 1258-1263).

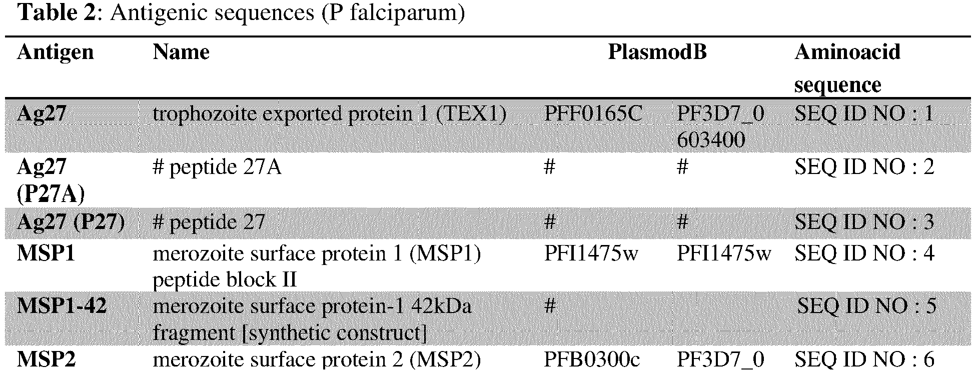

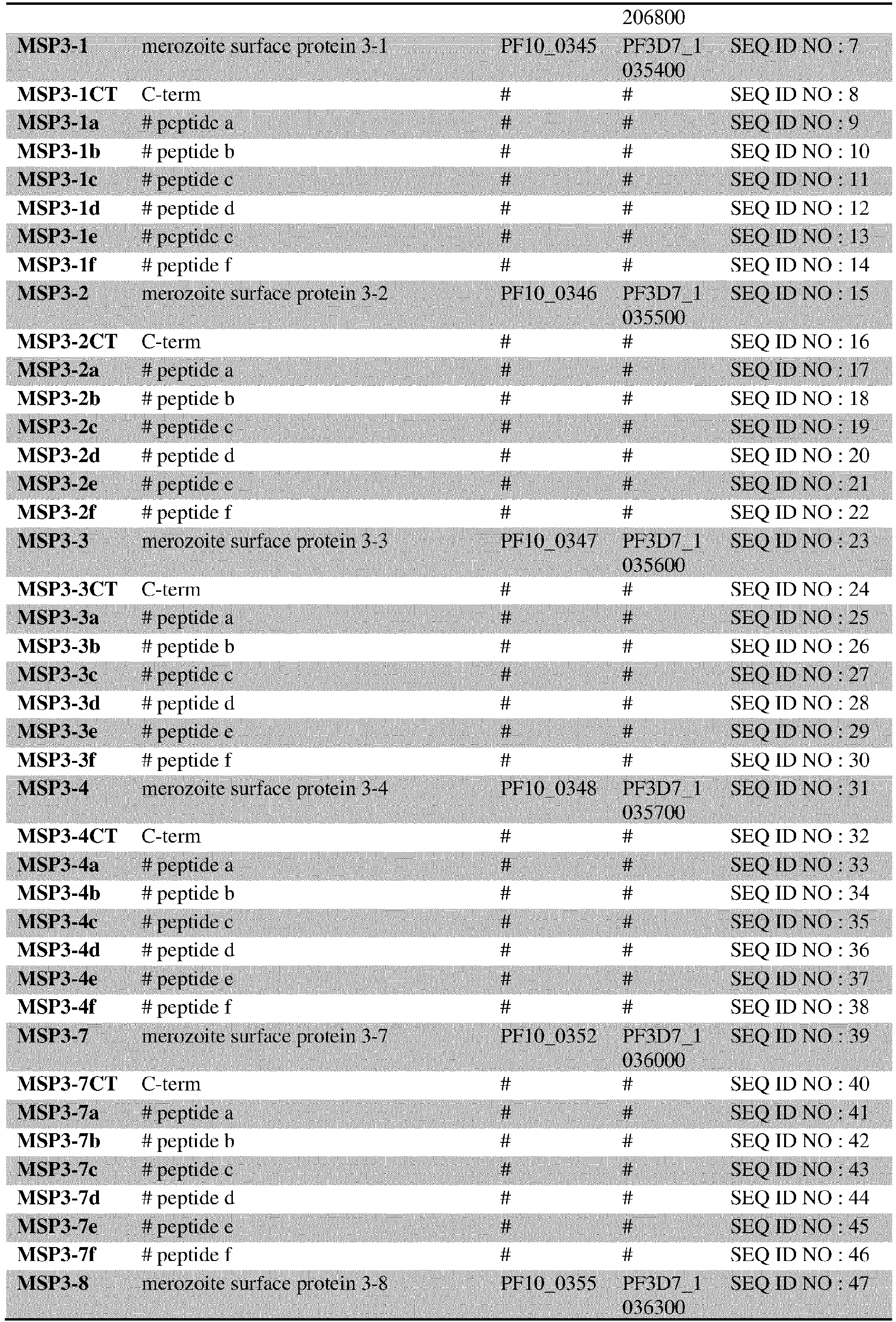

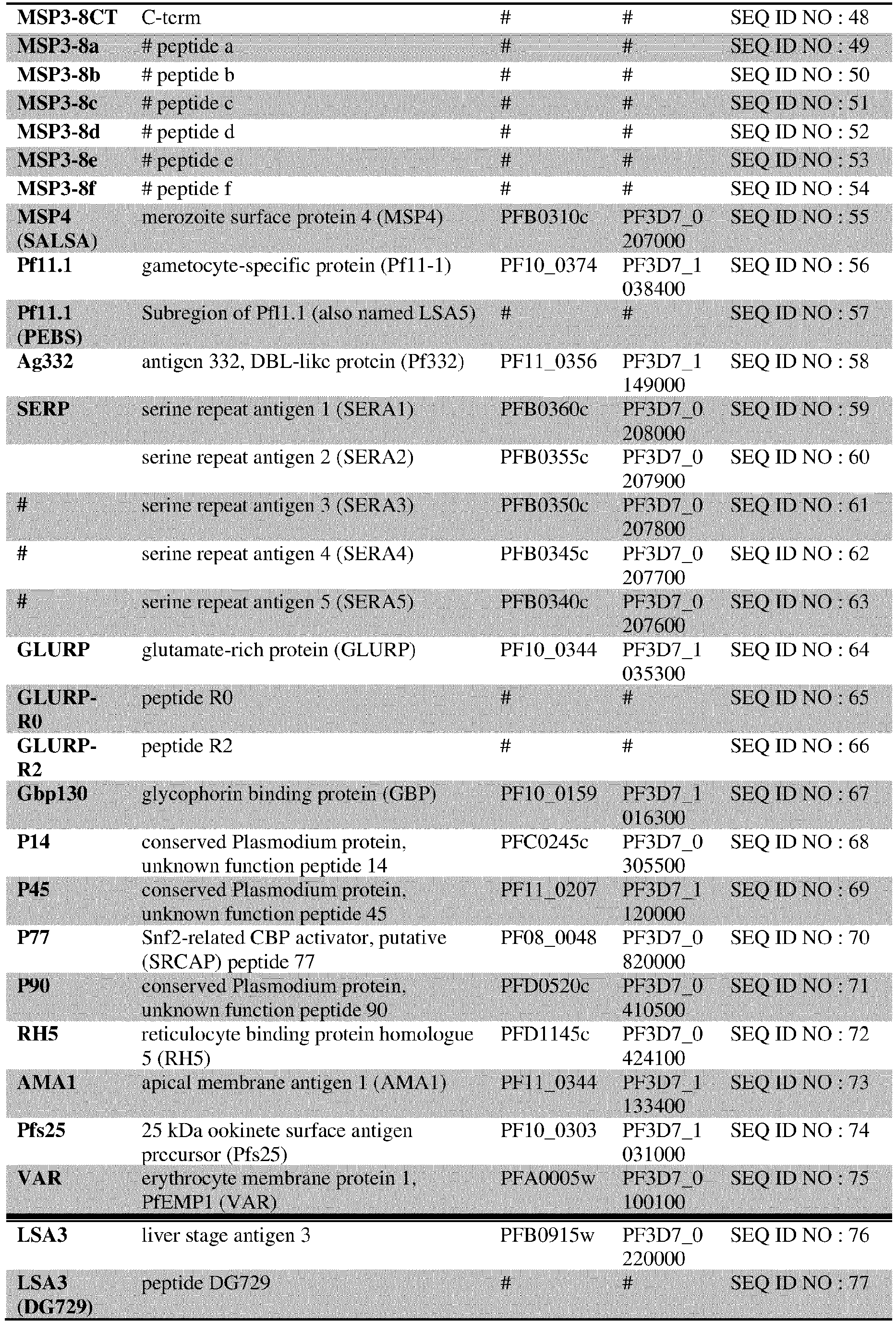

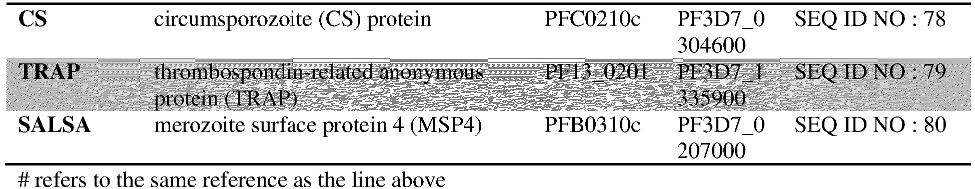

### The fusion construct

The fusion protein comprises or consists of MSP3 (preferably MSP3-1), LSA3, LSA5 (also named PEBS) or an epitopic fragment thereof as defined in the claims, as the antigenic sequence, fused to CRM197 or an immunogenic fragment thereof as defined in the claims, as the carrier sequence.

In a preferred aspect, the fusion protein comprises two antigenic sequences which are either each located in C-term and N-term of the carrier protein sequence, or both located at the same terminus, wherein the two antigenic sequences are the same or different from each other.

In a preferred embodiment, the fusion between the antigenic sequence and the carrier sequence is a direct fusion.

In another embodiment, the at least one amino acid sequence may be linked at the N-term and/or C-term of the carrier heterologous protein sequence by a peptide linker, wherein the peptide linker preferably is a sequence of 1 to 35 amino acids, preferably 5 to 20 amino acids. In a preferred aspect, the peptide linker is (Gly-Gly-Gly-Gly-Ser)n, (Gly)ₙ or (EAAAK)ₙ, wherein n is 1 to 4, preferably 1 to 3.

### Acid nucleic, vectors and host cells

The fusion protein of the present invention can be made by any recombinant technique. Accordingly, another aspect of the invention pertains to vectors, preferably expression vectors, containing a nucleic acid encoding said fusion protein.

The expression vectors used in the present invention can provide for expression in vitro and/or in vivo (e.g. in a suitable host cell, host organism and/or expression system). They typically comprise a polynucleotide sequence as defined above, and regulatory sequences (such as a suitable promoter(s), enhancer(s), terminator(s), etc.) allowing the expression (e.g. transcription and translation) of the protein product in the host cell or host organism.

The vectors according to the invention may be in the form of a vector, such as for example a plasmid, cosmid, YAC, a viral vector or transposon.

In a preferred but non-limiting aspect, a vector of the invention comprises i) at least one nucleic acid as described above; operably connected to ii) one or more regulatory elements, such as a promoter and optionally a suitable terminator; and optionally also iii) one or more further elements of genetic constructs such as 3'- or 5'-UTR sequences, leader sequences, selection markers, expression markers/reporter genes, and/or elements that may facilitate or increase (the efficiency of) transformation or integration.

A preferred host cell is E.coli. However any prokaryotic or eukaryotic expression systems may be used, such as bacteria, yeast, filamentous fungi, insect, plant cells and mammalian cells.

Preferred vectors, promoters, and host cells, are all vectors, promoters and host cells able to express the recombinant protein, glycosylated or not-glycosylated, such as: the pET26a (with T7 promoter), or the pTrcHis2 (with trp lac promoter) plasmids for procaryotic expression, the pUC19 , pUC 57, or pCR4 TOPO plasmids for transfer, and the E. coli BL21 (DE3), or E coli NiCo21 host cells for protein expression, or E coli Top 10 only for plasmids amplification.

### Vaccine formulation

The fusion protein is formulated in a pharmaceutical composition, in association with a physiologically acceptable vehicle, optionally combined with an adjuvant.

It is therefore provided a vaccine comprising the fusion protein, or a combination of at least two fusion proteins, which respectively comprise at least two different antigenic amino acid sequences, with a physiologically acceptable vehicle.

In another embodiment, it is provided a vaccine composition comprising a nucleic acid construct encoding said fusion protein(s).

In some embodiments, the vaccines may comprise one or more pharmaceutically acceptable vehicles or excipients. Excipients include any component that does not itself induce the production of antibodies and is not harmful to the subject receiving the composition. Suitable excipients are typically large, slowly metabolized macromolecules such as saccharides, polylactic acids, polyglycolic acids, polymeric amino acids, amino acid copolymers, sucrose, trehalose, lactose and lipid aggregates (such as oil droplets or liposomes). Suitable pharmaceutical vehicles are well known to those of ordinary skill in the art, including, but not limited to, diluents, such as water, saline, and others. Suitably, sterile pyrogen-free, phosphate buffered physiologic saline is a pharmaceutical vehicle. Additionally, additives, such as wetting or emulsifying agents, pH buffering substances, and the like, may be present.

The pharmaceutical composition, immunogenic composition or vaccine may further comprise an adjuvant. "Adjuvants" are used to enhance efficacy of the composition and include, but are not limited to, aluminum hydroxide (alum), aluminum phosphate, oil-in-water or water-in-oil emulsions, agonists of Toll-like receptors, N-acetyl-normuramyl-L-alanyl- D-isoglutamine (the-MDP), N-acetyl-muramyl-L-threonyl-D-isoglutamine (nor-MDP), PEI; AS01 or AS02 (GlaxoSmithKline), GLA-SE (IDRI) and similar formulations, ; and the like adjuvants known in the art. The Montanide^{®} adjuvants which are based on purified squalene and squalane, emulsified with highly purified mannide mono-oleate, may be used as well, including ISA 51 and 720. MF59^{®} is another example of an adjuvant, which is an oil-in-water emulsion of a squalene, polyoxyethylene sorbitan monooleate (Tween^{®} 80) and sorbitan trioleate.

Vaccines are formulated into suitable dosage for the subject to which it is to be administered. The dosage administered may vary with the condition, sex, weight and age of the individual; the route of administration; and the adjuvant used. The vaccine may be used in dosage forms such as suspensions or liquid solutions. The vaccine may be formulated with a pharmaceutically acceptable vehicle as described above. Suitable dosages include, but are not limited to, about 0.1 to about 100 micrograms, preferably about 1 to about 50 micrograms, still preferably 1 to 20, 1 to 15, or even 1 to 10 micrograms, of the fusion protein described herein.

### Vaccination

The immunogenic composition or vaccine may be a multi-component/multi- antigen immunogenic composition or vaccine.

Where the immunogenic composition or vaccine is intended for a multiple administration regime, such as a prime-boost regime, the prime composition may comprise the same or different composition as the boost composition. However a prime-boost regime is not compulsory at all in the context of the present invention.

The immunogenic composition or vaccine may be administered by any convenient route, preferably parenterally, intramuscularly, intradermally, subcutaneously, mucosally, or intravenously.

A variety of techniques are available for DNA vaccination, such as electroporation, needle-free approaches, such as particle bombardment and high-pressure delivery, dermal patches, formulation of DNA vaccine in microparticles or liposomes.

It is described a method for raising an immune response in a subject, comprising the step of administering an effective amount of a vaccine of the invention. The vaccines can be administered prophylactically (i.e. to prevent infection) or to provide protective and preferably involves induction of antibodies and/or T cell immunity. The method may raise a primary immune response, a secondary immune response, a booster response or a combination of immune responses.

The vaccine is meant to protect the subject against malaria at any stage, including pre-erythrocytic-stages, such as liver-stage, and/or sexual or asexual blood-stage.

The magnitude and/or memory of the immune response against the Plasmodium antigens is increased according to the invention.

Especially, the MSP3 fusion constructs of the invention trigger production of anti-MSP3 cytophilic antibodies such as IgG1 and IgG3 antibodies. The level of protection may be determined by measuring the titer of said antibodies.

The Figures and Examples illustrate the invention without limiting its scope.

### EXAMPLES:

Previous studies have shown that frequent malaria exposure leads to the development of immunity in adults, which prevents or alleviates disease. This immunity can be passively transferred by administering immunoglobulin (IgG) from malaria-immune adults.

A parasite-killing mechanism, called antibody-dependent cell-mediated inhibition (ADCI), was identified. The screening of a genome-wide DNA library by ADCI led to the identification of merozoite-surface-protein 3 (MSP3) and later to several other antigens, including PEBS.

Cytophilic antibodies were identified as surrogates of protection: Cytophilic (leucocyte-binding) anti-MSP3 antibodies IgG1 and IgG3, which mediate ADCI were found to be consistently associated with a reduction in the risk of malaria in epidemiological studies in many different settings. These antibodies also mediate protection against P.falciparum in infected animals.

These studies indicate the characteristics which an efficient immune response bears and guide the development of improved vaccine formulations.

On this basis, the inventors have now shown that a fusion protein CRM97-MSP3 increases immunogenicity by 20-100 fold, at B and T-cell levels, as compared to MSP3-LSP (long synthetic peptide covering the 186-271 region of MSP3-1). See Example 1. Using 10 fold lower immunizing doses, it translated into a major improvement in immunogenicity to lymphocytes from : humans, mice and saimiri monkeys, and a major improvement in duration of antibodies.

At pre-erythrocytic stage, previous studies have further shown that protective efficacy against massive sporozoites challenges can be elicited by LSA3 in Chimpanzees, which efficacy depends on IFN-γ likely inhibiting LS development, and on antibodies preventing sporozoite invasion.

In **Example 2,** the inventors have shown that a bio-fusion CRM-LSA3 construct strongly increases T and B cell responses.

The results obtained in Examples 1 and 2 proved to be reproducible using other antigens from the erytrocytic stages such as MSP3-2 and MSP3-3 in a construct with CRM (See **Example 3**).

**Example 4** provides results with a LSA5-CRM construct.

### Materials and methods

### Generation of expression constructs and production of the Bio-fusion proteins in Escherichia coli

The genetic sequences coding for the chimeric immunogens composed of the detoxified diphtheria toxin CRM197 covalently linked at both its N- and C-terminal end to malaria peptide antigens (Figure 1) were chemically synthetized (GenScript) and codon-optimized for the production in *E. coli.* Restriction sites for Nco I and Xho I were respectively added at the 5' and 3' extremities for cloning in the expression plasmid pET-26a (Novagen). The sequences inserted in the shuttle plasmid pUC57 were checked by sequencing and expedited lyophilized (4 µg).

The MSP3-1 peptide in PP21 (plasmodB acc. Nbr PF3D7_1035400) corresponds to amino acids (AA) 155 to 249 in MSP3-1.

Other hybrid immunogens (Table 3) have been designed around CRM197. In PP22, the MSP3-1 peptide was replaced by a MSP3-2 peptide (AA 178 to 257 of MSP3-2, PlasmodB acc. Nbr PF3D7_1035500). In PP23, the MSP3-2 peptide at the C-extremity was replaced by an MSP3-3 peptide (AA 246 to 307 in MSP3-3, PlasmodB acc. Nbr PF3D7_1035600). PP25 contains the same LSA3 peptide on both CRM197 extremities (AA 176 to 325, PlasmodB acc. Nbr PF3D7_0220000).

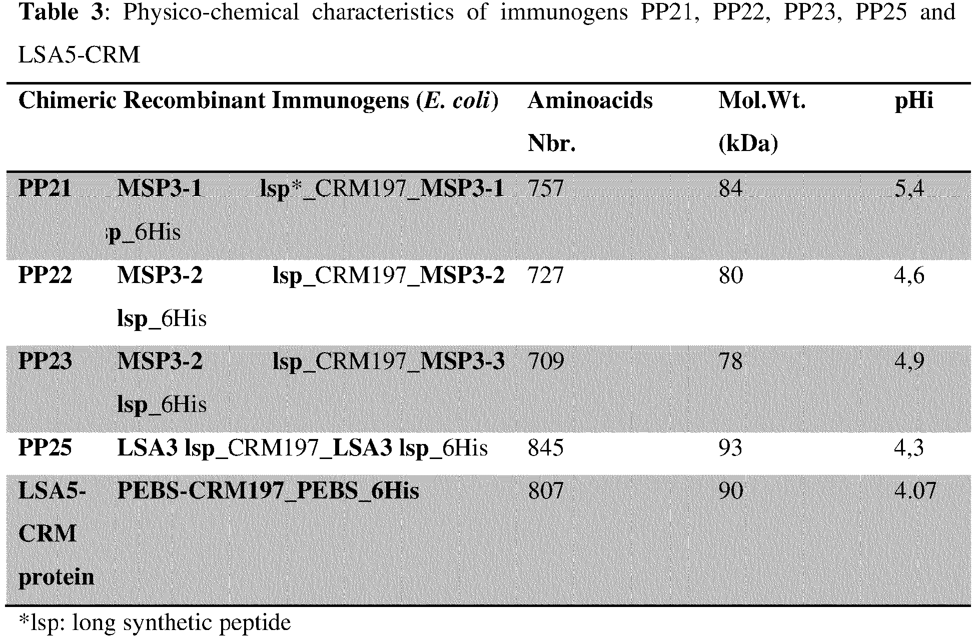

The *E. coli* transfer strain Top 10 (Invitrogen) [F- *mcrA* Δ(*mrr-hsd*RMS*-mcr*BC) Φ80*lac*ZΔM15 Δ*lac*X74 *rec*A1 *ara*D139 Δ(*ara leu*) 7697 *gal*U *gal*K *rps*L (StrR) *end*A1 *nup*G] was used as a for the cloning molecular procedures and plasmid propagation. Expression of recombinants was performed in strain BL21 (DE3) [F - *ompT hsdSB* (*rB- mB*) *gal dcm* (DE3)] (NEB).

The shuttle plasmid pUC57 containing the hybrid sequences (Genscript) and the expression plasmid pET26a were digested with Nco I and Xho I. The bands for the inserts and the linearized pET26a were recovered by gel extraction (QIAGEN) after electrophoresis. The gel-purified inserts were ligated inside pET26a with a ratio insert on plasmid of 3. The ligation reaction was used to transform chemically competent *E. coli* Top 10 and plated on LB agar media with 50µg/mL kanamycin in Petri dishes.

Six bacteria colonies were picked in liquid media with kanamycin for plasmid mini-preparations on silica column (QIAGEN). The correct insertion of inserts in pET26a was checked by digestion with informative restriction enzymes (**Figure 2**).

Plasmids with the correct restriction profile were selected to transform BL21 (DE3) bacteria. Transformation with the correct plasmid was confirmed on six BL21 (DE3) clones that were analyzed by restriction enzyme digestion as above.

### Optimization of the recombinant protein production in small volume bacteria cultures

Parameters such as the duration of recombinant protein production, the concentration of the inducer (IPTG) and temperature were analyzed on a small culture scale (1 L) in Erlenmeyer vessels. Five mL of a steady state overnight culture was diluted in 1 L (1:200) and further cultured at 30 °C to an optical density of 0,7-0,8 at 600nm. The induction of the recombinant protein expression was assayed with two IPTG inducer concentrations; 0.1 and 1 mM at three temperatures; 22, 30 and 37°C. The best recombinant yields were obtained after on overnight culture at 22°C and induction by 0.1 mM IPTG.

A yield of 1 to 2 mg of recombinant product per liter of bacteria culture was obtained after purification by immobilized metal affinity chromatography (IMAC).

### Production and purification of PP21

Productions at a research laboratory scale (1 to 2 liters) were conducted to obtain sufficient PP21 recombinant protein to conduct pre-clinical immunogenicity studies in murine and primate models.

The protocol for the bacteria culture and recombinant protein production has been established formerly in small scale cultures.

The C-terminally hexa-histidine tagged recombinant protein PP21 was purified by affinity on Ni-NTA resin (QIAGEN).

Two purification procedures, under native or denaturing conditions, were investigated.

Indeed, although in native conditions, no recombinant protein was seen in the bacteria lysate insoluble fraction, purification under denaturing conditions was also assayed to assess the effect of a putative conformational effect of the tri-dimensional structure of PP21 on the availability of the histidine tag for the immobilized Ni²⁺ metal on the Ni-NTA resin.

### Purification under native conditions:

- The bacteria cells were recovered by centrifugation (4000g, 30 mins, 4°C).
- The bacteria pellet was suspended in 5 mL of lysis buffer (50 mM NaH₂PO₄, 300 mM NaCl, 10 mM imidazole, pH 8.0) per gram of biomass.
- The lysate was rocked on ice 30 mins with 1 mg/mL of lysozyme.
- The bacteria were lysed by 5 30 sec bursts at 150 W with a 30 sec cooling intervals.
- The lysate was rocked on ice 30 min with 5µg/mL of DNAse I and 10µg/mL of RNAse A with a complete set of proteases inhibitors (Roche).
- The lysate was cleared by centrifugation at 10000g, 30 mins at 4°C.
- The lysate was filtered through a 0,22 µm sieve and incubated overnight on a rocking tray at 4°C with 200 µL of Ni NTA resin in a 20 mL volume.
- The resin was extensively washed in a 40 mL volume of lysis buffer five times. An aliquot of the first step supernatant (lysate post-resin incubation) was conserved for analysis. Centrifugation steps did not exceed 5 mins at 200g.
- The recombinant proteins were recovered by incubating the resin in 500 µL lysis buffer containing 250 mM imidazole 5-fold
- The eluted fractions were pooled and dialyzed against PBS pH 7.3 by ultrafiltration through a 30 kDa sieve.

The protein concentration was measured by spectrophotometry at 280 nM and further characterized by electrophoresis (SDS-PAGE 10%) and immunoblotting as shown in Figure 3 for PP21.

### Purification under denaturing conditions:

The bacteria cells were recovered by centrifugation (4000g, 30 mins, 4°C).

The bacteria pellet was suspended in 5 mL of denaturing lysis buffer (100 mM NaH2PO4, 10 mM Tris-Cl, 8 M urea, NaOH pH 8.0) per gram of biomass.

The lysate was rocked on ice 30 mins.

The bacteria were further lysed by 5 30 sec bursts at 150 W with a 30 sec cooling intervals. The lysate was cleared by centrifugation at 10000g, 30 min at 4°C.

The lysate was filtered through a 0,22 µm sieve and incubated overnight on a rocking tray at 4°C with 200 µL of Ni NTA resin in a 20 mL volume.

The resin was extensively washed in a 40 mL volume of denaturing lysis buffer five times. An aliquot of the first step supernatant (lysate post-resin incubation) was conserved for analysis. Centrifugation steps did not exceed 5 min at 200g.

The recombinant proteins were recovered by incubating the resin in 500 µL lysis buffer containing 250 mM imidazole 5-fold.

The eluted fractions were pooled and dialyzed against PBS pH 7.3 by ultrafiltration through a 30 kDa sieve.

The purification yield was similar by the two methods, suggesting that the affinity histidine tag is functional in the native form of PP21.

Production yields of 0,7 to 1mg of the recombinant protein PP21 per liter of bacteria culture have been reproducibly obtained in more than 10 production / purification trials at the 1 liter scale in Erlenmeyer vessels.

**Figure 3** shows recombinant protein PP21 purified by IMAC.

### Temperature stability of PP21

The stability of PP21 in function of the storage temperature assessed by *Western blotting* is shown in **Figure 4****.** No signs of degradations were observed after a period of 21 days storage in a fridge at a temperature of 6°C. At room temperature, PP21 started to show signs of degradation at the same period whereas it was completely degraded at 37°C after 8 days. However, this instability cannot be attributed to putative intrinsic molecule instability as it is not a GMP manufactured product and it might contain traces of proteases.

Amino acid sequences of recombinant proteins expressed in *E.coli* by pET26a are shown below. Sequences in brackets come from the expression plasmid, bold from the CRM197 diphtheria toxin.

The hexa-histidine tag at the C-terminal used for purification by immobilized metal affinity chromatography (IMAC) is underlined.
**PP21** sequence of MSP3-1 in italics-underlined (PlasmodB ID : PF3D7_1035400), residues 155 to 249 (95 aa)
**PP22** ; sequence of MSP3-2 in italics-underlined (PlasmodB ID : PF3D7_1035500), residues 178 to 257 (79 aa)
**PP23** ; sequence of MSP3-2 in italics-underlined before CRM197 (PlasmodB ID : PF3D7_1035500), residues 178 to 257 (79 aa) and MSP3-3 in italics after CRM197 (PlasmodB acc. Nbr PF3D7_1035600), residues 246 to 307 (61 aa).
**PP25** ; sequence of LSA-3 in italics-underlined (PlasmodB ID : PF3D7_0220000), residues 176 to 325 (150 aa)
LSA5-CRM Protein Sequence (SEQ ID NO: 89)

**The Human Immunogenicicty Mouse Model (HIMM):** the model is based upon the engraftment of immunodeficient NOD-SCID-IL-2rynull (NSG) mice with human spleen lymphocytes (Hu-SPL-NSG), and complements the information obtained using ordinary laboratory mice such as Balb/C and C57BL.

### Immunization of Balb/c or C57Bl/6 mice and blood sampling

Groups of 4 to 5 Balb/c or C57B1/6 mice received 2 or 3 injections of 10 µg of MSP3-1-LSP or 1 µg of PP21 diluted in 100 µl of Phosphate Buffer Saline pH 7.2 (PBS) and emulsified in 100 µl of adjuvant Montanide ISA 720. Immunizations were performed by subcutaneous injection and repeated once or twice fifteen days apart. Blood collection was performed by tail bleeding starting two weeks after the second injection and repeated at fifteen days interval up to 160 days afetr the first antigen injection. Sera were separated and stored at -20°C until used. Other groups handled simultaneously in parallel, received either 1 µg or 0.2 µg of PP21.

### Determination of anti-MSP3-LSP antibody titers in mice sera

MSP3-LSP specific antibody titers were determined in mice sera by Enzyme Linked Immunosorbent Assay (ELISA). Flat-bottomed microtitration plates (Nunc-Thermo Scientific, USA) were coated overnight at 4°C with 2µg/ml of PBS diluted MSP3-1-LSP or one of the MSP3-1-LSP peptides a, b, c or d,. After washing (PBS, pH 7.2) and saturation (PBS, 3% non-fat milk), serial dilutions of test sera (diluted in PBS, 3% non-fat milk, 0.05% Tween20) were added and incubated for 1 hour. Negative control consists of a preimmune mouse serum. Specific antibodies were revealed by subsequent addition of horseradish peroxidase-conjugated goat anti-mouse IgG (H+L) (Invitrogen, USA) for one hour, followed by the peroxidase substrate Tetramethylbenzidin (Amresco, USA). Specific antibody titer corresponds to the reverse of the last positive dilution.

### Human spleen donation and ethical considerations

The human spleens were obtained from deceased organ transplant donors according to an ethical agreement with the National Organization for Organ & Tissues Donation & Transplantation (NOOTDT) in Lebanon. The informed consent to donate organs for transplantation or scientific research was signed by the patients themselves during their lifetime or by their parents following the death.

### Human spleen cell preparation

Human spleens were processed within 24 hours after surgical excision as previously described (Brams P, 1998). Briefly, splenic tissue was dissected and a cell suspension was prepared. Red blood cells were lyzed using Gey's solution for 5-10 min at 25°C. After washing, leucocytes were resuspended in medium consisting of 37% fetal calf serum (FCS) (Sigma,USA), 10% dimethyl sulfoxide (DMSO) (Sigma,USA) and 53% RPMI 1640 (Sigma,USA), and then cryopreserved in liquid nitrogen until used. The mean number of cells isolated from each spleen donor was 10 ± 4 billion.

### Engraftment and immunization of human spleen cells in NSG mice

NOD.Cg-Prkdcscid-IL2rγtm1WjI /SzJ (NSG) mice were obtained from The Jackson Laboratories (USA) and housed in sterile microisolators. All food, water, caging and bedding was autoclaved before use. Six- to 8-week old NSG mice were included in the experiments. Human spleen cells were cultured on day 0 at 4 × 10⁶ cells/ml with or without 1 µg/ml of antigen in RPMI1640 medium supplemented with 10% Fetal Calf Serum, 1% non-essential amino acids (NEAA 100x), 2 mM glutamine, 2 mM sodium pyruvate and 50 µg/ml gentamicin (complete medium). All culture reagents were purchased from Sigma, USA. On day 1, recombinant human IL-2 (Gibco Invitrogen) was added at 25 IU/ml. On day 3, the spleen cells were harvested, washed and resuspended in Hank's Balanced Salt Solution (HBSS). Each NSG mouse received an intraperitoneal injection (ip) of 30 × 10⁶ antigen-primed or unprimed spleen cells. At day 7, and day 21, reconstituted mice (Hu-SPL-NSG) were boosted with 10 µg of antigen injected intraperitoneally (ip) in 200 µl of HBSS-Montanide ISA 720 or MF59 adjuvant (v/v). Mice reconstituted with unprimed spleen cells, received only adjuvant. Blood samples were collected one week after each booster.

### Determination of human MSP3-1-LSP specific antibodies in Hu-SPL-NSG mice sera

These determinations were performed by ELISA. Briefly, for the detection of total human IgG in mouse serum, flat-bottomed microtitration plates (Nunc-Thermo Scientific, USA) were coated overnight at 4°C with 2.5µg/ml purified goat anti-human IgG (H + L) (Invitrogen, USA) in 0.1M carbonate buffer, pH 9.5. After washing (PBS, pH 7.2) and saturation (PBS, 3% non-fat milk), test sera (diluted in PBS, 3% non-fat milk, 0.05% Tween 20) were added and incubated for 1 hour. Negative controls consisted of preimmune mouse serum of the same animals. Human IgG were revealed by subsequent addition of horseradish peroxidase-conjugated goat anti-human IgG (H+L) (Invitrogen, USA) for one hour, followed by the peroxidase substrate Tetramethylbenzidin (Amresco, USA). Total human IgG concentration in the Hu-SPL-NSG mouse serum was calculated in comparison with a standard human IgG solution (Zymed, USA). The same test was performed for the detection of antigen specific antibodies, except that plates were coated with MSP3-1-LSP at 25µg/ml in PBS. In this case, negative controls consisted of sera of individuals that have never been infected with *Plasmodium,* while positive controls consisted of a pool of sera from hyperimmune African adults. In both cases, serial dilutions of each serum were tested in order to determine antibody titer. For specific antibody titer determination, a test was considered positive when it yielded an absorbance above cut-off (negative control OD × 2). The detection of IgG subclasses was performed using secondary mouse monoclonal antibodies specific for human IgG subclasses (clones NL16 [IgG1; Skybio, UK], HP6002 [IgG2; Sigma-Aldrich, Germany], and Zg4 and GB7B [IgG3 and IgG4, respectively; Skybio, UK) were used at final dilutions of 1/4,000, 1/10,000, 1/10,000, and 1/60,000, respectively), followed by horseradish peroxydase conjugated goat anti-mouse IgG diluted 1/4000 in PBS (Invitrogen).

### Quantification of gene expression of different cytokines, chemokines and transcription factors by Real-Time Reverse Transcription PCR (RTqPCR)

Spleen cells of immunized and non immunized mice were cultured at 2×10⁶ cells/ml in complete RPMI1640 medium, with or without stimulation with the immunizing antigen. Total cellular RNA was extracted after a 24h culture using RNeasy Mini Kit (Quiagen, Germany). Reverse transcription was carried out using RevertAid M-MuLV enzyme (RevertAid kit First Strand Synthesis kit, Thermo Scientific Fermentas) in a 20 µl reaction mixture. Briefly, 1 µl of oligo dT18 primer was added to about 400 ng of total RNA, mixed and incubated at 65°C for 5 min. The tube was placed on ice for a few minutes and centrifuged briefly before the addition of 5x reaction buffer, Ribolock Rnase inhibitor (20 u/µl), dNTPs (10 mM) and reverse transcriptase (200 u/µl) as recommended by the manufacturer. The reaction mixture was incubated at 42°C for 60 min. The enzyme was inactivated by heating at 70°C for 5 min. Quantitative PCR was used to measure the relative expression of different human cytokines, chemokines and transcription factors considered as characteristic for Th1 or Treg cells. The PCR mixture was performed using LightCycler 480 SYBR Green Master, La Roche as recommended by the manufacturer. PCR amplifications were performed at 95°C for 5 min (1 cycle), followed by 45 cycles of incubation at 95°C for 10 sec and 55-57°C for 10 sec in the LightCycler 480 machine (La Roche). Each sample was run in triplicate. The housekeeping genes used as internal references were beta-actin (β-actin) and hypoxanthine phosphoribosyltransferase 1 (HPRT1) (geNorm analysis). The cycle threshold (Ct) value was calculated based on the produced PCR curve. The relative expression levels were calculated by the 2ΔΔCt method.

### Immunofluorescence assays.

Immunofluorescence assays were performed using air-dried, acetone-fixed, thin smears from red blood cell cultures containing predominantly mature schizonts of *P. falciparum* (3D7). Hu-SPL-NSG Sera diluted in PBS-1% bovine serum albumin were incubated at 37°C in a humid chamber for 1 h. After being washed with PBS, antibodies were detected by using an Alexa Fluor-conjugated goat anti-mouse IgG (Molecular Probes and Invitrogen) diluted to 1/400 in PBS with Evans blue counterstain (1/200).

### Results

### Example 1: Immunogenicity of PP21, a CRMP97-MS3 fusion construct

The immune responses induced by PP21 were assessed in the following animal models:
- BALB-C mice
- C57BL mice
- *Saimiri sciureus* monkeys
- The human immunogenicity mouse model (HIMM), where cells from human spleen tissue grafted in immunocompromised NSG mice are immunized in vivo and sera and cells from the immunized animals are used for immuno-assays.

All experiments were performed in comparative manner with the benchmark antigen MSP3-LSP (long synthetic peptide covering the 186-271 region of MSP3-1) used in clinical experiments, and using Montanide ISA 720 because laboratory mice respond usually poorly to alum, because the route of injection in HIMM does not allow to use alum, and above all because we were aiming at comparing constructs and not adjuvants, knowing that in humans alum adjuvated CRM (in meningococcal or pneumococcal vaccines) as MSP3 LSP provide very good responses.

Yet a succesful immunization with alum was also performed in lab mice, yielding titers of > 25 600 in Balb/C,even after two immunisarions, and 3200-25600 in C57bl (Figure 19).

### A major improvement of immunogenicity was observed using the PP21 Bio-fusion MSP3-1 construct of the invention, as compared to MSP3-LSP in mice.

PP21 induces higher serological responses of the target cytophilic subclasses than the benchmark antigen MSP3-LSP in all models used.
1. Low doses are very efficient: As with other CRM conjugated vaccines a major increase in immunogenicity was obtained, indicating that the fusion procedure produced as efficient products as chemical conjugation. We readily employed markedly lower dosages, which led to compare only 1 µg of PP21 with 10 µg of the reference MSP3-LSP (10 µG was found in the past not significantly different in lab mice from the 15 µG employed in the clinic). These differences were determined by different means see Figures 6 to 9. Results were reproducible with 4 distinct batches of PP21 in 4 groups of Balb/C and C57bl mice. Further dose-finding with PP21 focussed on lower dosage, eg 0.2 µg compared to 1 µg investigated in mice and in Saimiri monkeys. The high immunogenicity led us also to investigate only 2 as compared to 3 immunisations.
2. Wider breadth of recognition of ADCI targets : The peptides "b", "c" and "d" contained within the vaccine construct define distinct B-cell epitopes targeted by antibodies effective in ADCI. Reactivity with these peptides were higher in sera of mice vaccinated with PP21 as shown in Figure 9 (right-hand panel) as compared to the current MSP3-1-LSP (left-hand panel), at 1 and 10 µg respectively, suggesting that the magnitude and breadth of recognition of ADCI targets are higher and wider with PP21. In agreement with this finding the pattern of recognition of the 6 members of the MSP3 family, which exhibit cross-reactions through peptide « b » and « c-d » mainly, was wider with PP21. This also means that antibodies elicited by PP21 will trigger ADCI through triggering by antibody bridges to 6 proteins on the merozoite surface and not only one.
3. Very high B cell responses in Human lymphocytes : More importantly, human B-cell responses as assessed in the HIMM model showed similar major improvements with immunogenicity increased by 40-100 times. (Figure 10). In parallel experiments, using PP21 head to head with the present MSP3-LSP of 96 amino-acid, antibody titers were 20 to 100 fold higher with 1 µg of PP21 than with 10 µg of MSP3-LSP. Results were reproducible in 3 groups of 5 animals belonging to 3 HLA-classes.
4. Results are also reproducible when using either Montanide ISA720 or MF59 as adjuvants (Figure 14)
5. High IFN-γ secretion was obtained when Human lymphocytes were in vitro challenged with the parasite component MSP3-LSP (Figure 11). This important result indicates that CRM Th epitopes did not override the plasmodium-specific MSR3 T-cell epitopes and therefore that a Tcell help can be expected from vaccinated individuals when challenged by the parasite, yielding great chances for a fast anamnestic antibody rise when it is needed.
   Detailed analysis of cytokine profiles indicate that the responding cells are predominantly of a Thelper type 1 type (Figure 12 showing examples of results obtained with CXCL10 and Tbet)
6. Dominance of cytophilic IgG1: Isotype studies revealed as expected with strong Th1 responses a large dominance of the cytophilic isotypes, almost entirely made of IgG1 which is also the dominant isotype in MSP3-LSP immunized individuals. This was true when using either Montanide or MF59 as adjuvants (Figures 15 and 16). Although these result were obtained in a human model they bode very well of future responses which could be elicited in volunteers.
7. Reactivity with native parasite proteins: Antibodies elicited by PP21 vaccination reacted with the parasite native proteins in IFAT (Figure 13) and Western Blots The reactivity of antibodies, not only with the synthetic antigens, but above all with the parasite proteins is a critical feature to activate the ADCI defence mechanism during a malaria attack, and for the proper boosting of antibody responses by natural parasite challenges.
8. Very low immunizing doses are efficient: 0.2 µg and 1 µg per immunisation were assessed compared to 10 µg for MSP3-LSP (Figures 17) . At the one µg dose of PP21 a plateau was reached after the 2^{nd} immunisation, whereas at a 0.2 µg dose a third immunisation was required to reach about the same high levels of antibodies, and one animal did not respond. Yet the one microG profile indicates that 2 immunisations might be sufficient, and hence may deserve being explored.
9. Similar high immunogenicity is observed in South American Saimiri sciureus monkeys (Figure 20): 1 µg and 0.2 µg PP21 adjuvanted by Montanide 720 were used to immunize South American primates that respond usually poorly to immunisation. High responses close to titers seen in mice were reached at 1 µg, (see Figure 18) and about 5 fold lower titers at 0.2 µg (not shown). Though the comparison of titers in the same experiment has not yet be performed by our Brazilian colleagues, MSP3 LSP was very poorly immunogenic when it was employed in this model.
10. The duration of immune responses is markedly improved (Figure 8 upper and lower panels): Long term analysis showed a marked improvement of an essential component; the duration of antibody responses: Mice immunized by PP21 or LSP were followed up for 6 months post immunisation. The decay of titers is progressive but slow, so that at 6 months the titers remaining are higher than the peak obtained using MSP3-LSP. Titers remained with PP21 in the range 1/200 000 - 1/300 000 for more than 6 months, compared to a range of ca 1/50 000 for the current MSP3-LSP.

### Example 2: Immunogenicity of PP25, a CRMP97-LSA3 fusion construct

LSA3 is a pre-erythrocytic stage antigen, which has many attractive features, it was discovered by the differential responses of protected versus non-protected volunteers, it is conserved across strains, it is highly antigenic and immunogenic in a large range of animals including higher primates and chimpanzees, and induces in the latter immune responses protective against massive challenges by the human parasite Plasmodium falciparum at sporozoite stage. Protection is associated with antigen-specific Interferon gamma responses. The immunogenicity of PP25 was compared to control construction using LSA3 without conjugate nor any fusion (DG729). Conditions of immunization and immune-analysis are identical to those described above with PP21.

A similar major increase in antibody responses with LSA3-CRM97 biofusion, even using the antigen at low doses, was observed as was the case for MSP3 in PP21. The increase in IFN-γ responses was also seen but was less marked as LSA3 (DG729) without carrier is already a very good inducer of IFN-g responses.

The increase recorded in balb/C and C57bl mice, was also confirmed in the Human Immunogenicity model.

We further showed a very strong correlation between anti-LSA3 antibody titers with inhibition of P.falciparum parasites at sporozoite stage.

See results shown in **Figures 21-26****.**

### Example 3: Immunogenicity of PP23, a MSP3-2-CRMP97-MSP3-3 fusion constructs

The results obtained with PP21 relying on the fusion of MSP3-1 with CRM, and with LSA3, proved to be reproducible using other antigens from the erytrocytic stages such as MSP3-2 and MSP3-3 in a construct with CRM, named PP23. Indeed, strong B and T cell responses were elicited in Human Lymphocytes grafted in the HIMM model, by the construct PP23 (MSP3-2-CRMP97-MSP3-3) (**Figure 27**), the titers being on average 10 fold higher than in mice immunized by MSP3-2 CT alone in Montanide. Antibody sub-classes were predominantly of the IgG1 class, which is the main cytophilic class able to act in cooperation with monocytes in the ADCI mechanism. Similarly strong T-cells responses were induced as measured by secretion of IFN-γ by PP23-immunized human lymphocytes restimulated in vitro by either MSP3-2 or MSP3-3.

### Example 4 : LSA5-CRM

Liver-stage antigen-5 (LSA5), also known as PEBS (pre-erythrocytic and blood stages antigen), or SR 11.1 (sub-region of 11.1 P.falciparum gene) combines in a single molecule the features of both MSP3 and LSA3. It contains a unique sequence that differentiates it from the remaining of the mega-gene Pf 11.1, and is fully conserved across strains. It was recognized by sera from volunteers immunized with radiation- attenuated sporozoites and is unique as it induces protection against both pre-erythrocytic and asexual blood stages. It is expressed in both pre-erythrocytic and blood stages. The protective role of LSA5 against pre-erythrocytic stages is supported by convergent in vitro invasion inhibition studies, in vivo protection by passive transfer of anti-LSA5 antibodies, and by proof-of-concept studies in primates protected from challenge by vaccination with recombinant LSA5. Against the blood-stages, both naturally-occurring human and artificially-induced animal anti-Pf LSA5 antibodies exert a parasite-killing ADCI-mediated effect. Antigenicity is high in individuals from endemic areas with IgG3 antibodies predominating in individuals with premunition ie. exposure induced protection. A large number of epidemiological studies found strong association with protection against clinical malaria attacks and improved prognosis of drug-treated cerebral malaria. Finally LSA5 is highly immunogenic, achieving remarkably high titers in mice and greater ADCI activity than sera from MSP3-immunized mice.

A Bio-fusion of LSA5 with CRM197 similar in its principles to the previous 3 examples, was expressed in E.coli, its antigenicity ascertained and preliminary immunogenicity studies conducted. LSA5 immunogenicity was very high using the fusion with CRM (Figure 28); immunogenicity of 1 µg was compared to 10 µg dose of Bio-fusion LSA5-CRM/Montanide ISA720 in BALb/c mice (n=5). Experimental conditions were similar to those described in Figure 8 with MSP3. Immunization by Bio-fusion LSA5-CRM /Montanide ISA720 was performed on days 0,14 and 28 (arrows). Serum antibody detection was performed by ELISA titration on LSA5-LSP as antigen, using sera collected on days 0, 7, 21, 42.

ADCI-mediated effect. Antigenicity is high in individuals from endemic areas with IgG3 antibodies predominating in individuals with premunition ie. exposure induced protection. A large number of epidemiological studies found strong association with protection against clinical malaria attacks and improved prognosis of drug-treated cerebral malaria. Finally LSA5 is highly immunogenic, achieving remarkably high titers in mice and greater ADCI activity than sera from MSP3-immunized mice.

A Bio-fusion of LSA5 with CRM197 similar in its principles to the previous 3 examples, was expressed in E.coli, its antigenicity ascertained and preliminary immunogenicity studies conducted. LSA5 immunogenicity was very high using the fusion with CRM (Figure 28); immunogenicity of 1 µg was compared to 10 µg dose of Bio-fusion LSA5-CRM/Montanide ISA720 in BALb/c mice (n=5). Experimental conditions were similar to those described in Figure 8 with MSP3. Immunization by Bio-fusion LSA5-CRM /Montanide ISA720 was performed on days 0,14 and 28 (arrows). Serum antibody detection was performed by ELISA titration on LSA5-LSP as antigen, using sera collected on days 0, 7, 21, 42.

## Claims

1. A fusion protein which comprises at least one antigenic amino acid sequence fused at the N-term and/or C-term of a carrier heterologous protein sequence, wherein the antigenic sequence comprises an epitope sequence of a Plasmodium protein and the carrier heterologous protein sequence is a sequence that is immunogenic in humans, wherein the carrier heterologous protein sequence is CRM197 or an immunogenic fragment thereof; which immunogenic fragment is selected from the group consisting of Fragment A, Transmembrane Domain T, Receptor Binding domain R of CRM197, amino acid sequence 1-190 of CRM197, and amino acid sequence 1-389 of CRM197;
and wherein the antigenic sequence is MSP3, LSA3, or LSA5, or an epitopic fragment of MSP3 that encompasses motifs a, b, c, d, e and/or f of the C-terminal region of a Plasmodium MSP3 protein chosen among MSP3-1, MSP3-2, MSP3-3, MSP3-4, MSP3-7, MSP3-8, preferably selected from SEQ ID NO: 7 to SEQ ID NO: 54, or an epitopic fragment of LSA3 that is peptide DG729 of SEQ ID NO: 77.

2. The fusion protein of claim 1, wherein the antigenic sequence is MSP3-1.

3. The fusion protein according to any of claims 1-2, comprising two antigenic sequences which are either each located in C-term and N-term of the carrier protein sequence, or both located at the same terminus.

4. The fusion protein according to any of claims 1-3, wherein the at least one amino acid sequence is linked at the N-term and/or C-term of the carrier heterologous protein sequence by a peptide linker, wherein the peptide linker preferably is a sequence of 1 to 35 amino acids, preferably 5 to 20 amino acids.

5. The fusion protein according to claim 4, wherein the peptide linker is (Gly-Gly-Gly-Gly-Ser)n, (Gly)ₙ or (EAAAK)ₙ, wherein n is 1 to 4, preferably 1 to 3.

6. A nucleic acid construct encoding the fusion protein as defined in any of claims 1 to 5.

7. A vector comprising the nucleic acid construct of claim 6 in an expression cassette.

8. A host cell wherein the vector of claim 7 has been inserted.

9. An *in vitro* method for preparing a fusion protein as defined in any of claims 1 to 5, which method comprises allowing the host cell of claim 8 to reproduce under conditions which induce expression of the nucleic acid construct as defined in claim 6, and collecting the fusion protein which is so expressed.

10. A vaccine comprising the fusion protein as defined in any of claims 1 to 5, or a combination of at least two fusion proteins as defined in any of claims 1 to 5, which respectively comprise at least two different antigenic amino acid sequences, or a nucleic acid construct encoding said fusion protein(s), as defined in claim 6, with a physiologically acceptable vehicle.

11. The vaccine of claim 10, for use in vaccinating a human subject against malaria.

## Patentansprüche

1. Fusionsprotein, das mindestens eine antigene Aminosäuresequenz umfasst, die mit dem N-Terminus und/oder C-Terminus einer heterologen Trägerproteinsequenz fusioniert ist, wobei die antigene Sequenz eine Epitopsequenz eines Plasmodiumproteins umfasst und die heterologe Trägerproteinsequenz eine Sequenz ist, die beim Menschen immunogen ist, wobei die heterologe Trägerproteinsequenz CRM 197 oder ein immunogenes Fragment davon ist; wobei das immunogene Fragment ausgewählt wird aus der Gruppe bestehend aus dem Fragment A, der Transmembrandomäne T, der Rezeptorbindungsdomäne R von CRM 197, der Aminosäurensequenz 1-190 von CRM197 und der Aminosäurensequenz 1-389 von CRM197; wobei die antigene Sequenz MSP3, LSA3 oder LSA5 oder ein Epitop-Fragment von MSP3 ist, das die Muster a, b, c, d, e und/oder f der C-terminalen Region eines MSP3-Plasmodiumproteins umfasst, das ausgewählt wird aus MSP3-1, MSP3-2, MSP3-3, MSP3-4, MSP3-7, MSP3-8, vorzugsweise aber aus SEQ ID NO: 7 bis SEQ ID NO: 54, oder ein Epitop-Fragment von LSA3, bei dem es sich um das Peptid DG729 der SEQ ID NO: 77 handelt.

2. Fusionsprotein nach Anspruch 1, in dem die antigene Sequenz MSP3-1 ist.

3. Fusionsprotein nach einem der Ansprüche 1 und 2, das zwei antigene Sequenzen umfasst, die sich entweder jeweils am C-Terminus und am N-Terminus der Trägerproteinsequenz oder beide am selben Terminus befinden.

4. Fusionsprotein nach einem der Ansprüche 1 bis 3, in dem die mindestens eine Aminosäuresequenz über einen Peptidlinker an den N-Terminus und/oder C-Terminus der heterologen Trägerproteinsequenz gebunden ist, wobei der Peptidlinker vorzugsweise eine Sequenz von 1 bis 35 Aminosäuren, vorzugsweise aber von 5 bis 20 Aminosäuren ist.

5. Fusionsprotein nach Anspruch 4, in dem der Peptidlinker (Gly-Gly-Gly-Gly-Ser)n, (Gly)n oder (EAAAK)ₙ ist, wobei n 1 bis 4, vorzugsweise aber 1 bis 3 ist.

6. Nukleinsäurekonstrukt, das für das Fusionsprotein nach einem der Ansprüche 1 bis 5 kodiert.

7. Vektor, der das Nukleinsäurekonstrukt nach Anspruch 6 in einer Expressionskassette umfasst.

8. Wirtszelle, in die der Vektor nach Anspruch 7 inseriert wurde.

9. *In vitro*-Verfahren zur Herstellung eines Fusionsproteins nach einem der Ansprüche 1 bis 5, das darin besteht, die Reproduktion der Wirtszelle nach Anspruch 8 unter Bedingungen zuzulassen, die die Expression des Nukleinsäurekonstrukts nach Anspruch 6 induzieren, und das so exprimierte Fusionsprotein zu gewinnen.

10. Impfstoff, der das Fusionsprotein nach einem der Ansprüche 1 bis 5 umfasst, oder eine Kombination von mindestens zwei Fusionsproteinen nach einem der Ansprüche 1 bis 5, die jeweils mindestens zwei verschiedene antigene Aminosäuresequenzen umfassen, oder ein Nukleinsäurekonstrukt, das für das oder die Fusionsproteine nach Anspruch 6 kodiert, und zwar mit einem physiologisch verträglichen Träger.

11. Impfstoff nach Anspruch 10, das für das Impfen eines Menschen gegen Malaria bestimmt ist.

## Revendications

1. Protéine de fusion qui comprend au moins une séquence antigénique d'acides aminés fusionnée à l'extrémité N-terminale et / ou à l'extrémité C-terminale d'une séquence de protéine hétérologue porteuse, dans laquelle la séquence antigénique comprend une séquence épitope d'une protéine de Plasmodium et la séquence de protéine hétérologue porteuse est une séquence qui est immunogène chez l'homme, dans laquelle la séquence de protéine hétérologue porteuse est le CRM 197 ou un fragment immunogène de celui-ci ; lequel fragment immunogène est choisi dans le groupe constitué par le fragment A, le domaine transmembranaire T, le domaine de liaison au récepteur R de CRM 197, la séquence d'acides aminés 1-190 de CRM197, et la séquence d'acides aminés 1-389 de CRM197 ; et dans laquelle la séquence antigénique est MSP3, LSA3, ou LSA5, ou un fragment épitopique de MSP3 qui englobe les motifs a, b, c, d, e et / ou f de la région C-terminale d'une protéine MSP3 de Plasmodium choisie parmi MSP3-1, MSP3-2, MSP3-3, MSP3-4, MSP3-7, MSP3-8, de préférence choisie parmi SEQ ID NO: 7 à SEQ ID NO: 54, ou un fragment épitopique de LSA3 qui est le peptide DG729 de la SEQ ID NO: 77.

2. Protéine de fusion selon la revendication 1, dans laquelle la séquence antigénique est MSP3-1.

3. Protéine de fusion selon l'une quelconque des revendications 1 et 2, comprenant deux séquences antigéniques qui sont soit chacune située à l'extrémité C et à l'extrémité N de la séquence de la protéine porteuse, soit toutes deux situées à la même extrémité.

4. Protéine de fusion selon l'une quelconque des revendications 1 à 3, dans laquelle la au moins une séquence d'acides aminés est liée à l'extrémité N-terminale et / ou à l'extrémité C-terminale de la séquence de protéine hétérologue porteuse par un lieur peptidique, dans laquelle le lieur peptidique est de préférence une séquence de 1 à 35 acides aminés, de préférence de 5 à 20 acides aminés.

5. Protéine de fusion selon la revendication 4, dans laquelle le lieur peptidique est (Gly-Gly-Gly-Gly-Ser)n, (Gly)n ou (EAAAK)n, dans lequel n est 1 à 4, de préférence, 1 à 3.

6. Construction d'acide nucléique codant pour la protéine de fusion telle que définie dans l'une quelconque des revendications 1 à 5.

7. Vecteur comprenant la construction d'acide nucléique de la revendication 6 dans une cassette d'expression.

8. Cellule hôte dans laquelle le vecteur de la revendication 7 a été inséré.

9. Procédé *in vitro* pour préparer une protéine de fusion telle que définie dans l'une quelconque des revendications 1 à 5, lequel procédé consiste à permettre à la cellule hôte de la revendication 8 de se reproduire dans des conditions qui induisent l'expression de la construction d'acide nucléique telle que définie dans la revendication 6, et à recueillir la protéine de fusion qui est ainsi exprimée.

10. Vaccin comprenant la protéine de fusion telle que définie dans l'une quelconque des revendications 1 à 5, ou une combinaison d'au moins deux protéines de fusion telles que définies dans l'une quelconque des revendications 1 à 5, qui comprennent respectivement au moins deux séquences d'acides aminés antigéniques différentes, ou une construction d'acide nucléique codant pour ladite ou lesdites protéine(s) de fusion, telle que définie dans la revendication 6, avec un véhicule physiologiquement acceptable.

11. Vaccin selon la revendication 10, destiné à être utilisé pour vacciner un sujet humain contre le paludisme.


### SEQUENCE LISTING

<110> Vac4all
<120> Biofusion proteins as anti-malaria vaccines
<130> B2284PC00
<160> 89
<170> PatentIn version 3.5
<210> 1
   <211> 1103
   <212> PRT
   <213> Plasmodium falciparum
<400> 1
   Met Ser Asn Lys Lys Arg Ser Lys Asn Glu Asn Asp Glu Ser Thr Ser
   1               5                   10                  15
   Leu Pro Leu Glu Asn Ser Glu Leu Leu Ile Glu Tyr Ile His Asn Leu
               20                  25                  30
   Lys Ser Cys Leu Asn Val Tyr Arg Arg Glu Ile Gln Glu Lys Asn Lys
           35                  40                  45
   Tyr Ile Ser Ile Ile Lys Asn Asp Leu Ser Phe His Glu Cys Ile Leu
       50                  55                  60
   Thr Asn Val Asn Val Val Trp Ser Val Phe Asn Asn Asp Leu Leu Asn
   65                  70                  75                  80
   Leu Leu Cys Asn Asn Glu Gln Lys Glu Glu Gly Glu Glu Ile Ile Lys
                   85                  90                  95
   Gln Arg Asn Ile Gly Asp Glu Ile Asn Glu Tyr Asn Asn Leu Thr Lys
               100                 105                 110
   Leu Gln Asn Asp Glu Asn Ile Lys Asn Asn Asn Met Ile Lys Glu Asp
           115                 120                 125
   Leu Glu Asp Asp Ala Asn Gln Asn Ile Leu Met Lys Ser Pro Tyr Tyr
       130                 135                 140
   Asn Ile Glu Asn Phe Leu Gln Val Phe Leu Lys Tyr Ile Asn Lys Lys
   145                 150                 155                 160
   Lys Lys Lys Val Lys Val Lys Val Lys Asp Glu Gly Lys Lys Glu Lys
                   165                 170                 175
   Ile Glu Asp Lys Lys Tyr Glu Gln Asp Asp Glu Glu Glu Asn Glu Glu
               180                 185                 190
   Glu Glu Glu Glu Glu Glu Glu Glu Glu Gly Glu Glu Glu Asn Lys Glu
           195                 200                 205
   Asp Glu Glu Phe Phe Lys Thr Phe Val Ser Phe Asn Leu Tyr His Asn
       210                 215                 220
   Asn Asn Glu Lys Asn Ile Ser Tyr Asp Lys Asn Leu Val Lys Gln Glu
   225                 230                 235                 240
   Asn Asp Asn Lys Asp Glu Ala Arg Gly Asn Asp Asn Met Cys Gly Asn
                   245                 250                 255
   Tyr Asp Ile His Asn Glu Arg Gly Glu Met Leu Asp Lys Gly Lys Ser
               260                 265                 270
   Tyr Ser Gly Asp Glu Lys Ile Asn Thr Ser Asp Asn Ala Lys Ser Cys
           275                 280                 285
   Ser Gly Asp Glu Lys Val Ile Thr Ser Asp Asn Gly Lys Ser Tyr Asp
       290                 295                 300
   Tyr Val Lys Asn Glu Ser Glu Glu Gln Glu Glu Lys Glu Asn Met Leu
   305                 310                 315                 320
   Asn Asn Lys Lys Arg Ser Leu Glu Cys Asn Pro Asn Glu Ala Lys Lys
                   325                 330                 335
   Ile Cys Phe Ser Leu Glu Glu Lys Ile Gly Thr Val Gln Ser Val Lys
               340                 345                 350
   Leu Lys Glu Tyr Asn Glu Leu Ser Lys Glu Asn Ile Glu Lys Asn Lys
           355                 360                 365
   His Asp Asp Asn Asn Ile Cys Asn Tyr Leu Ser His Asn Glu Gly Glu
       370                 375                 380
   Asn Val Ile Glu Arg Glu Asp Lys Leu Phe Asn Lys Leu Asn Asn Lys
   385                 390                 395                 400
   Asn Tyr Arg Asn Glu Glu Glu Lys Lys Lys Asn Gln Ile Asn Phe Asp
                   405                 410                 415
   Tyr Leu Lys Lys Lys Ile Lys Asn Asn Gln Asp Val Phe Glu Glu Thr
               420                 425                 430
   Ile Gln Lys Cys Phe Leu Ile Asn Leu Lys Lys Thr Leu Asn Leu Ile
           435                 440                 445
   Asn Lys Ile Met Tyr Leu Lys Asn Val Glu Phe Arg Lys Tyr Asn Leu
       450                 455                 460
   Asp Tyr Ile Arg Lys Ile Asn Tyr Glu Lys Cys Phe Tyr Tyr Lys Asn
   465                 470                 475                 480
   Tyr Ile Asp Ile Lys Lys Lys Ile Ser Glu Leu Gln Lys Asp Asn Glu
                   485                 490                 495
   Ser Leu Lys Ile Gln Val Asp Arg Leu Glu Lys Lys Lys Ala Thr Leu
               500                 505                 510
   Ile Tyr Lys Leu Asn Asn Asp Asn Ile Arg Lys His Ile Leu Asp Asn
           515                 520                 525
   Asn Ile Lys Asp Tyr Gln Asn Gly Ile Asp Asn Ser Lys Val Ser Tyr
       530                 535                 540
   Phe Asp Glu Gly Glu Asn Pro Tyr Asn Arg Asn Asn Lys Asn Tyr Arg
   545                 550                 555                 560
   Thr Asp Asn Lys Asn Ser Asp Asp Asn Asn Asn Asn Asn Asn Tyr Tyr
                   565                 570                 575
   Tyr Asn Asn Tyr Asn Ser Asp Asp Asn Tyr Asn Ser Glu Asp Asn Glu
               580                 585                 590
   Tyr Asn Asn Gly Asn Tyr Arg Phe Arg Asn Asn Tyr Lys Lys Asp Ser
           595                 600                 605
   Leu Asn Glu Asp Asp Val Lys Lys Asn Pro Leu Lys Val Cys His Lys
       610                 615                 620
   Ile Asn Ser Asp Ser Asn Ile Phe Val Asn Phe Glu Asn Ile Ile Thr
   625                 630                 635                 640
   Lys Gln Asn Ile Ile His Ser Glu Pro Phe Arg Asn Leu Leu Lys Glu
                   645                 650                 655
   Ser Asn Glu Leu Tyr Ile Thr Leu Lys Glu Lys Glu Lys Glu Asn Ile
               660                 665                 670
   Ile Leu Lys Asn Glu Ile Leu Lys Met Glu Asn Lys Lys Asp Glu Glu
           675                 680                 685
   Tyr Glu His Leu Leu Asn Asn Thr Ile Glu Asp Lys Lys Glu Leu Thr
       690                 695                 700
   Arg Ser Ile Lys Glu Leu Glu Ile Asn Met Met Thr Cys Asn Met Glu
   705                 710                 715                 720
   Lys Asp Lys Ile Ser Asn Lys Val Asn Thr Leu Glu Tyr Glu Ile Asn
                   725                 730                 735
   Val Leu Lys Asn Ile Asp Lys Asn Gln Thr Met Gln Leu Gln Gln Lys
               740                 745                 750
   Glu Asn Asp Ile Leu Lys Met Lys Leu Tyr Ile Glu Lys Leu Lys Leu
           755                 760                 765
   Ser Glu Lys Asn Leu Lys Asp Lys Ile Ile Leu Leu Glu Asn Glu Lys
       770                 775                 780
   Asp Lys Met Leu Ser Gly Ile His Ile Lys Asp Asn Ser Phe Asn Glu
   785                 790                 795                 800
   Glu Ser Lys Ser Glu Glu Gly Lys Ile Gln Leu Arg Asp Ile Gln Asn
                   805                 810                 815
   Asp Asn Asp Glu Lys Tyr Asp Asp Glu Lys Lys Arg Phe Lys Glu Leu
               820                 825                 830
   Phe Ile Glu Asn Gln Lys Leu Lys Glu Glu Leu Asn Lys Lys Arg Asn
           835                 840                 845
   Val Glu Glu Glu Leu His Ser Leu Arg Lys Asn Tyr Asn Ile Ile Asn
       850                 855                 860
   Glu Glu Ile Glu Glu Ile Thr Lys Glu Phe Glu Lys Lys Gln Glu Gln
   865                 870                 875                 880
   Val Asp Glu Met Ile Leu Gln Ile Lys Asn Lys Glu Leu Glu Leu Leu
                   885                 890                 895
   Asp Lys Phe Asn Asn Lys Met Asn Lys Ala Tyr Val Glu Glu Lys Leu
               900                 905                 910
   Lys Glu Leu Lys Asn Thr Tyr Glu Glu Lys Met Lys His Ile Asn Asn
           915                 920                 925
   Ile Tyr Lys Lys His Asp Asp Phe Val Asn Ile Tyr Leu Asn Leu Phe
   930 935 940
   Phe Gln Ala Arg Lys Asn Ala Ile Leu Ser Asp Ser Gln Arg Glu Glu
   945 950 955 960
   Gln Met Asn Leu Phe Ile Lys Leu Lys Asp Lys Tyr Asp Ile Ile Phe
   965 970 975
   Gln Lys Lys Ile Glu Leu Thr Asp Ile Leu Lys Asn Val Tyr Asp Cys
   980 985 990
   Asn Lys Lys Leu Ile Gly His Cys Gln Asp Leu Glu Lys Glu Asn Ser
   995 1000 1005
   Thr Leu Gln Asn Lys Leu Ser Asn Glu Ile Lys Asn Ser Lys Met
   1010 1015 1020
   Leu Ser Lys Asn Leu Ser Lys Asn Ser Asp Asp His Leu Leu Ile
   1025 1030 1035
   Glu Glu Asn Asn Glu Leu Arg Arg Arg Leu Ile Cys Ser Val Cys
   1040 1045 1050
   Met Glu Asn Phe Arg Asn Tyr Ile Ile Ile Lys Cys Gly His Ile
   1055 1060 1065
   Tyr Cys Asn Asn Cys Ile Phe Asn Asn Leu Lys Thr Arg Asn Arg
   1070 1075 1080
   Lys Cys Pro Gln Cys Lys Val Pro Phe Asp Lys Lys Asp Leu Gln
   1085 1090 1095
   Lys Ile Phe Leu Asp
   1100
<210> 2
   <211> 104
   <212> PRT
   <213> Plasmodium falciparum
<400> 2
   His Asn Asn Asn Glu Lys Asn Ile Ser Tyr Asp Lys Asn Leu Val Lys
   1               5                   10                  15
   Gln Glu Asn Asp Asn Lys Asp Glu Ala Arg Gly Asn Asp Asn Met Cys
               20                  25                  30
   Gly Asn Tyr Asp Ile His Asn Glu Arg Gly Glu Met Leu Asp Lys Gly
           35                  40                  45
   Lys Ser Tyr Ser Gly Asp Glu Lys Ile Asn Thr Ser Asp Asn Ala Lys
       50                  55                  60
   Ser Cys Ser Gly Asp Glu Lys Val Ile Thr Ser Asp Asn Gly Lys Ser
   65                  70                  75                  80
   Tyr Asp Tyr Val Lys Asn Glu Ser Glu Glu Gln Glu Glu Lys Glu Asn
                   85                  90                  95
   Met Leu Asn Asn Lys Lys Arg Ser
               100
<210> 3
   <211> 27
   <212> PRT
   <213> Plasmodium falciparum
<400> 3
   Lys Lys Arg Asn Val Glu Glu Glu Leu His Ser Leu Arg Lys Asn Tyr
   1               5                   10                  15
   Asn Ile Ile Asn Glu Glu Ile Glu Glu Ile Thr
               20                  25
<210> 4
   <211> 80
   <212> PRT
   <213> Plasmodium falciparum
<400> 4
   Asn Glu Glu Glu Ile Thr Thr Lys Gly Ala Ser Ala Gln Ser Gly Ala
   1               5                   10                  15
   Ser Ala Gln Ser Gly Ala Ser Ala Gln Ser Gly Ala Ser Ala Gln Ser
               20                  25                  30
   Gly Ala Ser Ala Gln Ser Gly Ala Ser Ala Gln Ser Gly Thr Ser Gly
           35                  40                  45
   Pro Ser Gly Pro Ser Gly Thr Ser Pro Ser Ser Arg Ser Asn Thr Leu
       50                  55                  60
   Pro Arg Ser Asn Thr Ser Ser Gly Ala Ser Pro Pro Ala Asp Ala Ser
   65                  70                  75                  80
<210> 5
   <211> 390
   <212> PRT
   <213> Plasmodium falciparum
<400> 5
   Met Ala His His His His His His Pro Gly Gly Ser Gly Ser Gly Thr
   1               5                   10                  15
   Met Ala Ile Ser Val Thr Met Asp Asn Ile Leu Ser Gly Phe Glu Asn
               20                  25                  30
   Glu Tyr Asp Val Ile Tyr Leu Lys Pro Leu Ala Gly Val Tyr Arg Ser
           35                  40                  45
   Leu Lys Lys Gln Ile Glu Lys Asn Ile Phe Thr Phe Asn Leu Asn Leu
       50                  55                  60
   Asn Asp Ile Leu Asn Ser Arg Leu Lys Lys Arg Lys Tyr Phe Leu Asp
   65                  70                  75                  80
   Val Leu Glu Ser Asp Leu Met Gln Phe Lys His Ile Ser Ser Asn Glu
                   85                  90                  95
   Tyr Ile Ile Glu Asp Ser Phe Lys Leu Leu Asn Ser Glu Gln Lys Asn
               100                 105                 110
   Thr Leu Leu Lys Ser Tyr Lys Tyr Ile Lys Glu Ser Val Glu Asn Asp
           115                 120                 125
   Ile Lys Phe Ala Gln Glu Gly Ile Ser Tyr Tyr Glu Lys Val Leu Ala
       130                 135                 140
   Lys Tyr Lys Asp Asp Leu Glu Ser Ile Lys Lys Val Ile Lys Glu Glu
   145                 150                 155                 160
   Lys Glu Lys Phe Pro Ser Ser Pro Pro Thr Thr Pro Pro Ser Pro Ala
                   165                 170                 175
   Lys Thr Asp Glu Gln Lys Lys Glu Ser Lys Phe Leu Pro Phe Leu Thr
               180                 185                 190
   Asn Ile Glu Thr Leu Tyr Asn Asn Leu Val Asn Lys Ile Asp Asp Tyr
           195                 200                 205
   Leu Ile Asn Leu Lys Ala Lys Ile Asn Asp Cys Asn Val Glu Lys Asp
       210                 215                 220
   Glu Ala His Val Lys Ile Thr Lys Leu Ser Asp Leu Lys Ala Ile Asp
   225                 230                 235                 240
   Asp Lys Ile Asp Leu Phe Lys Asn Pro Thr Asp Phe Glu Ala Ile Lys
                   245                 250                 255
   Lys Leu Ile Asn Asp Asp Thr Lys Lys Asp Met Leu Gly Lys Leu Leu
               260                 265                 270
   Ser Thr Gly Leu Val Gln Asn Phe Pro Asn Thr Ile Ile Ser Lys Leu
           275                 280                 285
   Ile Glu Gly Lys Phe Gln Asp Met Leu Asn Ile Ser Gln His Gln Cys
       290                 295                 300
   Val Lys Lys Gln Cys Pro Glu Asn Ser Gly Cys Phe Arg His Leu Asp
   305                 310                 315                 320
   Glu Arg Glu Glu Cys Lys Cys Leu Leu Asn Tyr Lys Gln Glu Gly Asp
                   325                 330                 335
   Lys Cys Val Glu Asn Pro Asn Pro Thr Cys Asn Glu Asn Asn Gly Gly
               340                 345                 350
   Cys Asp Ala Asp Ala Thr Cys Thr Glu Glu Asp Ser Gly Ser Ser Arg
           355                 360                 365
   Lys Lys Ile Thr Cys Glu Cys Thr Lys Pro Asp Ser Tyr Pro Leu Phe
       370                 375                 380
   Asp Gly Ile Phe Cys Ser
   385                 390
<210> 6
   <211> 272
   <212> PRT
   <213> Plasmodium falciparum
<400> 6
   Met Lys Val Ile Lys Thr Leu Ser Ile Ile Asn Phe Phe Ile Phe Val
   1               5                   10                  15
   Thr Phe Asn Ile Lys Asn Glu Ser Lys Tyr Ser Asn Thr Phe Ile Asn
               20                  25                  30
   Asn Ala Tyr Asn Met Ser Ile Arg Arg Ser Met Ala Glu Ser Lys Pro
           35                  40                  45
   Ser Thr Gly Ala Gly Gly Ser Ala Gly Gly Ser Ala Gly Gly Ser Ala
       50                  55                  60
   Gly Gly Ser Ala Gly Gly Ser Ala Gly Gly Ser Ala Gly Ser Gly Asp
   65 70 75 80
   Gly Asn Gly Ala Asp Ala Glu Gly Ser Ser Ser Thr Pro Ala Thr Thr
   85 90 95
   Thr Thr Thr Lys Thr Thr Thr Thr Thr Thr Thr Thr Asn Asp Ala Glu
   100 105 110
   Ala Ser Thr Ser Thr Ser Ser Glu Asn Pro Asn His Lys Asn Ala Glu
   115 120 125
   Thr Asn Pro Lys Gly Lys Gly Glu Val Gln Glu Pro Asn Gln Ala Asn
   130 135 140
   Lys Glu Thr Gln Asn Asn Ser Asn Val Gln Gln Asp Ser Gln Thr Lys
   145 150 155 160
   Ser Asn Val Pro Pro Thr Gln Asp Ala Asp Thr Lys Ser Pro Thr Ala
   165 170 175
   Gln Pro Glu Gln Ala Glu Asn Ser Ala Pro Thr Ala Glu Gln Thr Glu
   180 185 190
   Ser Pro Glu Leu Gln Ser Ala Pro Glu Asn Lys Gly Thr Gly Gln His
   195 200 205
   Gly His Met His Gly Ser Arg Asn Asn His Pro Gln Asn Thr Ser Asp
   210 215 220
   Ser Gln Lys Glu Cys Thr Asp Gly Asn Lys Glu Asn Cys Gly Ala Ala
   225 230 235 240
   Thr Ser Leu Leu Asn Asn Ser Ser Asn Ile Ala Ser Ile Asn Lys Phe
   245 250 255
   Val Val Leu Ile Ser Ala Thr Leu Val Leu Ser Phe Ala Ile Phe Ile
   260 265 270
<210> 7
   <211> 354
   <212> PRT
   <213> Plasmodium falciparum
<400> 7
   Met Lys Ser Phe Ile Asn Ile Thr Leu Ser Leu Phe Leu Leu His Leu
   1 5 10 15
   Tyr Ile Tyr Ile Asn Asn Val Ala Ser Lys Glu Ile Val Lys Lys Tyr
               20                  25                  30
   Asn Leu Asn Leu Arg Asn Ala Ile Leu Asn Asn Asn Ser Gln Ile Glu
           35                  40                  45
   Asn Glu Glu Asn Val Asn Thr Thr Ile Thr Gly Asn Asp Phe Ser Gly
       50                  55                  60
   Gly Glu Phe Leu Trp Pro Gly Tyr Thr Glu Glu Leu Lys Ala Lys Lys
   65                  70                  75                  80
   Ala Ser Glu Asp Ala Glu Lys Ala Ala Asn Asp Ala Glu Asn Ala Ser
                   85                  90                  95
   Lys Glu Ala Glu Glu Ala Ala Lys Glu Ala Val Asn Leu Lys Glu Ser
               100                 105                 110
   Asp Lys Ser Tyr Thr Lys Ala Lys Glu Ala Cys Thr Ala Ala Ser Lys
           115                 120                 125
   Ala Lys Lys Ala Val Glu Thr Ala Leu Lys Ala Lys Asp Asp Ala Glu
       130                 135                 140
   Lys Ser Ser Lys Ala Asp Ser Ile Ser Thr Lys Thr Lys Glu Tyr Ala
   145                 150                 155                 160
   Glu Lys Ala Lys Asn Ala Tyr Glu Lys Ala Lys Asn Ala Tyr Gln Lys
                   165                 170                 175
   Ala Asn Gln Ala Val Leu Lys Ala Lys Glu Ala Ser Ser Tyr Asp Tyr
               180                 185                 190
   Ile Leu Gly Trp Glu Phe Gly Gly Gly Val Pro Glu His Lys Lys Glu
           195                 200                 205
   Glu Asn Met Leu Ser His Leu Tyr Val Ser Ser Lys Asp Lys Glu Asn
       210                 215                 220
   Ile Ser Lys Glu Asn Asp Asp Val Leu Asp Glu Lys Glu Glu Glu Ala
   225                 230                 235                 240
   Glu Glu Thr Glu Glu Glu Glu Leu Glu Glu Lys Asn Glu Glu Glu Thr
                   245                 250                 255
   Glu Ser Glu Ile Ser Glu Asp Glu Glu Glu Glu Glu Glu Glu Glu Glu
                   260                 265                 270
   Lys Glu Glu Glu Asn Asp Lys Lys Lys Glu Gln Glu Lys Glu Gln Ser
           275                 280                 285
   Asn Glu Asn Asn Asp Gln Lys Lys Asp Met Glu Ala Gln Asn Leu Ile
       290                 295                 300
   Ser Lys Asn Gln Asn Asn Asn Glu Lys Asn Val Lys Glu Ala Ala Glu
   305                 310                 315                 320
   Ser Ile Met Lys Thr Leu Ala Gly Leu Ile Lys Gly Asn Asn Gln Ile
                   325                 330                 335
   Asp Ser Thr Leu Lys Asp Leu Val Glu Glu Leu Ser Lys Tyr Phe Lys
               340                 345                 350
   Asn His
<210> 8
   <211> 188
   <212> PRT
   <213> Plasmodium falciparum
<400> 8
   Tyr Glu Lys Ala Lys Asn Ala Tyr Gln Lys Ala Asn Gln Ala Val Leu
   1 5 10 15
   Lys Ala Lys Glu Ala Ser Ser Tyr Asp Tyr Ile Leu Gly Trp Glu Phe
   20 25 30
   Gly Gly Gly Val Pro Glu His Lys Lys Glu Glu Asn Met Leu Ser His
   35 40 45
   Leu Tyr Val Ser Ser Lys Asp Lys Glu Asn Ile Ser Lys Glu Asn Asp
   50 55 60
   Asp Val Leu Asp Glu Lys Glu Glu Glu Ala Glu Glu Thr Glu Glu Glu
   65 70 75 80
   Glu Leu Glu Glu Lys Asn Glu Glu Glu Thr Glu Ser Glu Ile Ser Glu
   85 90 95
   Asp Glu Glu Glu Glu Glu Glu Glu Glu Glu Lys Glu Glu Glu Asn Asp
   100 105 110
   Lys Lys Lys Glu Gln Glu Lys Glu Gln Ser Asn Glu Asn Asn Asp Gln
   115 120 125
   Lys Lys Asp Met Glu Ala Gln Asn Leu Ile Ser Lys Asn Gln Asn Asn
   130 135 140
   Asn Glu Lys Asn Val Lys Glu Ala Ala Glu Ser Ile Met Lys Thr Leu
   145 150 155 160
   Ala Gly Leu Ile Lys Gly Asn Asn Gln Ile Asp Ser Thr Leu Lys Asp
   165 170 175
   Leu Val Glu Glu Leu Ser Lys Tyr Phe Lys Asn His
   180 185
<210> 9
   <211> 25
   <212> PRT
   <213> Plasmodium falciparum
<400> 9
   Tyr Glu Lys Ala Lys Asn Ala Tyr Gln Lys Ala Asn Gln Ala Val Leu
   1               5                   10                  15
   Lys Ala Lys Glu Ala Ser Ser Tyr Asp
               20                  25
<210> 10
   <211> 27
   <212> PRT
   <213> Plasmodium falciparum
<400> 10
   Ala Lys Glu Ala Ser Ser Tyr Asp Tyr Ile Leu Gly Trp Glu Phe Gly
   1               5                   10                  15
   Gly Gly Val Pro Glu His Lys Lys Glu Glu Asn
               20                  25
<210> 11
   <211> 28
   <212> PRT
   <213> Plasmodium falciparum
<400> 11
   Pro Glu His Lys Lys Glu Glu Asn Met Leu Ser His Leu Tyr Val Ser
   1               5                   10                  15
   Ser Lys Asp Lys Glu Asn Ile Ser Lys Glu Asn Asp
               20                  25
<210> 12
   <211> 41
   <212> PRT
   <213> Plasmodium falciparum
<400> 12
   Met Leu Ser His Leu Tyr Val Ser Ser Lys Asp Lys Glu Asn Ile Ser
   1               5                   10                  15
   Lys Glu Asn Asp Asp Val Leu Asp Glu Lys Glu Glu Glu Ala Glu Glu
               20                  25                  30
   Thr Glu Glu Glu Glu Leu Glu Glu Lys
           35                  40
<210> 13
   <211> 34
   <212> PRT
   <213> Plasmodium falciparum
<400> 13
   Glu Glu Glu Asn Asp Lys Lys Lys Glu Gln Glu Lys Glu Gln Ser Asn
   1 5 10 15
   Glu Asn Asn Asp Gln Lys Lys Asp Met Glu Ala Gln Asn Leu Ile Ser
   20 25 30
   Lys Asn
<210> 14
   <211> 53
   <212> PRT
   <213> Plasmodium falciparum
<400> 14
   Asn Leu Ile Ser Lys Asn Gln Asn Asn Asn Glu Lys Asn Val Lys Glu
   1 5 10 15
   Ala Ala Glu Ser Ile Met Lys Thr Leu Ala Gly Leu Ile Lys Gly Asn
   20 25 30
   Asn Gln Ile Asp Ser Thr Leu Lys Asp Leu Val Glu Glu Leu Ser Lys
   35 40 45
   Tyr Phe Lys Asn His
   50
<210> 15
   <211> 371
   <212> PRT
   <213> Plasmodium falciparum
<400> 15
   Met Asn Lys Ile Tyr Asn Ile Thr Phe Leu Phe Ile Leu Leu Asn Leu
   1               5                   10                  15
   Tyr Ile Asn Glu Asn Asn Phe Ile Arg Asn Glu Leu Ile Asn Glu Lys
               20                  25                  30
   Asn His Asn Leu Arg Asn Gly Ser Met Tyr Asn Asn Asp Lys Ile Leu
           35                  40                  45
   Ser Lys Asn Glu Val Asp Thr Asn Ile Glu Ser Asn Glu Asn Ser Ile
       50                  55                  60
   His Glu Ser Gly His Lys Ile Asp Gly Glu Glu Val Leu Lys Ala Asn
   65                  70                  75                  80
   Val Asp Asp Ile Thr Tyr Lys Lys Lys Asn Val Asp Asp Ser Glu Ile
                   85                  90                  95
   Pro Phe Ser Gly Tyr Asp Ile Gln Ala Thr Tyr Gln Phe Pro Ser Thr
               100                 105                 110
   Ser Gly Gly Asn Asn Val Ile Pro Leu Pro Ile Lys Gln Ser Gly Glu
           115                 120                 125
   Asn Gln Tyr Thr Val Thr Ser Ile Ser Gly Ile Gln Lys Gly Ala Asn
       130                 135                 140
   Gly Leu Thr Gly Ala Thr Glu Asn Ile Thr Gln Val Val Gln Ala Asn
   145                 150                 155                 160
   Ser Glu Thr Asn Lys Asn Pro Thr Ser His Ser Asn Ser Thr Thr Thr
                   165                 170                 175
   Ser Leu Asn Asn Asn Ile Leu Gly Trp Glu Phe Gly Gly Gly Ala Pro
               180                 185                 190
   Gln Asn Gly Ala Ala Glu Asp Lys Lys Thr Glu Tyr Leu Leu Glu Gln
           195                 200                 205
   Ile Lys Ile Pro Ser Trp Asp Arg Asn Asn Ile Pro Asp Glu Asn Glu
       210                 215                 220
   Gln Val Ile Glu Asp Pro Gln Glu Asp Asn Lys Asp Glu Asp Glu Asp
   225 230 235 240
   Glu Glu Thr Glu Thr Glu Asn Leu Glu Thr Glu Asp Asp Asn Asn Glu
   245 250 255
   Glu Ile Glu Glu Asn Glu Glu Asp Asp Ile Asp Glu Glu Ser Val Glu
   260 265 270
   Glu Lys Glu Glu Glu Glu Glu Lys Lys Glu Glu Glu Glu Lys Lys Glu
   275 280 285
   Glu Lys Lys Glu Glu Lys Lys Pro Asp Asn Glu Ile Thr Asn Glu Val
   290 295 300
   Lys Glu Glu Gln Lys Tyr Ser Ser Pro Ser Asp Ile Asn Ala Gln Asn
   305 310 315 320
   Leu Ile Ser Asn Lys Asn Lys Lys Asn Asp Glu Thr Lys Lys Thr Ala
   325 330 335
   Glu Asn Ile Val Lys Thr Leu Val Gly Leu Phe Asn Glu Lys Asn Glu
   340 345 350
   Ile Asp Ser Thr Ile Asn Asn Leu Val Gln Glu Met Ile His Leu Phe
   355 360 365
   Ser Asn Asn
   370
<210> 16
   <211> 211
   <212> PRT
   <213> Plasmodium falciparum
<400> 16
   Ser Glu Thr Asn Lys Asn Pro Thr Ser His Ser Asn Ser Thr Thr Thr
   1               5                   10                  15
   Ser Leu Asn Asn Asn Ile Leu Gly Trp Glu Phe Gly Gly Gly Ala Pro
               20                  25                  30
   Gln Asn Gly Ala Ala Glu Asp Lys Lys Thr Glu Tyr Leu Leu Glu Gln
           35                  40                  45
   Ile Lys Ile Pro Ser Trp Asp Arg Asn Asn Ile Pro Asp Glu Asn Glu
       50                  55                  60
   Gln Val Ile Glu Asp Pro Gln Glu Asp Asn Lys Asp Glu Asp Glu Asp
   65                  70                  75                  80
   Glu Glu Thr Glu Thr Glu Asn Leu Glu Thr Glu Asp Asp Asn Asn Glu
                   85                  90                  95
   Glu Ile Glu Glu Asn Glu Glu Asp Asp Ile Asp Glu Glu Ser Val Glu
               100                 105                 110
   Glu Lys Glu Glu Glu Glu Glu Lys Lys Glu Glu Glu Glu Lys Lys Glu
           115                 120                 125
   Glu Lys Lys Glu Glu Lys Lys Pro Asp Asn Glu Ile Thr Asn Glu Val
       130                 135                 140
   Lys Glu Glu Gln Lys Tyr Ser Ser Pro Ser Asp Ile Asn Ala Gln Asn
   145                 150                 155                 160
   Leu Ile Ser Asn Lys Asn Lys Lys Asn Asp Glu Thr Lys Lys Thr Ala
                   165                 170                 175
   Glu Asn Ile Val Lys Thr Leu Val Gly Leu Phe Asn Glu Lys Asn Glu
               180                 185                 190
   Ile Asp Ser Thr Ile Asn Asn Leu Val Gln Glu Met Ile His Leu Phe
           195                 200                 205
   Ser Asn Asn
       210
<210> 17
   <211> 21
   <212> PRT
   <213> Plasmodium falciparum
<400> 17
   Ser Glu Thr Asn Lys Asn Pro Thr Ser His Ser Asn Ser Thr Thr Thr
   1               5                   10                  15
   Ser Leu Asn Asn Asn
               20
<210> 18
   <211> 27
   <212> PRT
   <213> Plasmodium falciparum
<400> 18
   Leu Asn Asn Asn Ile Leu Gly Trp Glu Phe Gly Gly Gly Ala Pro Gln
   1               5                   10                  15
   Asn Gly Ala Ala Glu Asp Lys Lys Thr Glu Tyr
               20                  25
<210> 19
   <211> 33
   <212> PRT
   <213> Plasmodium falciparum
<400> 19
   Pro Gln Asn Gly Ala Ala Glu Asp Lys Lys Thr Glu Tyr Leu Leu Glu
   1               5                   10                  15
   Gln Ile Lys Ile Pro Ser Trp Asp Arg Asn Asn Ile Pro Asp Glu Asn
               20                  25                  30
   Glu
<210> 20
   <211> 53
   <212> PRT
   <213> Plasmodium falciparum
<400> 20
   Leu Leu Glu Gln Ile Lys Ile Pro Ser Trp Asp Arg Asn Asn Ile Pro
   1 5 10 15
   Asp Glu Asn Glu Gln Val Ile Glu Asp Pro Gln Glu Asp Asn Lys Asp
   20 25 30
   Glu Asp Glu Asp Glu Glu Thr Glu Thr Glu Asn Leu Glu Thr Glu Asp
   35 40 45
   Asp Asn Asn Glu Glu
   50
<210> 21
   <211> 42
   <212> PRT
   <213> Plasmodium falciparum
<400> 21
   Glu Glu Glu Lys Lys Glu Glu Lys Lys Glu Glu Lys Lys Pro Asp Asn
   1 5 10 15
   Glu Ile Thr Asn Glu Val Lys Glu Glu Gln Lys Tyr Ser Ser Pro Ser
   20 25 30
   Asp Ile Asn Ala Gln Asn Leu Ile Ser Asn
   35 40
<210> 22
   <211> 52
   <212> PRT
   <213> Plasmodium falciparum
<400> 22
   Asn Leu Ile Ser Asn Lys Asn Lys Lys Asn Asp Glu Thr Lys Lys Thr
   1 5 10 15
   Ala Glu Asn Ile Val Lys Thr Leu Val Gly Leu Phe Asn Glu Lys Asn
   20 25 30
   Glu Ile Asp Ser Thr Ile Asn Asn Leu Val Gln Glu Met Ile His Leu
   35 40 45
   Phe Ser Asn Asn
   50
<210> 23
   <211> 424
   <212> PRT
   <213> Plasmodium falciparum
<400> 23
   Met Lys Lys Ile Val Asn Ile Ile Phe Tyr Ile Leu Tyr Leu Tyr Ile
   1               5                   10                  15
   Tyr Lys Arg Asn Leu Val Gln Asn Glu Asn Val Asn Lys Ser Asn Leu
               20                  25                  30
   Arg Lys Gly Leu Ser Thr Asn Asn Ser Glu Asn Gly Ile Lys Ser Leu
           35                  40                  45
   Lys Asp Glu Asp Glu His Ile Asn Ile Ile Gly Asp Asp Phe Ser Ala
       50                  55                  60
   Phe Ser Tyr Gly Gly Tyr Pro Ile Tyr Glu Thr Thr Gly Ser Leu Gly
   65                  70                  75                  80
   Thr Gly Val Glu Ser Val Lys Ala Ile Asp Gly Glu Ser Gly Thr Ser
                   85                  90                  95
   Met Asp Ser Lys Pro Lys Glu Asn Lys Ile Ser Thr Glu Pro Gly Ala
               100                 105                 110
   Asp Gln Val Ser Ile Gly Leu Val Asn Glu Ser Asp Ser Ser Leu Glu
   115 120 125
   Asn Asp Lys Lys Lys Lys Glu Asn Val Lys Lys Glu Met Leu Gly Thr
   130 135 140
   Glu Lys Glu Gly Ser Pro Asp Ser His Asp Ser Ser Lys Glu Lys Leu
   145 150 155 160
   Asn Leu Asn Asp Asn Ser Lys Trp Ser Asp Phe Leu Lys Asn Ile Val
   165 170 175
   Thr Phe Gly Gly Phe Gly Pro Thr Val Val His Asp Val Ser Asp Thr
   180 185 190
   Leu Ser Asp Ile Ser Lys Asp Glu Val Thr Gln Lys Thr Thr Lys Asp
   195 200 205
   Ile Gly Ser Thr Leu Leu Asp Phe Phe Leu Pro Leu Pro Thr Lys Asn
   210 215 220
   Thr Asn Thr Tyr Glu Lys Lys Asn Glu Asn Lys Asn Val Ser Asn Val
   225 230 235 240
   Asp Ser Lys Thr Lys Ser Asn Glu Lys Gly Arg Pro Pro Thr Tyr Ser
   245 250 255
   Pro Ile Leu Asp Asp Gly Ile Glu Phe Ser Gly Gly Leu Tyr Phe Asn
   260 265 270
   Glu Lys Lys Ser Thr Glu Glu Asn Lys Gln Lys Asn Val Leu Glu Ser
   275 280 285
   Val Asn Leu Thr Ser Trp Asp Lys Glu Asp Ile Val Lys Glu Asn Glu
   290 295 300
   Asp Val Lys Asp Glu Lys Asp Glu Asp Asp Glu Glu Glu Glu Glu Lys
   305 310 315 320
   Tyr Glu Asn Glu Ile Ile Lys Gln Pro Glu Asp Ile Leu Asp Glu Glu
   325 330 335
   Glu Val Leu Glu Glu Glu Ile Leu Glu Glu Asn Lys Asn Asp Thr Val
   340 345 350
   Asp Thr Ser Asp Leu Glu Lys Lys Asn Ile Pro Asp Leu Ser Asn Asp
   355 360 365
   Asn Asn Tyr Tyr Ser Leu Ile Tyr Lys Asn Tyr Lys Asp Asn Asp Lys
   370 375 380
   Ser Glu Lys Thr Ala Gln Thr Leu Ile Thr Ala Leu Ile Ser Leu Leu
   385 390 395 400
   Asn Gly Lys Asn Glu Leu Asp Ala Thr Ile Arg Arg Leu Lys His Arg
   405 410 415
   Phe Met Glu Phe Phe Thr Tyr Asn
   420
<210> 24
   <211> 197
   <212> PRT
   <213> Plasmodium falciparum
<400> 24
   Tyr Glu Lys Lys Asn Glu Asn Lys Asn Val Ser Asn Val Asp Ser Lys
   1 5 10 15
   Thr Lys Ser Asn Glu Lys Gly Arg Pro Pro Thr Tyr Ser Pro Ile Leu
   20 25 30
   Asp Asp Gly Ile Glu Phe Ser Gly Gly Leu Tyr Phe Asn Glu Lys Lys
   35 40 45
   Ser Thr Glu Glu Asn Lys Gln Lys Asn Val Leu Glu Ser Val Asn Leu
   50 55 60
   Thr Ser Trp Asp Lys Glu Asp Ile Val Lys Glu Asn Glu Asp Val Lys
   65 70 75 80
   Asp Glu Lys Asp Glu Asp Asp Glu Glu Glu Glu Glu Lys Tyr Glu Asn
   85 90 95
   Glu Ile Ile Lys Gln Pro Glu Asp Ile Leu Asp Glu Glu Glu Val Leu
   100 105 110
   Glu Glu Glu Ile Leu Glu Glu Asn Lys Asn Asp Thr Val Asp Thr Ser
   115 120 125
   Asp Leu Glu Lys Lys Asn Ile Pro Asp Leu Ser Asn Asp Asn Asn Tyr
   130 135 140
   Tyr Ser Leu Ile Tyr Lys Asn Tyr Lys Asp Asn Asp Lys Ser Glu Lys
   145 150 155 160
   Thr Ala Gln Thr Leu Ile Thr Ala Leu Ile Ser Leu Leu Asn Gly Lys
   165 170 175
   Asn Glu Leu Asp Ala Thr Ile Arg Arg Leu Lys His Arg Phe Met Glu
   180 185 190
   Phe Phe Thr Tyr Asn
   195
<210> 25
   <211> 24
   <212> PRT
   <213> Plasmodium falciparum
<400> 25
   Tyr Glu Lys Lys Asn Glu Asn Lys Asn Val Ser Asn Val Asp Ser Lys
   1 5 10 15
   Thr Lys Ser Asn Glu Lys Gly Arg
   20
<210> 26
   <211> 26
   <212> PRT
   <213> Plasmodium falciparum
<400> 26
   Ser Asn Glu Lys Gly Arg Pro Pro Thr Tyr Ser Pro Ile Leu Asp Asp
   1 5 10 15
   Gly Ile Glu Phe Ser Gly Gly Leu Tyr Phe
   20 25
<210> 27
   <211> 29
   <212> PRT
   <213> Plasmodium falciparum
<400> 27
   Gly Ile Glu Phe Ser Gly Gly Leu Tyr Phe Asn Glu Lys Lys Ser Thr
   1 5 10 15
   Glu Glu Asn Lys Gln Lys Asn Val Leu Glu Ser Val Asn
   20 25
<210> 28
   <211> 32
   <212> PRT
   <213> Plasmodium falciparum
<400> 28
   Ser Thr Glu Glu Asn Lys Gln Lys Asn Val Leu Glu Ser Val Asn Leu
   1               5                   10                  15
   Thr Ser Trp Asp Lys Glu Asp Ile Val Lys Glu Asn Glu Asp Val Lys
               20                  25                  30
<210> 29
   <211> 33
   <212> PRT
   <213> Plasmodium falciparum
<400> 29
   Asp Thr Ser Asp Leu Glu Lys Lys Asn Ile Pro Asp Leu Ser Asn Asp
   1               5                   10                  15
   Asn Asn Tyr Tyr Ser Leu Ile Tyr Lys Asn Tyr Lys Asp Asn Asp Lys
               20                  25                  30
   Ser
<210> 30
   <211> 52
   <212> PRT
   <213> Plasmodium falciparum
<400> 30
   Ser Leu Ile Tyr Lys Asn Tyr Lys Asp Asn Asp Lys Ser Glu Lys Thr
   1               5                   10                  15
   Ala Gln Thr Leu Ile Thr Ala Leu Ile Ser Leu Leu Asn Gly Lys Asn
               20                  25                  30
   Glu Leu Asp Ala Thr Ile Arg Arg Leu Lys His Arg Phe Met Glu Phe
           35                  40                  45
   Phe Thr Tyr Asn
       50
<210> 31
   <211> 697
   <212> PRT
   <213> Plasmodium falciparum
<400> 31
   Met Lys Lys Ile Tyr Ser Ile Phe Phe Ser Leu Phe Ile Leu Asn Leu
   1               5                   10                  15
   His Ile Tyr Ile Lys Asn Ile Lys Cys Asn Asp Leu Ile Asn Tyr Asn
   20 25 30
   Asp Ser Asn Leu Arg Asn Gly Leu Leu Asn Asn Ser Leu Asp Leu Thr
   35 40 45
   Asn Gly Leu Asn Asn Lys Asp Asn Ser Phe Ile Asp Ser Lys Ile Glu
   50 55 60
   Glu His Glu Asn Lys Ser Tyr Gln Asn Lys Asp Asn Asn Ile Ser Ile
   65 70 75 80
   Val Gly Gln Asp Val Pro Ile Thr Ser Val Tyr Ser Ser Lys Ile Ile
   85 90 95
   Asn Ala Asn Asp Leu Glu Gly Asn Ser Ile Asp Asp Thr Lys Gly Leu
   100 105 110
   Ser Val Thr Asn Ser Gly Phe Asp Asp Gly Ser Ala Phe Gly Gly Gly
   115 120 125
   Leu Pro Phe Ser Gly Tyr Ser Pro Leu Gln Gly Asn His Asn Lys Cys
   130 135 140
   Pro Asp Glu Asn Phe Cys Lys Gly Ile Lys Asn Val Leu Ser Cys Pro
   145 150 155 160
   Pro Lys Asn Ser Thr Gly Arg Asn Gly Asp Trp Ile Ser Val Ala Val
   165 170 175
   Lys Glu Ser Ser Thr Thr Asn Lys Gly Val Leu Val Pro Pro Arg Arg
   180 185 190
   Thr Lys Leu Cys Leu Arg Asn Ile Asn Lys Val Trp His Arg Ile Lys
   195 200 205
   Asp Glu Lys Asn Phe Lys Glu Glu Phe Val Lys Val Ala Leu Gly Glu
   210 215 220
   Ser Asn Ala Leu Met Lys His Tyr Lys Glu Lys Asn Leu Asn Ala Leu
   225 230 235 240
   Thr Ala Ile Lys Tyr Gly Phe Ser Asp Met Gly Asp Ile Ile Lys Gly
   245 250 255
   Thr Asp Leu Ile Asp Tyr Gln Ile Thr Lys Asn Ile Asn Arg Ala Leu
   260 265 270
   Asp Lys Ile Leu Arg Asn Glu Thr Ser Asn Asp Lys Ile Lys Lys Arg
           275                 280                 285
   Val Asp Trp Trp Glu Ala Asn Lys Ser Ala Phe Trp Asp Ala Phe Met
       290                 295                 300
   Cys Gly Tyr Lys Val His Ile Gly Asn Lys Pro Cys Pro Glu His Asp
   305                 310                 315                 320
   Asn Met Asp Arg Ile Pro Gln Tyr Leu Arg Trp Phe Arg Glu Trp Gly
                   325                 330                 335
   Thr Tyr Val Cys Ser Glu Tyr Lys Asn Lys Phe Glu Asp Val Ile Lys
               340                 345                 350
   Leu Cys Asn Ile Gln Gln Phe Thr Asn Gln Asp Asp Ser Gln Leu Leu
           355                 360                 365
   Glu Ile Ser Lys Lys Asp Lys Cys Lys Glu Ala Leu Lys His Tyr Glu
       370                 375                 380
   Glu Trp Val Asn Arg Arg Arg Pro Glu Trp Lys Gly Gln Cys Asp Lys
   385                 390                 395                 400
   Phe Glu Lys Glu Lys Ser Lys Tyr Glu Asp Thr Lys Ser Ile Thr Ala
                   405                 410                 415
   Glu Lys Tyr Leu Lys Glu Ile Cys Ser Glu Cys Asp Cys Lys Tyr Lys
               420                 425                 430
   Asp Leu Asp Asn Thr Phe Lys Glu Phe Lys Asp Asn Val Thr Leu Leu
           435                 440                 445
   Lys Ala Val Ile Asp Asn Lys Lys Asn Gln Asp Ser Leu Thr Thr Thr
       450                 455                 460
   Ser Leu Ser Thr Ser Ile Asn Ser Val Arg Asp Ser Ser Asn Leu Asp
   465                 470                 475                 480
   Gln Arg Gly Asn Ile Thr Thr Ser Gln Gly Asn Ser His Arg Ala Thr
                   485                 490                 495
   Val Val Gln Gln Val Asp Gln Thr Asn Arg Leu Asp Asn Val Asn Ser
               500                 505                 510
   Val Thr Gln Arg Gly Asn Asn Asn Tyr Asn Asn Asn Leu Glu Arg Gly
           515                 520                 525
   Leu Gly Ser Gly Ala Leu Pro Gly Thr Asn Ile Ile Thr Glu Glu Lys
       530                 535                 540
   Tyr Ser Leu Glu Leu Ile Lys Leu Thr Ser Lys Asp Glu Glu Asp Ile
   545                 550                 555                 560
   Ile Lys His Asn Glu Asp Val Arg Glu Glu Ile Glu Glu Gln Gln Glu
                   565                 570                 575
   Asp Ile Glu Glu Asp Glu Glu Glu Leu Glu Asn Glu Gly Glu Glu Thr
               580                 585                 590
   Lys Glu Glu Asp Asp Glu Glu Lys Asn Glu Thr Asn Asp Thr Glu Asp
           595                 600                 605
   Thr Asp Asp Thr Glu Asp Thr Glu Asp Ile Glu Glu Glu Asn Lys Glu
       610                 615                 620
   Lys Glu Leu Ser Asn Gln Gln Gln Ser Glu Lys Lys Ser Ile Ser Lys
   625                 630                 635                 640
   Val Asp Glu Asp Ser Tyr Arg Ile Leu Ser Val Ser Tyr Lys Asp Asn
                   645                 650                 655
   Asn Glu Val Lys Asn Val Ala Glu Ser Ile Val Lys Lys Leu Phe Ser
               660                 665                 670
   Leu Phe Asn Asp Asn Asn Asn Leu Glu Thr Ile Phe Lys Gly Leu Thr
           675                 680                 685
   Glu Asp Met Thr Asp Leu Phe Gln Lys
       690                 695
<210> 32
   <211> 190
   <212> PRT
   <213> Plasmodium falciparum
<400> 32
   Asp Asn Val Asn Ser Val Thr Gln Arg Gly Asn Asn Asn Tyr Asn Asn
   1               5                   10                  15
   Asn Leu Glu Arg Gly Leu Gly Ser Gly Ala Leu Pro Gly Thr Asn Ile
               20                  25                  30
   Ile Thr Glu Glu Lys Tyr Ser Leu Glu Leu Ile Lys Leu Thr Ser Lys
           35                  40                  45
   Asp Glu Glu Asp Ile Ile Lys His Asn Glu Asp Val Arg Glu Glu Ile
   50 55 60
   Glu Glu Gln Gln Glu Asp Ile Glu Glu Asp Glu Glu Glu Leu Glu Asn
   65 70 75 80
   Glu Gly Glu Glu Thr Lys Glu Glu Asp Asp Glu Glu Lys Asn Glu Thr
   85 90 95
   Asn Asp Thr Glu Asp Thr Asp Asp Thr Glu Asp Thr Glu Asp Ile Glu
   100 105 110
   Glu Glu Asn Lys Glu Lys Glu Leu Ser Asn Gln Gln Gln Ser Glu Lys
   115 120 125
   Lys Ser Ile Ser Lys Val Asp Glu Asp Ser Tyr Arg Ile Leu Ser Val
   130 135 140
   Ser Tyr Lys Asp Asn Asn Glu Val Lys Asn Val Ala Glu Ser Ile Val
   145 150 155 160
   Lys Lys Leu Phe Ser Leu Phe Asn Asp Asn Asn Asn Leu Glu Thr Ile
   165 170 175
   Phe Lys Gly Leu Thr Glu Asp Met Thr Asp Leu Phe Gln Lys
   180 185 190
<210> 33
   <211> 25
   <212> PRT
   <213> Plasmodium falciparum
<400> 33
   Asp Asn Val Asn Ser Val Thr Gln Arg Gly Asn Asn Asn Tyr Asn Asn
   1 5 10 15
   Asn Leu Glu Arg Gly Leu Gly Ser Gly
   20 25
<210> 34
   <211> 27
   <212> PRT
   <213> Plasmodium falciparum
<400> 34
   Leu Glu Arg Gly Leu Gly Ser Gly Ala Leu Pro Gly Thr Asn Ile Ile
   1 5 10 15
   Thr Glu Glu Lys Tyr Ser Leu Glu Leu Ile Lys
   20 25
<210> 35
   <211> 28
   <212> PRT
   <213> Plasmodium falciparum
<400> 35
   Lys Tyr Ser Leu Glu Leu Ile Lys Leu Thr Ser Lys Asp Glu Glu Asp
   1 5 10 15
   Ile Ile Lys His Asn Glu Asp Val Arg Glu Glu Ile
   20 25
<210> 36
   <211> 28
   <212> PRT
   <213> Plasmodium falciparum
<400> 36
   Leu Thr Ser Lys Asp Glu Glu Asp Ile Ile Lys His Asn Glu Asp Val
   1 5 10 15
   Arg Glu Glu Ile Glu Glu Gln Gln Glu Asp Ile Glu
   20 25
<210> 37
   <211> 34
   <212> PRT
   <213> Plasmodium falciparum
<400> 37
   Glu Glu Asn Lys Glu Lys Glu Leu Ser Asn Gln Gln Gln Ser Glu Lys
   1 5 10 15
   Lys Ser Ile Ser Lys Val Asp Glu Asp Ser Tyr Arg Ile Leu Ser Val
   20 25 30
   Ser Tyr
<210> 38
   <211> 51
   <212> PRT
   <213> Plasmodium falciparum
<400> 38
   Arg Ile Leu Ser Val Ser Tyr Lys Asp Asn Asn Glu Val Lys Asn Val
   1 5 10 15
   Ala Glu Ser Ile Val Lys Lys Leu Phe Ser Leu Phe Asn Asp Asn Asn
   20 25 30
   Asn Leu Glu Thr Ile Phe Lys Gly Leu Thr Glu Asp Met Thr Asp Leu
   35 40 45
   Phe Gln Lys
   50
<210> 39
   <211> 405
   <212> PRT
   <213> Plasmodium falciparum
<400> 39
   Met Asn Lys Phe Leu Asn Ile Ile Phe Tyr Ile Phe Leu Ile Leu Asn
   1 5 10 15
   Phe Ser Phe Phe Gln Ser Asn Ala Thr Ser Lys Glu Ile Gln Lys Asp
   20 25 30
   Glu Gln Lys Asn Leu Arg Asn Gly Ser Ser Ile Asn Asn Asn Lys Asn
   35 40 45
   Ile Glu Asn Lys Asn Asp Asn Ile Glu Thr Gln Tyr Glu Ala Ser Glu
   50 55 60
   Tyr Ile Glu Lys Gln Asn Asp Ile Leu Asn Met Tyr Asn Asp Glu Lys
   65 70 75 80
   Glu Lys Asn Asn Asn Asn Ser Leu Asp Thr Asn Val Thr Lys Asn Thr
   85 90 95
   Val Ile Asp Asn Ser Asn Lys Phe Gln Ser Ile Glu Asp Asn Asn Val
   100 105 110
   Tyr Asn Lys Gly Ile Phe Val Gly Thr Gly Ile Lys Leu Asn Asp Ser
   115 120 125
   Gln Thr Thr Ser Asp Asn Tyr Lys Asn Glu Arg Tyr Gln Ile Asp Asp
   130 135 140
   Glu Lys Leu Lys Tyr Gly Gly Ser Phe Asp Thr Ile Phe Ser Gly Phe
   145 150 155 160
   Val Asn Leu Leu Thr Pro Ser Ser Pro Thr Gln Asn Asp Gly Ser Thr
   165 170 175
   Gly Arg Asn Val Pro Pro Pro Ser Glu Pro Asn Val Asp Thr Pro Asp
               180                 185                 190
   Pro Pro Thr Ala Pro Ala Pro Val Lys Val Pro Glu Asp Ala Lys Leu
           195                 200                 205
   Ser Ser Ser Pro Arg Pro Glu Gly Pro Arg Ala Asn Asn Arg Asn Glu
       210                 215                 220
   Asn Asn Gln Asn Thr Asp Pro Tyr Asn His Tyr Phe Ala Trp Glu Ile
   225                 230                 235                 240
   Gly Gly Gly Ala Pro Thr Tyr Lys Pro Glu Asn Asn Lys Asn Asp Asn
                   245                 250                 255
   Ile Leu Leu Glu His Val Lys Ile Thr Ser Trp Asp Lys Glu Asp Ile
               260                 265                 270
   Ile Lys Glu Asn Glu Asp Thr Lys Arg Glu Val Gln Glu Thr Glu Asp
           275                 280                 285
   Thr Asp Glu Thr Glu Asp Thr Asp Glu Thr Glu Glu Thr Glu Asp Met
       290                 295                 300
   Glu Asp Glu Asn Glu Ile Val Glu Asp Gln Leu Gln Glu Asn Glu Asp
   305                 310                 315                 320
   Asp Glu Asp Asn Val Asn Leu Glu Asp Ile Asn Lys Asn Thr Arg Asn
                   325                 330                 335
   Asp Ile Phe Glu Glu Gln Ile Lys Leu Asp Ser Thr Gln Asp Asp Lys
               340                 345                 350
   Ala Gln Lys Leu Ile Ser Asn Glu Tyr Lys Lys Thr Glu Glu Lys Lys
           355                 360                 365
   Ser Leu Glu Asp His Val Asn Leu Leu Phe Asn Phe Leu Gln Thr Asn
       370                 375                 380
   Asn Gln Leu Asp Pro Ser Leu Lys Asp Leu Glu Asn Glu Leu Thr Phe
   385                 390                 395                 400
   Phe Leu Asn Asn Tyr
                   405
<210> 40
   <211> 192
   <212> PRT
   <213> Plasmodium falciparum
<400> 40
   Pro Glu Gly Pro Arg Ala Asn Asn Arg Asn Glu Asn Asn Gln Asn Thr
   1               5                   10                  15
   Asp Pro Tyr Asn His Tyr Phe Ala Trp Glu Ile Gly Gly Gly Ala Pro
               20                  25                  30
   Thr Tyr Lys Pro Glu Asn Asn Lys Asn Asp Asn Ile Leu Leu Glu His
           35                  40                  45
   Val Lys Ile Thr Ser Trp Asp Lys Glu Asp Ile Ile Lys Glu Asn Glu
       50                  55                  60
   Asp Thr Lys Arg Glu Val Gln Glu Thr Glu Asp Thr Asp Glu Thr Glu
   65                  70                  75                  80
   Asp Thr Asp Glu Thr Glu Glu Thr Glu Asp Met Glu Asp Glu Asn Glu
                   85                  90                  95
   Ile Val Glu Asp Gln Leu Gln Glu Asn Glu Asp Asp Glu Asp Asn Val
               100                 105                 110
   Asn Leu Glu Asp Ile Asn Lys Asn Thr Arg Asn Asp Ile Phe Glu Glu
           115                 120                 125
   Gln Ile Lys Leu Asp Ser Thr Gln Asp Asp Lys Ala Gln Lys Leu Ile
       130                 135                 140
   Ser Asn Glu Tyr Lys Lys Thr Glu Glu Lys Lys Ser Leu Glu Asp His
   145                 150                 155                 160
   Val Asn Leu Leu Phe Asn Phe Leu Gln Thr Asn Asn Gln Leu Asp Pro
                   165                 170                 175
   Ser Leu Lys Asp Leu Glu Asn Glu Leu Thr Phe Phe Leu Asn Asn Tyr
               180                 185                 190
<210> 41
   <211> 26
   <212> PRT
   <213> Plasmodium falciparum
<400> 41
   Pro Glu Gly Pro Arg Ala Asn Asn Arg Asn Glu Asn Asn Gln Asn Thr
   1               5                   10                  15
   Asp Pro Tyr Asn His Tyr Phe Ala Trp Glu
   20 25
<210> 42
   <211> 22
   <212> PRT
   <213> Plasmodium falciparum
<400> 42
   Tyr Asn His Tyr Phe Ala Trp Glu Ile Gly Gly Gly Ala Pro Thr Tyr
   1 5 10 15
   Lys Pro Glu Asn Asn Lys
   20
<210> 43
   <211> 27
   <212> PRT
   <213> Plasmodium falciparum
<400> 43
   Pro Thr Tyr Lys Pro Glu Asn Asn Lys Asn Asp Asn Ile Leu Leu Glu
   1 5 10 15
   His Val Lys Ile Thr Ser Trp Asp Lys Glu Asp
   20 25
<210> 44
   <211> 29
   <212> PRT
   <213> Plasmodium falciparum
<400> 44
   Ile Leu Leu Glu His Val Lys Ile Thr Ser Trp Asp Lys Glu Asp Ile
   1 5 10 15
   Ile Lys Glu Asn Glu Asp Thr Lys Arg Glu Val Gln Glu
   20 25
<210> 45
   <211> 35
   <212> PRT
   <213> Plasmodium falciparum
<400> 45
   Val Asn Leu Glu Asp Ile Asn Lys Asn Thr Arg Asn Asp Ile Phe Glu
   1 5 10 15
   Glu Gln Ile Lys Leu Asp Ser Thr Gln Asp Asp Lys Ala Gln Lys Leu
   20 25 30
   Ile Ser Asn
   35
<210> 46
   <211> 52
   <212> PRT
   <213> Plasmodium falciparum
<400> 46
   Gln Lys Leu Ile Ser Asn Glu Tyr Lys Lys Thr Glu Glu Lys Lys Ser
   1 5 10 15
   Leu Glu Asp His Val Asn Leu Leu Phe Asn Phe Leu Gln Thr Asn Asn
   20 25 30
   Gln Leu Asp Pro Ser Leu Lys Asp Leu Glu Asn Glu Leu Thr Phe Phe
   35 40 45
   Leu Asn Asn Tyr
   50
<210> 47
   <211> 762
   <212> PRT
   <213> Plasmodium falciparum
<400> 47
   Met Ile Tyr Ile Leu Ser Ile Val Phe Tyr Ile Phe Phe Leu His Ile
   1 5 10 15
   Asp Ile Tyr Val Asn Ile Tyr Ser Thr Cys Phe Val Val Asn Glu Gly
   20 25 30
   Asn Pro Asn Leu Arg Asn Asn Ile Ile Asn Asp Asp Glu Leu Lys Gly
   35 40 45
   Lys Ala Tyr Asn Asn Thr Ile Asp Ala Asn Asn Gln Asn Ile Glu Tyr
   50 55 60
   Asn Lys Asn Leu Lys His Asn Val Asn Ser Ser His Ile Ser Lys Phe
   65 70 75 80
   Ser Asp Ile Met Asp Gln Glu Asp Lys Gly Asp Asn Glu Asn Ser His
   85 90 95
   Asp Ile Lys Phe Glu Glu Lys Lys Asn Ile Asn Lys Ser Leu Asp Ala
   100 105 110
   Glu Ser Asn Tyr Gly Ile Asn Glu Ile Ser Ile Thr Gly Asn Asp Ser
           115                 120                 125
   Asn Ser Asp Asn Ser Asn Gln Asn Ile Phe Pro Asp Gly Ser Glu Leu
       130                 135                 140
   Ala Gly Gly Ile Pro Arg Ser Ile Tyr Thr Ile Asn Leu Gly Phe Asn
   145                 150                 155                 160
   Lys Cys Pro Thr Glu Glu Ile Cys Lys Asp Phe Ser Asn Leu Pro Gln
                   165                 170                 175
   Cys Arg Lys Asn Val His Glu Arg Asn Asn Trp Leu Gly Ser Ser Val
               180                 185                 190
   Lys Asn Phe Ser Ser Asp Asn Lys Gly Val Leu Val Pro Pro Arg Arg
           195                 200                 205
   Gln Ser Leu Cys Leu Arg Ile Thr Leu Gln Asp Phe Arg Thr Lys Lys
       210                 215                 220
   Lys Lys Glu Gly Asp Phe Glu Lys Phe Ile Tyr Ser Tyr Ala Ser Ser
   225                 230                 235                 240
   Glu Ala Arg Lys Leu Arg Thr Ile His Asn Asn Asn Leu Glu Lys Ala
                   245                 250                 255
   His Gln Ala Ile Arg Tyr Ser Phe Ala Asp Ile Gly Asn Ile Ile Arg
               260                 265                 270
   Gly Asp Asp Met Met Asp Thr Pro Thr Ser Lys Glu Thr Ile Thr Tyr
           275                 280                 285
   Leu Glu Lys Val Leu Lys Ile Tyr Asn Glu Asn Asn Asp Lys Pro Lys
       290                 295                 300
   Asp Ala Lys Lys Trp Trp Thr Glu Asn Arg His His Val Trp Glu Ala
   305                 310                 315                 320
   Met Met Cys Gly Tyr Gln Ser Ala Gln Lys Asp Asn Gln Cys Thr Gly
                   325                 330                 335
   Tyr Gly Asn Ile Asp Asp Ile Pro Gln Phe Leu Arg Trp Phe Arg Glu
               340                 345                 350
   Trp Gly Thr Tyr Val Cys Glu Glu Ser Glu Lys Asn Met Asn Thr Leu
           355                 360                 365
   Lys Ala Val Cys Phe Pro Lys Gln Pro Arg Thr Glu Ala Asn Pro Ala
   370 375 380
   Leu Thr Val His Glu Asn Glu Met Cys Ser Ser Thr Leu Lys Lys Tyr
   385 390 395 400
   Glu Glu Trp Tyr Asn Lys Arg Lys Thr Glu Trp Thr Glu Gln Ser Ile
   405 410 415
   Lys Tyr Asn Asn Asp Lys Ile Asn Tyr Thr Asp Ile Lys Thr Leu Ser
   420 425 430
   Pro Ser Glu Tyr Leu Ile Glu Lys Cys Pro Glu Cys Lys Cys Thr Lys
   435 440 445
   Lys Asn Leu Gln Asp Val Phe Glu Leu Thr Phe Asp Gly Lys Ala Leu
   450 455 460
   Leu Glu Lys Leu Lys Lys Glu Glu Ser Pro Val Ser Asn Ser Val Asn
   465 470 475 480
   Ala Leu Pro Glu Pro Gly Gln Ile Thr Leu Pro Asp Pro Ser Leu Lys
   485 490 495
   Gln Thr Thr Gln Gln Glu Asn Gln Pro Val Val Glu Thr Pro Val Thr
   500 505 510
   Thr Ala Val Ile Asn Glu His Gln Gly Gln Thr Glu Pro Asn Lys Gly
   515 520 525
   Asp Asn Asn Asn Glu Arg Glu Asn His Glu Ser Asn Val Gly Ser Ile
   530 535 540
   Gln Glu Val Asn Gln Gly Ser Val Ser Glu Glu Ser His Ser Lys Thr
   545 550 555 560
   Ile Asp Pro Ser Lys Ile Asp Asp Arg Leu Glu Leu Ser Ser Gly Ser
   565 570 575
   Ser Ser Leu Glu Gln His Ser Lys Glu Asp Val Lys Lys Gly Cys Ala
   580 585 590
   Leu Glu Leu Val Pro Leu Ser Leu Ser Asp Ile Glu Gln Ile Ala Asn
   595 600 605
   Glu Ser Glu Asp Val Leu Glu Glu Ile Glu Glu Glu Ile Asn Thr Asp
           610                 615                 620
   Gly Glu Ile Glu Tyr Ile Thr Glu Glu Glu Ile Lys Glu Asp Ile Glu
   625                 630                 635                 640
   Glu Glu Thr Glu Glu Asp Ile Glu Glu Glu Thr Glu Glu Glu Thr Glu
                   645                 650                 655
   Glu Glu Thr Glu Glu Glu Ala Asp Glu Glu Thr Val Lys Glu Ile Glu
               660                 665                 670
   Asp Lys Pro Glu Gln Glu Ile Lys Asn Lys Ser Leu Glu Glu Lys Gln
           675                 680                 685
   Ile Asp Lys Asn Thr Asp Thr Ser Glu Lys Lys Gly Phe Asn Asn Ser
       690                 695                 700
   Glu Lys Asp Glu Lys Ala Arg Asn Leu Ile Ser Lys Asn Tyr Lys Asn
   705                 710                 715                 720
   Tyr Asn Glu Leu Asp Lys Asn Val His Thr Leu Val Asn Ser Ile Ile
                   725                 730                 735
   Ser Leu Leu Glu Glu Gly Asn Gly Ser Asp Ser Thr Leu Asn Ser Leu
               740                 745                 750
   Ser Lys Asp Ile Thr Asn Leu Phe Lys Asn
           755                 760
<210> 48
   <211> 226
   <212> PRT
   <213> Plasmodium falciparum
<400> 48
   His Glu Ser Asn Val Gly Ser Ile Gln Glu Val Asn Gln Gly Ser Val
   1               5                   10                  15
   Ser Glu Glu Ser His Ser Lys Thr Ile Asp Pro Ser Lys Ile Asp Asp
               20                  25                  30
   Arg Leu Glu Leu Ser Ser Gly Ser Ser Ser Leu Glu Gln His Ser Lys
           35                  40                  45
   Glu Asp Val Lys Lys Gly Cys Ala Leu Glu Leu Val Pro Leu Ser Leu
       50                  55                  60
   Ser Asp Ile Glu Gln Ile Ala Asn Glu Ser Glu Asp Val Leu Glu Glu
   65                  70                  75                  80
   Ile Glu Glu Glu Ile Asn Thr Asp Gly Glu Ile Glu Tyr Ile Thr Glu
                   85                  90                  95
   Glu Glu Ile Lys Glu Asp Ile Glu Glu Glu Thr Glu Glu Asp Ile Glu
               100                 105                 110
   Glu Glu Thr Glu Glu Glu Thr Glu Glu Glu Thr Glu Glu Glu Ala Asp
           115                 120                 125
   Glu Glu Thr Val Lys Glu Ile Glu Asp Lys Pro Glu Gln Glu Ile Lys
       130                 135                 140
   Asn Lys Ser Leu Glu Glu Lys Gln Ile Asp Lys Asn Thr Asp Thr Ser
   145                 150                 155                 160
   Glu Lys Lys Gly Phe Asn Asn Ser Glu Lys Asp Glu Lys Ala Arg Asn
                   165                 170                 175
   Leu Ile Ser Lys Asn Tyr Lys Asn Tyr Asn Glu Leu Asp Lys Asn Val
               180                 185                 190
   His Thr Leu Val Asn Ser Ile Ile Ser Leu Leu Glu Glu Gly Asn Gly
           195                 200                 205
   Ser Asp Ser Thr Leu Asn Ser Leu Ser Lys Asp Ile Thr Asn Leu Phe
       210                 215                 220
   Lys Asn
   225
<210> 49
   <211> 33
   <212> PRT
   <213> Plasmodium falciparum
<400> 49
   His Glu Ser Asn Val Gly Ser Ile Gln Glu Val Asn Gln Gly Ser Val
   1               5                   10                  15
   Ser Glu Glu Ser His Ser Lys Thr Ile Asp Pro Ser Lys Ile Asp Asp
               20                  25                  30
   Arg
<210> 50
   <211> 29
   <212> PRT
   <213> Plasmodium falciparum
<400> 50
   Ser Lys Thr Ile Asp Pro Ser Lys Ile Asp Asp Arg Leu Glu Leu Ser
   1               5                   10                  15
   Ser Gly Ser Ser Ser Leu Glu Gln His Ser Lys Glu Asp
               20                  25
<210> 51
   <211> 29
   <212> PRT
   <213> Plasmodium falciparum
<400> 51
   Glu Gln His Ser Lys Glu Asp Val Lys Lys Gly Cys Ala Leu Glu Leu
   1               5                   10                  15
   Val Pro Leu Ser Leu Ser Asp Ile Glu Gln Ile Ala Asn
               20                  25
<210> 52
   <211> 33
   <212> PRT
   <213> Plasmodium falciparum
<400> 52
   Ala Leu Glu Leu Val Pro Leu Ser Leu Ser Asp Ile Glu Gln Ile Ala
   1               5                   10                  15
   Asn Glu Ser Glu Asp Val Leu Glu Glu Ile Glu Glu Glu Ile Asn Thr
               20                  25                  30
   Asp
<210> 53
   <211> 41
   <212> PRT
   <213> Plasmodium falciparum
<400> 53
   Glu Ile Glu Asp Lys Pro Glu Gln Glu Ile Lys Asn Lys Ser Leu Glu
   1               5                   10                  15
   Glu Lys Gln Ile Asp Lys Asn Thr Asp Thr Ser Glu Lys Lys Gly Phe
               20                  25                  30
   Asn Asn Ser Glu Lys Asp Glu Lys Ala
           35                  40
<210> 54
   <211> 61
   <212> PRT
   <213> Plasmodium falciparum
<400> 54
   Asn Asn Ser Glu Lys Asp Glu Lys Ala Arg Asn Leu Ile Ser Lys Asn
   1               5                   10                  15
   Tyr Lys Asn Tyr Asn Glu Leu Asp Lys Asn Val His Thr Leu Val Asn
               20                  25                  30
   Ser Ile Ile Ser Leu Leu Glu Glu Gly Asn Gly Ser Asp Ser Thr Leu
           35                  40                  45
   Asn Ser Leu Ser Lys Asp Ile Thr Asn Leu Phe Lys Asn
       50                  55                  60
<210> 55
   <211> 272
   <212> PRT
   <213> Plasmodium falciparum
<400> 55
   Met Trp Ile Val Lys Phe Leu Ile Val Val His Phe Phe Ile Ile Cys
   1               5                   10                  15
   Thr Ile Asn Phe Asp Lys Leu Tyr Ile Ser Tyr Ser Tyr Asn Ile Val
               20                  25                  30
   Pro Glu Asn Gly Arg Met Leu Asn Met Arg Ile Leu Gly Glu Glu Lys
           35                  40                  45
   Pro Asn Val Asp Gly Val Ser Thr Ser Asn Thr Pro Gly Gly Asn Glu
       50                  55                  60
   Ser Ser Ser Ala Ser Pro Asn Leu Ser Asp Ala Ala Glu Lys Lys Asp
   65                  70                  75                  80
   Glu Lys Glu Ala Ser Glu Gln Gly Glu Glu Ser His Lys Lys Glu Asn
                   85                  90                  95
   Ser Gln Glu Ser Ala Asn Gly Lys Asp Asp Val Lys Glu Glu Lys Lys
               100                 105                 110
   Thr Asn Glu Lys Lys Asp Asp Gly Lys Thr Asp Lys Val Gln Glu Lys
   115 120 125
   Val Leu Glu Lys Ser Pro Lys Glu Ser Gln Met Val Asp Asp Lys Lys
   130 135 140
   Lys Thr Glu Ala Ile Pro Lys Lys Val Val Gln Pro Ser Ser Ser Asn
   145 150 155 160
   Ser Gly Gly His Val Gly Glu Glu Glu Asp His Asn Glu Gly Glu Gly
   165 170 175
   Glu His Glu Glu Glu Glu Glu His Glu Glu Asp Asp Asp Asp Glu Asp
   180 185 190
   Asp Asp Thr Tyr Asn Lys Asp Asp Leu Glu Asp Glu Asp Leu Cys Lys
   195 200 205
   His Asn Asn Gly Gly Cys Gly Asp Asp Lys Leu Cys Glu Tyr Val Gly
   210 215 220
   Asn Arg Arg Val Lys Cys Lys Cys Lys Glu Gly Tyr Lys Leu Glu Gly
   225 230 235 240
   Ile Glu Cys Val Glu Leu Leu Ser Leu Ala Ser Ser Ser Leu Asn Leu
   245 250 255
   Ile Phe Asn Ser Phe Ile Thr Ile Phe Val Val Ile Leu Leu Ile Asn
   260 265 270
<210> 56
   <211> 9563
   <212> PRT
   <213> Plasmodium falciparum
<400> 56
   Met Lys Lys Glu Ser Tyr Arg Phe Leu Asn Ile Ser Ile Asn Asn Gly
   1               5                   10                  15
   Ser Asp Lys Lys Lys Lys Ser Asn Phe Phe Val Lys Ile Lys Asn Gly
               20                  25                  30
   Thr Asp Leu Lys Asp Thr Lys Lys Asp Arg Ile Arg Phe His Phe Tyr
           35                  40                  45
   Phe Tyr Phe Phe Ile Val Lys Ile Ile Leu Leu Ser Leu Leu Ile Trp
       50                  55                  60
   Thr Val Ile Tyr Ser Asn Asn Cys Ser Asn Gly Lys Tyr Lys Ser Thr
   65                  70                  75                  80
   Leu Tyr Ile Val Gly Asn His Leu Arg Phe Arg Gly Phe Arg Ile Leu
                   85                  90                  95
   Ala Glu Asn Glu Tyr Glu Met Lys Thr Lys Tyr Lys Thr Leu Met Val
               100                 105                 110
   Asn Ile Asp Asp Asn Asp Leu Gly Lys Arg Gly Asn Lys Ile Lys Pro
           115                 120                 125
   Lys His Lys Lys Val Glu Ile Asn Leu Asn Asp Asn Asn Leu Ser Asn
       130                 135                 140
   Asn Glu Ser Lys Arg Lys Ser Arg Arg Lys Lys Val Lys Val Asn Leu
   145                 150                 155                 160
   Lys Asp Asn Leu Ala Lys Arg Glu Gly Lys Met Lys Arg Arg Asn Lys
                   165                 170                 175
   Lys Glu Lys Arg Arg Lys Glu Lys Asp Asn Lys Lys Met Asn Glu Leu
               180                 185                 190
   Lys Leu Lys Arg Glu Tyr Lys Glu Ser Tyr Leu Leu Asp Asn Lys Ser
           195                 200                 205
   Asp Met Asp Val Ile Arg Asn Asp Lys Phe Ser Tyr Phe Thr Leu Tyr
       210                 215                 220
   Lys Asn Asn Leu Phe Pro Asn Ser Asn Asn Tyr Phe Leu His Met Leu
   225                 230                 235                 240
   Lys Thr Tyr Asp Glu Glu Asn Lys Gly Ile Asp Glu Asn Thr Asp Ile
                   245                 250                 255
   Asn Glu Asn Val Glu Glu Asn Glu Tyr Val Asp Lys Asn Thr Glu Gly
               260                 265                 270
   Asp Lys Asp Gly Asp Glu Asp Lys Gly Gly Gly Glu Ser Glu Gly Gly
           275                 280                 285
   Asp Gly His Gly Ser Ala Gly Asp Ser Ala Gly Gly Asp Arg Gly Gly
       290                 295                 300
   Asp Glu Asn Lys Asp Lys Asp Asn Glu Glu Asn Lys Asp Lys Lys Gly
   305                 310                 315                 320
   Asn Lys Asp Asp Asp Asp Glu Glu Glu Glu Glu Glu Glu Glu Asp Asp
   325 330 335
   Glu Glu Lys Glu Glu Lys Asp Glu Lys Asp Glu Lys Asp Glu Lys Asp
   340 345 350
   Glu Lys Asp Glu Lys Asp Glu Lys Asp Glu Lys Asp Glu Lys Asp Glu
   355 360 365
   Lys Asp Glu Lys Asp Glu Lys Asp Glu Lys Asp Glu Lys Gly Glu Lys
   370 375 380
   Asp Glu Ser Lys Gly Glu Glu Lys Phe Leu Lys Glu Glu Lys Met Pro
   385 390 395 400
   Lys Lys Glu Glu Asn Met Gln Asp Lys Ile Glu Arg Asp Lys Glu Phe
   405 410 415
   Glu Gly Phe Lys Asp Glu Tyr Phe Thr Gln Met Glu Gly Gly Ala Tyr
   420 425 430
   Gly Ile Thr Val Glu His Glu Gly Gln Lys Leu Arg Glu Thr Leu Thr
   435 440 445
   Glu Gly Val Glu Gly Tyr Glu Lys Val Ile Glu Glu Met Glu Gln Thr
   450 455 460
   Lys Leu Asp Glu Phe Leu Leu Pro Thr Asn Glu Gly Lys Ser Asp Asp
   465 470 475 480
   Phe Val Ser Glu Met Glu Glu Glu Arg Leu Phe Asn Phe Val Ser Tyr
   485 490 495
   Met Glu Glu Glu Val Lys Asp Lys Met Phe Ile Glu Arg Glu Gln Gly
   500 505 510
   Thr Glu Glu Glu Val Ile His Glu Asp Leu Ser Asp Glu Lys Ile Gly
   515 520 525
   Ile Glu Lys Lys Glu Leu Glu Thr Phe Val Gln Asp Val Ser Asp Glu
   530 535 540
   Val Leu Gln Asp Glu Gln Leu Ile Glu Glu Glu Lys Leu Thr Glu Glu
   545 550 555 560
   Glu Lys Leu Thr Glu Glu Glu Lys Leu Thr Glu Glu Glu Lys Leu Thr
   565 570 575
   Glu Asp Glu Gln Leu Ile Glu Glu Glu Lys Leu Thr Glu Glu Glu Lys
               580                 585                 590
   Leu Thr Glu Glu Glu Lys Leu Thr Glu Glu Glu Lys Leu Thr Glu Glu
           595                 600                 605
   Glu Lys Leu Thr Glu Glu Glu Lys Leu Thr Glu Glu Glu Lys Leu Thr
       610                 615                 620
   Glu Glu Glu Lys Leu Thr Glu Glu Glu Lys Leu Thr Glu Glu Glu Ile
   625                 630                 635                 640
   Phe Ile Glu Glu Asp Lys Ile Ser Pro Lys Glu Val Glu Glu Pro Leu
                   645                 650                 655
   Glu Asp Val Leu Met Glu Lys Met Glu Lys Glu Tyr Lys Glu Ile Leu
               660                 665                 670
   His Glu Gly Lys Ser Val Val Thr Gln Asp Met Asp Glu Lys Leu Leu
           675                 680                 685
   Ser Glu Asn Leu Glu Asp Asp Leu Ile Pro Glu Glu Ile Ile Glu Glu
       690                 695                 700
   Leu Val Asp Glu Glu Leu Val Glu Glu Val Thr Ala Glu Glu Ile Val
   705                 710                 715                 720
   Glu Glu Phe Val Ala Glu Val Glu Ile Glu Glu Lys Ile Pro Lys Glu
                   725                 730                 735
   Leu Val Glu Glu Val Met Pro Glu Glu Asp Val Asp Glu Val Val Pro
               740                 745                 750
   Glu Glu Leu Val Glu Glu Val Val Pro Glu Glu Leu Ile Glu Gly Val
           755                 760                 765
   Ile Pro Glu Glu Leu Val Glu Asp Val Ile Pro Glu Lys Val Leu Glu
       770                 775                 780
   Lys Val Ile Pro Glu Glu Ile Ile Glu Glu Leu Val Leu Glu Glu Val
   785                 790                 795                 800
   Val Glu Glu Ile Lys His Glu Glu Leu Val Glu Glu Glu Ile Pro Glu
                   805                 810                 815
   Glu Val Thr Glu Glu Val Ile Pro Asp Glu Val Val Glu Glu Val Ile
               820                 825                 830
   Pro Glu Glu Leu Ile Glu Lys Val Val Lys Glu Glu Leu Val Glu Lys
           835                 840                 845
   Val Val Ala Glu Glu Ile Val Glu Glu Val Val Pro Glu Glu Leu Val
       850                 855                 860
   Glu Lys Val Val Ala Glu Glu Ile Val Glu Glu Val Ile Pro Glu Glu
   865                 870                 875                 880
   Leu Ile Glu Glu Val Ile Thr Glu Lys Pro Ala Glu Glu Val Ile Pro
                   885                 890                 895
   Glu Glu Leu Val Glu Glu Val Ile Pro Lys Glu Ile Val Glu Glu Val
               900                 905                 910
   Leu Pro Glu Glu Val Val Lys Glu Val Ile Pro Glu Glu Val Val Glu
           915                 920                 925
   Glu Val Ile Pro Glu Glu Leu Val Glu Glu Val Ile Pro Glu Glu Ile
       930                 935                 940
   Val Glu Glu Val Val Pro Glu Glu Leu Val Glu Glu Met Lys Pro Glu
   945                 950                 955                 960
   Glu Val Val Glu Glu Val Ile Ser Glu Glu Leu Val Glu Glu Met Lys
                   965                 970                 975
   Pro Glu Glu Val Val Glu Glu Met Lys Pro Glu Glu Val Val Glu Glu
               980                 985                 990
   Val Thr Pro Glu Lys Val Val Glu  Glu Val Val Pro Glu  Glu Leu Val
           995                 1000                 1005
   Glu Glu  Met Lys Pro Glu Glu  Val Val Glu Glu Val  Ile Ser Glu
       1010                 1015                 1020
   Glu Leu  Val Glu Glu Met Lys  Pro Glu Glu Val Val  Glu Glu Val
       1025                 1030                 1035
   Thr Pro  Glu Lys Val Val Glu  Glu Val Val Pro Glu  Glu Leu Val
       1040                 1045                 1050
   Glu Glu  Met Lys Pro Glu Glu  Val Val Glu Glu Val  Ile Ser Glu
       1055                 1060                 1065
   Glu Leu  Val Glu Glu Met Lys  Pro Glu Glu Val Val  Glu Glu Val
   1070 1075 1080
   Ile Ser Glu Glu Leu Val Glu Glu Met Lys Pro Glu Glu Val Val
   1085 1090 1095
   Glu Glu Val Thr Pro Glu Lys Val Val Glu Glu Val Val Pro Glu
   1100 1105 1110
   Glu Leu Val Glu Glu Met Lys Pro Glu Glu Val Val Glu Glu Val
   1115 1120 1125
   Ile Ser Glu Glu Leu Val Glu Glu Met Lys Pro Glu Glu Val Val
   1130 1135 1140
   Glu Glu Val Thr Pro Glu Lys Val Val Glu Glu Val Val Pro Glu
   1145 1150 1155
   Glu Leu Val Glu Glu Met Lys Pro Glu Glu Val Val Glu Glu Val
   1160 1165 1170
   Ile Ser Glu Glu Leu Val Glu Glu Met Lys Pro Glu Glu Val Val
   1175 1180 1185
   Glu Glu Val Val Pro Glu Glu Leu Val Glu Glu Val Ile Pro Glu
   1190 1195 1200
   Glu Val Val Glu Glu Ile Ile Pro Glu Glu Val Val His Glu Ala
   1205 1210 1215
   Lys His Glu Glu Leu Phe Glu Lys Thr Val Pro Glu Val Glu Val
   1220 1225 1230
   Gln Glu Ile Ile Pro Lys Glu Leu Val Glu Val Ile Pro Glu Glu
   1235 1240 1245
   Val Leu Pro Glu Glu Leu Ile Glu Glu Val Ile Pro Glu Glu Ile
   1250 1255 1260
   Ile Glu Glu Val Ile Pro Glu Glu Val Val Glu Glu Val Ile Pro
   1265 1270 1275
   Glu Glu Ile Val Gln Glu Val Ile Pro Glu Asp Leu Met Glu Glu
   1280 1285 1290
   Val Val Pro Glu Glu Ile Ile Glu Glu Lys Ile Leu Glu Glu Ile
   1295 1300 1305
   Val Glu Glu Glu Ile His Glu Glu Ile Val Glu Glu Ile Ile Ser
   1310 1315 1320
   Glu Glu Val Val Glu Glu Val Val Pro Ala Glu Val Val Glu Glu
   1325 1330 1335
   Met Ile Pro Glu Lys Leu Val Glu Glu Val Ile Pro Glu Glu Leu
   1340 1345 1350
   Val Glu Glu Val Ile Ser Glu Glu Leu Val Glu Glu Val Val Pro
   1355 1360 1365
   Val Glu Val Leu Glu Glu Val Val Pro Val Glu Val Leu Glu Glu
   1370 1375 1380
   Val Ile Pro Glu Glu Val Val Glu Glu Val Ile Pro Glu Glu Phe
   1385 1390 1395
   Ile Glu Glu Met Ile Pro Glu Glu Ile Val Glu Glu Val Val Pro
   1400 1405 1410
   Glu Val Val Glu Glu Val Ile Pro Glu Glu Leu Val Glu Glu Val
   1415 1420 1425
   Val Pro Val Glu Leu Leu Glu Glu Val Ile Pro Glu Glu Leu Leu
   1430 1435 1440
   Glu Glu Val Ile Pro Glu Glu Leu Val Glu Glu Val Val Pro Glu
   1445 1450 1455
   Glu Leu Val Glu Asp Val Ile Pro Glu Glu Leu Ile Ala Glu Val
   1460 1465 1470
   Val Ser Glu Glu Leu Val Glu Glu Glu Ile Pro Glu Glu Val Leu
   1475 1480 1485
   Glu Glu Val Ile Pro Glu Glu Leu Val Glu Glu Val Ile Pro Glu
   1490 1495 1500
   Glu Leu Val Glu Glu Val Ile Pro Glu Val Leu Val Glu Glu Val
   1505 1510 1515
   Ile Pro Glu Glu Leu Val Glu Glu Val Ile Pro Glu Val Leu Val
   1520 1525 1530
   Glu Glu Glu Val Pro Glu Glu Leu Val Glu Glu Val Ile Pro Glu
   1535 1540 1545
   Leu Leu Val Ala Glu Val Ile Pro Glu Glu Leu Val Ala Glu Val
   1550 1555 1560
   Ile Pro Glu Glu Phe Val Glu Glu Val Ile Pro Glu Glu Val Val
   1565 1570 1575
   Glu Glu Leu Val Pro Glu Glu Leu Val Glu Glu Val Ile Pro Glu
   1580 1585 1590
   Glu Ile Val Glu Glu Val Val Glu Glu Val Val Glu Glu Ile Ile
   1595 1600 1605
   Pro Glu Glu Val Ile Glu Glu Val Ile Pro Glu Glu Ile Val Lys
   1610 1615 1620
   Glu Val Val Pro Glu Val Leu Val Glu Glu Val Val Pro Glu Glu
   1625 1630 1635
   Ile Val Glu Glu Val Val Pro Glu Glu Ile Val Glu Glu Val Val
   1640 1645 1650
   Pro Glu Glu Leu Val Glu Glu Val Val Pro Glu Glu Leu Ile Glu
   1655 1660 1665
   Glu Val Ile Pro Glu Glu Leu Val Glu Glu Val Ile Pro Glu Val
   1670 1675 1680
   Leu Val Glu Glu Val Ile Pro Glu Val Leu Val Glu Glu Met Val
   1685 1690 1695
   Leu Glu Glu Leu Val Glu Glu Val Ile Pro Glu Val Leu Val Glu
   1700 1705 1710
   Glu Met Val Pro Glu Glu Val Val Glu Lys Met Val Leu Glu Glu
   1715 1720 1725
   Val Val Glu Glu Val Ile Pro Glu Glu Val Val Glu Glu Val Ile
   1730 1735 1740
   Pro Glu Glu Asp Val Glu Gln Val Ile Pro Glu Glu Leu Ile Glu
   1745 1750 1755
   Glu Val Val Pro Glu Glu Val Ile Glu Glu Val Ile Thr Glu Glu
   1760 1765 1770
   Val Val Glu Glu Val Leu Pro Glu Glu Leu Val Glu Lys Val Val
   1775 1780 1785
   Glu Glu  Glu Ile Leu Glu Glu  Val Ile Pro Glu Glu  Leu Val Glu
       1790                 1795                 1800
   Glu Val  Val Arg Glu Glu Leu  Ile Glu Glu Val Val  Thr Glu Glu
       1805                 1810                 1815
   Leu Val  Glu Lys Val Val Glu  Glu Glu Ile Val Glu  Glu Val Val
       1820                 1825                 1830
   Leu Ala  Glu Val Val Glu Glu  Val Ile Pro Gln Glu  Ile Val Glu
       1835                 1840                 1845
   Glu Val  Ile Pro Glu Glu Val  Val Glu Glu Val Ile  Pro Lys Glu
       1850                 1855                 1860
   Ile Val  Glu Glu Val Ile Pro  Glu Glu Leu Val Glu  Glu Val Val
       1865                 1870                 1875
   Pro Glu  Glu Leu Val Ala Glu  Val Ile Pro Glu Glu  Leu Val Ala
       1880                 1885                 1890
   Glu Val  Ile Pro Glu Glu Leu  Ile Glu Glu Val Val  Thr Glu Glu
       1895                 1900                 1905
   Leu Val  Glu Glu Val Ile Pro  Glu Glu Val Val Glu  Glu Val Ile
       1910                 1915                 1920
   Pro Glu  Glu Val Val Glu Glu  Val Val Pro Glu Glu  Leu Val Glu
       1925                 1930                 1935
   Glu Val  Val Pro Glu Glu Leu  Val Glu Glu Val Val  Pro Glu Glu
       1940                 1945                 1950
   Ile Val  Glu Glu Leu Leu Pro  Lys Glu Val Val Glu  Glu Val Ile
       1955                 1960                 1965
   Tyr Glu  Glu Gln Val Glu Glu  Val Ile Pro Glu Glu  Leu Val Ala
       1970                 1975                 1980
   Glu Val  Ile Pro Glu Lys Leu  Val Glu Glu Val Ile  Pro Glu Glu
       1985                 1990                 1995
   Leu Val  Glu Glu Val Ile Pro  Glu Glu Leu Val Glu  Glu Val Val
       2000                 2005                 2010
   Pro Glu  Val Val Glu Glu Val  Val Pro Glu Glu Leu  Val Glu Glu
   2015 2020 2025
   Val Ile Pro Glu Glu Leu Val Glu Glu Val Ile Pro Glu Val Leu
   2030 2035 2040
   Val Glu Glu Glu Val Pro Glu Glu Leu Val Glu Glu Val Ile Pro
   2045 2050 2055
   Glu Glu Leu Val Glu Glu Met Leu Pro Glu Glu Leu Ile Glu Val
   2060 2065 2070
   Val Val Pro Glu Glu Leu Val Glu Glu Val Val Pro Glu Glu Leu
   2075 2080 2085
   Val Glu Glu Val Val Pro Glu Glu Val Val Glu Glu Val Ile Pro
   2090 2095 2100
   Glu Glu Leu Ile Glu Glu Leu Val Pro Glu Val Glu Ile Glu Glu
   2105 2110 2115
   Ile Leu Pro Glu Glu Leu Ile Glu Glu Leu Val Pro Glu Glu Ile
   2120 2125 2130
   Ile Glu Glu Val Ile Pro Lys Glu Leu Ile Glu Glu Val Ile Pro
   2135 2140 2145
   Glu Val Val Val Glu Glu Val Val Pro Glu Val Val Glu Glu Val
   2150 2155 2160
   Val Pro Glu Ile Val Glu Glu Val Val Pro Glu Val Val Glu Glu
   2165 2170 2175
   Val Val Pro Glu Glu Ile Ile Glu Glu Lys Ile Ser Glu Glu Val
   2180 2185 2190
   Val Glu Glu Val Leu Pro Lys Glu Ile Val Glu Glu Val Ile Pro
   2195 2200 2205
   Glu Glu Leu Val Glu Glu Val Val Pro Glu Glu Leu Val Ala Glu
   2210 2215 2220
   Val Ile Pro Glu Glu Leu Val Glu Glu Val Ile Pro Glu Val Leu
   2225 2230 2235
   Phe Glu Glu Glu Ile Pro Glu Val Val Val Glu Glu Val Val Pro
   2240 2245 2250
   Glu Val Val Glu Glu Glu Ile Pro Glu Val Val Val Glu Glu Val
   2255 2260 2265
   Val Pro Glu Val Val Glu Glu Val Ile Pro Glu Glu Leu Val Glu
   2270 2275 2280
   Glu Val Ile Pro Glu Glu Leu Ile Glu Glu Val Ile Pro Glu Glu
   2285 2290 2295
   Val Ala Glu Glu Val Leu Pro Glu Glu Leu Ile Glu Glu Val Ile
   2300 2305 2310
   Pro Glu Glu Leu Val Glu Glu Val Val Pro Glu Glu Leu Val Ala
   2315 2320 2325
   Glu Val Ile Pro Glu Glu Ile Val Glu Glu Val Val Arg Glu Glu
   2330 2335 2340
   Leu Val Glu Glu Val Val Pro Glu Glu Val Val Glu Glu Val Ile
   2345 2350 2355
   Pro Lys Glu Ile Val Glu Glu Val Val Ser Glu Glu Val Val Glu
   2360 2365 2370
   Glu Val Ile Pro Lys Glu Ile Val Glu Glu Glu Val Pro Glu Glu
   2375 2380 2385
   Leu Val Glu Val Val Pro Glu Glu Leu Val Glu Glu Val Val Pro
   2390 2395 2400
   Glu Glu Leu Val Glu Glu Val Val Pro Glu Glu Leu Val Glu Glu
   2405 2410 2415
   Val Val Pro Glu Glu Leu Val Glu Glu Val Val Pro Glu Glu Leu
   2420 2425 2430
   Val Glu Glu Val Val Pro Glu Val Val Glu Glu Val Gln Glu Gln
   2435 2440 2445
   Leu Val Glu Glu Phe Ile Pro Glu Glu Val Val Glu Glu Leu Val
   2450 2455 2460
   Pro Glu Glu Leu Val Glu Glu Val Ile Pro Glu Glu Leu Val Glu
   2465 2470 2475
   Glu Val Ile Pro Glu Glu Ile Val Glu Glu Val Leu Pro Glu Glu
   2480 2485 2490
   Val Val Lys Glu Val Ile Pro Glu Glu Val Val Glu Glu Val Ile
       2495                2500                2505
   Pro Glu Glu Leu Val Glu Glu Val Ile Pro Glu Glu Ile Val Glu
       2510                2515                2520
   Glu Val Leu Pro Glu Glu Val Val Lys Glu Val Ile Pro Glu Glu
       2525                2530                2535
   Val Val Glu Glu Val Ile Pro Glu Glu Leu Val Glu Glu Val Ile
       2540                2545                2550
   Pro Glu Glu Leu Val Glu Glu Val Ile Pro Glu Glu Leu Val Glu
       2555                2560                2565
   Glu Val Val Ser Glu Glu Leu Val Glu Glu Val Ile Pro Glu Glu
       2570                2575                2580
   Ile Val Glu Glu Val Ile Pro Glu Glu Leu Leu Glu Glu Val Val
       2585                2590                2595
   Glu Glu Val Val Pro Glu Ile Val Glu Glu Val Val Pro Glu Val
       2600                2605                2610
   Val Glu Glu Val Ile Thr Glu Glu Val Ile Pro Glu Glu Leu Ile
       2615                2620                2625
   Lys Glu Val Ile Pro Glu Glu Leu Val Glu Glu Val Ile Pro Glu
       2630                2635                2640
   Glu Ile Val Glu Glu Val Ile Pro Glu Glu Ile Val Glu Glu Val
       2645                2650                2655
   Ile Ser Glu Glu Val Val Glu Glu Val Val Pro Glu Val Val Val
       2660                2665                2670
   Glu Glu Val Ile Pro Glu Glu Ile Val Glu Glu Val Leu Pro Lys
       2675                2680                2685
   Glu Ile Val Glu Glu Val Ile Ser Glu Lys Val Val Glu Glu Met
       2690                2695                2700
   Ile Pro Glu Glu Leu Val Glu Glu Val Ile Pro Glu Glu Val Val
       2705                2710                2715
   Glu Glu Val Ile Pro Glu Val Glu Ile Glu Glu Ile Leu Pro Glu
       2720                2725                2730
   Glu Leu Ile Glu Glu Leu Val Ala Glu Glu Val Ile Glu Glu Val
   2735 2740 2745
   Ile Pro Lys Glu Leu Ile Glu Glu Val Ile Pro Glu Lys Leu Ile
   2750 2755 2760
   Glu Asp Val Val Pro Glu Glu Ile Val Glu Glu Val Leu Pro Glu
   2765 2770 2775
   Glu Val Val Lys Glu Val Ile Pro Glu Glu Val Val Glu Glu Val
   2780 2785 2790
   Leu Pro Glu Glu Leu Val Glu Glu Val Ile Pro Glu Val Val Glu
   2795 2800 2805
   Glu Val Val Pro Glu Glu Leu Val Glu Glu Val Leu Pro Lys Glu
   2810 2815 2820
   Val Val Glu Glu Val Ile Pro Glu Val Glu Ile Glu Glu Ile Leu
   2825 2830 2835
   Pro Glu Glu Ile Ile Glu Glu Leu Val Ala Lys Glu Val Ile Glu
   2840 2845 2850
   Glu Val Ile Pro Lys Glu Leu Ile Glu Glu Ala Ile Thr Glu Glu
   2855 2860 2865
   Val Val Glu Glu Met Ile Pro Glu Glu Ile Ile Glu Glu Val Val
   2870 2875 2880
   Pro Glu Glu Val Ile Glu Glu Val Ile Thr Glu Glu Val Val Glu
   2885 2890 2895
   Glu Val Val Pro Glu Glu Val Ile Glu Glu Val Ile Thr Glu Glu
   2900 2905 2910
   Val Val Glu Glu Ala Ile Pro Glu Glu Val Val Glu Glu Val Ile
   2915 2920 2925
   Pro Glu Val Glu Ile Glu Glu Val Leu Pro Glu Glu Leu Ile Glu
   2930 2935 2940
   Glu Leu Val Pro Glu Glu Val Thr Glu Glu Val Ile Thr Glu Glu
   2945 2950 2955
   Val Val Glu Glu Ala Ile Ser Glu Val Val Glu Glu Val Ile Pro
   2960 2965 2970
   Glu Glu Ile Val Lys Glu Val Ile Pro Glu Glu Val Val Glu Glu
   2975 2980 2985
   Ile Ile Thr Glu Glu Ile Ala Glu Glu Ile Ile Pro Lys Glu Leu
   2990 2995 3000
   Val Glu Glu Val Ile Pro Glu Glu Val Val Val Glu Ile Ile Pro
   3005 3010 3015
   Glu Lys Leu Val Glu Glu Phe Val Ser Glu Glu Leu Val Glu Glu
   3020 3025 3030
   Leu Val Pro Glu Glu Ile Val Glu Lys Val Val Pro Glu Glu Leu
   3035 3040 3045
   Val Glu Glu Ile Val Pro Glu Glu Leu Val Glu Glu Val Val Pro
   3050 3055 3060
   Glu Glu Leu Val Glu Glu Val Ile Pro Glu Glu Leu Val Glu Glu
   3065 3070 3075
   Phe Ile Pro Glu Glu Val Val Glu Glu Val Leu Pro Glu Gln Leu
   3080 3085 3090
   Val Glu Glu Val Ile Ser Glu Val Val Glu Glu Val Val Pro Glu
   3095 3100 3105
   Glu Leu Val Glu Glu Val Ile Pro Glu Glu Val Val Glu Glu Leu
   3110 3115 3120
   Val Ala Glu Glu Val Ile Glu Glu Val Ile Pro Lys Glu Leu Ile
   3125 3130 3135
   Glu Glu Ala Ile Thr Glu Glu Val Glu Glu Val Ile Pro Glu Glu
   3140 3145 3150
   Ile Val Glu Glu Val Ile Pro Glu Glu Val Val Lys Glu Val Ile
   3155 3160 3165
   Pro Glu Val Glu Ile Glu Glu Ile Leu Pro Glu Glu Leu Ile Glu
   3170 3175 3180
   Glu Leu Val Ala Lys Glu Val Ile Glu Glu Val Ile Thr Glu Glu
   3185 3190 3195
   Val Ala Glu Glu Val Ile Pro Glu Glu Ile Val Gln Glu Val Ile
   3200 3205 3210
   Pro Glu Glu Val Val Glu Glu Ile Ile Thr Glu Val Glu Ile Glu
   3215 3220 3225
   Glu Val Leu Pro Glu Glu Leu Ile Asp Glu Val Val Pro Glu Glu
   3230 3235 3240
   Val Thr Glu Glu Val Ile Thr Glu Glu Val Met Pro Glu Glu Val
   3245 3250 3255
   Val Lys Glu Val Ile Pro Glu Glu Ile Ala Glu Glu Val Ile Pro
   3260 3265 3270
   Lys Glu Leu Val Glu Glu Val Ile Pro Glu Glu Val Val Glu Glu
   3275 3280 3285
   Ile Ile Pro Glu Val Glu Ile Glu Glu Val Leu Pro Glu Glu Val
   3290 3295 3300
   Ile Glu Glu Val Val Pro Glu Val Glu Ile Glu Glu Leu Ile Pro
   3305 3310 3315
   Asp Glu Val Val Glu Glu Val Leu Pro Glu Glu Leu Val Glu Glu
   3320 3325 3330
   Val Ile Pro Glu Glu Val Ala Val Glu Val Val Pro Glu Glu Val
   3335 3340 3345
   Ile Glu Glu Ala Val Pro Glu Glu Ile Val Glu Glu Phe Ile Pro
   3350 3355 3360
   Glu Glu Gln Ile Glu Glu Val Ile Gln Glu Glu Ile Ile Glu Gln
   3365 3370 3375
   Val Val Pro Glu Glu Leu Ile Glu Glu Val Val Pro Glu Glu Ile
   3380 3385 3390
   Ile Glu Glu Val Ile Pro Glu Glu Ile Val Glu Glu Val Ile Tyr
   3395 3400 3405
   Glu Glu Val Ile Pro Glu Glu Leu Val Glu Glu Val Ile Ala Glu
   3410 3415 3420
   Lys Leu Val Lys Glu Ile Val Pro Glu Gln Val Arg Glu Glu Val
   3425 3430 3435
   Thr Leu Glu Glu Ile Val Glu Glu Met Ile Pro Glu Glu Phe Val
   3440 3445 3450
   Glu Glu Val Ala Pro Glu Val Glu Ile Glu Glu Ile Ile Pro Glu
   3455 3460 3465
   Glu Leu Ile Glu Glu Val Ile Pro Glu Val Leu Val Glu Glu Ala
   3470 3475 3480
   Val Pro Glu Glu Leu Ile Glu Lys Val Ile Pro Glu Val Leu Val
   3485 3490 3495
   Glu Glu Ile Ile Tyr Lys Glu Gly Ile Pro Glu Val Leu Ile Glu
   3500 3505 3510
   Glu Val Ile Pro Glu Glu Leu Val Gln Glu Val Ile Pro Glu Glu
   3515 3520 3525
   Ile Val Glu Glu Val Val Pro Glu Glu Phe Ile Glu Glu Met Ile
   3530 3535 3540
   Pro Gln Glu Val Val Lys Glu Val Ile Thr Glu Lys Phe Ile Glu
   3545 3550 3555
   Glu Met Ile Pro Glu Glu Val Val Glu Glu Val Ile Pro Glu Glu
   3560 3565 3570
   Phe Ile Glu Glu Met Ile Pro Glu Val Val Val Glu Glu Val Val
   3575 3580 3585
   Pro Glu Glu Leu Val Glu Glu Met Leu Pro Glu Glu Leu Ile Glu
   3590 3595 3600
   Val Val Val Pro Glu Glu Leu Ile Glu Glu Val Ile Pro Glu Glu
   3605 3610 3615
   Leu Val Glu Glu Val Val Ala Glu Glu Leu Val Glu Glu Val Ile
   3620 3625 3630
   Pro Glu Glu Leu Val Glu Lys Val Val Pro Glu Val Val Glu Glu
   3635 3640 3645
   Phe Ile His Glu Val Val Glu Glu Val Val Pro Glu Val Val Glu
   3650 3655 3660
   Glu Val Val Pro Glu Val Val Glu Glu Val Val Pro Glu Val Val
   3665 3670 3675
   Glu Glu Val Val Pro Glu Val Val Glu Glu Val Val Pro Glu Val
   3680 3685 3690
   Val Glu Glu Val Val Pro Glu Val Val Glu Glu Val Val Pro Glu
   3695 3700 3705
   Val Val Glu Glu Val Val Pro Glu Val Val Glu Glu Val Val Pro
   3710 3715 3720
   Glu Val Val Glu Glu Val Val Pro Glu Glu Leu Val Glu Glu Val
   3725 3730 3735
   Ile Pro Glu Val Leu Phe Glu Glu Glu Ile Pro Glu Glu Leu Val
   3740 3745 3750
   Glu Val Val Pro Val Glu Leu Leu Glu Glu Ala Ile Pro Glu Glu
   3755 3760 3765
   Leu Val Glu Glu Val Ile Pro Glu Glu Leu Val Glu Glu Val Ile
   3770 3775 3780
   Pro Glu Glu Leu Val Glu Glu Val Ile Pro Glu Glu Leu Val Glu
   3785 3790 3795
   Glu Val Ile Leu Glu Glu Leu Val Glu Glu Val Ile Pro Glu Glu
   3800 3805 3810
   Leu Val Glu Glu Val Ile Pro Glu Glu Leu Val Glu Glu Val Ile
   3815 3820 3825
   Pro Glu Val Leu Val Glu Glu Glu Ile Pro Glu Lys Leu Val Glu
   3830 3835 3840
   Glu Asp Ile Pro Glu Lys Leu Val Glu Glu Val Val Pro Glu Glu
   3845 3850 3855
   Leu Val Glu Val Val Ile Pro Glu Glu Leu Val Glu Glu Val Ile
   3860 3865 3870
   Pro Glu Glu Leu Val Lys Glu Val Ile Pro Glu Glu Leu Val Lys
   3875 3880 3885
   Glu Val Val Pro Glu Glu Leu Val Glu Glu Glu Ile Pro Lys Val
   3890 3895 3900
   Val Glu Glu Val Leu Pro Glu Glu Leu Val Glu Glu Val Met Leu
   3905 3910 3915
   Glu Glu Val Val Glu Glu Val Val Pro Glu Ile Val Glu Glu Val
   3920 3925 3930
   Val Pro Glu Glu Leu Val Glu Glu Val Val Pro Glu Val Val Glu
   3935 3940 3945
   Glu Val Val Pro Glu Glu Ile Val Glu Glu Val Ile Pro Glu Glu
   3950 3955 3960
   Leu Val Glu Glu Val Val Lys Glu Glu Leu Val Glu Glu Val Val
   3965 3970 3975
   Lys Glu Glu Leu Val Glu Glu Val Val Lys Glu Glu Leu Val Glu
   3980 3985 3990
   Glu Val Ile Pro Glu Val Leu Val Glu Glu Val Val Pro Glu Glu
   3995 4000 4005
   Leu Leu Glu Glu Val Ile Pro Lys Val Leu Val Glu Glu Val Ile
   4010 4015 4020
   Pro Glu Leu Leu Val Glu Glu Val Val Pro Glu Val Ile Glu Glu
   4025 4030 4035
   Val Val Pro Glu Val Val Glu Glu Val Val Pro Glu Glu Leu Leu
   4040 4045 4050
   Glu Glu Val Ile Pro Glu Val Leu Val Glu Glu Glu Ile Pro Glu
   4055 4060 4065
   Lys Leu Val Glu Glu Val Val Pro Glu Glu Leu Val Glu Glu Val
   4070 4075 4080
   Ile Pro Glu Val Leu Val Glu Glu Val Val Pro Glu Glu Leu Val
   4085 4090 4095
   Glu Glu Val Ile Ser Glu Glu Leu Val Glu Glu Val Ile Pro Glu
   4100 4105 4110
   Glu Leu Val Glu Glu Val Ile Pro Glu Glu Val Val Glu Glu Val
   4115 4120 4125
   Val Pro Glu Val Val Glu Glu Val Val Pro Glu Val Val Glu Glu
   4130 4135 4140
   Val Val Pro Glu Val Val Glu Glu Val Val Pro Glu Val Val Glu
   4145 4150 4155
   Glu Val Val Pro Glu Val Val Glu Glu Val Val Pro Glu Val Val
   4160 4165 4170
   Glu Glu Val Val Pro Glu Val Val Glu Glu Val Val Pro Glu Val
   4175 4180 4185
   Val Glu Glu Val Val Pro Glu Val Val Glu Glu Val Val Pro Glu
   4190 4195 4200
   Glu Leu Ile Glu Glu Val Ile Pro Glu Glu Ile Val Glu Glu Val
   4205 4210 4215
   Ile Pro Glu Glu Leu Val Ala Glu Val Ile Pro Glu Lys Leu Val
   4220 4225 4230
   Glu Glu Val Ile Pro Glu Glu Leu Val Glu Glu Val Ile Pro Glu
   4235 4240 4245
   Glu Leu Val Glu Glu Val Ile Pro Glu Glu Leu Val Glu Glu Val
   4250 4255 4260
   Leu Pro Glu Glu Val Val Glu Glu Ala Val Pro Glu Val Val Glu
   4265 4270 4275
   Glu Ile Val Pro Glu Glu Leu Ile Glu Glu Val Ile Pro Glu Glu
   4280 4285 4290
   Leu Ile Lys Glu Val Val Pro Glu Glu Val Ile Glu Glu Val Ile
   4295 4300 4305
   Thr Glu Glu Val Val Glu Glu Val Leu Pro Glu Glu Ile Val Glu
   4310 4315 4320
   Glu Val Leu Pro Glu Glu Val Ile Glu Glu Val Val Pro Glu Glu
   4325 4330 4335
   Leu Val Glu Glu Val Leu Pro Glu Glu Val Val Glu Glu Val Val
   4340 4345 4350
   Pro Glu Glu Leu Val Glu Glu Val Val Pro Glu Glu Leu Val Glu
   4355 4360 4365
   Glu Val Ile Pro Glu Glu Leu Val Glu Glu Val Leu Pro Glu Glu
   4370 4375 4380
   Val Val Glu Glu Val Val Pro Lys Glu Leu Val Glu Glu Leu Ile
   4385 4390 4395
   Pro Glu Glu Leu Val Glu Glu Val Ile Pro Glu Glu Leu Val Glu
   4400 4405 4410
   Glu Val Val Pro Glu Glu Leu Val Ala Glu Val Ile Pro Glu Glu
   4415 4420 4425
   Leu Val Glu Glu Val Ile Pro Glu Glu Leu Val Glu Glu Val Ile
   4430 4435 4440
   Pro Glu Glu Leu Val Glu Glu Glu Ile Pro Glu Glu Leu Val Glu
   4445 4450 4455
   Glu Val Ile Leu Glu Glu Leu Val Glu Glu Val Val Pro Glu Glu
   4460 4465 4470
   Leu Ile Glu Glu Val Val Pro Glu Glu Leu Ile Glu Glu Val Val
   4475 4480 4485
   Pro Glu Glu Leu Ile Glu Glu Val Val Glu Lys Val Ile Pro Glu
   4490 4495 4500
   Glu Ile Ile Glu Glu Val Val Pro Glu Glu Val Ile Glu Glu Val
   4505 4510 4515
   Ile Thr Glu Glu Val Val Glu Glu Val Val Pro Glu Lys Val Ile
   4520 4525 4530
   Glu Glu Val Ile Thr Glu Glu Val Val Glu Glu Val Val Pro Glu
   4535 4540 4545
   Glu Val Ile Asp Glu Val Ile Thr Glu Glu Val Val Glu Glu Val
   4550 4555 4560
   Val Pro Glu Glu Val Ile Glu Glu Val Ile Ile Glu Glu Val Val
   4565 4570 4575
   Glu Glu Val Val Pro Glu Glu Val Ile Glu Glu Val Ile Thr Glu
   4580 4585 4590
   Glu Val Val Glu Glu Ala Ile Pro Glu Glu Leu Val Glu Glu Val
   4595 4600 4605
   Ile Pro Glu Val Leu Val Glu Glu Glu Ile Pro Glu Glu Leu Val
   4610 4615 4620
   Glu Glu  Val Ile Leu Glu Glu  Leu Val Glu Glu Val  Val Ala Glu
       4625                 4630                 4635
   Glu Leu  Ile Glu Glu Val Val  Glu Glu Val Ile Pro  Glu Lys Leu
       4640                 4645                 4650
   Val Glu  Glu Val Ile Pro Glu  Glu Ile Val Glu Glu  Val Ile Pro
       4655                 4660                 4665
   Glu Glu  Leu Val Glu Val Val  Val Pro Glu Glu Leu  Val Glu Glu
       4670                 4675                 4680
   Val Ile  Pro Glu Glu Leu Val  Glu Glu Val Ile His  Glu Glu Leu
       4685                 4690                 4695
   Val Glu  Glu Val Ile Pro Glu  Glu Leu Val Glu Glu  Val Ile His
       4700                 4705                 4710
   Glu Glu  Ile Val Glu Glu Val  Ile Pro Glu Glu Val  Val Glu Glu
       4715                 4720                 4725
   Val Ile  Pro Glu Glu Ile Val  Glu Glu Val Val Pro  Glu Glu Leu
       4730                 4735                 4740
   Leu Glu  Glu Val Ile Pro Glu  Leu Leu Val Glu Glu  Val Val Pro
       4745                 4750                 4755
   Glu Val  Val Glu Glu Val Val  Pro Val Glu Leu Leu  Glu Glu Val
       4760                 4765                 4770
   Val Glu  Glu Val Ile Thr Glu  Glu Val Ile Pro Glu  Glu Leu Ile
       4775                 4780                 4785
   Lys Glu  Val Ile Ser Glu Glu  Leu Val Glu Glu Leu  Ile Pro Glu
       4790                 4795                 4800
   Glu Val  Val Gln Glu Val Ile  Thr Glu Glu Val Leu  Glu Glu Val
       4805                 4810                 4815
   Ile Pro  Glu Glu Ile Val Glu  Glu Leu Ile Pro Glu  Lys Leu Ile
       4820                 4825                 4830
   Gly Glu  Val Val Pro Glu Lys  Val Val Glu Glu Val  Val Leu Glu
       4835                 4840                 4845
   Glu Val  Val Glu Glu Val Ile  Pro Glu Glu Ile Val  Glu Glu Val
   4850 4855 4860
   Ile Thr Glu Glu Val Leu Ala Glu Glu Ile Val Glu Glu Phe Val
   4865 4870 4875
   Pro Glu Glu Leu Val Glu Glu Met Leu Pro Glu Glu Val Val Glu
   4880 4885 4890
   Glu Arg Val Arg Glu Val Glu Ile Glu Glu Met Val Pro Glu Val
   4895 4900 4905
   Leu Val Glu Glu Val Leu Pro Glu Val Val Glu Glu Val Ile Thr
   4910 4915 4920
   Glu Glu Val Val Glu Glu Met Val Pro Glu Glu Val Val Glu Glu
   4925 4930 4935
   Met Val Pro Lys Glu Ile Val Glu Glu Val Leu Pro Glu Glu Val
   4940 4945 4950
   Val Glu Glu Val Ile Pro Glu Glu Val Val Glu Glu Met Val Pro
   4955 4960 4965
   Glu Val Leu Val Glu Glu Val Val Pro Glu Glu Ile Val Glu Lys
   4970 4975 4980
   Val Val Pro Glu Glu Val Val Glu Glu Val Val Ala Glu Glu Ile
   4985 4990 4995
   Ile Glu Glu Val Ile Thr Glu Glu Val Val Glu Glu Ile Val Pro
   5000 5005 5010
   Gly Glu Val Val Glu Glu Met Val Pro Glu Glu Ile Ile Glu Glu
   5015 5020 5025
   Val Val Pro Glu Glu Val Ile Glu Glu Val Ile Thr Glu Glu Val
   5030 5035 5040
   Val Glu Glu Val Ile Pro Glu Glu Leu Val Glu Glu Val Ile Pro
   5045 5050 5055
   Glu Ile Ser Val Glu Val Glu Ile Pro Glu Val Leu Val Glu Glu
   5060 5065 5070
   Val Ile Pro Glu Lys Leu Val Lys Val Val Pro Glu Glu Leu Val
   5075 5080 5085
   Glu Glu Val Ile Pro Glu Glu Leu Val Glu Glu Val Ile Thr Glu
   5090 5095 5100
   Ala Leu Val Glu Glu Val Val Asp Val Val Val Pro Glu Glu Ile
   5105 5110 5115
   Phe Glu Glu Val Leu Pro Glu Glu Val Val Glu Glu Val Ile Pro
   5120 5125 5130
   Glu Glu Val Val Glu Glu Met Val Pro Glu Glu Val Val Glu Glu
   5135 5140 5145
   Val Val Pro Glu Glu Leu Val Glu Glu Val Ile Pro Glu Glu Val
   5150 5155 5160
   Val Glu Lys Val Val Pro Glu Gln Leu Val Glu Lys Val Val Pro
   5165 5170 5175
   Glu Glu Leu Val Glu Lys Val Leu Pro Glu Glu Leu Val Glu Glu
   5180 5185 5190
   Val Val Pro Glu Glu Leu Ile Glu Glu Val Val Thr Glu Glu Val
   5195 5200 5205
   Ile Glu Glu Ile Ile Pro Glu Glu Ile Ile Glu Glu Ile Ile Pro
   5210 5215 5220
   Glu Glu Val Val Glu Glu Arg Val Ser Ile Glu Val Val Glu Glu
   5225 5230 5235
   Leu Val Pro Glu Glu Leu Ile Glu Glu Val Val Ala Glu Glu Ile
   5240 5245 5250
   Phe Glu Glu Val Val Pro Glu Lys Leu Ile Glu Glu Val Val Ala
   5255 5260 5265
   Glu Glu Leu Ile Glu Glu Met Val Pro Glu Val Val Glu Glu Val
   5270 5275 5280
   Val Glu Glu Ile Ile Pro Glu Glu Val Ile Glu Glu Val Ile Pro
   5285 5290 5295
   Glu Glu Ile Arg Glu Glu Ile Ile Pro Glu Glu Val Val Glu Glu
   5300 5305 5310
   Val Ile Glu Glu Val Ile Pro Glu Glu Leu Ile Glu Glu Glu Ile
   5315 5320 5325
   Ser Arg  Glu Ile Val Glu Glu  Val Ile Pro Glu Glu  Val Val Glu
       5330                 5335                 5340
   Glu Met  Met Pro Glu Glu Val  Ile Glu Glu Val Ile  Pro Glu Glu
       5345                 5350                 5355
   Ile Arg  Glu Glu Met Ile Pro  Glu Glu Val Val Glu  Glu Met Ile
       5360                 5365                 5370
   Pro Glu  Ile Glu Ile Glu Gln  Val Leu Pro Glu Glu  Leu Ile Glu
       5375                 5380                 5385
   Glu Leu  Val Pro Glu Val Glu  Ile Glu Glu Ile Leu  Pro Glu Glu
       5390                 5395                 5400
   Leu Ile  Glu Glu Leu Val Pro  Glu Gly Val Ile Glu  Glu Val Ile
       5405                 5410                 5415
   Pro Lys  Glu Leu Ile Glu Glu  Val Ile Pro Glu Glu  Leu Ile Lys
       5420                 5425                 5430
   Glu Val  Val Pro Glu Glu Val  Val Glu Glu Val Ile  Pro Lys Glu
       5435                 5440                 5445
   Ile Val  Glu Glu Val Val Pro  Glu Glu Leu Ile Glu  Glu Val Ile
       5450                 5455                 5460
   Pro Glu  Glu Leu Ile Glu Glu  Val Val Pro Glu Glu  Met Ile Glu
       5465                 5470                 5475
   Glu Val  Ile Pro Glu Glu Leu  Ile Glu Glu Val Val  Pro Glu Glu
       5480                 5485                 5490
   Met Ile  Glu Glu Val Ile Pro  Glu Glu Leu Ile Glu  Glu Val Val
       5495                 5500                 5505
   Pro Glu  Glu Val Lys Glu Glu  Val Ile Pro Val Glu  Leu Ile Glu
       5510                 5515                 5520
   Glu Val  Val Pro Glu Glu Met  Ile Glu Glu Val Ile  Ser Glu Glu
       5525                 5530                 5535
   Val Val  Glu Glu Val Ile Pro  Glu Glu Leu Val Glu  Glu Val Leu
       5540                 5545                 5550
   Pro Glu  Val Phe Val Glu Glu  Ile Val Pro Lys Glu  Leu Val Glu
       5555                 5560                 5565
   Glu Val Ile Pro Glu Glu Ile Val Glu Glu Val Ile Pro Glu Glu
   5570 5575 5580
   Leu Val Glu Glu Val Ile Pro Glu Glu Leu Val Glu Glu Val Ile
   5585 5590 5595
   Ser Glu Glu Leu Leu Glu Glu Val Ile Pro Glu Leu Leu Val Glu
   5600 5605 5610
   Glu Val Val Pro Glu Val Ile Glu Glu Val Val Pro Glu Val Val
   5615 5620 5625
   Glu Glu Val Ile Pro Glu Val Val Glu Glu Val Val Thr Glu Glu
   5630 5635 5640
   Leu Val Glu Glu Val Ile Pro Glu Glu Val Val Glu Glu Met Ile
   5645 5650 5655
   Pro Gly Val Glu Ile Glu Glu Val Leu Pro Glu Glu Leu Val Glu
   5660 5665 5670
   Glu Val Ile Pro Glu Glu Ile Val Glu Glu Val Ile Ser Glu Glu
   5675 5680 5685
   Asp Phe Glu Glu Val Ile Pro Glu Ala Leu Val Glu Glu Val Ile
   5690 5695 5700
   Pro Glu Leu Leu Val Glu Glu Val Val Leu Glu Glu Leu Ile Glu
   5705 5710 5715
   Glu Val Ile Pro Glu Val Val Glu Glu Val Ile Pro Glu Glu Val
   5720 5725 5730
   Val Glu Glu Val Ile Pro Glu Glu Val Val Glu Lys Val Val Pro
   5735 5740 5745
   Glu Glu Leu Val Glu Glu Val Ile Pro Glu Glu Leu Val Glu Glu
   5750 5755 5760
   Val Ile Pro Glu Glu Val Pro Glu Glu Val Val Pro Glu Glu Leu
   5765 5770 5775
   Val Glu Glu Val Ile Ser Glu Glu Leu Val Glu Glu Val Ile Pro
   5780 5785 5790
   Glu Glu Ile Val Glu Glu Val Ile Pro Glu Glu Ile Val Glu Glu
   5795 5800 5805
   Val Met Pro Glu Glu Val Ala Glu Glu Val Val Pro Glu Glu Leu
   5810 5815 5820
   Val Glu Glu Glu Val Pro Glu Glu Leu Val Glu Glu Val Ile Pro
   5825 5830 5835
   Glu Val Leu Val Glu Glu Val Ile Pro Glu Glu Ile Val Glu Glu
   5840 5845 5850
   Val Val Pro Glu Glu Val Val Glu Val Val Ile Pro Glu Glu Phe
   5855 5860 5865
   Ile Glu Glu Ile Ile Pro Glu Glu Val Val Val Glu Val Thr Pro
   5870 5875 5880
   Glu Glu Leu Ile Glu Glu Met Lys Pro Glu Glu Leu Phe Glu Glu
   5885 5890 5895
   Val Ile Pro Lys Glu Leu Ile Glu Glu Ile Ile Pro Glu Glu Leu
   5900 5905 5910
   Ile Lys Glu Val Ile Pro Glu Glu Ile Ile Glu Glu Val Ile Ser
   5915 5920 5925
   Lys Glu Ile Val Glu Glu Val Val Glu Glu Glu Ile Ile Glu Glu
   5930 5935 5940
   Val Ile Pro Glu Glu Ile Val Glu Glu Val Val Pro Glu Glu Leu
   5945 5950 5955
   Val Glu Lys Lys Val Pro Glu Glu Ile Val Glu Glu Val Ile Tyr
   5960 5965 5970
   Glu Glu Phe Leu Glu Glu Ile Met Pro Glu Glu Leu Ile Glu Glu
   5975 5980 5985
   Met Lys Pro Glu Glu Leu Ile Glu Glu Ile Ile Pro Arg Glu Ile
   5990 5995 6000
   Val Glu Glu Ile Ile Thr Glu Glu Val Val Glu Glu Met Lys Pro
   6005 6010 6015
   Glu Glu Leu Val Glu Glu Met Val Pro Glu Glu Val Val Glu Glu
   6020 6025 6030
   Met Val Pro Glu Glu Val Val Glu Glu Met Val Pro Glu Glu Val
   6035 6040 6045
   Val Glu Glu Val Ile Thr Glu Lys Leu Ile Glu Glu Val Val Pro
   6050 6055 6060
   Glu Glu Ile Ile Glu Glu Leu Ile Pro Glu Glu Leu Val Glu Glu
   6065 6070 6075
   Val Ile Pro Glu Lys Leu Ile Glu Glu Met Ile Pro Glu Glu Val
   6080 6085 6090
   Val Glu Glu Met Leu Leu Glu Glu Val Val Glu Glu Val Val Pro
   6095 6100 6105
   Glu Val Glu Ile Glu Glu Met Ile Pro Glu Gln Leu Val Glu Glu
   6110 6115 6120
   Val Val Pro Glu Glu Leu Val Glu Lys Glu Ile Pro Glu Val Val
   6125 6130 6135
   Glu Glu Ile Ile Thr Glu Glu Val Val Glu Glu Met Val Pro Glu
   6140 6145 6150
   Glu Val Val Glu Glu Met Val Pro Glu Glu Val Val Glu Glu Val
   6155 6160 6165
   Ile Leu Glu Lys Leu Ile Glu Glu Val Val Pro Val Glu Val Ile
   6170 6175 6180
   Glu Glu Val Val Pro Glu Glu Val Ile Glu Glu Val Ile Pro Glu
   6185 6190 6195
   Lys Leu Ile Glu Glu Ile Ile Pro Glu Val Leu Val Glu Glu Val
   6200 6205 6210
   Val Pro Glu Glu Leu Ile Glu Glu Val Ile Pro Glu Val Leu Phe
   6215 6220 6225
   Glu Glu Val Val Pro Glu Val Val Glu Glu Val Val Pro Glu Glu
   6230 6235 6240
   Leu Val Glu Lys Val Val Pro Glu Glu Ile Val Glu Glu Val Ile
   6245 6250 6255
   Pro Glu Phe Val Glu Glu Val Ile Ser Glu Glu Ile Val Glu Glu
   6260 6265 6270
   Val Val Thr Glu Glu Leu Ile Glu Glu Met Ile Pro Glu Glu Val
   6275 6280 6285
   Val Glu Glu Val Ile Pro Glu Glu Leu Val Glu Glu Val Val Pro
   6290 6295 6300
   Val Glu Leu Ile Glu Glu Val Val Pro Val Glu Leu Leu Glu Glu
   6305 6310 6315
   Val Ile Pro Glu Glu Leu Val Glu Glu Val Val Pro Val Glu Leu
   6320 6325 6330
   Ile Glu Glu Val Val Pro Val Glu Leu Leu Glu Glu Val Ile Pro
   6335 6340 6345
   Glu Glu Val Val Glu Glu Val Ile Pro Glu Val Val Lys Glu Val
   6350 6355 6360
   Val Pro Glu Val Val Glu Glu Val Val Pro Glu Val Val Glu Glu
   6365 6370 6375
   Val Val Pro Glu Val Val Glu Glu Val Val Pro Glu Val Val Glu
   6380 6385 6390
   Glu Val Val Pro Glu Glu Leu Ile Glu Glu Val Ile Pro Glu Glu
   6395 6400 6405
   Leu Ile Lys Glu Val Leu Pro Glu Glu Val Ile Glu Glu Leu Lys
   6410 6415 6420
   Pro Glu Glu Leu Ile Glu Glu Ile Ile Pro Glu Glu Val Val Glu
   6425 6430 6435
   Glu Val Ile Pro Glu Glu Leu Ile Glu Glu Met Ile Pro Glu Leu
   6440 6445 6450
   Leu Val Glu Glu Glu Val Pro Glu Glu Leu Val Glu Glu Val Ile
   6455 6460 6465
   Pro Glu Val Leu Val Glu Glu Met Val Pro Glu Glu Ile Val Glu
   6470 6475 6480
   Glu Val Ile Pro Glu Glu Leu Ile Ala Glu Phe Ala Pro Glu Glu
   6485 6490 6495
   Leu Phe Glu Lys Val Ile Pro Glu Glu Val Val Glu Glu Leu Val
   6500 6505 6510
   Ser Glu  Glu Leu Val Glu Glu  Val Leu Pro Glu Val  Val Glu Glu
       6515                 6520                 6525
   Val Leu  Pro Glu Val Phe Glu  Glu Val Ile Pro Glu  Glu Val Ile
       6530                 6535                 6540
   Glu Glu  Ile Val Pro Glu Glu  Val Met Glu Glu Val  Val Pro Lys
       6545                 6550                 6555
   Glu Leu  Ile Glu Glu Val Ile  Pro Glu Glu Leu Ile  Glu Glu Val
       6560                 6565                 6570
   Ile Pro  Glu Glu Val Ala Glu  Glu Val Val Pro Glu  Lys Leu Ile
       6575                 6580                 6585
   Glu Glu  Val Ile Pro Glu Glu  Leu Ile Lys Glu Val  Val Pro Glu
       6590                 6595                 6600
   Glu Leu  Ile Glu Glu Val Ile  Thr Glu Glu Val Val  Glu Glu Ala
       6605                 6610                 6615
   Ile Ser  Glu Glu Leu Val Glu  Glu Val Val Pro Glu  Glu Val Val
       6620                 6625                 6630
   Glu Glu  Val Ile Pro Glu Glu  Leu Ile Glu Glu Val  Val Pro Glu
       6635                 6640                 6645
   Glu Leu  Ile Glu Glu Ile Ile  Pro Glu Glu Leu Val  Glu Glu Val
       6650                 6655                 6660
   Ile Pro  Glu Glu Leu Val Glu  Glu Val Val Pro Glu  Glu Leu Val
       6665                 6670                 6675
   Glu Glu  Val Ile Pro Glu Glu  Leu Val Glu Glu Val  Val Pro Glu
       6680                 6685                 6690
   Glu Leu  Val Glu Lys Leu Ile  Pro Glu Glu Leu Val  Glu Glu Val
       6695                 6700                 6705
   Ile Pro  Glu Leu Leu Val Glu  Glu Val Val Pro Glu  Glu Val Val
       6710                 6715                 6720
   Glu Glu  Leu Ile Pro Glu Glu  Leu Val Glu Glu Val  Ile Pro Glu
       6725                 6730                 6735
   Glu Leu  Val Glu Glu Val Ile  Pro Glu Glu Val Pro  Glu Glu Val
   6740 6745 6750
   Ile Arg Glu Glu Leu Val Glu Glu Val Ile Pro Glu Glu Leu Val
   6755 6760 6765
   Glu Glu Val Ile Pro Glu Glu Val Pro Glu Glu Val Val Pro Glu
   6770 6775 6780
   Glu Leu Val Gln Glu Val Ile Pro Glu Glu Leu Val Glu Glu Val
   6785 6790 6795
   Val His Glu Glu Val Pro Glu Glu Val Val Pro Glu Lys Leu Val
   6800 6805 6810
   Glu Glu Val Ile Pro Glu Glu Leu Phe Glu Glu Val Val Pro Glu
   6815 6820 6825
   Glu Val Pro Glu Glu Val Val Pro Glu Glu Leu Val Glu Glu Val
   6830 6835 6840
   Ile Ser Glu Glu Leu Val Glu Glu Val Ile Pro Glu Glu Leu Val
   6845 6850 6855
   Glu Glu Phe Ile Pro Glu Glu Leu Val Glu Glu Val Ile Pro Glu
   6860 6865 6870
   Glu Leu Val Glu Glu Val Ile Pro Glu Glu Leu Val Glu Glu Phe
   6875 6880 6885
   Ile Pro Glu Glu Leu Val Glu Glu Val Ile Pro Glu Glu Leu Val
   6890 6895 6900
   Glu Glu Val Ile Pro Glu Val Leu Val Glu Glu Glu Ile Pro Glu
   6905 6910 6915
   Lys Leu Val Glu Glu Val Val Pro Glu Glu Leu Val Glu Glu Val
   6920 6925 6930
   Ile Pro Glu Val Leu Val Glu Glu Val Val Thr Glu Glu Val Pro
   6935 6940 6945
   Glu Glu Val Val Pro Glu Glu Leu Val Ala Glu Val Val Pro Glu
   6950 6955 6960
   Glu Leu Val Lys Glu Val Ile Pro Glu Glu Leu Val Glu Glu Val
   6965 6970 6975
   Ile Pro Glu Glu Ile Val Glu Glu Val Ile Pro Glu Glu Val Val
   6980 6985 6990
   Glu Glu Val Val Pro Glu Glu Leu Val Glu Glu Val Ile Pro Glu
   6995 7000 7005
   Glu Val Val Glu Glu Val Ile Pro Glu Glu Leu Val Glu Glu Val
   7010 7015 7020
   Val Pro Val Glu Leu Val Glu Glu Val Val Pro Glu Glu Leu Val
   7025 7030 7035
   Glu Glu Val Ile Pro Glu Glu Leu Val Glu Glu Val Ile Pro Glu
   7040 7045 7050
   Glu Ile Val Glu Glu Val Ile Pro Glu Glu Val Val Glu Glu Val
   7055 7060 7065
   Ile Pro Glu Glu Ile Val Glu Glu Val Ile Ser Glu Glu Leu Ile
   7070 7075 7080
   Glu Glu Val Val Pro Val Glu Leu Leu Glu Glu Val Val Pro Val
   7085 7090 7095
   Glu Val Leu Glu Asp Val Ile Pro Glu Glu Val Val Glu Glu Val
   7100 7105 7110
   Ile Pro Glu Glu Phe Ile Glu Glu Met Ile Pro Glu Glu Ile Ile
   7115 7120 7125
   Glu Glu Ala Ile Pro Glu Glu Ile Val Glu Glu Val Ile Pro Glu
   7130 7135 7140
   Val Val Glu Glu Val Val Pro Glu Glu Leu Val Glu Glu Val Ile
   7145 7150 7155
   Pro Glu Glu Val Val Glu Lys Val Val Pro Glu Glu Leu Val Glu
   7160 7165 7170
   Glu Val Ile Pro Glu Glu Leu Val Glu Glu Val Ile Pro Glu Glu
   7175 7180 7185
   Val Pro Glu Glu Val Val Pro Glu Glu Leu Val Glu Glu Val Ile
   7190 7195 7200
   Ser Glu Glu Leu Val Glu Glu Ile Ile Pro Glu Glu Leu Val Glu
   7205 7210 7215
   Glu Val Ile Pro Glu Glu Val Val Glu Glu Val Val Pro Glu Glu
   7220 7225 7230
   Leu Val Glu Glu Val Ile Pro Glu Glu Leu Val Glu Glu Val Ile
   7235 7240 7245
   Pro Glu Glu Leu Val Glu Glu Val Ile Pro Glu Val Val Glu Glu
   7250 7255 7260
   Val Ile Pro Glu Lys Leu Val Glu Glu Val Val Pro Glu Glu Leu
   7265 7270 7275
   Ile Glu Glu Met Ile Pro Glu Glu Ile Val Glu Glu Val Val Pro
   7280 7285 7290
   Glu Val Val Glu Glu Val Ile Pro Glu Glu Leu Ile Glu Glu Val
   7295 7300 7305
   Ile Pro Glu Glu Leu Val Glu Glu Val Ile Pro Glu Lys Leu Ile
   7310 7315 7320
   Glu Glu Met Ile Pro Glu Glu Leu Val Glu Glu Val Ile Leu Glu
   7325 7330 7335
   Glu Val Val Glu Glu Val Ile Pro Glu Glu Val Val Glu Glu Val
   7340 7345 7350
   Leu Pro Glu Glu Leu Val Glu Glu Val Val Pro Glu Glu Leu Val
   7355 7360 7365
   Glu Glu Val Ala Pro Val Glu Leu Leu Glu Glu Val Ile Pro Glu
   7370 7375 7380
   Glu Leu Leu Glu Glu Val Ile Pro Glu Glu Leu Val Glu Glu Val
   7385 7390 7395
   Ile Pro Glu Glu Leu Val Glu Asp Val Ile Pro Glu Glu Leu Val
   7400 7405 7410
   Glu Glu Val Ile Pro Glu Glu Leu Val Glu Glu Ile Ile Pro Glu
   7415 7420 7425
   Glu Leu Val Glu Glu Val Ile Pro Glu Val Leu Val Glu Glu Glu
   7430 7435 7440
   Val Pro Glu Glu Leu Val Glu Glu Val Ile Pro Glu Glu Leu Val
   7445 7450 7455
   Glu Glu  Val Ile Pro Glu Glu  Leu Val Glu Glu Leu  Ile Pro Glu
       7460                 7465                 7470
   Val Leu  Glu Glu Val Ile Pro  Glu Lys Leu Val Glu  Glu Val Val
       7475                 7480                 7485
   Pro Val  Glu Leu Leu Glu Glu  Val Ile Pro Glu Glu  Leu Ile Glu
       7490                 7495                 7500
   Glu Val  Val Pro Glu Val Leu  Val Glu Val Ile Pro  Glu Lys Val
       7505                 7510                 7515
   Val Glu  Glu Val Ile His Glu  Glu Ile Val Lys Glu  Val Val Pro
       7520                 7525                 7530
   Glu Glu  Leu Val Glu Glu Val  Val Pro Glu Glu Ile  Val Glu Glu
       7535                 7540                 7545
   Val Val  Pro Glu Glu Val Leu  Glu Glu Val Ile Pro  Lys Val Leu
       7550                 7555                 7560
   Leu Glu  Glu Glu Ile Pro Glu  Lys Leu Val Glu Glu  Val Ile Pro
       7565                 7570                 7575
   Glu Glu  Leu Ile Glu Glu Val  Val Pro Glu Glu Leu  Val Glu Glu
       7580                 7585                 7590
   Val Met  Pro Glu Glu Val Val  Glu Glu Val Val Pro  Glu Glu Leu
       7595                 7600                 7605
   Val Glu  Glu Val Ile Pro Glu  Glu Val Val Glu Glu  Val Ile Pro
       7610                 7615                 7620
   Glu Glu  Leu Val Glu Glu Val  Val Pro Val Glu Leu  Leu Glu Glu
       7625                 7630                 7635
   Ile Val  Pro Val Glu Leu Leu  Glu Glu Val Ile Pro  Glu Glu Leu
       7640                 7645                 7650
   Val Glu  Glu Val Ile Pro Glu  Glu Leu Val Glu Glu  Val Val Pro
       7655                 7660                 7665
   Glu Glu  Leu Val Ala Glu Val  Ile Pro Glu Lys Leu  Val Glu Glu
       7670                 7675                 7680
   Val Ile  Pro Glu Lys Leu Val  Glu Glu Val Ile Pro  Glu Glu Leu
   7685 7690 7695
   Val Glu Glu Val Val Pro Glu Glu Leu Val Ala Glu Val Ile Pro
   7700 7705 7710
   Glu Lys Leu Val Glu Glu Val Ile Pro Glu Glu Leu Val Glu Glu
   7715 7720 7725
   Ile Ile Pro Glu Glu Leu Val Glu Glu Val Val Pro Glu Glu Leu
   7730 7735 7740
   Val Glu Glu Val Val Pro Glu Glu Leu Val Glu Glu Val Ile Pro
   7745 7750 7755
   Glu Glu Leu Val Glu Glu Val Ile Pro Glu Val Leu Val Glu Glu
   7760 7765 7770
   Glu Val Pro Glu Glu Leu Val Glu Glu Val Ile Pro Glu Glu Leu
   7775 7780 7785
   Val Glu Glu Met Leu Pro Glu Glu Leu Ile Glu Val Val Val Pro
   7790 7795 7800
   Glu Glu Leu Val Glu Glu Val Val Pro Glu Glu Leu Val Glu Glu
   7805 7810 7815
   Val Val Pro Glu Glu Val Val Glu Glu Val Ile Pro Glu Glu Leu
   7820 7825 7830
   Ile Glu Glu Leu Val Pro Glu Val Glu Ile Glu Glu Ile Leu Pro
   7835 7840 7845
   Glu Glu Leu Ile Glu Glu Leu Val Pro Glu Glu Ile Ile Glu Glu
   7850 7855 7860
   Val Ile Pro Lys Glu Leu Ile Glu Glu Val Ile Pro Glu Val Val
   7865 7870 7875
   Val Glu Glu Val Val Pro Glu Val Leu Val Glu Glu Val Val Pro
   7880 7885 7890
   Glu Glu Leu Val Glu Glu Val Ile Pro Glu Val Leu Val Glu Glu
   7895 7900 7905
   Glu Val Pro Glu Glu Leu Val Glu Asp Val Ile Pro Glu Glu Leu
   7910 7915 7920
   Val Glu Glu Val Val Pro Glu Glu Leu Val Glu Glu Val Ile Pro
   7925 7930 7935
   Glu Glu Val Val Glu Glu Val Leu Pro Glu Glu Leu Val Glu Glu
   7940 7945 7950
   Val Val Pro Glu Glu Ile Val Glu Glu Val Ile Pro Glu Glu Leu
   7955 7960 7965
   Val Glu Glu Glu Val Pro Glu Glu Leu Val Glu Glu Val Ile Pro
   7970 7975 7980
   Glu Glu Val Val Glu Glu Val Ile Pro Glu Glu Val Pro Glu Lys
   7985 7990 7995
   Val Ile Pro Glu Lys Leu Val Glu Glu Leu Ile Pro Glu Glu Val
   8000 8005 8010
   Val Glu Glu Leu Ile Pro Glu Glu Val Val Glu Glu Met Val Pro
   8015 8020 8025
   Ile Glu Val Val Glu Glu Val Val Pro Thr Glu Val Leu Glu Glu
   8030 8035 8040
   Val Ile Pro Glu Ile Val Val Glu Glu Val Val Pro Glu Glu Leu
   8045 8050 8055
   Val Glu Glu Val Ile Pro Glu Glu Leu Val Glu Glu Val Ile Pro
   8060 8065 8070
   Glu Ile Val Val Glu Glu Val Val Pro Glu Glu Leu Val Glu Glu
   8075 8080 8085
   Val Ile Pro Glu Glu Leu Val Glu Glu Val Val Pro Glu Glu Leu
   8090 8095 8100
   Val Glu Glu Val Val Pro Glu Glu Ile Val Glu Glu Leu Leu Pro
   8105 8110 8115
   Lys Glu Ile Val Glu Glu Val Ile Tyr Glu Glu Gln Val Glu Glu
   8120 8125 8130
   Val Ile His Glu Glu Leu Val Ala Glu Val Ile Pro Glu Glu Val
   8135 8140 8145
   Val Glu Glu Val Val Pro Glu Val Val Glu Glu Val Val Pro Glu
   8150 8155 8160
   Ile Val Ala Glu Val Ile Pro Glu Glu Val Val Glu Glu Val Val
   8165 8170 8175
   Pro Glu Val Val Glu Glu Val Val Pro Glu Val Val Glu Glu Val
   8180 8185 8190
   Ile Pro Glu Glu Val Val Glu Glu Val Ile Pro Glu Glu Phe Ile
   8195 8200 8205
   Glu Glu Met Ile Pro Glu Glu Ile Val Glu Glu Val Val Pro Glu
   8210 8215 8220
   Val Val Glu Glu Val Ile Pro Glu Glu Leu Val Ala Glu Val Ile
   8225 8230 8235
   Pro Glu Glu Leu Val Glu Glu Val Ile Pro Glu Val Leu Phe Glu
   8240 8245 8250
   Glu Glu Ile Pro Glu Val Val Val Glu Glu Val Val Pro Glu Val
   8255 8260 8265
   Val Glu Glu Glu Ile Pro Glu Val Val Val Glu Glu Val Val Pro
   8270 8275 8280
   Glu Val Val Glu Glu Val Ile Pro Glu Glu Val Val Glu Glu Leu
   8285 8290 8295
   Ile Pro Glu Glu Val Val Glu Glu Met Val Pro Ile Glu Ile Val
   8300 8305 8310
   Glu Glu Val Val Pro Thr Glu Val Leu Glu Glu Val Ile Pro Glu
   8315 8320 8325
   Ile Val Val Glu Glu Val Val Pro Glu Glu Leu Val Glu Glu Val
   8330 8335 8340
   Ile Pro Glu Glu Leu Val Glu Glu Val Val Pro Glu Glu Leu Val
   8345 8350 8355
   Glu Glu Val Val Pro Glu Val Val Glu Glu Val Val Pro Glu Val
   8360 8365 8370
   Val Glu Glu Val Val Pro Glu Glu Leu Val Glu Glu Val Ile Pro
   8375 8380 8385
   Glu Val Val Val Glu Glu Val Val Pro Glu Glu Leu Val Glu Glu
   8390 8395 8400
   Val Ile Pro Glu Glu Leu Val Glu Glu Ile Ile Pro Glu Glu Ile
   8405 8410 8415
   Val Glu Glu Val Ile Pro Glu Glu Leu Ile Glu Glu Val Val Pro
   8420 8425 8430
   Glu Val Leu Val Glu Glu Val Ile Pro Glu Glu Leu Val Glu Glu
   8435 8440 8445
   Val Ile Pro Glu Glu Leu Phe Glu Glu Val Val Pro Glu Glu Leu
   8450 8455 8460
   Val Ala Glu Val Ile Pro Glu Glu Ile Val Glu Glu Val Ile Pro
   8465 8470 8475
   Glu Glu Leu Ile Glu Glu Phe Val Pro Glu Glu Leu Val Glu Glu
   8480 8485 8490
   Glu Val Pro Glu Glu Leu Val Glu Glu Val Ile Pro Glu Val Leu
   8495 8500 8505
   Val Glu Glu Val Ile Pro Glu Glu Phe Val Glu Glu Val Ile Pro
   8510 8515 8520
   Glu Glu Ile Val Glu Glu Val Ile Pro Glu Glu Ile Val Glu Glu
   8525 8530 8535
   Val Ile Pro Glu Glu Val Val Glu Glu Val Val Pro Glu Ile Val
   8540 8545 8550
   Ala Glu Val Ile Pro Glu Glu Val Val Glu Glu Val Val Pro Glu
   8555 8560 8565
   Val Val Glu Glu Val Val Pro Glu Val Ile Glu Glu Val Val Pro
   8570 8575 8580
   Glu Val Ile Glu Glu Val Val Pro Glu Val Val Glu Glu Val Val
   8585 8590 8595
   Pro Glu Val Val Glu Glu Val Val Pro Glu Val Val Glu Glu Val
   8600 8605 8610
   Val Pro Val Glu Val Leu Glu Glu Val Ile Pro Glu Glu Val Val
   8615 8620 8625
   Glu Glu Val Ile Pro Glu Glu Phe Ile Glu Glu Met Ile Pro Glu
   8630 8635 8640
   Glu Ile Val Glu Glu Val Val Pro Glu Val Val Glu Asp Val Ile
   8645 8650 8655
   Pro Glu Glu Leu Ile Ala Glu Val Val Ser Glu Glu Leu Phe Glu
   8660 8665 8670
   Glu Glu Ile Pro Glu Glu Leu Val Glu Val Val Pro Val Glu Leu
   8675 8680 8685
   Leu Glu Glu Val Ile Pro Glu Glu Leu Val Glu Glu Val Ile Pro
   8690 8695 8700
   Glu Glu Leu Val Glu Glu Val Val Pro Glu Glu Leu Val Glu Glu
   8705 8710 8715
   Val Val Pro Glu Glu Leu Val Glu Glu Val Ile Pro Glu Glu Leu
   8720 8725 8730
   Phe Glu Glu Val Val Pro Glu Glu Leu Val Glu Glu Val Ile Pro
   8735 8740 8745
   Glu Glu Ile Val Glu Glu Val Ile Pro Glu Glu Leu Ile Glu Glu
   8750 8755 8760
   Phe Val Pro Glu Glu Leu Val Glu Glu Glu Val Pro Glu Glu Leu
   8765 8770 8775
   Val Glu Glu Val Ile Pro Glu Val Leu Val Glu Glu Val Ile Pro
   8780 8785 8790
   Glu Glu Phe Val Glu Glu Val Ile Pro Glu Glu Leu Val Glu Glu
   8795 8800 8805
   Val Ile Pro Glu Glu Leu Phe Glu Glu Val Val Pro Glu Glu Leu
   8810 8815 8820
   Val Glu Glu Val Ile Pro Glu Glu Ile Val Glu Glu Val Ile Pro
   8825 8830 8835
   Glu Glu Leu Ile Glu Glu Val Val Pro Glu Val Leu Val Glu Glu
   8840 8845 8850
   Val Ile Pro Glu Glu Leu Val Glu Glu Val Ile Pro Glu Glu Leu
   8855 8860 8865
   Phe Glu Glu Val Val Pro Glu Glu Leu Val Ala Glu Val Ile Pro
   8870 8875 8880
   Glu Glu Ile Val Glu Glu Val Ile Pro Glu Glu Leu Ile Glu Glu
   8885 8890 8895
   Phe Val Pro Glu Glu Leu Val Glu Glu Glu Val Pro Glu Glu Leu
   8900 8905 8910
   Val Glu Glu Val Ile Pro Glu Val Leu Val Glu Glu Glu Val Pro
   8915 8920 8925
   Glu Glu Leu Val Glu Glu Val Ile Pro Glu Glu Leu Val Glu Glu
   8930 8935 8940
   Met Leu Pro Glu Glu Leu Ile Glu Val Val Val Pro Glu Glu Leu
   8945 8950 8955
   Val Glu Glu Val Val Pro Glu Ile Val Glu Glu Val Val Pro Glu
   8960 8965 8970
   Glu Leu Val Glu Glu Val Ile Pro Glu Glu Val Val Glu Glu Val
   8975 8980 8985
   Leu Pro Glu Lys Leu Val Glu Glu Val Val Pro Glu Ile Val Glu
   8990 8995 9000
   Glu Val Ala Pro Glu Val Val Glu Glu Val Ile Pro Glu Glu Leu
   9005 9010 9015
   Val Glu Glu Ile Ile Pro Glu Ile Leu Val Glu Glu Val Val Pro
   9020 9025 9030
   Ile Glu Val Val Glu Glu Ala Val Pro Glu Val Val Val Glu Glu
   9035 9040 9045
   Val Val Pro Glu Lys Ile Val Glu Glu Val Val Pro Glu Lys Val
   9050 9055 9060
   Leu Glu Glu Val Ile Pro Glu Leu Leu Val Glu Glu Ile Val Pro
   9065 9070 9075
   Ile Glu Val Val Gly Glu Ile Val Pro Glu Glu Val Val Glu Lys
   9080 9085 9090
   Ile Val Pro Met Glu Val Val Glu Glu Leu Leu Pro Glu Val Val
   9095 9100 9105
   Glu Glu Val Pro His Glu Ile Val Glu Glu Glu Val Val Lys Glu
   9110 9115 9120
   Val Ile Pro Glu Asp Glu Lys Lys Val Glu Ile Val Glu Ile Ile
   9125 9130 9135
   Ile Glu Glu Lys Val Glu Lys Ile Pro Lys Lys Lys Val Ile Glu
   9140 9145 9150
   Lys Lys Lys Glu Lys Glu Lys Ile Arg Lys Pro Lys Lys Lys Val
   9155 9160 9165
   Glu Lys Glu Ile Lys Pro Lys Glu Val Lys Gly Val Lys Gly Lys
   9170 9175 9180
   Leu Lys Lys Asp Val Lys Ala Lys His Leu Lys Gly Ser Lys Val
   9185 9190 9195
   Lys Ile Thr Glu Asp Ile Gln Lys Lys Met Glu Asp Asp Ile Lys
   9200 9205 9210
   Glu Lys Ile Met Glu Lys Glu Leu Lys Glu Glu Ile Lys Asp Lys
   9215 9220 9225
   Glu Tyr Leu Arg Lys Val Ile Pro Lys Glu Glu Lys Lys Glu Pro
   9230 9235 9240
   Ile Pro Lys Lys Gly Lys Ser Val Glu Pro Lys Lys Glu Lys Gln
   9245 9250 9255
   Ala Gly Val Lys Lys Glu Lys Val Pro Glu Thr Lys Lys Glu Lys
   9260 9265 9270
   Ala Pro Ala Pro Lys Lys Glu Lys Val Gln Ala Pro Lys Lys Glu
   9275 9280 9285
   Lys Val Pro Ala Pro Lys Lys Glu Lys Pro Ala Val Pro Lys Lys
   9290 9295 9300
   Glu Lys Val Pro Glu Pro Lys Lys Glu Lys Val Pro Glu Pro Lys
   9305 9310 9315
   Lys Glu Lys Ala Pro Ala Pro Lys Lys Glu Lys Val Gln Ala Pro
   9320 9325 9330
   Lys Lys Glu Lys Ala Pro Ala Pro Lys Lys Glu Lys Ala Pro Ala
   9335 9340 9345
   Pro Lys Lys Glu Lys Ala Pro Ala Pro Lys Lys Glu Lys Ala Pro
   9350 9355 9360
   Ala Pro Lys Lys Glu Lys Pro Ala Val Pro Lys Lys Glu Lys Pro
   9365 9370 9375
   Ala Val Pro Lys Lys Glu Lys Val Pro Glu Thr Lys Lys Glu Lys
   9380 9385 9390
   Val Pro Glu Thr Lys Lys Glu Lys Val Pro Glu Thr Lys Lys Glu
   9395 9400 9405
   Lys Val Pro Val Pro Lys Lys Glu Lys Val Pro Ala Pro Lys Lys
   9410 9415 9420
   Glu Lys Val Pro Ala Pro Lys Lys Glu Lys Val Pro Ala Pro Lys
   9425 9430 9435
   Lys Glu Lys Pro Ala Val Pro Lys Lys Glu Lys Val Pro Glu Thr
   9440 9445 9450
   Lys Lys Glu Lys Ala Pro Glu Pro Lys Lys Glu Lys Ala Pro Glu
   9455 9460 9465
   Pro Lys Lys Glu Lys Ala Pro Glu Pro Lys Lys Glu Lys Ala Pro
   9470 9475 9480
   Glu Pro Lys Lys Glu Lys Ala Pro Ala Pro Lys Lys Glu Lys Val
   9485 9490 9495
   Pro Val Pro Lys Lys Glu Lys Val Pro Ala Pro Lys Lys Glu Lys
   9500 9505 9510
   Val Pro Ala Pro Lys Lys Glu Lys Pro Ala Val Pro Lys Lys Glu
   9515 9520 9525
   Lys Val Pro Glu Thr Lys Lys Glu Lys Glu Lys Asp Ser Gln Pro
   9530 9535 9540
   Lys Lys Glu Lys Ala Lys Glu Thr Ser Arg Pro Pro Lys Lys Glu
   9545 9550 9555
   Lys Glu Lys Gly Lys
   9560
<210> 57
   <211> 133
   <212> PRT
   <213> Plasmodium falciparum
<400> 57
   Ile Pro Glu Glu Gln Ile Glu Glu Val Ile Gln Glu Glu Ile Ile Glu
   1               5                   10                  15
   Gln Val Val Pro Glu Glu Leu Ile Glu Glu Val Val Pro Glu Glu Ile
               20                  25                  30
   Ile Glu Glu Val Ile Pro Glu Glu Ile Val Glu Glu Val Ile Tyr Glu
           35                  40                  45
   Glu Val Ile Pro Glu Glu Leu Val Glu Glu Val Ile Ala Glu Lys Leu
       50                  55                  60
   Val Lys Glu Ile Val Pro Glu Gln Val Arg Glu Glu Val Thr Leu Glu
   65                  70                  75                  80
   Glu Ile Val Glu Glu Met Ile Pro Glu Glu Phe Val Glu Glu Val Ala
                   85                  90                  95
   Pro Glu Val Glu Ile Glu Glu Ile Ile Pro Glu Glu Leu Ile Glu Glu
               100                 105                 110
   Val Ile Pro Glu Val Leu Val Glu Glu Ala Val Pro Glu Glu Leu Ile
           115                 120                 125
   Glu Lys Val Ile Pro
       130
<210> 58
   <211> 6093
   <212> PRT
   <213> Plasmodium falciparum
<400> 58
   Met Ser Asn Ile Asn Asn Lys Asp Ser Ser Thr Glu Trp Asn Cys Lys
   1               5                   10                  15
   Glu Asp Val Gly Cys Val Pro Pro Arg Arg Gln Asn Leu Asn Met Glu
               20                  25                  30
   Arg Leu Asp Asn Glu Asn Glu Asp Ser Val Pro Asp Phe Met Lys Lys
           35                  40                  45
   Thr Phe Tyr Leu Ala Ala Ala Gly Glu Gly Lys Lys Leu Arg Glu Lys
       50                  55                  60
   His Asp Glu Ser Cys Asp Glu Phe Cys Asp Ala Trp Asn Arg Ser Leu
   65 70 75 80
   Ala Asp Tyr Lys Asp Ile Phe Gln Gly Lys Asp Met Trp Asn Asp Gly
   85 90 95
   Lys Tyr Gly Glu Ala Lys Asn His Ile Lys Asn Ala Phe Gly Asp Met
   100 105 110
   Asn Asn Arg Lys Thr Met Leu Asn Glu Ile Glu Lys Gly Ile Lys Asp
   115 120 125
   Glu Thr Phe Ser Arg Glu Asn Gly Leu Asp Val Cys Lys Ser Gln Cys
   130 135 140
   Glu Glu Arg Ser Arg Asp Asp Thr Glu Asp Gln Phe Leu Arg Phe Phe
   145 150 155 160
   Ala Glu Trp Glu Glu Glu Phe Cys Asp Gly Leu Asn Lys His Glu Glu
   165 170 175
   Gln Leu Lys Ser Cys Thr Lys Asp Ile Asn Cys Asp Ile Lys Cys Ser
   180 185 190
   Asn Phe Lys Asp Trp Leu Glu Thr Lys Lys Asp Glu Tyr Asp Ile Gln
   195 200 205
   Ser Arg Val Phe Glu Lys Lys Tyr Ala Asn Asp Asn Lys Ser Lys His
   210 215 220
   Leu Asn Tyr Leu Lys Glu Gly Met Asn Lys Cys Lys Val Lys Asn Pro
   225 230 235 240
   Glu Met Val Phe Lys Ser Gly Phe Ala Asn Val Ala Glu Cys Arg Asn
   245 250 255
   Leu Asn Val Glu Gly Ala Gly Asn Lys Asn Ser Asn Asn Leu Lys Asp
   260 265 270
   Leu Asp Ser Asn Ser Asp Lys Asp Gly Ile Val Ser Glu Ser Tyr Lys
   275 280 285
   Ala Thr Lys Lys Asn Gly Glu Ser Ile Met Asp Arg Ile Pro Lys Ser
   290 295 300
   Phe Asn Lys Leu Phe Gly Tyr Phe Ser Gly Ser Gln Glu Glu Glu Gln
   305 310 315 320
   Lys Glu Asn Asp Val Ser His Arg Asn Asn Tyr Asp Asn Ile Leu Val
   325 330 335
   Asp Lys Phe His Arg Ser Ser Leu Leu Asp Lys Leu Asp Asp Arg Met
   340 345 350
   Phe Phe Asp Glu Leu Asn Arg Asp Asn Ile Met Glu Glu Val Leu Ser
   355 360 365
   Lys Ile Pro Glu Pro Ile Ile Arg Glu Ala Pro Lys Tyr Val Pro Lys
   370 375 380
   Lys Pro Val Pro Pro Gln His Ile Pro Arg Gly Asp Asn Val Pro Arg
   385 390 395 400
   Asn Ile Asp Val Asn Gly Ser Lys Asp Glu Tyr Ser Pro Glu Thr Glu
   405 410 415
   Ser Ala Asn Ser Lys Ile Lys Pro Thr Tyr Glu Glu Asn Asp Glu Asp
   420 425 430
   Lys Ser Lys Ile Ser Ile Glu Thr Ser Glu Ile Asp Arg Asp Lys Glu
   435 440 445
   Pro Phe Arg Ile Ser Glu Glu Lys Lys Val Val Lys Glu Asp Val Gln
   450 455 460
   Glu Leu Glu Asn Ile Glu Tyr Glu Phe Asp Glu Thr Phe Asp Phe Phe
   465 470 475 480
   Asp Glu Asp Ala Lys Arg Asn Ile Asp Asp Ile Arg Lys Asp Ile Gln
   485 490 495
   Ser Gln Ile Met Lys Ser Val Glu Asn Tyr Asn Ser Glu Lys Glu Glu
   500 505 510
   Phe Lys Arg Asn Ile Glu Thr Gln Leu Ile Glu Ser Gly Asp Gly Met
   515 520 525
   Asn Ala Gly Asn Tyr Ser Ser Ala Leu Gln Asp Asn Ser Thr Glu Ile
   530 535 540
   Pro Thr Met Val Leu Val Pro Gly Val Leu Thr Val Phe Leu Leu Thr
   545 550 555 560
   Ile Ile Trp Val Leu Val Tyr Lys His Ser Leu Ile Asp Arg Val His
   565 570 575
   Gly Thr Gly Lys Thr Lys Lys Glu Glu Lys Met Lys Glu Leu Glu Ser
   580 585 590
   Glu Glu Phe Pro Lys Glu Lys Tyr Asn Ile Glu Asp Met Glu Glu Thr
   595 600 605
   Glu Lys Glu Asn Glu Ile Glu Lys Ile Met Asp Glu Asn Lys Glu Thr
   610 615 620
   Glu Gln Thr Glu Glu Gly Asn Thr Glu Glu Phe Val Gln Glu Lys Glu
   625 630 635 640
   Leu Asn Gln Glu Thr Leu Glu Asn Glu Ile Ile Val Asp His Ile Lys
   645 650 655
   Glu Glu Glu Asp Thr Arg Asn Val Lys Glu Gln Glu Ser Val Leu Glu
   660 665 670
   Glu Ser Pro Ile Glu Glu Leu Pro Val Glu Asn Asn Ile Gly Lys Ile
   675 680 685
   Asn Glu Glu Val Glu Glu Phe Ile Leu Asn Glu Ile Pro Leu Glu Glu
   690 695 700
   Gln Val Pro Lys Glu Leu Pro Gln Glu Glu Ile Glu Glu Ile Val Val
   705 710 715 720
   Glu Glu Leu Pro Ile Asp Glu His Leu Ser Ser Glu Glu Thr Thr Val
   725 730 735
   Thr Glu Glu Asp Thr Phe Lys Gly Gln Leu Ile Asn Glu Glu Lys Pro
   740 745 750
   Val Glu Glu Lys Ser Val Ser Glu Glu Ile Pro Val Glu Glu Lys Ser
   755 760 765
   Val Ser Glu Glu Ile Pro Val Glu Glu Lys Ser Val Ser Glu Glu Ile
   770 775 780
   Pro Val Glu Glu Lys Ser Val Ser Glu Glu Ile Pro Val Glu Glu Lys
   785 790 795 800
   Asn Val Ser Glu Glu Ile Pro Val Glu Glu Lys Asn Val Ser Glu Glu
   805 810 815
   Ile Pro Val Glu Glu Lys Asn Val Ser Glu Glu Ile Pro Val Glu Glu
               820                 825                 830
   Glu Asn Val Ser Glu Glu Ile Pro Glu Gly Gly Ile Ala Ile Glu Asp
           835                 840                 845
   Val Pro Val Asp Glu Glu Thr Val Thr Glu Glu Ile Thr Val Asp Glu
       850                 855                 860
   Lys Ile Tyr Asp Lys Leu Pro Asn Glu Ile Glu Thr Val Asn Glu Glu
   865                 870                 875                 880
   Met Pro Val Glu Asp Glu Thr Leu Thr Glu Gln Ile Ser Ser Glu His
                   885                 890                 895
   Glu Arg Val Pro Glu Glu Ile Ile Glu Glu Lys Pro Phe Thr Glu Gly
               900                 905                 910
   Glu Glu Thr Glu Ser Leu Thr Asp Glu Ile Val Glu Glu Gly Val Val
           915                 920                 925
   Thr Asp Asp Ile Pro Glu Glu Gln Ile Ile Thr Glu Lys Val Gln Glu
       930                 935                 940
   Glu Glu Glu Phe Val Thr Gly Glu Leu Ser Glu Glu Asp Ile Ile Asn
   945                 950                 955                 960
   Glu Lys Val Gln Glu Glu Asp Glu Ser Val Thr Glu Glu Leu Pro Glu
                   965                 970                 975
   Glu Asp Ile Ile Asn Glu Lys Val Gln Glu Glu Glu Glu Ser Ala Tyr
               980                 985                 990
   Gln Glu Ile Val Gln Asp Gly Ser  Val Thr Lys Asp Val  Glu Tyr Lys
           995                 1000                1005
   Glu Leu  Val Asn Asp Asp Val  Arg Asp Lys Glu Asn  Phe Val Ile
       1010                1015                1020
   Glu Glu  Asp Pro Phe Lys Gly  Gln Leu Ile Asn Glu  Gly Leu Pro
       1025                1030                1035
   Val Glu  Glu Glu Phe Val Thr  Lys Glu Leu Pro Val  Lys Glu Glu
       1040                1045                1050
   Ser Val  Phe Glu Glu Leu Thr  Glu Glu Asp Gln Ser  Val Thr Lys
       1055                1060                1065
   Glu Ile  Pro Val Glu Glu His  Ser Val Phe Lys Glu  Val Asp Glu
       1070                 1075                 1080
   Ile Glu  Ser Val Thr Asp Glu  Ile Val Glu Glu Glu  Gly Ser Val
       1085                 1090                 1095
   Asn Glu  Glu Val Glu Glu Glu  Val Ser Val Ser Gly  Glu Val Asp
       1100                 1105                 1110
   Glu Thr  Glu Tyr Val Thr Glu  Glu Val Glu Glu Thr  Glu Leu Val
       1115                 1120                 1125
   Asn Glu  Glu Val Leu Lys Glu  Glu Gly Ser Ala Ser  Glu Lys Val
       1130                 1135                 1140
   Val Lys  Glu Glu Gly Ser Ala  Ser Glu Lys Ile Val  Glu Glu Glu
       1145                 1150                 1155
   Gly Ser  Val Thr Glu Glu Thr  Ala Ser Glu Glu Ile  Val Glu Glu
       1160                 1165                 1170
   Glu Gly  Ser Val Thr Glu Glu  Ser Ser Ser Glu Glu  Ile Val Glu
       1175                 1180                 1185
   Asp Glu  Gly Ser Val Thr Glu  Glu Ser Ser Ser Glu  Glu Ile Val
       1190                 1195                 1200
   Glu Glu  Glu Gly Ser Val Ile  Glu Glu Ser Ala Ser  Glu Val Ile
       1205                 1210                 1215
   Val Glu  Asp Glu Val Ser Ala  Ser Lys Glu Ile Val  Glu Asp Glu
       1220                 1225                 1230
   Ala Ser  Val Thr Glu Glu Val  Val Glu Glu Glu Gly  Ala Val Ser
       1235                 1240                 1245
   Asp Glu  Val Gln Val Thr Glu  Ser Val Glu Asp Glu  Ile Ile Asn
       1250                 1255                 1260
   Gln Gly  Ile Val Asp Glu Val  Ile Val Glu Gln Glu  Ala Ser Val
       1265                 1270                 1275
   Thr Asp  Glu Ile Val Lys Glu  Asp Glu Ser Val Ile  Glu Glu Ile
       1280                 1285                 1290
   Ala Val  Glu Glu Thr Val Thr  Glu Val Val Glu Glu  Thr Lys Pro
   1295 1300 1305
   Met Asp Glu Glu Ile Val Asp Gln Gly Ser Val Val Glu Asn Val
   1310 1315 1320
   Glu Glu Lys Lys Thr Met Asp Glu Glu Ile Val Asp Gln Gly Ser
   1325 1330 1335
   Val Val Glu Asn Val Glu Glu Lys Lys Thr Met Asp Glu Glu Ile
   1340 1345 1350
   Gly Asp Gln Gly Ser Val Ala Glu Lys Val Glu Glu Glu Glu Leu
   1355 1360 1365
   Val Thr Glu Glu Val Ile Glu Arg Glu Gly Ser Ile Asn Glu Asp
   1370 1375 1380
   Ile Val Lys Glu Ala Ser Ile Thr Glu Glu Val Glu Gln Ile Glu
   1385 1390 1395
   Pro Val Thr Gly Lys Val Glu Lys Ile Glu Ser Ile Thr Asp Glu
   1400 1405 1410
   Ile Lys Glu Gln Leu Val Pro Glu Asp Ile Lys Glu Glu Gln Leu
   1415 1420 1425
   Asp Phe Glu Glu Ile Val Ala Gln Arg Ala Ser Val Asn Asp Glu
   1430 1435 1440
   Lys Val Glu Val Ala Ser Ile Thr Glu Glu Val Glu Glu Glu Glu
   1445 1450 1455
   Lys Ser Val Ser Glu Glu Val Leu Glu Glu Glu Gly Ser Thr Thr
   1460 1465 1470
   Glu Lys Val Val Lys Gly Ser Ser Thr Glu Val Val Val Glu Glu
   1475 1480 1485
   Gln Gly Ser Val Thr Glu Asn Leu Leu Glu Glu Glu Ser Ala Ser
   1490 1495 1500
   Gln Gly Ile Val Glu Lys Glu Glu Phe Val Asp Glu Glu Asp Ser
   1505 1510 1515
   Val Lys Asp Gln Asn Val Phe Glu Lys Glu Gly Ser Val Thr Glu
   1520 1525 1530
   Gln Leu  Val Glu Glu Glu Lys  Gly Leu Ile Asn Glu  Asp Asn Glu
       1535                 1540                 1545
   Lys Glu  Glu Leu Ile Thr Glu  Met Ser Glu Glu Ile  Lys Ser Val
       1550                 1555                 1560
   Asn Glu  Glu Ile Glu Glu Thr  Asp Leu Ser Thr Glu  Glu Ile Ile
       1565                 1570                 1575
   Lys Gln  Gln Gly Leu Ala Thr  Tyr Glu Phe Val Glu  Glu Glu Lys
       1580                 1585                 1590
   Ser Leu  Thr Asp Lys Leu Leu  Glu Glu Glu Ser Val  Thr Lys Glu
       1595                 1600                 1605
   Val Gly  Glu Thr Glu Leu Ser  Thr Gln Glu Val Val  Asp Glu Lys
       1610                 1615                 1620
   Val Ser  Val Thr Glu Glu Val  Ile Glu Glu Glu Lys  Ser Val Ser
       1625                 1630                 1635
   Glu Glu  Val Leu Glu Glu Gly  Ser Ala Thr Glu Glu  Val Val Glu
       1640                 1645                 1650
   Glu Gly  Ser Cys Thr Glu Val  Val Val Glu Glu Gly  Ser Cys Thr
       1655                 1660                 1665
   Glu Val  Val Val Glu Glu Gly  Ser Cys Thr Glu Ile  Val Val Glu
       1670                 1675                 1680
   Lys Gly  Ser Asp Thr Glu Ile  Val Val Glu Glu Gly  Ser Ala Thr
       1685                 1690                 1695
   Glu Ile  Val Val Glu Glu Gly  Ser Ala Thr Glu Val  Val Val Glu
       1700                 1705                 1710
   Glu Gly  Ser Ala Thr Glu Glu  Val Val Glu Glu Gly  Ser Val Ser
       1715                 1720                 1725
   Glu Glu  Met Leu Glu Glu Glu  Gly Ser Ala Thr Glu  Glu Val Val
       1730                 1735                 1740
   Glu Glu  Gly Leu Ser Ser Asp  Asn Val Gln Lys Ser  Lys Gly Val
       1745                 1750                 1755
   Ile Glu  Asn Val Gly Glu Ile  Tyr Ser Val Lys Thr  Ala Lys Asp
       1760                 1765                 1770
   Glu Ser Met Asn Glu Lys Ile Pro Leu Glu Lys Ser Ser Phe Val
   1775 1780 1785
   Asp Asp Glu Ser Phe Lys Gly Gln Gly Pro Thr Asp Asn Val Ser
   1790 1795 1800
   Val Glu Asp Val Asn Ser Glu Asp Ile Ile Asn Glu His Thr Pro
   1805 1810 1815
   Leu Glu Glu Thr Lys Ile Glu Glu Leu Pro Thr Glu Tyr Ile Thr
   1820 1825 1830
   Thr Ala Asp Ile His Thr Lys Gly Glu Thr Glu Thr Lys Tyr Asn
   1835 1840 1845
   Leu Ile Tyr Glu Lys Ile Asn Glu Glu Val Glu Lys Ala Lys Phe
   1850 1855 1860
   Gln Glu Glu Lys Ile Thr Glu Asn Ile Pro Val Glu Arg Glu Ser
   1865 1870 1875
   Val Thr Glu Asp Ile Val Gln Glu Pro Ser Leu Ala Gln Glu Val
   1880 1885 1890
   Glu Gln Lys Glu Ser Asp Thr Asn Glu Ile Glu Glu Thr Lys Leu
   1895 1900 1905
   Ala Asn Glu Lys Ile Ile Pro Glu Val Ser Val Thr Glu Asn Val
   1910 1915 1920
   Val Glu Lys Glu Gly Leu Asp Thr Glu Glu Val Leu Glu Glu Asp
   1925 1930 1935
   Glu Ser Ile Thr Glu Glu Ile Val Glu Glu Glu Val Ser Ser Ser
   1940 1945 1950
   Glu Glu Ile Val Glu Glu Glu Glu Ser Ser Ser Glu Glu Ile Val
   1955 1960 1965
   Glu Glu Glu Glu Ser Ser Ser Glu Glu Ile Val Glu Glu Glu Glu
   1970 1975 1980
   Ser Ser Ser Glu Glu Ile Val Glu Glu Glu Glu Ser Ser Ser Glu
   1985 1990 1995
   Glu Ile Val Glu Glu Glu Glu Ser Ser Ser Glu Glu Ile Ile Glu
   2000 2005 2010
   Glu Val Ser Ser Thr Glu Glu Val Leu Glu Glu Glu Gly Ser Val
   2015 2020 2025
   Thr Glu Glu Ile Val Glu Glu Glu Val Ser Thr Thr Glu Glu Val
   2030 2035 2040
   Lys Asp Ile Gly Ser Val Ser Glu Glu Val Leu Glu Glu Glu Gly
   2045 2050 2055
   Phe Gly Thr Glu Glu Phe Val Gly Gln Gln Gly Ser Val Ile Glu
   2060 2065 2070
   Glu Ile Val Glu Thr Glu Ser Ser Thr Glu Lys Val Leu Glu Asp
   2075 2080 2085
   Val Gly Ser Asn Val Glu Glu Ile Val Gln Glu Glu Gly Pro Val
   2090 2095 2100
   Ala Gln Glu Ile Val His Glu Glu Val Ser Thr Thr Glu Lys His
   2105 2110 2115
   Asp Glu Val Asp Arg Ser Thr Thr Glu Glu Ile Val Glu Lys Val
   2120 2125 2130
   Gly Ser Val Ser Glu Glu Ile Ile Val Glu Glu Val Ser Ala Ser
   2135 2140 2145
   Glu Glu Ile Val Glu Glu Gly Ser Val Thr Glu Glu Val Val Glu
   2150 2155 2160
   Glu Glu Lys Leu Ile Asn Glu Val Gly Glu Thr Glu Ser Val Thr
   2165 2170 2175
   Glu Glu Ile Val Gln Lys Glu Val Ser Asp Ala Glu Glu Val Leu
   2180 2185 2190
   Gly Gln Glu Gly Ser Met Asn Glu Glu Ile Leu Glu Lys Glu Ser
   2195 2200 2205
   Ile Val Glu Glu Ile Val Gly Pro Glu Gly Ser Val Thr Glu Glu
   2210 2215 2220
   Ile Val Asp His Gly Ser Phe Ala Glu Glu Val Lys Glu Glu Glu
   2225 2230 2235
   Leu Val Thr Glu Glu Ala Val Gln Tyr Glu Gly Ser Val Thr Glu
   2240 2245 2250
   Glu Ile Lys Glu Glu Glu Ser Ile Thr Glu Asn Glu Ala Ile Glu
   2255 2260 2265
   Glu Ser Ala Phe Ala Glu Ile Ile Glu Glu Lys Gly Pro Asn Thr
   2270 2275 2280
   Asp Glu Ile Val Lys Glu Glu Gly Leu Asp Thr Glu Glu Ile Val
   2285 2290 2295
   Asn Glu Val Ser Val Thr Asp Glu Val Ile Glu Glu Glu Lys Leu
   2300 2305 2310
   Val Asn Glu Gln Ile Val Gly Glu Glu Arg Ser Val Thr Glu Lys
   2315 2320 2325
   Pro Val Glu Val Glu Arg Ser Ala Thr Glu Asp Leu Val Glu Glu
   2330 2335 2340
   Glu Ala Ser Val Thr Glu Lys Val Ser Val His Glu Gly Ser Thr
   2345 2350 2355
   Thr Glu Gln Ile Leu Asp Glu Ser Val Ala Glu Glu Ile Val Glu
   2360 2365 2370
   Glu Glu Val Ser Val Asp Asp Lys Ile Ile Glu Glu Glu Val Ser
   2375 2380 2385
   Val Asp Glu Val Val Glu Glu Glu Gly Ser Val Ile Glu Glu Ile
   2390 2395 2400
   Val Glu Glu Glu Glu Ser Val Pro Glu Glu Ile Leu Glu Glu Glu
   2405 2410 2415
   Leu Ser Gly Ser Glu Glu Val Leu Glu Asp Glu Trp Val Thr Asp
   2420 2425 2430
   Ala Phe Met Gly Gln Glu Gly Ser Val Ile Glu Glu Ile Glu Glu
   2435 2440 2445
   Ile Val Asp Gly Glu Gly Ser Ile Thr Glu Glu Ile Val Glu Asp
   2450 2455 2460
   Gly Ser Ala Asn Glu Lys Ile Val Glu Glu Glu Pro Ser Arg Val
   2465 2470 2475
   Glu Glu  Val Leu Gly Lys Glu  Gly Phe Val Ile Glu  Glu Ile Ile
       2480                 2485                 2490
   Glu Glu  Gly Ser Val Ile Glu  Gln Val Glu Asp Thr  Lys Thr Val
       2495                 2500                 2505
   Ser Glu  Lys Ser Glu Glu Ser  Ser Ala Ile Glu Glu  Val Lys Glu
       2510                 2515                 2520
   Val Lys  Glu Glu Glu Ser Ile  Ser Glu Lys Ile Val  Glu Lys Glu
       2525                 2530                 2535
   Glu Ser  Val Thr Glu Glu Ile  Val Arg Gln Glu Glu  Ser Thr Thr
       2540                 2545                 2550
   Glu Lys  Ile Val Lys Asp Val  Ser Pro Thr Glu Asp  Phe Val Glu
       2555                 2560                 2565
   Gln Thr  Asp Ser Val Thr Glu  Lys Val Ile Glu Gln  Glu Gly Ser
       2570                 2575                 2580
   Asn Thr  Glu Val Ala Glu Asp  Val Glu Glu Lys Glu  Ser Ala Ser
       2585                 2590                 2595
   Asp Glu  His Glu Gln Glu Asp  Val Ser Val Asn Ala  Gln Val Thr
       2600                 2605                 2610
   Tyr Glu  Lys Lys Ser Val Thr  Lys Glu Ile Val Asp  Glu Val Ser
       2615                 2620                 2625
   Arg Thr  Glu Glu Ile Val Glu  Glu Asn Gly Ser Val  Thr Glu Gly
       2630                 2635                 2640
   Val Asp  Glu Thr Gly Ser Val  Thr Glu Glu Ile Ile  Glu Glu Ala
       2645                 2650                 2655
   Thr Val  Thr Glu Glu Val Val  Glu Asp Gly Ser Val  Thr Glu Glu
       2660                 2665                 2670
   Val Val  Glu Asp Gly Ser Val  Ile Gln Glu Val Val  Glu Asp Gly
       2675                 2680                 2685
   Ser Val  Thr Glu Glu Ile Val  Gln Glu Asn Gly Ser  Val Thr Glu
       2690                 2695                 2700
   Glu Ile  Val Glu Glu Glu Gly  Ser Val Asn Glu Glu  Val Glu Glu
       2705                 2710                 2715
   Glu Val Ser Val Ser Gly Glu Val Asp Glu Thr Glu Tyr Val Thr
   2720 2725 2730
   Glu Glu Val Glu Glu Glu Gly Ser Val Val Glu Glu Ile Val Glu
   2735 2740 2745
   Glu Glu Gly Ser Val Ser Gly Glu Val Asp Glu Thr Glu Tyr Val
   2750 2755 2760
   Thr Glu Glu Val Glu Glu Glu Gly Ser Val Val Glu Glu Ile Val
   2765 2770 2775
   Glu Glu Glu Gly Ser Val Ser Gly Glu Val Asp Glu Thr Glu Tyr
   2780 2785 2790
   Val Thr Glu Glu Val Glu Glu Glu Gly Ser Val Val Glu Glu Ile
   2795 2800 2805
   Val Glu Glu Glu Gly Ser Val Ser Gly Glu Val Asp Glu Thr Glu
   2810 2815 2820
   Tyr Val Thr Glu Glu Val Glu Glu Glu Gly Ser Val Val Glu Glu
   2825 2830 2835
   Ile Val Glu Glu Glu Gly Ser Val Val Glu Glu Ile Val Glu Glu
   2840 2845 2850
   Glu Gly Ser Val Ser Glu Val Val Asp Glu Thr Glu Leu Val Asn
   2855 2860 2865
   Asp Glu Ile Val Glu Gln Ala Pro Phe Thr Glu Glu Val Glu Glu
   2870 2875 2880
   Gln Val Ser Val Asn Asp Glu Ile Ile Glu Asp Ala Ser Val Ala
   2885 2890 2895
   Glu Ala Val Glu Glu Ser Glu Ser Ile Thr Glu Ser Val Ser Gln
   2900 2905 2910
   Glu Glu Glu Thr Glu Lys Gly Phe Val Ile Glu Lys Val Glu Glu
   2915 2920 2925
   Thr Gly Ala Val Thr Glu Glu Ile Val Gln Asp Gly Leu Ile Thr
   2930 2935 2940
   Glu Glu Ile Leu Glu Glu Ser Glu Ser Val Asn Gly Glu Ile Ile
   2945 2950 2955
   Asn Lys Glu Ser Asp Ala Glu Glu Ile Leu Glu Thr Glu Phe Leu
       2960                2965                2970
   Thr Glu Glu Val Val Gly Gln Ala Gly Ser Thr Ser Glu Glu Ile
       2975                2980                2985
   Val Glu Glu Glu Gly Ser Val Thr Lys Glu Val Glu Glu Lys Glu
       2990                2995                3000
   Ser Val Thr Glu Glu Leu Val Asp Glu Gly Ser Val Thr Glu Glu
       3005                3010                3015
   Leu Val Asp Glu Gly Ser Val Thr Glu Glu Val Val Glu Gln Gly
       3020                3025                3030
   Gly Ser Ile Ala Gln Glu Ile Val Glu Glu Glu Ser Ala Thr Glu
       3035                3040                3045
   Glu Ile Ile Arg Asp Glu Thr Asn Val Glu Glu Val Leu Glu Lys
       3050                3055                3060
   Glu Gly Ser Ala Thr Glu Glu Ile Val Gln Asp Gly Ser Gly Thr
       3065                3070                3075
   Asn Asp Phe Val Gly Lys Gln Gly Ser Val Ile Glu Glu Val Val
       3080                3085                3090
   Glu Glu Glu Ile Ser Thr Thr Glu Glu Lys Leu Lys Glu Glu Ala
       3095                3100                3105
   Ser Ala Ile Glu Glu Phe Val Glu Glu Glu Ser Ile Arg Glu Asp
       3110                3115                3120
   Val Leu Glu Glu Ser Leu Val Thr Glu Asn Val Val Gly Gln Gln
       3125                3130                3135
   Glu Ser Val Thr Glu Glu Ile Val Asp Gly Glu Gly Ser Phe Thr
       3140                3145                3150
   Glu Asp Ile Val Glu Glu Glu Glu Ser Val Thr Glu Glu Ile Val
       3155                3160                3165
   Val Asp Glu Glu Ser Val Thr Lys Glu Ile Val Glu Asp Glu Glu
       3170                3175                3180
   Leu Val Thr Glu Glu Ile Val Glu Asp Glu Gly Ser Phe Thr Glu
   3185 3190 3195
   Glu Ile Val Glu Asp Glu Gly Ser Phe Thr Glu Glu Val Ile Glu
   3200 3205 3210
   Glu Arg Ser Leu Ile Glu Glu Val Glu Asp Thr Glu Thr Val Ala
   3215 3220 3225
   Glu Lys Glu Glu Gly Ser Val Ile Lys Glu Ile Ile Asp Glu Lys
   3230 3235 3240
   Ser Leu Thr Glu Lys Ile Val Glu Glu Glu Lys Ser Val Thr Glu
   3245 3250 3255
   Glu Val Glu Glu Lys Glu Ser Val Lys Glu Glu Val Glu Glu Gln
   3260 3265 3270
   Arg Leu Val Val Glu Glu Glu Gly Ser Ala Thr Glu Gly Ile Val
   3275 3280 3285
   Glu Asp Arg Leu Ala Thr Glu Gly Ile Val Asp Asp Ile Leu Val
   3290 3295 3300
   Thr Glu Glu Ile Val Glu Asp Gly Leu Ala Thr Asp Glu Phe Val
   3305 3310 3315
   Glu Gln Gln Gly Ser Ile Ile Glu Glu Val Leu Asp Asp Glu Gly
   3320 3325 3330
   Ser Val Thr Glu Glu Ile Val Glu Glu Glu Gly Ser Pro Asn Glu
   3335 3340 3345
   Glu Ile Val Glu Gly Val Ser Val Ile Glu Glu Asp Asp Asn Ile
   3350 3355 3360
   Glu Pro Val Ser Glu Glu Ile Val Glu Gly Ser Val Thr Glu Glu
   3365 3370 3375
   Met Ile Lys Glu Gly Leu Glu Asn Glu Val Ile Leu Asp Glu Asp
   3380 3385 3390
   Ser Ile Thr Glu Glu Ala Leu Glu Lys Glu Gly Ser Val Ser Glu
   3395 3400 3405
   Glu Ile Val Glu Glu Met Gly Ser Leu Thr Glu Glu Ile Val Asp
   3410 3415 3420
   Glu Glu Arg Ser Thr Ser Glu Asp Met Ile Glu Glu Gly Ser Ala
   3425 3430 3435
   Ser Glu Glu Ile Ile Gln Glu Glu Ser Gln Val Glu Glu Val Val
   3440 3445 3450
   Glu Glu Val Ser Val Ile Asp Glu Ile Val Glu Glu Asp Glu Leu
   3455 3460 3465
   Asp Thr Lys Glu Val Val Glu Glu Ile Glu Phe Asn Thr Glu Glu
   3470 3475 3480
   Val Val Glu His Lys Glu Glu Glu Gly Ser Val Ala Glu Glu Ile
   3485 3490 3495
   Val Gln Glu Glu Lys Glu Gly Ser Val Asn Glu Glu Ile Ile Glu
   3500 3505 3510
   Glu Val Gly Ser Ile Thr Glu Glu Met Val Glu Gln Asp Val Ser
   3515 3520 3525
   Asp Asn Glu Glu Ile Val Glu Glu Arg Ser Val Ile Glu Glu Ala
   3530 3535 3540
   Glu Glu Asn Val Trp Ile Glu Lys Glu Val Glu Glu Glu Gly Leu
   3545 3550 3555
   Asp Asn Glu Glu Val Ile Asp Glu Glu Asp Ser Val Ser Glu Gln
   3560 3565 3570
   Ala Glu Glu Glu Val Tyr Ile Asn Glu Glu Ile Leu Lys Glu Ser
   3575 3580 3585
   Ser Asp Val Glu Asp Val Lys Val Glu Asn Glu Leu Met Asn Glu
   3590 3595 3600
   Glu Val Asn Glu Glu Thr Gln Ser Val Ala Glu Asn Asn Glu Glu
   3605 3610 3615
   Asp Lys Glu Leu Asp Asn Tyr Val Val Glu Glu Thr Glu Ser Val
   3620 3625 3630
   Thr Glu Glu Val Val Val Asp Glu Val Pro Asn Ser Lys Glu Val
   3635 3640 3645
   Gln Glu Ile Glu Ser Ile Ile Glu Glu Ile Val Glu Asp Gly Leu
   3650 3655 3660
   Thr Thr Asp Asp Leu Val Gly Gln Gln Gly Ser Val Ile Glu Glu
   3665 3670 3675
   Val Val Glu Glu Val Gly Ser Asp Ser Glu Glu Ile Val Glu Glu
   3680 3685 3690
   Ala Ser Ile Thr Glu Glu Val Glu Lys Lys Glu Ser Val Thr Glu
   3695 3700 3705
   Asp Ile Leu Val Glu Glu Ser Val Thr Gly Asp Ile Leu Val Glu
   3710 3715 3720
   Gly Ser Val Thr Glu Glu Val Val Gly Glu Glu Lys Leu Val Ser
   3725 3730 3735
   Glu Glu Ile Val Thr Glu Glu Gly Ser Val Ala Gln Glu Ile Val
   3740 3745 3750
   Glu Glu Asp Ala Pro Ala Thr Glu Glu Ile Asp Glu Ile Glu Ser
   3755 3760 3765
   Val Thr Glu Glu Val Val Glu Glu Glu Gly Pro Val Asp Glu Glu
   3770 3775 3780
   Ile Val Gln Glu Glu Gly Ser Val Thr Glu Glu Ile Ile Gln Gly
   3785 3790 3795
   Glu Ser Lys Val Glu Glu Val Val Glu Glu Gln Gly Ser Glu Asn
   3800 3805 3810
   Glu Glu Ile Phe Val Glu Glu Val Ser Ala Ser Gln Glu Ile Val
   3815 3820 3825
   Gln Asn Glu Ser Gly Thr Glu Glu Ile Leu Glu Lys Val Ser Ala
   3830 3835 3840
   Ser Gln Glu Ile Val Gln Asp Gly Ser Val Thr Glu Gln Ile Ile
   3845 3850 3855
   Glu Glu Gln Lys Pro Val Thr Glu Glu Val Val Asn Glu Glu Glu
   3860 3865 3870
   Ser Ile Thr His Glu Ile Ile Gln Glu Glu Ser His Val Glu Lys
   3875 3880 3885
   Val Val Gln Gln Gly Ser Val Ala Glu Glu Val Val Glu Asn Pro
   3890 3895 3900
   Val Ser Val Thr Glu Glu Ile Val Glu Lys Glu Gly Ser Val Thr
   3905 3910 3915
   Glu Asp Ile Gly Gln Glu Gly Tyr Val Ala Glu Glu Ile Val Glu
   3920 3925 3930
   Glu Glu Glu Phe Asp Asn Glu Glu Ile Leu Glu Glu Glu Ser Val
   3935 3940 3945
   Ala Glu Glu Phe Val Glu Glu Gly Phe Asp Asn Glu Glu Ile Phe
   3950 3955 3960
   Val Glu Gln Ile Ser Asp Ser Glu Ile Val Lys Glu Glu Asn Ser
   3965 3970 3975
   Val Asn Glu Glu Val Leu Glu Glu Glu Gly Ser Tyr Thr Glu Glu
   3980 3985 3990
   Ile Leu Glu Glu Glu Gly Ser Tyr Thr Glu Glu Ile Leu Glu Glu
   3995 4000 4005
   Glu Gly Ser Phe Thr Glu Glu Ile Leu Glu Glu Glu Gly Ser Tyr
   4010 4015 4020
   Asn Glu Glu Ile Leu Glu Glu Glu Gly Ser Tyr Asn Glu Glu Ile
   4025 4030 4035
   Leu Glu Glu Glu Gly Ser Tyr Asn Glu Glu Ile Leu Glu Glu Glu
   4040 4045 4050
   Gly Ser Tyr Asn Glu Glu Ile Leu Glu Glu Glu Gly Ser Ala Thr
   4055 4060 4065
   Asp Tyr Phe Val Gly Gln Gly Ser Asp Asn Glu Glu Ile Ile Glu
   4070 4075 4080
   Glu Gly Ser Ala Thr Asp Tyr Phe Val Gly Gln Gly Ser Asp Asn
   4085 4090 4095
   Glu Glu Ile Ile Glu Glu Gly Ser Ala Thr Asp Tyr Phe Val Gly
   4100 4105 4110
   Gln Gly Ser Ile Ile Glu Glu Val Leu Glu Glu Glu Gly Leu Asp
   4115 4120 4125
   Thr Glu Lys Val Phe Glu Asn Glu Gly Ser Ala Thr Glu Phe Glu
   4130 4135 4140
   Glu Thr Glu Ser Phe Thr Glu Val Val Glu Glu Thr Glu Ser Val
   4145 4150 4155
   Asn Glu Asn Ile Leu Glu Glu Thr Ser Ile Asn Glu Val Gln Lys
   4160 4165 4170
   Ile Glu Ser Ile Thr Glu Asp Ile Lys Glu Gln Leu Val Pro Glu
   4175 4180 4185
   Glu Ile Lys Glu Glu Gln Leu Asp Ser Glu Glu Ile Lys Glu Glu
   4190 4195 4200
   Gln Leu Asp Ser Glu Glu Ile Lys Glu Glu Gln Leu Val Pro Glu
   4205 4210 4215
   Glu Ile Lys Glu Glu Gln Leu Asp Ser Glu Glu Ile Lys Glu Glu
   4220 4225 4230
   Gln Leu Asp Ser Glu Glu Ile Lys Glu Glu Gln Leu Asp Ser Glu
   4235 4240 4245
   Glu Ile Lys Glu Glu Gln Leu Asp Ser Glu Glu Ile Lys Glu Glu
   4250 4255 4260
   Gln Leu Val Pro Glu Glu Ile Lys Glu Glu Gln Leu Asp Ser Glu
   4265 4270 4275
   Glu Ile Lys Glu Glu Gln Leu Val Pro Glu Glu Ile Lys Glu Glu
   4280 4285 4290
   Gln Leu Asp Ser Glu Glu Ile Lys Glu Glu Gln Leu Asp Ser Glu
   4295 4300 4305
   Glu Ile Lys Glu Glu Gln Leu Asp Ser Glu Glu Ile Lys Glu Glu
   4310 4315 4320
   Gln Leu Asp Ser Glu Glu Ile Lys Glu Gln Lys Gly Ser Val Asn
   4325 4330 4335
   Glu Glu Val Val Glu Glu Glu Gly Ser Val Thr Glu Glu Ile Lys
   4340 4345 4350
   Glu Gln Glu Glu Ser Val Asn Glu Glu Val Leu Glu Glu Val Glu
   4355 4360 4365
   Glu Thr Glu Ser Ile Lys Glu Glu Ile Val Glu Gly Gly Ile Ala
   4370 4375 4380
   Thr Gln Glu Ile Ile Glu Glu Glu Ser Asp Thr Lys Glu Val Val
   4385 4390 4395
   Glu Glu Glu Val Ile Asp Ser Glu Lys Leu Val Asp Ala Gly Ser
   4400 4405 4410
   Val Thr Gly Glu Val Met Pro Glu Glu Val Ser Val Thr Asp Glu
   4415 4420 4425
   Val Val Glu Glu Gly Ser Thr Thr Glu Glu Val Leu Glu Glu Gln
   4430 4435 4440
   Lys Ser Val Asn Glu Glu Val Val Glu Asp Gly Leu Thr Ile Asp
   4445 4450 4455
   Asp Phe Val Gly Leu Gln Gly Ser Thr Thr Glu Glu Val Val Glu
   4460 4465 4470
   Glu Asp Gly Ser Ala Ile Glu Lys Ile Leu Glu Glu Glu Thr Ala
   4475 4480 4485
   Thr Glu Glu Ile Val Glu Lys Gln Val Ser Val Thr Glu Asp Ile
   4490 4495 4500
   Val Glu Lys Glu Gly Ser Val Asn Glu Glu Ile Ile Glu Glu Ala
   4505 4510 4515
   Ser Val Ala Glu Glu Ile Ile Gln Gly Gly Ser Phe Thr Glu Glu
   4520 4525 4530
   Ile Val Gly Gln Glu Glu Ser Ala Thr Glu Glu Val Ile Asp Glu
   4535 4540 4545
   Glu Gly Leu Ile Ser Asn Glu Ile Glu Glu Glu Glu Glu Lys Ser
   4550 4555 4560
   Val Thr Glu Glu Met Ile Glu Glu Val Glu Glu Val Ser Val Asp
   4565 4570 4575
   Asp Glu Val Glu Glu Val Ser Val Ala Glu Glu Ile Val Glu Glu
   4580 4585 4590
   Glu Leu Val Asp Asp Glu Ile Leu Pro Glu Glu Leu Ser Ala Thr
   4595 4600 4605
   Glu Asp Val Ile Glu Glu Val Arg Ser Val Thr Asp Glu Ile Val
   4610 4615 4620
   Gln Glu Glu Ser Val Cys Glu Glu Ile Leu Glu Gln Glu Val Ser
   4625 4630 4635
   Ala Ser Glu Glu Tyr Val Asp Asp Lys Ser Val Thr Asp Asp Phe
   4640 4645 4650
   Val Gly His Glu Arg Ser Val Ile Gln Asp Val Glu Asn Thr Glu
   4655 4660 4665
   Ser Val Thr Glu Glu Ile Ala Glu Val Asp Lys Ser Val Ile Glu
   4670 4675 4680
   Glu Ala Val Glu Lys Gln Gly Ser Val Thr Glu Glu Lys Val Gln
   4685 4690 4695
   Glu Gly Val Ser Ala Ile Glu Glu Ile Glu Glu Leu Glu Ser Val
   4700 4705 4710
   Thr Glu Glu Ile Ala Glu Glu Asp Lys Ser Val Ile Glu Glu Ala
   4715 4720 4725
   Val Glu Lys Gln Gly Ser Val Thr Glu Glu Ile Val Glu Glu Glu
   4730 4735 4740
   Glu Leu Asp Thr Glu Glu Val Leu Glu Asp Lys Ser Val Thr Gly
   4745 4750 4755
   Asp Val Val Glu Gln Glu Gly Ser Gly Lys Asp Glu Ser Glu Ala
   4760 4765 4770
   Lys Glu Ser Phe Thr Glu Glu Val Asp Glu Leu Lys Ser Val Lys
   4775 4780 4785
   Glu Glu Asp Gln Glu Thr Glu Tyr Ile Ser Arg Glu Ile Glu Glu
   4790 4795 4800
   Glu Ser Ala Thr Glu Gln His Ser Glu Gln Glu Leu Ser Ile Asn
   4805 4810 4815
   Lys Glu Val Val Glu Thr Glu Ser Leu Thr Lys Asp Ile Glu Glu
   4820 4825 4830
   Glu Lys Ser Thr Thr Gln Glu Ile Leu Glu Glu Thr Gln Ser Val
   4835 4840 4845
   Asn Glu Glu Ile Val Glu Glu Glu Arg Asp Thr Asp Glu Val Leu
   4850 4855 4860
   Lys Glu Lys Val Ser Pro Ser Glu Glu Val Ile Glu Glu Gln Ala
   4865 4870 4875
   Ser Thr Thr Glu Glu Phe Val Glu Glu Arg Ser Ser Thr Asp Glu
   4880 4885 4890
   Ile Val Glu Val Glu Asp Leu Phe Thr Glu Glu Val Lys Glu Arg
   4895 4900 4905
   Glu Gly Ser Val Thr Glu Glu Ile Val Glu Glu Gly Ser Asp Thr
   4910 4915 4920
   Gly Glu Ile Val Glu Glu Glu Gly Ser Asp Thr Glu Glu Ile Leu
   4925 4930 4935
   Glu Glu Gly Ser Phe Asn Glu Glu Ile Val Glu Glu Glu Gly Ser
   4940 4945 4950
   Ile Thr Glu Glu Ile Leu Gln Gly Ser Val Thr Glu Glu Ile Leu
   4955 4960 4965
   Gln Gly Ser Val Thr Glu Glu Phe Val Gly Gln Gln Gly Ser Val
   4970 4975 4980
   Ile Glu Glu Ile Val Glu Thr Glu Ser Ala Ile Glu Glu Arg Val
   4985 4990 4995
   Glu Glu Glu Ser Ala Thr Glu Glu Val Asp Glu Arg Glu Ser Val
   5000 5005 5010
   Thr Glu Val Val Glu Glu Glu Val Ser Ser Ser Asp Glu Val Val
   5015 5020 5025
   Glu Gly Ser Ile Glu Glu Val Ile Glu Asn Glu Gly Ser Val Thr
   5030 5035 5040
   Glu Glu Ile Leu Glu His Glu Val Ser Ala Asp Glu Asn Phe Val
   5045 5050 5055
   Gly Gln Ala Val Ser Val Ile Glu Glu Val Glu Gly Thr Glu Ser
   5060 5065 5070
   Val Thr Glu Glu Val Val Glu Glu Thr Glu Ser Val Ser Glu Glu
   5075 5080 5085
   Ile Val Glu Val Ser Pro Thr Glu Asn Val Val Gln Gln Thr Asp
   5090 5095 5100
   Ser Val Ile Glu Glu Val Val Glu Gln Lys Glu Gly Ser Phe Asn
   5105 5110 5115
   Glu Glu Ile Asp Ile Arg Glu Leu Gly Asp Asp Gly Val Glu Glu
   5120 5125 5130
   Arg Glu Lys Ile Ser Thr Glu Glu Val Val Gly Gln Asp Lys Ser
   5135 5140 5145
   Ala Thr Gly Asp Val Glu Glu Val Ser Ser Thr Glu Asp Glu Glu
   5150 5155 5160
   Glu Val Ser Ser Thr Glu Gly Leu Glu Glu Val Ser Ser Thr Glu
   5165 5170 5175
   Gly Leu Glu Glu Val Ser Asn Thr Glu Asp Val Glu Glu Val Ser
   5180 5185 5190
   Ser Thr Glu Asp Val Glu Glu Gly Ser Val Ala Glu Asn Val Lys
   5195 5200 5205
   Glu Thr Lys Ser Ile Thr Glu Glu Val Ser Val Glu Glu Asp Ile
   5210 5215 5220
   Ile Thr Asp Lys Val Ser Val Glu Gln Glu Val Met Ala Glu Ala
   5225 5230 5235
   Ser Val Glu Glu Asn Ile Leu Thr Glu Val Pro Val Glu Glu Glu
   5240 5245 5250
   Ile Met Thr Glu Lys Leu Ser Val Glu Asp Lys Ala Leu Asn Glu
   5255 5260 5265
   Lys Ile Met Ser Glu Glu Glu Ile Val Ile Glu Asp Gly Asn Val
   5270 5275 5280
   His Glu Val Val Pro Ala Glu Val Ser Val Thr Glu Glu Ile Pro
   5285 5290 5295
   Gly Val Glu Glu Thr Thr Asn Asn Glu Ser His Val Lys Gly Glu
   5300 5305 5310
   Asn Val  Val Asn Glu Val Val  Val Asp Asp Asn Ser  Val Asn Asp
       5315                 5320                 5325
   Glu Ile  Gln Phe Asp Asp Asp  Ser Ser Ile Glu Ile  Tyr Thr Val
       5330                 5335                 5340
   Asp Ser  Lys Asp Val Phe His  Lys Glu Glu Asn Tyr  Asp Ser Phe
       5345                 5350                 5355
   Arg Glu  Glu Val Arg Ser Asp  Glu Asn Ile His Ile  Phe Arg Lys
       5360                 5365                 5370
   Lys Asn  Glu Asn Phe Val Lys  Lys Ile Glu Asn Glu  Lys Ser Ser
       5375                 5380                 5385
   Val Gly  His Val Pro Thr Glu  Tyr Thr Ser Lys Glu  Asn Ile Ala
       5390                 5395                 5400
   Glu Glu  Val Pro Ser His Ile  Met Phe Lys Glu Asn  Val Thr Glu
       5405                 5410                 5415
   Glu Val  Pro Lys Glu Val Lys  Tyr Glu Glu Asn Thr  Val Glu Glu
       5420                 5425                 5430
   Val Phe  Glu Glu Val Thr Ser  Lys Glu Asn Ile Ile  Glu Glu Ala
       5435                 5440                 5445
   Pro Gly  Asp Ile Asn Ser Glu  Glu Asn Ile Ile Glu  Glu Val Pro
       5450                 5455                 5460
   Glu Val  Val Thr Ser Glu Glu  Ile Val Val Gln Asn  Asp Val Asn
       5465                 5470                 5475
   Asn Ile  Asn Thr Asn Ile Asp  His Met Phe Asp Phe  Asp Leu Asp
       5480                 5485                 5490
   Asp Ile  Leu Lys Ile Pro Glu  Ala Gln Arg Lys Leu  Asn Lys Leu
       5495                 5500                 5505
   Arg Thr  Leu Val Asp Val His  Leu Asp Val Ile Glu  Lys Met Gln
       5510                 5515                 5520
   Arg Asp  Glu Trp Lys Lys Asn  Lys Arg Asp Phe Leu  Tyr Ile Cys
       5525                 5530                 5535
   Leu Lys  Glu Ile Asn Lys Leu  Ser Glu Asp Thr Leu  Met Lys Phe
       5540                 5545                 5550
   Tyr Gly Ser Asp Ser Asn Lys Ser Ala Asn Asp Asn Asp Thr Val
   5555 5560 5565
   Met Val Ile Lys Ile Leu Lys Asp Lys Trp Gly Thr Gly Arg Ile
   5570 5575 5580
   Val Asp Thr Ile Ala Asn Ser Leu Asn Lys Ser Tyr Asn Ser Leu
   5585 5590 5595
   Tyr His Asn Leu Tyr Ile Glu Met Glu Lys Asp Leu Leu Ile Asn
   5600 5605 5610
   Lys Thr Glu Thr Phe Asn Lys Trp Ser Lys Gln His Trp Asn Lys
   5615 5620 5625
   Leu Asp Asn Trp Lys Glu Glu Lys Trp Phe Lys Leu Phe Lys Arg
   5630 5635 5640
   Asp Leu Lys Ile Asp Met Arg Asn Thr Tyr Glu Ser Asp Gln Asn
   5645 5650 5655
   Asp Glu Glu Asn Asn Glu Glu Ser Met Asn Glu Leu Ser Asp Glu
   5660 5665 5670
   Leu Ile Lys Asn Asn Thr Ser Asp Asn Met Arg Asn Glu Gln Lys
   5675 5680 5685
   Thr Leu Glu Lys Asn Lys Glu Tyr Ser Asn Leu Ser Lys Asp Leu
   5690 5695 5700
   Gly Leu Ile Glu Lys Gln Lys Ile Ile Trp Lys Ser Trp Ile Val
   5705 5710 5715
   Lys Asn Val Asn Asn Ile Glu Asn Trp Phe Asp Glu Met Trp Phe
   5720 5725 5730
   Lys Asn Val Val Asn Glu Leu Lys Glu Lys Asn Asp Val Ser Asn
   5735 5740 5745
   Val Leu Gln Glu Asn Ala Ser Glu Ala Ser Val Glu Asn Tyr Ile
   5750 5755 5760
   Ser Asp Val Glu Asp Ser Lys Asp Met Thr Asn Ser Ile Asn Asp
   5765 5770 5775
   Ser Glu Lys Ser Lys Ile Val Ala Ser Ser Ser Lys Thr Thr Asn
   5780 5785 5790
   Glu Glu Tyr Ile Ile Leu Ser Arg Lys Asp Leu Ile Tyr Asn Ile
   5795 5800 5805
   Ile Val Met Val His Met Met Val Leu Asp Gln Phe Lys Tyr Asp
   5810 5815 5820
   Glu Leu Lys Tyr Ala Lys Lys Arg Phe Leu Asn Arg Ser Ile Asp
   5825 5830 5835
   Lys Phe Ile Lys Glu Lys Lys Ile Lys Asp Lys Glu Thr Val Leu
   5840 5845 5850
   Asp Tyr Tyr Ile Asp Asp Ile Ile Lys Arg Ile Val Glu Asp Thr
   5855 5860 5865
   Ser His Val Asn Asn Ile Lys Glu Lys Ala Ile Asp His Tyr Thr
   5870 5875 5880
   Ser His Asp Trp Phe Arg Leu Leu Arg Gln Gly Asn Lys Ala Gln
   5885 5890 5895
   Asn Ser Ile Ser Gln Glu Val Asp Ile Leu Thr Glu Lys Tyr Lys
   5900 5905 5910
   Lys Leu Ile Ser Glu Glu Asp Asn Lys Lys Glu Asn Asn Asn Asn
   5915 5920 5925
   Asn Asp Asp Glu Val Lys Pro Glu Leu Lys Asp Asn Ile Lys Cys
   5930 5935 5940
   Glu Glu Asn Val Ser Phe Asn Ile Leu Arg Arg Ser Asn Lys Lys
   5945 5950 5955
   Asp Gln Leu Pro Leu Glu Glu Lys Lys Asn Lys Asn Gly Asp Leu
   5960 5965 5970
   Asn Asn Thr Leu Thr Glu Ser Asp Gly Lys Lys Ile Asn Ile Asn
   5975 5980 5985
   Glu Ala Ile Glu Glu Ser Lys Asn Gly Asn Glu Asp Lys Asn Ser
   5990 5995 6000
   Gly Asn Met Glu Lys Ser Arg Lys Pro Arg Asp Arg Arg Thr Glu
   6005 6010 6015
   Arg Lys Glu Lys Asp Gln Asp Leu Arg Val Gln Leu Leu Gln Asp
   6020 6025 6030
   Tyr Glu Asn Ile Phe Glu Gln Ile Gln Asn Met Glu Asn Gln Asn
   6035 6040 6045
   Lys Asp Lys Asn Lys Lys Glu Thr Lys Asn Ile Leu Lys Thr Ser
   6050 6055 6060
   Ile Asp Leu Asp Lys Asn Leu Leu Arg Glu Tyr Lys Lys Glu Lys
   6065 6070 6075
   Asn Ile Ile Asn Gln Ser Glu Lys Glu Phe Ser Val Asn Glu Asn
   6080 6085 6090
<210> 59
   <211> 994
   <212> PRT
   <213> Plasmodium falciparum
<400> 59
   Met Lys Phe Phe Ile Pro Tyr Val Tyr Ile Ile Cys Val Ile Phe Ile
   1               5                   10                  15
   Ile Asn Val Ile Arg Thr Arg Gly Glu Glu Asp Asp Asp Asn Asn Asn
               20                  25                  30
   Ile Ser Gly Lys Ser Ile Leu Gly Thr Ser His Asn Asn Ile Ser Asn
           35                  40                  45
   Ile Asp Leu Ser Ser Ile Pro Asn Leu Asp Ser Asn Ile His Ala Ser
       50                  55                  60
   Phe Ser Ser Asp Thr Lys Glu Trp Ser Pro Asn Asn Leu Thr Ser Lys
   65                  70                  75                  80
   Lys Lys Lys Lys Lys Lys Glu Ile Arg Pro Lys Asp Ile Met Ser Asn
                   85                  90                  95
   Ser Asp Ser Ser Asn Thr Ser Ser Ile Asn Lys Gln Asn Asn Asn Gln
               100                 105                 110
   Ile Lys Ser Val Leu Leu Lys Glu Asn Lys Gly Val Lys Ile Thr Gly
           115                 120                 125
   Pro Cys Asn Val Asn Leu Ser Ile Phe Leu Val Pro His Ile Tyr Ile
       130                 135                 140
   Asp Val Glu Thr Lys Tyr Asn Asn Ile Glu Leu Lys Tyr Glu Leu Asp
   145                 150                 155                 160
   Glu Phe Ser Asp Ser Ile Lys Phe Lys Asp Thr Thr Thr Glu Leu Arg
                   165                 170                 175
   Thr Ser Asp Asp Thr Leu Met Asn Thr Asn Phe Asn Val Gly Val Ser
               180                 185                 190
   Arg Asp Thr Leu Asp Lys Asp Arg Leu Tyr Asn Ile Cys Ala Glu Asn
           195                 200                 205
   Lys Thr Phe Lys Phe Val Val Tyr Ile Lys Asp Asn Ile Leu Thr Leu
       210                 215                 220
   Lys Trp Lys Val Tyr Glu Thr Gly Val Thr Asn Asn Lys Val Asp Ile
   225                 230                 235                 240
   Arg Gln Tyr Lys Met Lys Glu Leu Thr Arg Pro Ile Thr Thr Ile Gln
                   245                 250                 255
   Ile His Ser Val Ser Glu Asn Lys Asp Thr His Leu Leu Glu Ser Lys
               260                 265                 270
   Asn Tyr Val Ile Lys Thr Asp Ile Pro Glu Thr Cys Asp Val Met Ala
           275                 280                 285
   Thr Asn Cys Phe Leu Ser Gly Asn Ile Asn Ile Glu Lys Cys Leu Glu
       290                 295                 300
   Cys Thr Leu Leu Val Gln Asn Asn Asp Thr Ser Ser Glu Cys Phe Thr
   305                 310                 315                 320
   Tyr Val Ser Asn Asp Val Arg Glu Asn Phe Asn Gln Ile Lys Ala Glu
                   325                 330                 335
   Ala Glu Asp Asp Glu Asn Phe Arg Asn Tyr His Leu Thr Asp Thr Ile
               340                 345                 350
   Asn Asn Ile Leu Lys Arg Ile Tyr Lys Ile Asn Lys Asn Glu Gly Lys
           355                 360                 365
   Lys Glu Leu Ile Thr Leu Glu Glu Leu Asp Asn Phe Leu Lys Glu Ser
       370                 375                 380
   Ile Thr Asp Tyr Cys Lys Ile Leu Arg Glu Ile Asp Thr Asn Gly Thr
   385                 390                 395                 400
   Leu Val Asn His Glu Leu Gly Asn Asn Val Asp Val Phe Asn Asn Leu
   405 410 415
   Ile Arg Leu Leu Lys Leu His Lys Asn Glu Ser Ile Ser Thr Leu His
   420 425 430
   Asn Lys Leu Arg Asn Ser Ala Ile Cys Met Lys Tyr Pro Asp Lys Trp
   435 440 445
   Ile Glu Lys Lys Thr Gly Leu Ile Leu Pro Asn Val Val Asn Asn Asn
   450 455 460
   Ile Ile Tyr Asn Asn Lys Tyr Glu Lys Leu Asn Glu Glu Lys Lys Arg
   465 470 475 480
   Lys Ile Tyr Asp Asn Lys Asp Asp Ser Lys Ile Ser Asp Ile Ile Asn
   485 490 495
   Ile Lys Lys Tyr Ile Phe Thr Asn Asn Thr Leu Lys Tyr Phe Asn Asn
   500 505 510
   Asp Lys Gln Phe Cys Asn Ser Ser Phe Cys Asn Arg Leu Lys Asp Glu
   515 520 525
   Asn Asn Cys Ile Ser Lys Ile Gln Ile Glu Asp Gln Gly Asn Cys Ala
   530 535 540
   Ile Ser Trp Ile Phe Ala Ser Lys Tyr Tyr Leu Glu Thr Leu Lys Cys
   545 550 555 560
   Met Lys Gly Tyr Glu Pro His Ala Ile Ser Ala Leu Tyr Ile Ala Asn
   565 570 575
   Cys Ser Lys Arg Lys His Lys Asn Arg Cys Asn Val Gly Ser Asn Pro
   580 585 590
   Leu Glu Phe Leu Gln Ile Ile Glu Glu Asn Gln Phe Leu Pro Met Asp
   595 600 605
   Thr Asn Tyr Leu Tyr Ser Tyr Thr Lys Val Gly Asn Asp Cys Pro Asp
   610 615 620
   Glu Glu Lys Asn Trp Val Asn Leu Leu Lys His Thr Arg Met Leu Asn
   625 630 635 640
   Tyr Asn Asn Lys His Arg Ser Thr Leu Ser Thr Lys Ala Tyr Arg Ala
   645 650 655
   Tyr Glu Ser Glu His Phe Lys Asp Lys Met Asp Thr Phe Ile Lys Leu
   660 665 670
   Ile Lys Asp Glu Ile Met Asn Asn Gly Ser Val Ile Ala Tyr Val Lys
   675 680 685
   Ala Glu Asn Val Leu Gly Tyr Glu Leu Asn Gly Lys Asn Val Gln Asn
   690 695 700
   Leu Cys Gly Asp Lys Thr Pro Asp His Ala Val Asn Ile Val Gly Tyr
   705 710 715 720
   Gly Asn Tyr Ile Asn Asp Glu Asp Glu Lys Lys Ser Tyr Trp Ile Val
   725 730 735
   Arg Asn Ser Trp Gly Lys Tyr Trp Gly Asp Glu Gly Tyr Phe Lys Val
   740 745 750
   Asp Met Tyr Gly Pro Ser Thr Cys Glu Asp Asn Phe Ile His Thr Val
   755 760 765
   Val Val Phe Asn Ile Asn Met Pro Lys Ser Lys Lys Ser Pro Val Lys
   770 775 780
   Ile Thr Phe Pro Leu Tyr Asn Tyr Tyr Leu Lys Tyr Ser Pro Asp Phe
   785 790 795 800
   Tyr His Asn Leu Tyr Tyr Lys Asn Phe Asn Ser Lys Lys Ser Met Lys
   805 810 815
   Leu Val Asn Ala Ser Asp Glu His Lys Asn Ile Tyr Ser Gln Glu Asp
   820 825 830
   Lys Val Asn His Lys Lys Gly Ser Lys Ile Leu Asn Ser Glu Val Thr
   835 840 845
   Thr Ser Leu Leu Ser Gln Glu Ile Ser Gln Arg Arg Gly Glu Asp Asp
   850 855 860
   Asp Ile Asp Thr Leu Ile Gly Asp Ser Pro Asp Ile Asn Glu Gln Glu
   865 870 875 880
   Lys Asn Ile Lys Asp Glu Phe Asn Lys Ser Ser Ile Thr His Asn Ser
   885 890 895
   Val Ser Ser Ser Asn Ile Thr Lys Ser Asn Lys Asn Thr Asn Lys Val
   900 905 910
   Lys Ile Tyr His Ile Ile Lys His Val Lys Asn Thr Lys Ile Lys Ile
           915                 920                 925
   Gly Phe Val Lys Tyr Asp Asn Tyr Asn Thr Ile Gly Thr Asn His Thr
       930                 935                 940
   Cys Ser Arg Ser Tyr Ser Glu Asp Gln Glu Lys His Glu Gly Cys Ile
   945                 950                 955                 960
   Lys Phe Cys Glu Leu His Trp Asn Glu Cys Lys Asp Lys Thr Ser Pro
                   965                 970                 975
   Gly Tyr Cys Leu Thr Lys Leu Lys Gly Ser Asn Glu Cys Phe Phe Cys
               980                 985                 990
   Tyr Val
<210> 60
   <211> 1105
   <212> PRT
   <213> Plasmodium falciparum
<400> 60
   Met Lys Phe His Ile Ser Phe Phe Leu Ile Leu Tyr Ile Val Phe Phe
   1               5                   10                  15
   Lys Asn Thr Ile Lys Ser Glu Thr Thr Thr Asp Glu Ser Ala Thr Gly
               20                  25                  30
   Ser Leu Ser Ser Asp Gly Ser Arg Val Thr Thr Gln Ala Arg Ile Glu
           35                  40                  45
   Lys Pro Lys Gln Gln Pro Thr Leu Pro Thr Leu Ala Gln Glu Thr Gln
       50                  55                  60
   Pro Gln Gln Gln Gln Gln Gln Lys Glu Val Gly Ser Gly Ile Gly Ala
   65                  70                  75                  80
   Glu Gln Lys Val Glu Ser Ala Arg Pro Gly Ala Glu Val Ser Gln Ser
                   85                  90                  95
   Asp Val Glu Arg Ala Gly Arg Ser Ser Gly Thr Gly Gly Ser Val Gly
               100                 105                 110
   Thr Lys Ile Ser Pro Gly Ser Gln Gly Gln Gly Lys Val Ala Gly Pro
           115                 120                 125
   Gln Leu Pro Arg Leu Pro Gln Leu Pro Gln Ser Phe Glu Gln Ser Arg
       130                 135                 140
   Asn Gln Gln Ser Ser Pro Val Thr Pro Lys Arg Asn Gly Ile Ser Pro
   145                 150                 155                 160
   Thr Asn Ala Lys Ser Pro Glu Ser Val Leu Pro Pro Ala Gln Ser Phe
                   165                 170                 175
   Thr Asn Leu Asn Lys Ser Thr Ile Pro His Thr Ile Pro Ile Lys Ser
               180                 185                 190
   Ser Phe Leu Lys Tyr Tyr Lys Gly Val Lys Ile Thr Gly Ser Cys Gly
           195                 200                 205
   Val Gln Phe Gln Leu Val Ile Val Pro His Leu Phe Ile Tyr Val Glu
       210                 215                 220
   Thr Lys Glu Asn Asn Ile Gln Leu Glu Pro Arg Phe Met Lys Leu Asn
   225                 230                 235                 240
   Glu Arg Ile Asp Phe Glu Lys Asp Lys Ser Asn Leu Lys Asn Lys Cys
                   245                 250                 255
   Asp Val Asn Lys Lys Gln Ser Phe Lys Phe Ile Leu Tyr Leu Gln His
               260                 265                 270
   Asp Leu Ile Thr Ile Lys Trp Lys Val Tyr Glu Glu Lys Pro Asp Thr
           275                 280                 285
   Thr Thr Arg Ile Ala Pro Gly Gln Val Asp Leu Asn Val Asp Val Lys
       290                 295                 300
   Arg Tyr Lys Leu Pro Lys Leu Asp Gln Pro Val Ile Ser Ile Gln Ile
   305                 310                 315                 320
   His Ser Leu Ala Gln Asp Gly Glu Thr Tyr Leu Met Glu Ser Lys Asp
                   325                 330                 335
   Tyr Asn Leu Glu Asp Gln Ile Pro Glu Lys Cys Glu Ala Ile Ala Ser
               340                 345                 350
   Asp Cys Phe Leu Ser Gly Asn Val Asp Ile Glu Lys Cys Leu Gln Cys
           355                 360                 365
   Thr Leu Leu Val Ile Lys Ala Asp Lys Asn Asp Glu Cys Leu Lys Tyr
   370 375 380
   Val Ser Lys Asn Val Lys Asp Arg Phe Glu Glu Ile Leu Thr Lys Gly
   385 390 395 400
   Glu Asp Asp Ala Asp Ser Asp Glu Tyr Asp Phe Ile Ala Pro Ala Asn
   405 410 415
   Tyr Ile Leu Lys Asn Ile Tyr Lys Lys Asn Asp Thr Asn Gly Lys Lys
   420 425 430
   Glu Leu Leu His Phe Lys Asp Ile Asn Asn Asn Leu Lys Leu Glu Leu
   435 440 445
   Ile Asn Tyr Cys Asn Leu Leu Lys Asp Asn Asp Val Ser Gly Ile Leu
   450 455 460
   Thr Tyr Glu Lys Leu Gly Asn Val Gln Asp Ile Phe Asn Asn Leu Thr
   465 470 475 480
   Lys Leu Leu Glu Glu His Lys Glu Glu Asn Asn Tyr Val Leu Tyr His
   485 490 495
   Lys Met Lys Asn Glu Val Leu Cys Leu Lys Asn Ala Asn Asp Trp Met
   500 505 510
   Lys Asn Lys Thr Gly Leu Val Leu Pro Gln Leu Lys Tyr Ser Leu Asn
   515 520 525
   Lys Phe Asn Lys Asn Lys Glu Asn Tyr Ile Lys Glu Asn Ile Phe Glu
   530 535 540
   Glu Asp Glu Asn Gly Ile Val Asp Leu Thr Lys Phe Pro Val Asp Thr
   545 550 555 560
   Ser Tyr Ser Ser Tyr Asn Tyr Ala Asp Ser Leu Tyr Cys Asn Arg Glu
   565 570 575
   Tyr Cys Asn Arg Leu Lys Asp His Asn Asn Cys Ile Ser Lys Ile Asn
   580 585 590
   Val Glu Asp Gln Lys Asn Cys Ala Leu Ser Trp Ala Phe Ala Ser Ile
   595 600 605
   Tyr His Leu Glu Thr Ile Lys Cys Met Lys Gly Tyr Glu Pro Leu Asn
   610 615 620
   Ala Ser Val Leu Tyr Val Thr Asn Cys Leu Lys Asn Lys Asn Asn Asp
   625                 630                 635                 640
   Val Cys Thr Glu Gly Ser Asn Pro Leu Val Phe Leu Glu Thr Ile Glu
                   645                 650                 655
   Glu Lys Gly Phe Leu Pro Thr Glu Ser Asn Tyr Pro Tyr Asp Gln Ser
               660                 665                 670
   Lys Val Gly Asp Val Cys Pro Gln Val Gln Asn Asp Trp Asp Asn Val
           675                 680                 685
   Phe Glu Asn Thr Lys Val Leu Glu Tyr Asn Asn Ala Pro Phe Ser Val
       690                 695                 700
   Gly Thr Lys Gly Tyr Ile Ala Tyr Glu Ser Glu Val Phe Gln Lys Asp
   705                 710                 715                 720
   Ile Asp Ser Phe Val Lys Leu Ile Lys Asp Glu Ile Met Asn Lys Gly
                   725                 730                 735
   Ser Val Ile Ala Tyr Val Lys Ala Lys Asn Val Leu Gly Tyr Glu Leu
               740                 745                 750
   Asn Gly Lys Lys Val Gln Asn Leu Cys Gly Asp Lys Lys Pro Asp His
           755                 760                 765
   Ala Val Asn Ile Val Gly Tyr Gly Asn Tyr Ile Asn Asn Lys Gly Glu
       770                 775                 780
   Lys Lys Ser Tyr Trp Ile Val Arg Asn Ser Trp Gly Lys Tyr Trp Gly
   785                 790                 795                 800
   Asp Asp Gly Tyr Phe Lys Val Asp Met Tyr Gly Pro Pro Thr Cys Glu
                   805                 810                 815
   Asp Asn Phe Ile His Ser Val Val Val Phe Asn Val Glu Val Pro Val
               820                 825                 830
   Asn Glu Asn Phe Asp Lys Lys Glu His Asp Ile Tyr Lys Ser Tyr Leu
           835                 840                 845
   Lys Asn Ser Pro Asp Phe Tyr His Asn Ile Tyr Tyr Lys Asn Tyr Asn
       850                 855                 860
   Phe Glu Asn Tyr Val Ser Pro Ile Ser Thr Trp Ser Glu Val Leu His
   865                 870                 875                 880
   Asn Thr Leu Tyr Asn Asn Asn Leu Ile Trp Gly Gln Glu Thr Thr Asp
                   885                 890                 895
   Pro Ile Gly Glu Gly Lys Leu Pro Ala Ser Glu Ala Gly His Leu Arg
               900                 905                 910
   Gly Glu Gly Lys Gly Asn Ser Glu Glu Asn Gln Gly Gln Arg Ser Asn
           915                 920                 925
   Ala Asn Gly Ala Ile Ser Ser Asn Gln Ser Thr Gly Gly Lys Ile Thr
       930                 935                 940
   Lys Gln Gly Glu Ser Lys Asp Asp Gly Val Ala Leu Pro Val Ser Thr
   945                 950                 955                 960
   Asp Gly Lys Pro Asn Thr Ser Ser Ser Val Val Asp Thr Asn Asp Gln
                   965                 970                 975
   Arg Ser Leu Pro Asn Pro Arg Ala Thr Ser Leu Gln Pro Pro Ser Val
               980                 985                 990
   Gln Ile Pro Asn His Glu Gly Thr  Ser Ala Pro Gly Asn  Ser Arg Thr
           995                 1000                 1005
   Pro Ser  Ile Val Ser Pro Thr  Ala Ala Glu Lys Ser  Arg Lys Ala
       1010                 1015                 1020
   Gln Ile  Phe His Val Leu Lys  His Ile Lys Asn Ser  Arg Ile Lys
       1025                 1030                 1035
   Met Gly  Leu Val Lys Tyr Asp  Asn Ser Asp Asn Ile  Gly Gly Asp
       1040                 1045                 1050
   His Val  Cys Ser Arg Thr Tyr  Ala Val Asn Pro Glu  Lys Gln Glu
       1055                 1060                 1065
   Glu Cys  Val Lys Phe Cys Glu  Glu Asn Trp Glu Asn  Cys Lys Asn
       1070                 1075                 1080
   Lys Pro  Ser Pro Gly Tyr Cys  Leu Ala Lys Leu Lys  Asn Thr Asn
       1085                 1090                 1095
   Glu Cys  Phe Phe Cys Tyr Val
       1100                 1105
<210> 61
   <211> 930
   <212> PRT
   <213> Plasmodium falciparum
<400> 61
   Met Lys Phe Ser Ile Ser Leu Phe Leu Ile Leu Cys Val Leu Phe Cys
   1               5                   10                  15
   Lys Asn Asp Ile Lys Cys Thr Thr Val Asp Glu Ser Thr Lys Glu Gly
               20                  25                  30
   Ser Gln Asn Pro Lys Asn Ser Ser Ser Thr Thr Pro Ala Ser Gly Ser
           35                  40                  45
   Gln Lys Gly Ser Ser Ser Glu Ser Pro Gly Ser Ser Val Glu Lys Gln
       50                  55                  60
   Ser Gln Glu Ser Asn Lys Glu Ser Thr Asn Gly Gly Asn Val Val Ser
   65                  70                  75                  80
   Gln Gly Thr Pro Ala Asn Thr Phe Gly Gln Asn Ser Asn Asn Pro Ser
                   85                  90                  95
   Asp Ser Pro Gln Gly Thr Ser Thr Leu Pro Ser Pro Pro Lys Ser Ile
               100                 105                 110
   Asp Val Lys Ser Ala Phe Leu Lys His Tyr Lys Gly Val Lys Val Thr
           115                 120                 125
   Gly Ser Cys Asn Ala Asn Phe Gln Leu Phe Leu Val Pro His Ile Phe
       130                 135                 140
   Ile Asn Val Glu Thr Lys Glu Asn Asn Ile Gln Leu Asp Val Lys Phe
   145                 150                 155                 160
   Leu Lys Leu Thr Lys Arg Ile Asp Phe Ala Lys Asp Lys Ser Met Leu
                   165                 170                 175
   Lys Asn Lys Cys Glu Ser Gly Lys Asn Gln Thr Phe Lys Phe Val Leu
               180                 185                 190
   Tyr Phe Lys Asp Asp Ile Leu Thr Ile Lys Trp Lys Val Tyr Glu Glu
           195                 200                 205
   Lys Ser Ala Thr Pro Gln Lys Ser Glu Glu Asn Thr Val Asp Ile Lys
       210                 215                 220
   Leu Tyr Lys Leu Pro Lys Leu Asp Gln Thr Ile Thr Ser Ile Gln Val
   225                 230                 235                 240
   His Thr Leu Ser Ile Glu Gly Thr Ser Tyr Leu Met Glu Ser Lys Asp
   245 250 255
   Tyr Ser Leu Gly Asn Asn Leu Pro Glu Lys Cys Asp Ala Ile Ala Ser
   260 265 270
   Asp Cys Phe Leu Ser Gly Asn Ile Asn Val Glu Lys Cys Leu Lys Cys
   275 280 285
   Thr Leu Lys Val Lys Lys Val Glu Ala Ser Asp Glu Cys Tyr Lys Tyr
   290 295 300
   Val Ser Lys Asp Lys Pro Lys Glu Thr Lys Leu Ala Val Ser Gly Ser
   305 310 315 320
   Glu Val Lys Glu Val Lys Ala Ala Ser Val Asp His Ser Asn Asp Lys
   325 330 335
   Glu Tyr Glu Leu Ser Gln Ser Ile Asn Asn Ile Leu Asn Lys Met Tyr
   340 345 350
   Lys Lys Glu Ser Asn Asp Glu Lys Asn Asn Lys Lys Glu Leu Ile Lys
   355 360 365
   Leu Glu Asp Ala Asp Asp Ser Leu Gln Lys Glu Leu Asn Lys Tyr Cys
   370 375 380
   Asn Ser Leu Lys Glu Val Asp Leu Asn Gly Val Leu Ser Lys Asn Glu
   385 390 395 400
   Val Gly Asn Glu Lys Asp Val Phe Asn Asn Leu Thr Thr Leu Leu Lys
   405 410 415
   Glu His Met Leu Glu Ser His His Val Val Phe Glu Lys Leu Lys Asn
   420 425 430
   Ser Ala Leu Cys Leu Lys Asn Ile Asp Asp Trp Leu Lys Asn Lys Asn
   435 440 445
   Gly Leu Ile Val Pro Pro Ser Lys Tyr Lys Leu Lys Asp Thr Asn Glu
   450 455 460
   Lys Lys Glu Leu Asn Asn Asn Val Glu Val Ile Glu Asp Met Phe Lys
   465 470 475 480
   Ala Asn Glu His Gly Ile Val Asp Leu Thr Lys Phe Pro Ile Asp Thr
   485 490 495
   Asn Tyr Ser Ser Tyr Lys His Ile Asp His Thr Tyr Cys Asn Asn Asp
   500 505 510
   Tyr Cys Asn Trp Ser Lys Asp Lys Asn Ser Cys Ile Ser Lys Ile Asn
   515 520 525
   Val Glu Asp Gln Lys Asn Cys Ala Leu Ser Trp Ala Phe Ala Ser Lys
   530 535 540
   Tyr His Leu Glu Thr Ile Lys Cys Met Lys Gly Tyr Glu His Ile Pro
   545 550 555 560
   Ile Ser Ser Leu Tyr Ile Ala Asn Cys Ser Lys Asn Glu Lys Lys Asp
   565 570 575
   Val Cys Thr Glu Gly Ser Asn Pro Leu Lys Val Leu Gln Met Ile Val
   580 585 590
   Glu Lys Gly Phe Leu Pro Thr Glu Gly Asp Tyr Ser Tyr Glu Gln Ser
   595 600 605
   Lys Val Gly Glu Thr Cys Pro Glu Val Gln Asn Gly Trp Val Asn Leu
   610 615 620
   Trp Ala Asn Ala Lys Leu Leu Glu Gln Asn Asn Asp Glu His Asn Ser
   625 630 635 640
   Leu Ser Thr Lys Gly Tyr Thr Ala Tyr Glu Ser Glu Ala Phe Gln Lys
   645 650 655
   Asp Met His Ser Phe Val Lys Leu Ile Lys Asp Glu Ile Met Asn Lys
   660 665 670
   Gly Ser Val Ile Ala Tyr Val Lys Ala Asp Lys Ile Met Ala Tyr Glu
   675 680 685
   Phe Asn Gly Lys Lys Val Gln Asn Leu Cys Gly Asp Lys Thr Pro Asp
   690 695 700
   His Ala Val Asn Ile Ile Gly Tyr Gly Asn Tyr Ile Asn Asp Glu His
   705 710 715 720
   Gln Lys Lys Ser Tyr Trp Ile Val Arg Asn Ser Trp Gly Lys His Trp
   725 730 735
   Gly Asp Lys Gly His Phe Lys Val Asp Met Tyr Gly Pro Ser Asp Cys
   740 745 750
   Glu Asp Asn Phe Ile His Ser Val Val Ile Phe Asn Val Asp Leu Pro
   755 760 765
   Ile Asn Gln Glu Ser Val Lys Lys Glu Pro Lys Ile Tyr Asn Tyr Tyr
   770 775 780
   Leu Lys Ala Ser Pro Asp Phe Tyr His Asn Leu Tyr Tyr Lys Asn Phe
   785 790 795 800
   Asp Ser Gln Lys Gly Lys Ala Asp Gln Ala Glu Asn Lys Lys Ser Tyr
   805 810 815
   Leu Tyr Gly Gln Glu Glu Ser Thr Ser Glu Gln Leu Pro Ser Ser Leu
   820 825 830
   Ser Ser Pro Gln Asn Asn Lys Gln Ser Glu Arg Ser Lys Glu Lys Val
   835 840 845
   Asp Ile Phe His Val Leu Lys His Ile Lys Asp Ser Lys Ile Lys Met
   850 855 860
   Gly Ile Val Lys Tyr Asp His Ser Asp Ala Leu Gly Glu Asp Asn Val
   865 870 875 880
   Cys Ser Arg Ser Tyr Ser Ser Asn Pro Glu Lys Gln Glu Gly Cys Val
   885 890 895
   Lys Phe Cys Asn Glu Asn Trp Gly Lys Cys Lys Asp Ala Ala Ser Pro
   900 905 910
   Gly Phe Cys Leu Ser Glu Leu Glu Lys Thr Asn Asp Cys Phe Phe Cys
   915 920 925
   Tyr Ile
   930
<210> 62
   <211> 962
   <212> PRT
   <213> Plasmodium falciparum
<400> 62
   Met Lys Ile His Ile Phe Leu Ile Ala Thr Ile Tyr Val Leu Phe Ser
   1 5 10 15
   Glu Lys Leu Ile Lys Trp Thr Thr Ala Ser Thr Thr Gln Gly Gly Asp
               20                  25                  30
   Thr Asp Thr His Pro Gly Thr Pro Pro Gly Glu Gly Ser Asp Val Ser
           35                  40                  45
   Gln Gly Ala Gly Gln Asp Ala Ser Gln Gly Ala Gly Gln Asp Ala Asn
       50                  55                  60
   Pro Asp Pro Thr Leu Pro Lys Pro Pro Ser Pro Pro Ala Asp Asp Thr
   65                  70                  75                  80
   Lys Asp Thr Gly Ser Gln Gly Asp Ala Asp Ser Ser Ser Ser Lys Ile
                   85                  90                  95
   Glu Ile Pro Pro Leu Val Lys Pro Glu Asn His Lys Thr Ile Val Ser
               100                 105                 110
   Ala Met Leu Lys Asn Tyr Lys Gly Val Lys Val Thr Gly Thr Cys Gly
           115                 120                 125
   Ala Asp Phe Gly Leu Phe Leu Val Pro His Ile Tyr Val His Val Lys
       130                 135                 140
   Ser Glu Asp Thr Glu Ile Glu Leu Ser Ser Glu Leu Ala Pro Pro Glu
   145                 150                 155                 160
   Met Gln Thr Lys Phe Asp Lys Thr Gln Leu Lys Lys Phe Cys Val Lys
                   165                 170                 175
   Asp Asp Thr Lys Lys Phe Asp Phe Ile Ala Tyr Ile Tyr Lys Asp Ile
               180                 185                 190
   Leu Val Phe Lys Trp Lys Val Tyr Glu Glu Gly Leu Ser Lys Glu Gln
           195                 200                 205
   Asp Val Asp Glu Met Lys Tyr Leu Leu Pro Asn Leu Lys Gln Pro Ile
       210                 215                 220
   Thr Ser Ile Gln Val His Ser Trp Thr Gly Thr Lys Glu Ser Tyr Ile
   225                 230                 235                 240
   Leu Glu Ser Lys Asp Tyr Val Leu Gly Glu Gly Met Pro Glu Lys Cys
                   245                 250                 255
   Asp Ala Ile Ala Thr Asp Cys Phe Leu Ser Gly Phe Thr Asp Ile Gly
               260                 265                 270
   Lys Cys Phe Gln Cys Lys Leu Leu Met Gln Glu Lys Asn Ile Asn Asp
           275                 280                 285
   Ser Cys Phe Lys Tyr Val Ser Ser Asn Gln Lys Glu Leu Ile Lys Lys
       290                 295                 300
   Gln Leu Lys Ile Thr Ala Gln Asp Asp Glu Glu Ser Ser Glu Tyr His
   305                 310                 315                 320
   Leu Ser Glu Ser Ile Lys Asn Leu Leu Lys Asn Ile Tyr Lys Lys Asn
                   325                 330                 335
   Asn Asp Asp Asn Lys Lys Lys Glu Leu Leu His Phe Glu Asn Val Asn
               340                 345                 350
   Ser Ala Leu Lys Ser Glu Leu Leu Asn Tyr Cys Asn Leu Leu Lys Glu
           355                 360                 365
   Val Asn Met Asn Gly Val Leu Lys Asp His Gln Leu Gly Asn Val Gln
       370                 375                 380
   Asp Val Phe Asn Asn Leu Thr Lys Leu Leu Glu Glu His Lys Glu Glu
   385                 390                 395                 400
   Asn Asp Asn Val Leu Tyr His Lys Met Lys Asn Glu Ala Leu Cys Leu
                   405                 410                 415
   Lys Asn Val Asn Asp Trp Met Lys Asn Lys Thr Gly Leu Leu Leu Pro
               420                 425                 430
   Gln Leu Ser Tyr Asp Leu Thr Tyr Lys Asn Asn Asn Phe Thr Glu Phe
           435                 440                 445
   Thr Gln Asn Lys Ser Tyr Thr Ser Gln Asn Ile Val Asp Lys Leu Tyr
       450                 455                 460
   Cys Asn His Glu Tyr Cys Asn Arg Leu Lys Asp His Asn Asn Cys Ile
   465                 470                 475                 480
   Ser Lys Ile Asn Val Glu Asp Gln Lys Asn Cys Ala Leu Ser Trp Ala
                   485                 490                 495
   Phe Ala Ser Lys Tyr His Leu Glu Thr Ile Lys Cys Met Lys Gly Tyr
               500                 505                 510
   Glu Pro Leu Asn Ala Ser Val Leu Tyr Val Thr Asn Cys Leu Lys Asn
           515                 520                 525
   Lys Asn Lys Asp Val Cys Thr Glu Gly Ser Asn Pro Leu Val Phe Leu
       530                 535                 540
   Glu Thr Ile Glu Glu Lys Gly Phe Leu Pro Thr Glu Ser Asn Tyr Pro
   545                 550                 555                 560
   Tyr Asp Gln Ser Lys Val Gly Asp Ile Cys Pro Gln Leu Gln Asn Asp
                   565                 570                 575
   Trp Asp Asn Val Phe Glu Asn Thr Lys Val Leu Asp Tyr Asn Asn Gly
               580                 585                 590
   Pro Phe Ser Val Gly Thr Lys Gly Tyr Ile Ala Tyr Glu Ser Glu Ala
           595                 600                 605
   Phe Gln Lys Asp Met His Ser Phe Val Lys Leu Val Lys Asp Glu Ile
       610                 615                 620
   Met Asn Lys Gly Ser Val Ile Ala Tyr Val Lys Ala Glu Asn Val Leu
   625                 630                 635                 640
   Gly Tyr Glu Leu Asn Gly Lys Lys Val Gln Asn Leu Cys Gly Asp Lys
                   645                 650                 655
   Thr Pro Asp His Val Val Asn Ile Val Gly Tyr Gly Asn Tyr Ile Asn
               660                 665                 670
   Asn Lys Gly Glu Lys Lys Ser Tyr Trp Ile Val Arg Asn Ser Trp Gly
           675                 680                 685
   Lys Tyr Trp Gly Asp Asp Gly Tyr Phe Lys Val Asp Met Tyr Gly Pro
       690                 695                 700
   Ser Thr Cys Glu Asp Asn Phe Ile His Thr Val Val Val Phe Asn Val
   705                 710                 715                 720
   Gln Val Pro Ile Asn Glu Lys Phe Asp Lys Lys Glu His Asp Ile Tyr
                   725                 730                 735
   Asn Tyr Tyr Leu Lys Thr Ser Pro Glu Phe Tyr His Asn Leu Tyr Tyr
               740                 745                 750
   Lys Thr Phe Asn Ser Asn Lys Glu Glu Lys Ser Met Asn Lys Asn Ser
           755                 760                 765
   Tyr Val Tyr Gly Gln Asp Thr Thr Pro Val Glu Asn Glu Ala Pro Arg
   770 775 780
   Ser Gly Val Gln Lys Pro Thr Glu Leu Ser Ser Thr Glu Ser Gln Thr
   785 790 795 800
   Val Ser Pro Pro Asn Glu Ser Gln Thr Glu Ser Leu Leu Ser Gly Gly
   805 810 815
   Ser Gln Val Thr Asn Pro Thr Leu Thr Gln Ser Thr Ser Ser Ser Ser
   820 825 830
   Gly Gln Gln Glu Thr Gly Pro Leu Ser Thr Gln Gly Leu Ser Pro Ala
   835 840 845
   Thr Gly Asp Pro Lys Gly Lys Glu Gln Glu Ala Ser Pro Ala Glu Gly
   850 855 860
   Leu Ser Gly Val Leu Asn Pro Thr Lys Glu Val Thr Ser Glu Glu Lys
   865 870 875 880
   Ile Gln Ile Ile His Leu Leu Lys His Ile Lys Asn Ser Lys Ile Arg
   885 890 895
   Arg Gly Leu Val Lys Tyr Asn His Glu Phe Glu Val Gly Asp Asn Ser
   900 905 910
   Cys Ser Arg Ser Thr Ser Lys Asn Ala Glu Met His Asp Glu Cys Val
   915 920 925
   Asn Ile Cys Glu Lys Tyr Trp Pro Glu Cys Arg Gly Thr Ala Val Pro
   930 935 940
   Gly Tyr Cys Leu Ser Thr His Asp Asp Lys Asn Glu Cys Asp Phe Cys
   945 950 955 960
   Tyr Val
<210> 63
   <211> 997
   <212> PRT
   <213> Plasmodium falciparum
<400> 63
   Met Lys Ser Tyr Ile Ser Leu Phe Phe Ile Leu Cys Val Ile Phe Asn
   1 5 10 15
   Lys Asn Val Ile Lys Cys Thr Gly Glu Ser Gln Thr Gly Asn Thr Gly
   20 25 30
   Gly Gly Gln Ala Gly Asn Thr Gly Gly Asp Gln Ala Gly Ser Thr Gly
   35 40 45
   Gly Ser Pro Gln Gly Ser Thr Gly Ala Ser Pro Gln Gly Ser Thr Gly
   50 55 60
   Ala Ser Pro Gln Gly Ser Thr Gly Ala Ser Gln Pro Gly Ser Ser Glu
   65 70 75 80
   Pro Ser Asn Pro Val Ser Ser Gly His Ser Val Ser Thr Val Ser Val
   85 90 95
   Ser Gln Thr Ser Thr Ser Ser Glu Lys Gln Asp Thr Ile Gln Val Lys
   100 105 110
   Ser Ala Leu Leu Lys Asp Tyr Met Gly Leu Lys Val Thr Gly Pro Cys
   115 120 125
   Asn Glu Asn Phe Ile Met Phe Leu Val Pro His Ile Tyr Ile Asp Val
   130 135 140
   Asp Thr Glu Asp Thr Asn Ile Glu Leu Arg Thr Thr Leu Lys Lys Thr
   145 150 155 160
   Asn Asn Ala Ile Ser Phe Glu Ser Asn Ser Gly Ser Leu Glu Lys Lys
   165 170 175
   Lys Tyr Val Lys Leu Pro Ser Asn Gly Thr Thr Gly Glu Gln Gly Ser
   180 185 190
   Ser Thr Gly Thr Val Arg Gly Asp Thr Glu Pro Ile Ser Asp Ser Ser
   195 200 205
   Ser Ser Ser Ser Ser Ser Ser Ser Ser Ser Ser Ser Ser Ser Ser Ser
   210 215 220
   Ser Ser Ser Ser Ser Ser Ser Ser Ser Glu Ser Leu Pro Ala Asn Gly
   225 230 235 240
   Pro Asp Ser Pro Thr Val Lys Pro Pro Arg Asn Leu Gln Asn Ile Cys
   245 250 255
   Glu Thr Gly Lys Asn Phe Lys Leu Val Val Tyr Ile Lys Glu Asn Thr
   260 265 270
   Leu Ile Leu Lys Trp Lys Val Tyr Gly Glu Thr Lys Asp Thr Thr Glu
   275 280 285
   Asn Asn Lys Val Asp Val Arg Lys Tyr Leu Ile Asn Glu Lys Glu Thr
   290 295 300
   Pro Phe Thr Asn Ile Leu Ile His Ala Tyr Lys Glu His Asn Gly Thr
   305 310 315 320
   Asn Leu Ile Glu Ser Lys Asn Tyr Ala Ile Gly Ser Asp Ile Pro Glu
   325 330 335
   Lys Cys Asp Thr Leu Ala Ser Asn Cys Phe Leu Ser Gly Asn Phe Asn
   340 345 350
   Ile Glu Lys Cys Phe Gln Cys Ala Leu Leu Val Glu Lys Glu Asn Lys
   355 360 365
   Asn Asp Val Cys Tyr Lys Tyr Leu Ser Glu Asp Ile Val Ser Lys Phe
   370 375 380
   Lys Glu Ile Lys Ala Glu Thr Glu Asp Asp Asp Glu Asp Asp Tyr Thr
   385 390 395 400
   Glu Tyr Lys Leu Thr Glu Ser Ile Asp Asn Ile Leu Val Lys Met Phe
   405 410 415
   Lys Thr Asn Glu Asn Asn Asp Lys Ser Glu Leu Ile Lys Leu Glu Glu
   420 425 430
   Val Asp Asp Ser Leu Lys Leu Glu Leu Met Asn Tyr Cys Ser Leu Leu
   435 440 445
   Lys Asp Val Asp Thr Thr Gly Thr Leu Asp Asn Tyr Gly Met Gly Asn
   450 455 460
   Glu Met Asp Ile Phe Asn Asn Leu Lys Arg Leu Leu Ile Tyr His Ser
   465 470 475 480
   Glu Glu Asn Ile Asn Thr Leu Lys Asn Lys Phe Arg Asn Ala Ala Val
   485 490 495
   Cys Leu Lys Asn Val Asp Asp Trp Ile Val Asn Lys Arg Gly Leu Val
   500 505 510
   Leu Pro Glu Leu Asn Tyr Asp Leu Glu Tyr Phe Asn Glu His Leu Tyr
   515 520 525
   Asn Asp Lys Asn Ser Pro Glu Asp Lys Asp Asn Lys Gly Lys Gly Val
       530                 535                 540
   Val His Val Asp Thr Thr Leu Glu Lys Glu Asp Thr Leu Ser Tyr Asp
   545                 550                 555                 560
   Asn Ser Asp Asn Met Phe Cys Asn Lys Glu Tyr Cys Asn Arg Leu Lys
                   565                 570                 575
   Asp Glu Asn Asn Cys Ile Ser Asn Leu Gln Val Glu Asp Gln Gly Asn
               580                 585                 590
   Cys Asp Thr Ser Trp Ile Phe Ala Ser Lys Tyr His Leu Glu Thr Ile
           595                 600                 605
   Arg Cys Met Lys Gly Tyr Glu Pro Thr Lys Ile Ser Ala Leu Tyr Val
       610                 615                 620
   Ala Asn Cys Tyr Lys Gly Glu His Lys Asp Arg Cys Asp Glu Gly Ser
   625                 630                 635                 640
   Ser Pro Met Glu Phe Leu Gln Ile Ile Glu Asp Tyr Gly Phe Leu Pro
                   645                 650                 655
   Ala Glu Ser Asn Tyr Pro Tyr Asn Tyr Val Lys Val Gly Glu Gln Cys
               660                 665                 670
   Pro Lys Val Glu Asp His Trp Met Asn Leu Trp Asp Asn Gly Lys Ile
           675                 680                 685
   Leu His Asn Lys Asn Glu Pro Asn Ser Leu Asp Gly Lys Gly Tyr Thr
       690                 695                 700
   Ala Tyr Glu Ser Glu Arg Phe His Asp Asn Met Asp Ala Phe Val Lys
   705                 710                 715                 720
   Ile Ile Lys Thr Glu Val Met Asn Lys Gly Ser Val Ile Ala Tyr Ile
                   725                 730                 735
   Lys Ala Glu Asn Val Met Gly Tyr Glu Phe Ser Gly Lys Lys Val Gln
               740                 745                 750
   Asn Leu Cys Gly Asp Asp Thr Ala Asp His Ala Val Asn Ile Val Gly
           755                 760                 765
   Tyr Gly Asn Tyr Val Asn Ser Glu Gly Glu Lys Lys Ser Tyr Trp Ile
       770                 775                 780
   Val Arg Asn Ser Trp Gly Pro Tyr Trp Gly Asp Glu Gly Tyr Phe Lys
   785 790 795 800
   Val Asp Met Tyr Gly Pro Thr His Cys His Phe Asn Phe Ile His Ser
   805 810 815
   Val Val Ile Phe Asn Val Asp Leu Pro Met Asn Asn Lys Thr Thr Lys
   820 825 830
   Lys Glu Ser Lys Ile Tyr Asp Tyr Tyr Leu Lys Ala Ser Pro Glu Phe
   835 840 845
   Tyr His Asn Leu Tyr Phe Lys Asn Phe Asn Val Gly Lys Lys Asn Leu
   850 855 860
   Phe Ser Glu Lys Glu Asp Asn Glu Asn Asn Lys Lys Leu Gly Asn Asn
   865 870 875 880
   Tyr Ile Ile Phe Gly Gln Asp Thr Ala Gly Ser Gly Gln Ser Gly Lys
   885 890 895
   Glu Ser Asn Thr Ala Leu Glu Ser Ala Gly Thr Ser Asn Glu Val Ser
   900 905 910
   Glu Arg Val His Val Tyr His Ile Leu Lys His Ile Lys Asp Gly Lys
   915 920 925
   Ile Arg Met Gly Met Arg Lys Tyr Ile Asp Thr Gln Asp Val Asn Lys
   930 935 940
   Lys His Ser Cys Thr Arg Ser Tyr Ala Phe Asn Pro Glu Asn Tyr Glu
   945 950 955 960
   Lys Cys Val Asn Leu Cys Asn Val Asn Trp Lys Thr Cys Glu Glu Lys
   965 970 975
   Thr Ser Pro Gly Leu Cys Leu Ser Lys Leu Asp Thr Asn Asn Glu Cys
   980 985 990
   Tyr Phe Cys Tyr Val
   995
<210> 64
   <211> 1233
   <212> PRT
   <213> Plasmodium falciparum
<400> 64
   Met Arg Asn Leu Phe His Ile Thr Ile Cys Leu Val Thr Leu Asn Leu
   1               5                   10                  15
   Phe Ile Leu Glu Ile Asn Ala Lys Thr Asn Thr Ser Glu Asn Arg Asn
               20                  25                  30
   Lys Arg Ile Gly Gly Pro Lys Leu Arg Gly Asn Val Thr Ser Asn Ile
           35                  40                  45
   Lys Phe Pro Ser Asp Asn Lys Gly Lys Ile Ile Arg Gly Ser Asn Asp
       50                  55                  60
   Lys Leu Asn Lys Asn Ser Glu Asp Val Leu Glu Gln Ser Glu Lys Ser
   65                  70                  75                  80
   Leu Val Ser Glu Asn Val Pro Ser Gly Leu Asp Ile Asp Asp Ile Pro
                   85                  90                  95
   Lys Glu Ser Ile Phe Ile Gln Glu Asp Gln Glu Gly Gln Thr His Ser
               100                 105                 110
   Glu Leu Asn Pro Glu Thr Ser Glu His Ser Lys Asp Leu Asn Asn Asn
           115                 120                 125
   Asp Ser Lys Asn Glu Ser Ser Asp Ile Ile Ser Val Asn Asn Lys Ser
       130                 135                 140
   Asn Lys Val Gln Asn His Phe Glu Ser Leu Ser Asp Leu Glu Leu Leu
   145                 150                 155                 160
   Glu Asn Ser Ser Gln Asp Asn Leu Asp Lys Asp Thr Ile Ser Thr Glu
                   165                 170                 175
   Pro Phe Pro Asn Gln Lys His Lys Asp Leu Gln Gln Asp Leu Asn Asp
               180                 185                 190
   Glu Pro Leu Glu Pro Phe Pro Thr Gln Ile His Lys Asp Tyr Lys Glu
           195                 200                 205
   Lys Asn Leu Ile Asn Glu Glu Asp Ser Glu Pro Phe Pro Arg Gln Lys
       210                 215                 220
   His Lys Lys Val Asp Asn His Asn Glu Glu Lys Asn Val Phe His Glu
   225                 230                 235                 240
   Asn Gly Ser Ala Asn Gly Asn Gln Gly Ser Leu Lys Leu Lys Ser Phe
                   245                 250                 255
   Asp Glu His Leu Lys Asp Glu Lys Ile Glu Asn Glu Pro Leu Val His
               260                 265                 270
   Glu Asn Leu Ser Ile Pro Asn Asp Pro Ile Glu Gln Ile Leu Asn Gln
           275                 280                 285
   Pro Glu Gln Glu Thr Asn Ile Gln Glu Gln Leu Tyr Asn Glu Lys Gln
       290                 295                 300
   Asn Val Glu Glu Lys Gln Asn Ser Gln Ile Pro Ser Leu Asp Leu Lys
   305                 310                 315                 320
   Glu Pro Thr Asn Glu Asp Ile Leu Pro Asn His Asn Pro Leu Glu Asn
                   325                 330                 335
   Ile Lys Gln Ser Glu Ser Glu Ile Asn His Val Gln Asp His Ala Leu
               340                 345                 350
   Pro Lys Glu Asn Ile Ile Asp Lys Leu Asp Asn Gln Lys Glu His Ile
           355                 360                 365
   Asp Gln Ser Gln His Asn Ile Asn Val Leu Gln Glu Asn Asn Ile Asn
       370                 375                 380
   Asn His Gln Leu Glu Pro Gln Glu Lys Pro Asn Ile Glu Ser Phe Glu
   385                 390                 395                 400
   Pro Lys Asn Ile Asp Ser Glu Ile Ile Leu Pro Glu Asn Val Glu Thr
                   405                 410                 415
   Glu Glu Ile Ile Asp Asp Val Pro Ser Pro Lys His Ser Asn His Glu
               420                 425                 430
   Thr Phe Glu Glu Glu Thr Ser Glu Ser Glu His Glu Glu Ala Val Ser
           435                 440                 445
   Glu Lys Asn Ala His Glu Thr Val Glu His Glu Glu Thr Val Ser Gln
       450                 455                 460
   Glu Ser Asn Pro Glu Lys Ala Asp Asn Asp Gly Asn Val Ser Gln Asn
   465                 470                 475                 480
   Ser Asn Asn Glu Leu Asn Glu Asn Glu Phe Val Glu Ser Glu Lys Ser
                   485                 490                 495
   Glu His Glu Pro Ala Glu Asn Glu Glu Ser Ser Leu Glu Glu Gly His
   500 505 510
   His Glu Glu Ile Val Pro Glu Gln Asn Asn Glu Glu Ser Gly Glu Ser
   515 520 525
   Lys Leu Val Asp Asn Asp Glu Gly Gly Phe Glu Glu Ala His His Glu
   530 535 540
   Asn Phe Ser Ser Glu Val Ser Asn Ser Glu Leu Asn Glu Asn Glu Phe
   545 550 555 560
   Val Glu Ser Asp Lys Ser Val Thr Glu Pro Ala Glu His Glu Glu Val
   565 570 575
   Val Ser Glu Glu Ser Asn Pro Glu Pro Ala Glu Asn Glu Glu Ser Ser
   580 585 590
   Ile Glu Glu Ala His Gln Glu Glu Ile Val Pro Glu Gln Asn Asp Glu
   595 600 605
   Glu Ser Gly Glu Ser Gly Leu Val Asp Asn Glu Glu Gly Asp Phe Glu
   610 615 620
   Glu Pro Asn His Glu Glu Phe Glu Pro Asp Gln Asn Asp Ser Glu Leu
   625 630 635 640
   Ser Glu Asn Glu Leu Val Glu Ser Glu Lys Ser Val Ser Glu Pro Ala
   645 650 655
   Glu His Val Glu Ile Val Ser Glu Lys Ser Val Ser Glu Pro Ala Glu
   660 665 670
   His Val Glu Ile Val Ser Glu Lys Ser Thr Ser Glu Pro Ala Glu His
   675 680 685
   Val Glu Ser Val Ser Glu Gln Ser Asn Asn Glu Pro Ser Glu Lys Lys
   690 695 700
   Asp Gly Pro Val Pro Ser Lys Pro Phe Glu Glu Ile Glu Lys Val Asp
   705 710 715 720
   Val Gln Pro Lys Ile Val Asp Leu Gln Ile Ile Glu Pro Asn Phe Val
   725 730 735
   Asp Ser Gln Pro Asn Pro Gln Glu Pro Val Glu Pro Ser Phe Val Lys
   740 745 750
   Ile Glu Lys Val Pro Ser Glu Glu Asn Lys His Ala Ser Val Asp Pro
   755 760 765
   Glu Val Lys Glu Lys Glu Asn Val Ser Glu Val Val Glu Glu Lys Gln
   770 775 780
   Asn Ser Gln Glu Ser Val Glu Glu Ile Pro Val Asn Glu Asp Glu Phe
   785 790 795 800
   Glu Asp Val His Thr Glu Gln Leu Asp Leu Asp His Lys Thr Val Asp
   805 810 815
   Pro Glu Ile Val Glu Val Glu Glu Ile Pro Ser Glu Leu His Glu Asn
   820 825 830
   Glu Val Ala His Pro Glu Ile Val Glu Ile Glu Glu Val Phe Pro Glu
   835 840 845
   Pro Asn Gln Asn Asn Glu Phe Gln Glu Ile Asn Glu Asp Asp Lys Ser
   850 855 860
   Ala His Ile Gln His Glu Ile Val Glu Val Glu Glu Ile Leu Pro Glu
   865 870 875 880
   Asp Asp Lys Asn Glu Lys Val Glu His Glu Ile Val Glu Val Glu Glu
   885 890 895
   Ile Leu Pro Glu Asp Lys Asn Glu Lys Val Gln His Glu Ile Val Glu
   900 905 910
   Val Glu Glu Ile Leu Pro Glu Asp Lys Asn Glu Lys Val Glu His Glu
   915 920 925
   Ile Val Glu Val Glu Glu Ile Leu Pro Glu Asp Lys Asn Glu Lys Gly
   930 935 940
   Gln His Glu Ile Val Glu Val Glu Glu Ile Leu Pro Glu Asp Lys Asn
   945 950 955 960
   Glu Lys Val Gln His Glu Ile Val Glu Val Glu Glu Ile Leu Pro Glu
   965 970 975
   Asp Lys Asn Glu Lys Gly Gln His Glu Ile Val Glu Val Glu Glu Ile
   980 985 990
   Leu Pro Glu Asp Lys Asn Glu Lys Val Glu His Glu Ile Val Glu Val
   995 1000 1005
   Glu Glu Ile Leu Pro Glu Asp Lys Asn Glu Lys Gly Gln His Glu
   1010 1015 1020
   Ile Val Glu Val Glu Glu Ile Leu Pro Glu Asp Asp Lys Asn Glu
   1025 1030 1035
   Lys Gly Gln His Glu Ile Val Glu Val Glu Glu Ile Leu Pro Glu
   1040 1045 1050
   Ile Val Glu Ile Glu Glu Val Pro Ser Gln Thr Asn Asn Asn Glu
   1055 1060 1065
   Asn Ile Glu Thr Ile Lys Pro Glu Glu Lys Lys Asn Glu Phe Ser
   1070 1075 1080
   Val Val Glu Glu Lys Ala Ile Pro Gln Glu Pro Val Val Pro Thr
   1085 1090 1095
   Leu Asn Glu Asn Glu Asn Val Thr Pro Lys Pro Ser Glu Gly Glu
   1100 1105 1110
   Ser Thr Lys Pro Asp Ile Val Gln Ile Lys Ile Val Gln Glu Asn
   1115 1120 1125
   Lys Pro Asn Lys Lys Glu Thr Pro Val Val Asp Gly Pro Lys His
   1130 1135 1140
   Val Glu Gln Asn Ile Gln Glu Asp Asp Asn Asp Glu Glu Asp Asp
   1145 1150 1155
   Asp Asp Ile Asp Phe Glu Gly Leu Ser Arg Lys Asp Asp Glu Lys
   1160 1165 1170
   Asp Ser Ser Asn Lys Asn Lys Lys Lys Ser Ser Phe Ile Thr Tyr
   1175 1180 1185
   Ile Ser Thr Lys Lys Phe Lys Lys Val Ser Gln Thr Ile Val Ser
   1190 1195 1200
   Val Met Ile Asn Ala Tyr Asp Gly Val Ile Gln Val Val Ser Thr
   1205 1210 1215
   Ile Lys Gly Ile Ala Lys Asp Ile Val Ile Phe Phe Gln Asn Ile
   1220 1225 1230
<210> 65
   <211> 396
   <212> PRT
   <213> Plasmodium falciparum
<400> 65
   Asp Ile Pro Lys Glu Ser Ile Phe Ile Gln Glu Asp Gln Glu Gly Gln
   1               5                   10                  15
   Thr His Ser Glu Leu Asn Pro Glu Thr Ser Glu His Ser Lys Asp Leu
               20                  25                  30
   Asn Asn Asn Asp Ser Lys Asn Glu Ser Ser Asp Ile Ile Ser Val Asn
           35                  40                  45
   Asn Lys Ser Asn Lys Val Gln Asn His Phe Glu Ser Leu Ser Asp Leu
       50                  55                  60
   Glu Leu Leu Glu Asn Ser Ser Gln Asp Asn Leu Asp Lys Asp Thr Ile
   65                  70                  75                  80
   Ser Thr Glu Pro Phe Pro Asn Gln Lys His Lys Asp Leu Gln Gln Asp
                   85                  90                  95
   Leu Asn Asp Glu Pro Leu Glu Pro Phe Pro Thr Gln Ile His Lys Asp
               100                 105                 110
   Tyr Lys Glu Lys Asn Leu Ile Asn Glu Glu Asp Ser Glu Pro Phe Pro
           115                 120                 125
   Arg Gln Lys His Lys Lys Val Asp Asn His Asn Glu Glu Lys Asn Val
       130                 135                 140
   Phe His Glu Asn Gly Ser Ala Asn Gly Asn Gln Gly Ser Leu Lys Leu
   145                 150                 155                 160
   Lys Ser Phe Asp Glu His Leu Lys Asp Glu Lys Ile Glu Asn Glu Pro
                   165                 170                 175
   Leu Val His Glu Asn Leu Ser Ile Pro Asn Asp Pro Ile Glu Gln Ile
               180                 185                 190
   Leu Asn Gln Pro Glu Gln Glu Thr Asn Ile Gln Glu Gln Leu Tyr Asn
           195                 200                 205
   Glu Lys Gln Asn Val Glu Glu Lys Gln Asn Ser Gln Ile Pro Ser Leu
       210                 215                 220
   Asp Leu Lys Glu Pro Thr Asn Glu Asp Ile Leu Pro Asn His Asn Pro
   225                 230                 235                 240
   Leu Glu Asn Ile Lys Gln Ser Glu Ser Glu Ile Asn His Val Gln Asp
                   245                 250                 255
   His Ala Leu Pro Lys Glu Asn Ile Ile Asp Lys Leu Asp Asn Gln Lys
               260                 265                 270
   Glu His Ile Asp Gln Ser Gln His Asn Ile Asn Val Leu Gln Glu Asn
           275                 280                 285
   Asn Ile Asn Asn His Gln Leu Glu Pro Gln Glu Lys Pro Asn Ile Glu
       290                 295                 300
   Ser Phe Glu Pro Lys Asn Ile Asp Ser Glu Ile Ile Leu Pro Glu Asn
   305                 310                 315                 320
   Val Glu Thr Glu Glu Ile Ile Asp Asp Val Pro Ser Pro Lys His Ser
                   325                 330                 335
   Asn His Glu Thr Phe Glu Glu Glu Thr Ser Glu Ser Glu His Glu Glu
               340                 345                 350
   Ala Val Ser Glu Lys Asn Ala His Glu Thr Val Glu His Glu Glu Thr
           355                 360                 365
   Val Ser Gln Glu Ser Asn Pro Glu Lys Ala Asp Asn Asp Gly Asn Val
       370                 375                 380
   Ser Gln Asn Ser Asn Asn Glu Leu Asn Glu Asn Glu
   385                 390                 395
<210> 66
   <211> 436
   <212> PRT
   <213> Plasmodium falciparum
<400> 66
   Asp Gly Pro Val Pro Ser Lys Pro Phe Glu Glu Ile Glu Lys Val Asp
   1               5                   10                  15
   Val Gln Pro Lys Ile Val Asp Leu Gln Ile Ile Glu Pro Asn Phe Val
               20                  25                  30
   Asp Ser Gln Pro Asn Pro Gln Glu Pro Val Glu Pro Ser Phe Val Lys
           35                  40                  45
   Ile Glu Lys Val Pro Ser Glu Glu Asn Lys His Ala Ser Val Asp Pro
   50 55 60
   Glu Val Lys Glu Lys Glu Asn Val Ser Glu Val Val Glu Glu Lys Gln
   65 70 75 80
   Asn Ser Gln Glu Ser Val Glu Glu Ile Pro Val Asn Glu Asp Glu Phe
   85 90 95
   Glu Asp Val His Thr Glu Gln Leu Asp Leu Asp His Lys Thr Val Asp
   100 105 110
   Pro Glu Ile Val Glu Val Glu Glu Ile Pro Ser Glu Leu His Glu Asn
   115 120 125
   Glu Val Ala His Pro Glu Ile Val Glu Ile Glu Glu Val Phe Pro Glu
   130 135 140
   Pro Asn Gln Asn Asn Glu Phe Gln Glu Ile Asn Glu Asp Asp Lys Ser
   145 150 155 160
   Ala His Ile Gln His Glu Ile Val Glu Val Glu Glu Ile Leu Pro Glu
   165 170 175
   Asp Asp Lys Asn Glu Lys Val Glu His Glu Ile Val Glu Val Glu Glu
   180 185 190
   Ile Leu Pro Glu Asp Lys Asn Glu Lys Val Gln His Glu Ile Val Glu
   195 200 205
   Val Glu Glu Ile Leu Pro Glu Asp Lys Asn Glu Lys Val Glu His Glu
   210 215 220
   Ile Val Glu Val Glu Glu Ile Leu Pro Glu Asp Lys Asn Glu Lys Gly
   225 230 235 240
   Gln His Glu Ile Val Glu Val Glu Glu Ile Leu Pro Glu Asp Lys Asn
   245 250 255
   Glu Lys Val Gln His Glu Ile Val Glu Val Glu Glu Ile Leu Pro Glu
   260 265 270
   Asp Lys Asn Glu Lys Gly Gln His Glu Ile Val Glu Val Glu Glu Ile
   275 280 285
   Leu Pro Glu Asp Lys Asn Glu Lys Val Glu His Glu Ile Val Glu Val
   290 295 300
   Glu Glu Ile Leu Pro Glu Asp Lys Asn Glu Lys Gly Gln His Glu Ile
   305 310 315 320
   Val Glu Val Glu Glu Ile Leu Pro Glu Asp Asp Lys Asn Glu Lys Gly
   325 330 335
   Gln His Glu Ile Val Glu Val Glu Glu Ile Leu Pro Glu Ile Val Glu
   340 345 350
   Ile Glu Glu Val Pro Ser Gln Thr Asn Asn Asn Glu Asn Ile Glu Thr
   355 360 365
   Ile Lys Pro Glu Glu Lys Lys Asn Glu Phe Ser Val Val Glu Glu Lys
   370 375 380
   Ala Ile Pro Gln Glu Pro Val Val Pro Thr Leu Asn Glu Asn Glu Asn
   385 390 395 400
   Val Thr Pro Lys Pro Ser Glu Gly Glu Ser Thr Lys Pro Asp Ile Val
   405 410 415
   Gln Ile Lys Ile Val Gln Glu Asn Lys Pro Asn Lys Lys Glu Thr Pro
   420 425 430
   Val Val Asp Gly
   435
<210> 67
   <211> 824
   <212> PRT
   <213> Plasmodium falciparum
<400> 67
   Met Arg Leu Ser Lys Val Ser Asp Ile Lys Ser Thr Gly Val Ser Asn
   1 5 10 15
   Tyr Lys Asn Phe Asn Ser Lys Asn Ser Ser Lys Tyr Ser Leu Met Glu
   20 25 30
   Val Ser Lys Lys Asn Glu Lys Lys Asn Ser Leu Gly Ala Phe His Ser
   35 40 45
   Lys Lys Ile Leu Leu Ile Phe Gly Ile Ile Tyr Val Val Leu Leu Asn
   50 55 60
   Ala Tyr Ile Cys Gly Asp Lys Tyr Glu Lys Ala Val Asp Tyr Gly Phe
   65 70 75 80
   Arg Glu Ser Arg Ile Leu Ala Glu Gly Glu Asp Thr Cys Ala Arg Lys
                   85                  90                  95
   Glu Lys Thr Thr Leu Arg Lys Ser Lys Gln Lys Thr Ser Thr Arg Thr
               100                 105                 110
   Val Ala Thr Gln Thr Lys Lys Asp Glu Glu Asn Lys Ser Val Val Thr
           115                 120                 125
   Glu Glu Gln Lys Val Glu Ser Asp Ser Glu Lys Gln Lys Arg Thr Lys
       130                 135                 140
   Lys Val Val Lys Lys Gln Ile Asn Ile Gly Asp Thr Glu Asn Gln Lys
   145                 150                 155                 160
   Glu Gly Lys Asn Val Lys Lys Val Ile Lys Lys Glu Lys Lys Lys Glu
                   165                 170                 175
   Glu Ser Gly Lys Pro Glu Glu Asn Lys His Ala Asn Glu Ala Ser Lys
               180                 185                 190
   Lys Gln Glu Pro Lys Ala Ser Lys Val Ser Gln Lys Pro Ser Thr Ser
           195                 200                 205
   Thr Arg Ser Asn Asn Glu Val Lys Ile Arg Ala Ala Ser Asn Gln Glu
       210                 215                 220
   Thr Leu Thr Ser Ala Asp Pro Glu Gly Gln Ile Met Arg Glu Tyr Ala
   225                 230                 235                 240
   Ala Asp Pro Glu Tyr Arg Lys His Leu Glu Ile Phe Tyr Lys Ile Leu
                   245                 250                 255
   Thr Asn Thr Asp Pro Asn Asp Glu Val Glu Arg Arg Asn Ala Asp Asn
               260                 265                 270
   Lys Glu Asp Leu Thr Ser Ala Asp Pro Glu Gly Gln Ile Met Arg Glu
           275                 280                 285
   Tyr Ala Ser Asp Pro Glu Tyr Arg Lys His Leu Glu Ile Phe Tyr Lys
       290                 295                 300
   Ile Leu Thr Asn Thr Asp Pro Asn Asp Asp Val Glu Arg Arg Asn Ala
   305                 310                 315                 320
   Asp Asn Lys Glu Asp Leu Thr Ser Ala Asp Pro Glu Gly Gln Ile Met
                   325                 330                 335
   Arg Glu Tyr Ala Ala Asp Pro Glu Tyr Arg Lys His Leu Glu Val Phe
               340                 345                 350
   His Lys Ile Leu Thr Asn Thr Asp Pro Asn Asp Glu Val Glu Arg Arg
           355                 360                 365
   Asn Ala Asp Asn Lys Glu Asp Leu Thr Ser Ala Asp Pro Glu Gly Gln
       370                 375                 380
   Ile Met Arg Glu Tyr Ala Ala Asp Pro Glu Tyr Arg Lys His Leu Glu
   385                 390                 395                 400
   Val Phe His Lys Ile Leu Thr Asn Thr Asp Pro Asn Asp Glu Val Glu
                   405                 410                 415
   Arg Arg Asn Ala Asp Asn Lys Glu Leu Thr Ser Ser Asp Pro Glu Gly
               420                 425                 430
   Gln Ile Met Arg Glu Tyr Ala Ala Asp Pro Glu Tyr Arg Lys His Leu
           435                 440                 445
   Glu Val Phe His Lys Ile Leu Thr Asn Thr Asp Pro Asn Asp Glu Val
       450                 455                 460
   Glu Arg Arg Asn Ala Asp Asn Lys Glu Asp Leu Thr Ser Ala Asp Pro
   465                 470                 475                 480
   Glu Gly Gln Ile Met Arg Glu Tyr Ala Ala Asp Pro Glu Tyr Arg Lys
                   485                 490                 495
   His Leu Glu Val Phe His Lys Ile Leu Thr Asn Thr Asp Pro Asn Asp
               500                 505                 510
   Glu Val Glu Arg Arg Asn Ala Asp Asn Lys Glu Asp Leu Thr Ser Ala
           515                 520                 525
   Asp Pro Glu Gly Gln Ile Met Arg Glu Tyr Ala Ala Asp Pro Glu Tyr
       530                 535                 540
   Arg Lys His Leu Glu Ile Phe His Lys Ile Leu Thr Asn Thr Asp Pro
   545                 550                 555                 560
   Asn Asp Glu Val Glu Arg Arg Asn Ala Asp Asn Lys Glu Asp Leu Thr
                   565                 570                 575
   Ser Ala Asp Pro Glu Gly Gln Ile Met Arg Glu Tyr Ala Ala Asp Pro
               580                 585                 590
   Glu Tyr Arg Lys His Leu Glu Ile Phe Tyr Lys Ile Leu Thr Asn Thr
   595 600 605
   Asp Pro Asn Asp Glu Val Glu Arg Arg Asn Ala Asp Asn Lys Glu Glu
   610 615 620
   Leu Thr Ser Ser Asp Pro Glu Gly Gln Ile Met Arg Glu Tyr Ala Ala
   625 630 635 640
   Asp Pro Glu Tyr Arg Lys His Leu Glu Ile Phe His Lys Ile Leu Thr
   645 650 655
   Asn Thr Asp Pro Asn Asp Glu Val Glu Arg Arg Asn Ala Asp Asn Lys
   660 665 670
   Glu Asp Leu Thr Ser Ala Asp Pro Glu Gly Gln Ile Met Arg Glu Tyr
   675 680 685
   Ala Ala Asp Pro Glu Tyr Arg Lys His Leu Glu Ile Phe Tyr Lys Ile
   690 695 700
   Leu Thr Asn Thr Asp Pro Asn Asp Glu Val Glu Arg Arg Asn Ala Asp
   705 710 715 720
   Asn Lys Glu Asp Leu Thr Ser Ala Asp Pro Glu Gly Gln Ile Met Arg
   725 730 735
   Glu Tyr Ala Ser Asp Pro Glu Tyr Arg Lys His Leu Glu Ile Phe Tyr
   740 745 750
   Lys Ile Leu Thr Asn Thr Asp Pro Asn Asp Asp Val Glu Arg Arg Asn
   755 760 765
   Ala Asp Asn Lys Glu Asp Leu Thr Ser Ala Asp Pro Glu Gly Gln Ile
   770 775 780
   Met Arg Glu Tyr Ala Ala Asp Pro Glu Tyr Arg Lys His Leu Glu Val
   785 790 795 800
   Phe His Lys Ile Leu Thr Asn Thr Asp Pro Asn Asp Glu Val Glu Arg
   805 810 815
   Gln Asn Ala Asp Asn Asn Glu Ala
   820
<210> 68
   <211> 41
   <212> PRT
   <213> Plasmodium falciparum
<400> 68
   Gly Met Asn Asn Met Asn Gly Asp Ile Asn Asn Ile Asn Gly Asp Ile
   1               5                   10                  15
   Asn Asn Met Asn Gly Asp Ile Asn Asn Met Asn Gly Asp Ile Asn Asn
               20                  25                  30
   Met Asn Gly Asp Ile Asn Asn Met Asn
           35                  40
<210> 69
   <211> 37
   <212> PRT
   <213> Plasmodium falciparum
<400> 69
   Glu Glu Ile Lys Glu Glu Ile Lys Glu Val Lys Glu Glu Ile Lys Glu
   1               5                   10                  15
   Val Lys Glu Glu Ile Lys Glu Val Lys Glu Glu Ile Lys Glu Val Lys
               20                  25                  30
   Glu Glu Ile Lys Glu
           35
<210> 70
   <211> 28
   <212> PRT
   <213> Plasmodium falciparum
<400> 70
   Glu Lys Leu Lys Lys Tyr Asn Asn Glu Ile Ser Ser Leu Lys Lys Glu
   1               5                   10                  15
   Leu Asp Ile Leu Asn Glu Lys Met Gly Lys Cys Thr
               20                  25
<210> 71
   <211> 38
   <212> PRT
   <213> Plasmodium falciparum
<400> 71
   Thr Lys Lys Leu Asn Lys Glu Leu Ser Glu Gly Asn Lys Glu Leu Glu
   1               5                   10                  15
   Lys Leu Glu Lys Asn Ile Lys Glu Leu Glu Glu Thr Asn Asn Thr Leu
   20 25 30
   Glu Asn Asp Ile Lys Val
   35
<210> 72
   <211> 526
   <212> PRT
   <213> Plasmodium falciparum
<400> 72
   Met Ile Arg Ile Lys Lys Lys Leu Ile Leu Thr Ile Ile Tyr Ile His
   1 5 10 15
   Leu Phe Ile Leu Asn Arg Leu Ser Phe Glu Asn Ala Ile Lys Lys Thr
   20 25 30
   Lys Asn Gln Glu Asn Asn Leu Thr Leu Leu Pro Ile Lys Ser Thr Glu
   35 40 45
   Glu Glu Lys Asp Asp Ile Lys Asn Gly Lys Asp Ile Lys Lys Glu Ile
   50 55 60
   Asp Asn Asp Lys Glu Asn Ile Lys Thr Asn Asn Ala Lys Asp His Ser
   65 70 75 80
   Thr Tyr Ile Lys Ser Tyr Leu Asn Thr Asn Val Asn Asp Gly Leu Lys
   85 90 95
   Tyr Leu Phe Ile Pro Ser His Asn Ser Phe Ile Lys Lys Tyr Ser Val
   100 105 110
   Phe Asn Gln Ile Asn Asp Gly Met Leu Leu Asn Glu Lys Asn Asp Val
   115 120 125
   Lys Asn Asn Glu Asp Tyr Lys Asn Val Asp Tyr Lys Asn Val Asn Phe
   130 135 140
   Leu Gln Tyr His Phe Lys Glu Leu Ser Asn Tyr Asn Ile Ala Asn Ser
   145 150 155 160
   Ile Asp Ile Leu Gln Glu Lys Glu Gly His Leu Asp Phe Val Ile Ile
   165 170 175
   Pro His Tyr Thr Phe Leu Asp Tyr Tyr Lys His Leu Ser Tyr Asn Ser
   180 185 190
   Ile Tyr His Lys Ser Ser Thr Tyr Gly Lys Cys Ile Ala Val Asp Ala
           195                 200                 205
   Phe Ile Lys Lys Ile Asn Glu Thr Tyr Asp Lys Val Lys Ser Lys Cys
       210                 215                 220
   Asn Asp Ile Lys Asn Asp Leu Ile Ala Thr Ile Lys Lys Leu Glu His
   225                 230                 235                 240
   Pro Tyr Asp Ile Asn Asn Lys Asn Asp Asp Ser Tyr Arg Tyr Asp Ile
                   245                 250                 255
   Ser Glu Glu Ile Asp Asp Lys Ser Glu Glu Thr Asp Asp Glu Thr Glu
               260                 265                 270
   Glu Val Glu Asp Ser Ile Gln Asp Thr Asp Ser Asn His Thr Pro Ser
           275                 280                 285
   Asn Lys Lys Lys Asn Asp Leu Met Asn Arg Thr Phe Lys Lys Met Met
       290                 295                 300
   Asp Glu Tyr Asn Thr Lys Lys Lys Lys Leu Ile Lys Cys Ile Lys Asn
   305                 310                 315                 320
   His Glu Asn Asp Phe Asn Lys Ile Cys Met Asp Met Lys Asn Tyr Gly
                   325                 330                 335
   Thr Asn Leu Phe Glu Gln Leu Ser Cys Tyr Asn Asn Asn Phe Cys Asn
               340                 345                 350
   Thr Asn Gly Ile Arg Tyr His Tyr Asp Glu Tyr Ile His Lys Leu Ile
           355                 360                 365
   Leu Ser Val Lys Ser Lys Asn Leu Asn Lys Asp Leu Ser Asp Met Thr
       370                 375                 380
   Asn Ile Leu Gln Gln Ser Glu Leu Leu Leu Thr Asn Leu Asn Lys Lys
   385                 390                 395                 400
   Met Gly Ser Tyr Ile Tyr Ile Asp Thr Ile Lys Phe Ile His Lys Glu
                   405                 410                 415
   Met Lys His Ile Phe Asn Arg Ile Glu Tyr His Thr Lys Ile Ile Asn
               420                 425                 430
   Asp Lys Thr Lys Ile Ile Gln Asp Lys Ile Lys Leu Asn Ile Trp Arg
           435                 440                 445
   Thr Phe Gln Lys Asp Glu Leu Leu Lys Arg Ile Leu Asp Met Ser Asn
   450 455 460
   Glu Tyr Ser Leu Phe Ile Thr Ser Asp His Leu Arg Gln Met Leu Tyr
   465 470 475 480
   Asn Thr Phe Tyr Ser Lys Glu Lys His Leu Asn Asn Ile Phe His His
   485 490 495
   Leu Ile Tyr Val Leu Gln Met Lys Phe Asn Asp Val Pro Ile Lys Met
   500 505 510
   Glu Tyr Phe Gln Thr Tyr Lys Lys Asn Lys Pro Leu Thr Gln
   515 520 525
<210> 73
   <211> 622
   <212> PRT
   <213> Plasmodium falciparum
<400> 73
   Met Arg Lys Leu Tyr Cys Val Leu Leu Leu Ser Ala Phe Glu Phe Thr
   1 5 10 15
   Tyr Met Ile Asn Phe Gly Arg Gly Gln Asn Tyr Trp Glu His Pro Tyr
   20 25 30
   Gln Asn Ser Asp Val Tyr Arg Pro Ile Asn Glu His Arg Glu His Pro
   35 40 45
   Lys Glu Tyr Glu Tyr Pro Leu His Gln Glu His Thr Tyr Gln Gln Glu
   50 55 60
   Asp Ser Gly Glu Asp Glu Asn Thr Leu Gln His Ala Tyr Pro Ile Asp
   65 70 75 80
   His Glu Gly Ala Glu Pro Ala Pro Gln Glu Gln Asn Leu Phe Ser Ser
   85 90 95
   Ile Glu Ile Val Glu Arg Ser Asn Tyr Met Gly Asn Pro Trp Thr Glu
   100 105 110
   Tyr Met Ala Lys Tyr Asp Ile Glu Glu Val His Gly Ser Gly Ile Arg
   115 120 125
   Val Asp Leu Gly Glu Asp Ala Glu Val Ala Gly Thr Gln Tyr Arg Leu
   130 135 140
   Pro Ser Gly Lys Cys Pro Val Phe Gly Lys Gly Ile Ile Ile Glu Asn
   145                 150                 155                 160
   Ser Asn Thr Thr Phe Leu Thr Pro Val Ala Thr Gly Asn Gln Tyr Leu
                   165                 170                 175
   Lys Asp Gly Gly Phe Ala Phe Pro Pro Thr Glu Pro Leu Met Ser Pro
               180                 185                 190
   Met Thr Leu Asp Glu Met Arg His Phe Tyr Lys Asp Asn Lys Tyr Val
           195                 200                 205
   Lys Asn Leu Asp Glu Leu Thr Leu Cys Ser Arg His Ala Gly Asn Met
       210                 215                 220
   Ile Pro Asp Asn Asp Lys Asn Ser Asn Tyr Lys Tyr Pro Ala Val Tyr
   225                 230                 235                 240
   Asp Asp Lys Asp Lys Lys Cys His Ile Leu Tyr Ile Ala Ala Gln Glu
                   245                 250                 255
   Asn Asn Gly Pro Arg Tyr Cys Asn Lys Asp Glu Ser Lys Arg Asn Ser
               260                 265                 270
   Met Phe Cys Phe Arg Pro Ala Lys Asp Ile Ser Phe Gln Asn Tyr Thr
           275                 280                 285
   Tyr Leu Ser Lys Asn Val Val Asp Asn Trp Glu Lys Val Cys Pro Arg
       290                 295                 300
   Lys Asn Leu Gln Asn Ala Lys Phe Gly Leu Trp Val Asp Gly Asn Cys
   305                 310                 315                 320
   Glu Asp Ile Pro His Val Asn Glu Phe Pro Ala Ile Asp Leu Phe Glu
                   325                 330                 335
   Cys Asn Lys Leu Val Phe Glu Leu Ser Ala Ser Asp Gln Pro Lys Gln
               340                 345                 350
   Tyr Glu Gln His Leu Thr Asp Tyr Glu Lys Ile Lys Glu Gly Phe Lys
           355                 360                 365
   Asn Lys Asn Ala Ser Met Ile Lys Ser Ala Phe Leu Pro Thr Gly Ala
       370                 375                 380
   Phe Lys Ala Asp Arg Tyr Lys Ser His Gly Lys Gly Tyr Asn Trp Gly
   385                 390                 395                 400
   Asn Tyr Asn Thr Glu Thr Gln Lys Cys Glu Ile Phe Asn Val Lys Pro
                   405                 410                 415
   Thr Cys Leu Ile Asn Asn Ser Ser Tyr Ile Ala Thr Thr Ala Leu Ser
               420                 425                 430
   His Pro Ile Glu Val Glu Asn Asn Phe Pro Cys Ser Leu Tyr Lys Asp
           435                 440                 445
   Glu Ile Met Lys Glu Ile Glu Arg Glu Ser Lys Arg Ile Lys Leu Asn
       450                 455                 460
   Asp Asn Asp Asp Glu Gly Asn Lys Lys Ile Ile Ala Pro Arg Ile Phe
   465                 470                 475                 480
   Ile Ser Asp Asp Lys Asp Ser Leu Lys Cys Pro Cys Asp Pro Glu Met
                   485                 490                 495
   Val Ser Asn Ser Thr Cys Arg Phe Phe Val Cys Lys Cys Val Glu Arg
               500                 505                 510
   Arg Ala Glu Val Thr Ser Asn Asn Glu Val Val Val Lys Glu Glu Tyr
           515                 520                 525
   Lys Asp Glu Tyr Ala Asp Ile Pro Glu His Lys Pro Thr Tyr Asp Lys
       530                 535                 540
   Met Lys Ile Ile Ile Ala Ser Ser Ala Ala Val Ala Val Leu Ala Thr
   545                 550                 555                 560
   Ile Leu Met Val Tyr Leu Tyr Lys Arg Lys Gly Asn Ala Glu Lys Tyr
                   565                 570                 575
   Asp Lys Met Asp Glu Pro Gln Asp Tyr Gly Lys Ser Asn Ser Arg Asn
               580                 585                 590
   Asp Glu Met Leu Asp Pro Glu Ala Ser Phe Trp Gly Glu Glu Lys Arg
           595                 600                 605
   Ala Ser His Thr Thr Pro Val Leu Met Glu Lys Pro Tyr Tyr
       610                 615                 620
<210> 74
   <211> 217
   <212> PRT
   <213> Plasmodium falciparum
<400> 74
   Met Asn Lys Leu Tyr Ser Leu Phe Leu Phe Leu Phe Ile Gln Leu Ser
   1               5                   10                  15
   Ile Lys Tyr Asn Asn Ala Lys Val Thr Val Asp Thr Val Cys Lys Arg
               20                  25                  30
   Gly Phe Leu Ile Gln Met Ser Gly His Leu Glu Cys Lys Cys Glu Asn
           35                  40                  45
   Asp Leu Val Leu Val Asn Glu Glu Thr Cys Glu Glu Lys Val Leu Lys
       50                  55                  60
   Cys Asp Glu Lys Thr Val Asn Lys Pro Cys Gly Asp Phe Ser Lys Cys
   65                  70                  75                  80
   Ile Lys Ile Asp Gly Asn Pro Val Ser Tyr Ala Cys Lys Cys Asn Leu
                   85                  90                  95
   Gly Tyr Asp Met Val Asn Asn Val Cys Ile Pro Asn Glu Cys Lys Asn
               100                 105                 110
   Val Thr Cys Gly Asn Gly Lys Cys Ile Leu Asp Thr Ser Asn Pro Val
           115                 120                 125
   Lys Thr Gly Val Cys Ser Cys Asn Ile Gly Lys Val Pro Asn Val Gln
       130                 135                 140
   Asp Gln Asn Lys Cys Ser Lys Asp Gly Glu Thr Lys Cys Ser Leu Lys
   145                 150                 155                 160
   Cys Leu Lys Glu Asn Glu Thr Cys Lys Ala Val Asp Gly Ile Tyr Lys
                   165                 170                 175
   Cys Asp Cys Lys Asp Gly Phe Ile Ile Asp Asn Glu Ser Ser Ile Cys
               180                 185                 190
   Thr Ala Phe Ser Ala Tyr Asn Ile Leu Asn Leu Ser Ile Met Phe Ile
           195                 200                 205
   Leu Phe Ser Val Cys Phe Phe Ile Met
       210                 215
<210> 75
   <211> 2163
   <212> PRT
   <213> Plasmodium falciparum
<400> 75
   Met Val Thr Gln Ser Ser Gly Gly Gly Ala Ala Gly Ser Ser Gly Glu
   1               5                   10                  15
   Glu Asp Ala Lys His Val Leu Asp Glu Phe Gly Gln Gln Val Tyr Asn
               20                  25                  30
   Glu Lys Val Glu Lys Tyr Ala Asn Ser Lys Ile Tyr Lys Glu Ala Leu
           35                  40                  45
   Lys Gly Asp Leu Ser Gln Ala Ser Ile Leu Ser Glu Leu Ala Gly Thr
       50                  55                  60
   Tyr Lys Pro Cys Ala Leu Glu Tyr Glu Tyr Tyr Lys His Thr Asn Gly
   65                  70                  75                  80
   Gly Gly Lys Gly Lys Arg Tyr Pro Cys Thr Glu Leu Gly Glu Lys Val
                   85                  90                  95
   Glu Pro Arg Phe Ser Asp Thr Leu Gly Gly Gln Cys Thr Asn Lys Lys
               100                 105                 110
   Ile Glu Gly Asn Lys Tyr Ile Lys Gly Lys Asp Val Gly Ala Cys Ala
           115                 120                 125
   Pro Tyr Arg Arg Leu His Leu Cys Ser His Asn Leu Glu Ser Ile Gln
       130                 135                 140
   Thr Asn Asn Tyr Asn Ser Gly Asn Ala Lys His Asn Leu Leu Val Asp
   145                 150                 155                 160
   Val Cys Met Ala Ala Lys Tyr Glu Gly Asp Ser Ile Lys Asn Tyr Tyr
                   165                 170                 175
   Pro Lys Tyr Gln Arg Thr Tyr Pro Asp Thr Asn Ser Gln Leu Cys Thr
               180                 185                 190
   Val Leu Ala Arg Ser Phe Ala Asp Ile Gly Asp Ile Val Arg Gly Lys
           195                 200                 205
   Asp Leu Tyr Leu Gly Asn Pro Gln Glu Ser Thr Gln Arg Ile Ile Leu
       210                 215                 220
   Glu Asn Asn Leu Lys Asp Ile Phe Ala Lys Ile His Ser Asp Val Met
   225                 230                 235                 240
   Ser Thr Ser Gly Ser Asn Gly Arg Ala Leu Gln Lys Arg Tyr Lys Asp
                   245                 250                 255
   Thr Asp Asn Tyr Tyr Glu Leu Arg Glu Asp Trp Trp Ala Leu Asn Arg
               260                 265                 270
   Asp Gln Val Trp Lys Ala Ile Thr Cys Asn Ala Gly Gly Gly Asn Arg
           275                 280                 285
   Tyr Phe Arg Gln Thr Cys Gly Ser Gly Glu Trp Ala Lys Asp Lys Cys
       290                 295                 300
   Arg Cys Lys Asp Asp Lys Val Pro Thr Tyr Phe Asp Tyr Val Pro Gln
   305                 310                 315                 320
   Tyr Leu Arg Trp Phe Glu Glu Trp Ala Glu Asp Phe Cys Arg Leu Arg
                   325                 330                 335
   Lys His Lys Leu Lys Asp Ala Lys Asn Lys Cys Arg Gly Asp Ser Gly
               340                 345                 350
   Asn Asp Arg Tyr Cys Asp Leu Asn Arg Tyr Asp Cys Thr Gln Thr Ile
           355                 360                 365
   Arg Gly Asn Glu His Phe Val Glu Lys Asp Asp Cys Lys Gly Cys Gln
       370                 375                 380
   Tyr Ser Cys Ala His Phe Val Asn Trp Ile Asp Asn Gln Lys Leu Glu
   385                 390                 395                 400
   Phe Glu Lys Gln Lys Glu Lys Tyr Thr Lys Glu Ile Lys Lys Lys His
                   405                 410                 415
   Pro Thr Thr Ile Ile Ile Lys Thr Ala Asn Arg Lys Thr Thr Ile Asn
               420                 425                 430
   Asn Leu Tyr Val Lys Glu Phe Tyr Lys Lys Leu Gln Glu Lys Tyr Gly
           435                 440                 445
   Asp Val Glu Asn Phe Leu Gln Lys Leu Asn Glu Glu Gln Ile Cys Lys
       450                 455                 460
   Asn Gln Pro Tyr Asn Asp Glu Ser Ser Ile Asp Ile Asn Phe Lys Ser
   465                 470                 475                 480
   Ile Lys Asp Ile Asp Ile Phe Ser His Thr Glu Tyr Cys Gln Ala Cys
                   485                 490                 495
   Pro Trp Cys Gly Ala Lys Arg Lys Gly Lys Gly Trp Glu Pro Lys Glu
   500 505 510
   Lys Thr Cys Gly Lys Thr Lys Thr Tyr Asp Pro Lys Lys Thr Thr Asn
   515 520 525
   Ile Pro Ile Leu Thr Pro Tyr Ile Ser Gln Gln Ser Ile Leu Lys Lys
   530 535 540
   Tyr Asn Lys Phe Cys Asn Gly Asn Gly Gly Asn Gly Ala Pro Ala Thr
   545 550 555 560
   Ala Thr Gly Gly Gly Gln Ile Lys Asn Trp Gln Cys His Tyr Glu Gly
   565 570 575
   Asp Asn Asn Asp Asn Cys Val Glu Gly Glu Trp Lys Glu Phe Lys Glu
   580 585 590
   Gly Lys Asn Val Met Ser Tyr Asn Ala Phe Phe Trp Lys Trp Val His
   595 600 605
   Asp Met Leu Ile Asp Ser Met Gln Trp Arg Asn Glu His Gly Asn Cys
   610 615 620
   Ile Asn Lys Asp Asn Asp Asn Thr Cys Lys Asn Ser Cys Lys Arg Pro
   625 630 635 640
   Cys Glu Cys Phe Lys Arg Trp Val Asp Gln Lys Lys Lys Asn Glu Trp
   645 650 655
   Glu Ala Ile Lys Asp His Phe Lys Lys Gln Asn Ile Ala Ala Glu Thr
   660 665 670
   Gln Cys Asp Pro Gly Val Thr Leu Gln Trp Val Leu Ile Leu Asp Phe
   675 680 685
   Leu Lys Asp Glu Ser Thr Glu Asp Lys Glu Asn Lys Val Ser Ala Glu
   690 695 700
   Glu Ala Lys Glu Ile Lys His Leu Arg Gln Met Leu Gln Gln Ala Gly
   705 710 715 720
   Val Asp Asp Pro Ala Ala Phe Ala Arg Pro Cys Thr Glu Asp Gly Val
   725 730 735
   Ala Glu Gln Asp Thr Ile Met Asp Lys Leu Leu Asn Arg Glu Glu Asn
   740 745 750
   Asp Ala Thr Glu Cys Lys Lys Cys Asp Lys Pro Pro Pro Ala Pro Thr
           755                 760                 765
   Ala Gly Asp Arg Gly Pro Gly Ala Arg Ala Asp Pro His Asp Val Gln
       770                 775                 780
   Gln Pro Arg Pro Pro Gly Ser Gly Pro Gly Thr Asp Ala Asn Asp Glu
   785                 790                 795                 800
   Asp Asp Asp Asp Asp Asp Asp Asp Asp Asp Glu Glu Asp Gly Glu Ala
                   805                 810                 815
   Lys Glu Glu Glu Glu Asp Glu Glu Lys Gln Glu Asp Val His Gln Glu
               820                 825                 830
   Glu Lys Ala Lys Lys Glu Glu Pro Gln Lys Glu Glu Val Ala Arg Thr
           835                 840                 845
   Pro Lys Asp Asp Val Asn Val Cys Asn Ile Val Asn Asn Val Phe Thr
       850                 855                 860
   Asp Gly Ser Ser Leu Gln Ala Ala Cys Ser Leu Lys Tyr Gly Lys Asn
   865                 870                 875                 880
   Ala Pro Thr Ser Trp Lys Cys Val Thr Pro Ser Gly Asn Thr Ser Asp
                   885                 890                 895
   Thr Thr Val Lys Ser Gly Asp Thr Thr Gly Gly Ser Ile Cys Val Pro
               900                 905                 910
   Pro Arg Arg Arg Arg Leu Tyr Val Thr Pro Leu Thr Arg Leu Thr Gly
           915                 920                 925
   Gly Asp Ser Thr Thr Gln Ala Ser Gln Ala Ser Glu Val Gln Thr Gln
       930                 935                 940
   Ala Arg Gly Ser Asn Thr Asp Lys Ser Pro Gly Ser Ser Glu Ala Ala
   945                 950                 955                 960
   Gln Gly Asp Gly Val Ser Lys Asp Pro Gln Lys Ala Leu Leu Lys Ala
                   965                 970                 975
   Phe Val Glu Ser Ala Ala Val Glu Thr Phe Phe Leu Trp Asp Arg Tyr
               980                 985                 990
   Lys Lys Ile Lys Glu Lys Glu Lys  Lys Glu Lys Lys Lys  Thr Tyr Glu
           995                 1000                1005
   Gln Ile  Tyr Glu Ser Thr Asp  Tyr Asp Asp Glu Glu  Lys Asp Pro
       1010                 1015                 1020
   Gln Glu  Glu Leu Lys Lys Gly  Ile Ile Pro Asp Glu  Phe Lys Arg
       1025                 1030                 1035
   Gln Met  Phe Tyr Thr Leu Gly  Asp Tyr Lys Asp Ile  Leu Tyr Ser
       1040                 1045                 1050
   Gly Asp  Thr Val Asn Gly Gly  Asn Glu Asp Lys Ile  Lys Lys Ala
       1055                 1060                 1065
   Ile Asn  Asn Tyr Phe Gln Lys  Ile Arg Glu Gln Ser  Ser Ser Asp
       1070                 1075                 1080
   Asn Asn  Pro Ser Pro Arg Ser  Val Lys Thr Pro Ser  Thr Ser Asp
       1085                 1090                 1095
   Lys Asp  Pro Gln Thr Trp Trp  Asn Ala His Ala Pro  Ser Ile Trp
       1100                 1105                 1110
   Asn Ala  Met Val Cys Ala Leu  Thr Tyr Asp Thr Asn  Ser Gly Gly
       1115                 1120                 1125
   Glu Gly  Lys Thr Thr Thr Ile  Thr Gln Asp Pro Asn  Leu Lys Thr
       1130                 1135                 1140
   Ala Leu  Trp Asp Glu Asn Gly  Lys Lys Pro Leu Lys  Thr Lys Tyr
       1145                 1150                 1155
   Gln Tyr  Asp Ser Val Thr Ile  Gly Ala Ser Gly Ala  Lys Pro Gln
       1160                 1165                 1170
   Thr Lys  Ala Lys Pro Thr Gly  Gly Asp Thr Pro Leu  Thr Gln Phe
       1175                 1180                 1185
   Val Leu  Arg Pro Thr Tyr Phe  Arg Tyr Leu Glu Glu  Trp Gly Gln
       1190                 1195                 1200
   Asn Phe  Cys Lys Lys Arg Thr  Glu Met Leu Glu Lys  Ile Lys Tyr
       1205                 1210                 1215
   Glu Cys  Lys Val Gly Gln Gly  Arg Gly Gly Arg Lys  Gln Lys Thr
       1220                 1225                 1230
   Pro Gln  Cys Ser Cys Tyr Gly  Glu Asn Cys Asp Asp  Gln Leu Asp
   1235 1240 1245
   Asp Asn Pro Ser Thr Asp Ala Asp Leu Lys Cys Pro Gly Cys Gly
   1250 1255 1260
   Arg Glu Cys Arg Lys Tyr Lys Lys Trp Ile Glu Lys Lys Lys Glu
   1265 1270 1275
   Glu Phe Thr Lys Gln Ser Asn Val Tyr Glu Glu Gln Lys Thr Lys
   1280 1285 1290
   Cys Gln Lys Glu Ser Lys Ser Ala Lys Gly Asn Asn His Gly Asn
   1295 1300 1305
   Glu Phe Cys Gly Thr Gln Gly Thr Cys Asp Thr Ala Gly Asp Phe
   1310 1315 1320
   Leu Asn Arg Leu Lys Ser Gly Pro Cys Lys Lys Glu Asn Gly Lys
   1325 1330 1335
   Asp Asn Gln Glu Asp Glu Ile Asn Phe Lys Asp Glu Asp Lys Thr
   1340 1345 1350
   Phe Gly His Glu Asn Tyr Cys Ala Pro Cys Pro Val Phe Lys Asp
   1355 1360 1365
   Ile Cys Lys Lys Lys Asp Cys Arg Asn Ala Ser Asn Asn Met Cys
   1370 1375 1380
   Asn Gly Lys Asp Phe Ile Thr Ala Glu Asp Ile Lys Ile Met Asp
   1385 1390 1395
   Ser Ser Ser Glu Glu Val Asn Met Leu Val Ser Asp Asn Asp Thr
   1400 1405 1410
   Asn Lys Phe Asp Gly Gly Leu Asp Ala Cys Lys Asp Ala His Ile
   1415 1420 1425
   Phe Lys Gly Ile Lys Glu Asn Lys Trp Ser Cys Gly Asn Val Cys
   1430 1435 1440
   Gly Tyr Asn Val Cys Lys Pro Lys Lys Val Asn Gly Glu Lys Gly
   1445 1450 1455
   Ser Gly Glu Asn Asn Asp Gln Ile Ile Thr Ile Arg Gly Leu Val
   1460 1465 1470
   Thr His Trp Val Gln Asn Phe Leu Asp Asp Tyr Asn Lys Ile Arg
   1475 1480 1485
   Thr Lys Leu Lys Pro Cys Arg Asn Asn Gly Glu Val Ser Lys Cys
   1490 1495 1500
   Ile Lys Asp Cys Val Lys Lys Trp Val Glu Lys Lys Thr Glu Glu
   1505 1510 1515
   Trp Pro Lys Ile Arg Asp Arg Tyr Leu Glu Pro Tyr Lys Ser Asp
   1520 1525 1530
   Asp Gly Tyr Asn Lys Lys Ser Leu Val Arg Ser Phe Met Glu Thr
   1535 1540 1545
   Leu Ile Pro Leu Met Asp Leu Thr Asn Gly Lys Glu Lys Ile Gln
   1550 1555 1560
   Glu Leu Asn Lys Phe Leu Arg Ser Tyr Glu Cys Asn Cys Ala Asp
   1565 1570 1575
   Asn Ser Gln Gln Lys Gly Asp Thr Pro Lys Asp Ile Val Glu Cys
   1580 1585 1590
   Leu Leu Glu Lys Leu Glu Asp Lys Ala Asn Lys Cys Lys Thr Gln
   1595 1600 1605
   Thr Ser Gly Thr Asp Cys His Pro Ser Thr Pro Leu Glu Asp Asp
   1610 1615 1620
   Asp Glu Pro Leu Glu Glu Thr Glu Glu Asn Thr Val Glu Gln Pro
   1625 1630 1635
   Asn Ile Cys Pro Thr Lys Gln Pro Gln Pro Glu Lys Glu Asp Gly
   1640 1645 1650
   Cys Glu Ala Ala Pro Thr Thr Ala Glu Glu Thr Ser Pro Thr Ala
   1655 1660 1665
   Thr Ser Glu Gly Thr Glu Asn Gln Ser Pro Pro Pro Pro Pro Pro
   1670 1675 1680
   Ala Pro Ala Pro Ala Pro Ala Pro Ala Pro Glu Lys Ser Gln Pro
   1685 1690 1695
   Lys Glu Asp Lys Lys Val Glu Pro Gln Pro Lys Pro Gln Pro Thr
   1700 1705 1710
   Asn Pro  Pro Pro Asn Leu Phe  Asn Asn Pro Ala Val  Ile Pro Ala
       1715                 1720                 1725
   Leu Met  Ser Ser Thr Ile Met  Trp Ser Ile Gly Ile  Gly Phe Ala
       1730                 1735                 1740
   Ala Phe  Thr Tyr Phe Leu Leu  Lys Lys Lys Thr Lys  Ser Ser Val
       1745                 1750                 1755
   Gly Asn  Leu Phe Gln Ile Leu  Gln Ile Pro Gln Asn  Asp Tyr Gly
       1760                 1765                 1770
   Ile Pro  Thr Leu Lys Ser Lys  Asn Arg Tyr Ile Pro  Tyr Arg Ser
       1775                 1780                 1785
   Gly Thr  Tyr Lys Gly Lys Thr  Tyr Ile Tyr Met Glu  Gly Asp Ser
       1790                 1795                 1800
   Ser Gly  Asp Glu Lys Tyr Ala  Phe Met Ser Asp Thr  Thr Asp Val
       1805                 1810                 1815
   Thr Ser  Ser Glu Ser Glu Tyr  Glu Glu Leu Asp Ile  Asn Asp Ile
       1820                 1825                 1830
   Tyr Val  Pro His Ala Pro Lys  Tyr Lys Thr Leu Ile  Glu Val Val
       1835                 1840                 1845
   Leu Glu  Pro Ser Gly Asn Asn  Thr Thr Ala Ser Gly  Lys Asn Thr
       1850                 1855                 1860
   Pro Ser  Asp Thr Gln Asn Asp  Ile Pro Thr Ser Asp  Thr Pro Pro
       1865                 1870                 1875
   Pro Ile  Thr Asp Asn Glu Trp  Asn Thr Leu Lys Asp  Glu Phe Ile
       1880                 1885                 1890
   Ser Gln  Tyr Leu Gln Ser Glu  Gln Pro Lys Asp Val  Pro Asn Asp
       1895                 1900                 1905
   Tyr Lys  Ser Gly Asp Ile Pro  Leu Asn Thr Gln Pro  Asn Thr Leu
       1910                 1915                 1920
   Tyr Phe  Asn Lys Pro Glu Glu  Lys Pro Phe Ile Thr  Ser Ile His
       1925                 1930                 1935
   Asp Arg  Asp Leu Tyr Thr Gly  Glu Gln Ile Ser Tyr  Asn Ile His
       1940                 1945                 1950
   Met Ser Thr Asn Thr Met Asp Asp Pro Lys Tyr Val Ser Asn Asn
   1955 1960 1965
   Val Tyr Ser Gly Ile Asp Leu Ile Asn Asp Ala Leu Asn Gly Asp
   1970 1975 1980
   Tyr Asp Ile Tyr Asp Glu Ile Leu Lys Arg Lys Glu Asn Glu Leu
   1985 1990 1995
   Phe Gly Thr Asn His Val Lys Gln Thr Ser Ile His Ser Val Ala
   2000 2005 2010
   Lys Leu Thr Asn Ser Asp Pro Ile His Asn Gln Leu Glu Leu Phe
   2015 2020 2025
   His Lys Trp Leu Asp Arg His Arg Asn Met Cys Glu Lys Trp Lys
   2030 2035 2040
   Asn Asp Asn Glu Arg Leu Ala Lys Leu Lys Glu Glu Trp Glu Asn
   2045 2050 2055
   Glu Thr His Ser Gly Asn Thr His Pro Ser Asp Ser Asn Lys Thr
   2060 2065 2070
   Leu Asn Thr Asp Val Ser Ile Gln Ile Asp Met Asp His Glu Lys
   2075 2080 2085
   Arg Met Lys Glu Phe Thr Asn Met Asp Thr Ile Leu Asp Asp Leu
   2090 2095 2100
   Lys Thr Tyr Asn Glu Pro Tyr Tyr Asp Val Gln Asp Asp Ile Tyr
   2105 2110 2115
   Tyr Asp Val Asn Asp His Asp Ala Ser Thr Val Asp Ser Asn Asn
   2120 2125 2130
   Met Asp Val Pro Ser Arg Val Gln Ile Glu Met Asp Val Asn Thr
   2135 2140 2145
   Lys Leu Val Lys Glu Lys Tyr Pro Ile Ala Asp Val Trp Asp Ile
   2150 2155 2160
<210> 76
   <211> 1558
   <212> PRT
   <213> Plasmodium falciparum
<400> 76
   Met Thr Asn Ser Asn Tyr Lys Ser Asn Asn Lys Thr Tyr Asn Glu Asn
   1 5 10 15
   Asn Asn Glu Gln Ile Thr Thr Ile Phe Asn Arg Thr Asn Met Asn Pro
   20 25 30
   Ile Lys Lys Cys His Met Arg Glu Lys Ile Asn Lys Tyr Phe Phe Leu
   35 40 45
   Ile Lys Ile Leu Thr Cys Thr Ile Leu Ile Trp Ala Val Gln Tyr Ala
   50 55 60
   Asn Asn Ser Asp Ile Asn Lys Ser Trp Lys Lys Asn Thr Tyr Val Asp
   65 70 75 80
   Lys Lys Leu Asn Lys Leu Phe Asn Arg Ser Leu Gly Glu Ser Gln Val
   85 90 95
   Asn Gly Glu Leu Ala Ser Glu Glu Val Lys Glu Lys Ile Leu Asp Leu
   100 105 110
   Leu Glu Glu Gly Asn Thr Leu Thr Glu Ser Val Asp Asp Asn Lys Asn
   115 120 125
   Leu Glu Glu Ala Glu Asp Ile Lys Glu Asn Ile Leu Leu Ser Asn Ile
   130 135 140
   Glu Glu Pro Lys Glu Asn Ile Ile Asp Asn Leu Leu Asn Asn Ile Gly
   145 150 155 160
   Gln Asn Ser Glu Lys Gln Glu Ser Val Ser Glu Asn Val Gln Val Ser
   165 170 175
   Asp Glu Leu Phe Asn Glu Leu Leu Asn Ser Val Asp Val Asn Gly Glu
   180 185 190
   Val Lys Glu Asn Ile Leu Glu Glu Ser Gln Val Asn Asp Asp Ile Phe
   195 200 205
   Asn Ser Leu Val Lys Ser Val Gln Gln Glu Gln Gln His Asn Val Glu
   210 215 220
   Glu Lys Val Glu Glu Ser Val Glu Glu Asn Asp Glu Glu Ser Val Glu
   225 230 235 240
   Glu Asn Val Glu Glu Asn Val Glu Glu Asn Asp Asp Glu Ser Val Ala
   245 250 255
   Ser Ser Val Glu Glu Ser Ile Ala Ser Ser Val Asp Glu Ser Ile Asp
   260 265 270
   Ser Ser Ile Glu Glu Asn Val Ala Pro Thr Val Glu Glu Ile Val Ala
   275 280 285
   Pro Thr Val Glu Glu Ile Val Ala Pro Ser Val Val Glu Ser Val Ala
   290 295 300
   Pro Ser Val Glu Glu Ser Val Glu Glu Asn Val Glu Glu Ser Val Ala
   305 310 315 320
   Glu Asn Val Glu Glu Ser Val Ala Glu Asn Val Glu Glu Ser Val Ala
   325 330 335
   Glu Asn Val Glu Glu Ser Val Ala Glu Asn Val Glu Glu Ser Val Ala
   340 345 350
   Glu Asn Val Glu Glu Ser Val Ala Glu Asn Val Glu Glu Ile Val Ala
   355 360 365
   Pro Thr Val Glu Glu Ser Val Ala Pro Thr Val Glu Glu Ile Val Ala
   370 375 380
   Pro Ser Val Glu Glu Ser Val Ala Pro Ser Val Glu Glu Ile Val Val
   385 390 395 400
   Pro Thr Val Glu Glu Ser Val Ala Glu Asn Val Glu Glu Ile Val Ala
   405 410 415
   Pro Ser Val Glu Glu Ile Val Ala Pro Ser Val Glu Glu Ile Val Ala
   420 425 430
   Pro Thr Val Glu Glu Ser Val Ala Pro Thr Val Glu Glu Ile Val Ala
   435 440 445
   Pro Ser Val Glu Glu Ser Val Ala Pro Ser Val Glu Glu Ile Val Val
   450 455 460
   Pro Thr Val Glu Glu Ser Val Ala Glu Asn Val Glu Glu Ser Val Ala
   465 470 475 480
   Glu Asn Val Glu Glu Ile Val Ala Pro Ser Val Glu Glu Ile Val Ala
   485 490 495
   Pro Ser Val Glu Glu Ile Val Ala Pro Ser Val Glu Glu Ile Val Ala
               500                 505                 510
   Pro Ser Val Glu Glu Ile Val Ala Pro Ser Val Glu Glu Ile Val Ala
           515                 520                 525
   Pro Ser Val Glu Glu Ile Val Ala Pro Ser Val Glu Glu Ile Val Ala
       530                 535                 540
   Pro Ser Val Glu Glu Ile Val Ala Pro Thr Val Glu Glu Ile Val Ala
   545                 550                 555                 560
   Pro Thr Val Glu Glu Ile Val Ala Pro Ser Val Glu Glu Ile Val Ala
                   565                 570                 575
   Pro Thr Val Glu Glu Ser Val Ala Glu Asn Val Ala Thr Asn Leu Ser
               580                 585                 590
   Asp Asn Leu Leu Ser Asn Leu Leu Gly Gly Ile Glu Thr Glu Glu Ile
           595                 600                 605
   Lys Asp Ser Ile Leu Asn Glu Ile Glu Glu Val Lys Glu Asn Val Val
       610                 615                 620
   Thr Thr Ile Leu Glu Asn Val Glu Glu Thr Thr Ala Glu Ser Val Thr
   625                 630                 635                 640
   Thr Phe Ser Asn Ile Leu Glu Glu Ile Gln Glu Asn Thr Ile Thr Asn
                   645                 650                 655
   Asp Thr Ile Glu Glu Lys Leu Glu Glu Leu His Glu Asn Val Leu Ser
               660                 665                 670
   Ala Ala Leu Glu Asn Thr Gln Ser Glu Glu Glu Lys Lys Glu Val Ile
           675                 680                 685
   Asp Val Ile Glu Glu Val Lys Glu Glu Val Ala Thr Thr Leu Ile Glu
       690                 695                 700
   Thr Val Glu Gln Ala Glu Glu Glu Ser Ala Ser Thr Ile Thr Glu Ile
   705                 710                 715                 720
   Phe Glu Asn Leu Glu Glu Asn Ala Val Glu Ser Asn Glu Asn Val Ala
                   725                 730                 735
   Glu Asn Leu Glu Lys Leu Asn Glu Thr Val Phe Asn Thr Val Leu Asp
               740                 745                 750
   Lys Val Glu Glu Thr Val Glu Ile Ser Gly Glu Ser Leu Glu Asn Asn
   755 760 765
   Glu Met Asp Lys Ala Phe Phe Ser Glu Ile Phe Asp Asn Val Lys Gly
   770 775 780
   Ile Gln Glu Asn Leu Leu Thr Gly Met Phe Arg Ser Ile Glu Thr Ser
   785 790 795 800
   Ile Val Ile Gln Ser Glu Glu Lys Val Asp Leu Asn Glu Asn Val Val
   805 810 815
   Ser Ser Ile Leu Asp Asn Ile Glu Asn Met Lys Glu Gly Leu Leu Asn
   820 825 830
   Lys Leu Glu Asn Ile Ser Ser Thr Glu Gly Val Gln Glu Thr Val Thr
   835 840 845
   Glu His Val Glu Gln Asn Val Tyr Val Asp Val Asp Val Pro Ala Met
   850 855 860
   Lys Asp Gln Phe Leu Gly Ile Leu Asn Glu Ala Gly Gly Leu Lys Glu
   865 870 875 880
   Met Phe Phe Asn Leu Glu Asp Val Phe Lys Ser Glu Ser Asp Val Ile
   885 890 895
   Thr Val Glu Glu Ile Lys Asp Glu Pro Val Gln Lys Glu Val Glu Lys
   900 905 910
   Glu Thr Val Ser Ile Ile Glu Glu Met Glu Glu Asn Ile Val Asp Val
   915 920 925
   Leu Glu Glu Glu Lys Glu Asp Leu Thr Asp Lys Met Ile Asp Ala Val
   930 935 940
   Glu Glu Ser Ile Glu Ile Ser Ser Asp Ser Lys Glu Glu Thr Glu Ser
   945 950 955 960
   Ile Lys Asp Lys Glu Lys Asp Val Ser Leu Val Val Glu Glu Val Gln
   965 970 975
   Asp Asn Asp Met Asp Glu Ser Val Glu Lys Val Leu Glu Leu Lys Asn
   980 985 990
   Met Glu Glu Glu Leu Met Lys Asp Ala Val Glu Ile Asn Asp Ile Thr
   995 1000 1005
   Ser Lys Leu Ile Glu Glu Thr Gln Glu Leu Asn Glu Val Glu Ala
   1010 1015 1020
   Asp Leu Ile Lys Asp Met Glu Lys Leu Lys Glu Leu Glu Lys Ala
   1025 1030 1035
   Leu Ser Glu Asp Ser Lys Glu Ile Ile Asp Ala Lys Asp Asp Thr
   1040 1045 1050
   Leu Glu Lys Val Ile Glu Glu Glu His Asp Ile Thr Thr Thr Leu
   1055 1060 1065
   Asp Glu Val Val Glu Leu Lys Asp Val Glu Glu Asp Lys Ile Glu
   1070 1075 1080
   Lys Val Ser Asp Leu Lys Asp Leu Glu Glu Asp Ile Leu Lys Glu
   1085 1090 1095
   Val Lys Glu Ile Lys Glu Leu Glu Ser Glu Ile Leu Glu Asp Tyr
   1100 1105 1110
   Lys Glu Leu Lys Thr Ile Glu Thr Asp Ile Leu Glu Glu Lys Lys
   1115 1120 1125
   Glu Ile Glu Lys Asp His Phe Glu Lys Phe Glu Glu Glu Ala Glu
   1130 1135 1140
   Glu Ile Lys Asp Leu Glu Ala Asp Ile Leu Lys Glu Val Ser Ser
   1145 1150 1155
   Leu Glu Val Glu Glu Glu Lys Lys Leu Glu Glu Val His Glu Leu
   1160 1165 1170
   Lys Glu Glu Val Glu His Ile Ile Ser Gly Asp Ala His Ile Lys
   1175 1180 1185
   Gly Leu Glu Glu Asp Asp Leu Glu Glu Val Asp Asp Leu Lys Gly
   1190 1195 1200
   Ser Ile Leu Asp Met Leu Lys Gly Asp Met Glu Leu Gly Asp Met
   1205 1210 1215
   Asp Lys Glu Ser Leu Glu Asp Val Thr Ala Lys Leu Gly Glu Arg
   1220 1225 1230
   Val Glu Ser Leu Lys Asp Val Leu Ser Ser Ala Leu Gly Met Asp
       1235                1240                1245
   Glu Glu  Gln Met Lys Thr Arg  Lys Lys Ala Gln Arg  Pro Lys Leu
       1250                1255                1260
   Glu Glu  Val Leu Leu Lys Glu  Glu Val Lys Glu Glu  Pro Lys Lys
       1265                1270                1275
   Lys Ile  Thr Lys Lys Lys Val  Arg Phe Asp Ile Lys  Asp Lys Glu
       1280                1285                1290
   Pro Lys  Asp Glu Ile Val Glu  Val Glu Met Lys Asp  Glu Asp Ile
       1295                1300                1305
   Asp Glu  Asp Ile Glu Glu Asp  Val Glu Glu Asp Ile  Glu Glu Asp
       1310                1315                1320
   Lys Val  Glu Asp Ile Asp Glu  Asp Ile Asp Glu Asp  Ile Asp Glu
       1325                1330                1335
   Asp Ile  Gly Glu Asp Lys Asp  Glu Val Ile Asp Leu  Ile Val Gln
       1340                1345                1350
   Lys Glu  Lys Arg Ile Glu Lys  Val Lys Glu Lys Lys  Lys Lys Leu
       1355                1360                1365
   Glu Lys  Lys Val Glu Glu Gly  Val Ser Gly Leu Lys  Lys His Val
       1370                1375                1380
   Asp Glu  Val Met Lys Tyr Val  Gln Lys Ile Asp Lys  Glu Val Asp
       1385                1390                1395
   Lys Glu  Val Ser Lys Ala Leu  Glu Ser Lys Asn Asp  Val Thr Asn
       1400                1405                1410
   Val Leu  Lys Gln Asn Gln Asp  Phe Phe Ser Lys Val  Lys Asn Phe
       1415                1420                1425
   Val Lys  Lys Tyr Lys Val Phe  Ala Ala Pro Phe Ile  Ser Ala Val
       1430                1435                1440
   Ala Ala  Phe Ala Ser Tyr Val  Val Gly Phe Phe Thr  Phe Ser Leu
       1445                1450                1455
   Phe Ser  Ser Cys Val Thr Ile  Ala Ser Ser Thr Tyr  Leu Leu Ser
       1460                1465                1470
   Lys Val Asp Lys Thr Ile Asn Lys Asn Lys Glu Arg Pro Phe Tyr
   1475 1480 1485
   Ser Phe Val Phe Asp Ile Phe Lys Asn Leu Lys His Tyr Leu Gln
   1490 1495 1500
   Gln Met Lys Glu Lys Phe Ser Lys Glu Lys Asn Asn Asn Val Ile
   1505 1510 1515
   Glu Val Thr Asn Lys Ala Glu Lys Lys Gly Asn Val Gln Val Thr
   1520 1525 1530
   Asn Lys Thr Glu Lys Thr Thr Lys Val Asp Lys Asn Asn Lys Val
   1535 1540 1545
   Pro Lys Lys Ser Arg Thr Gln Lys Ser Lys
   1550 1555
<210> 77
   <211> 150
   <212> PRT
   <213> Plasmodium falciparum
<400> 77
   Ser Asp Glu Leu Phe Asn Glu Leu Leu Asn Ser Val Asp Val Asn Gly
   1 5 10 15
   Glu Val Lys Glu Asn Ile Leu Glu Glu Ser Gln Val Asn Asp Asp Ile
   20 25 30
   Phe Asn Ser Leu Val Lys Ser Val Gln Gln Glu Gln Gln His Asn Val
   35 40 45
   Glu Glu Lys Val Glu Glu Ser Val Glu Glu Asn Asp Glu Glu Ser Val
   50 55 60
   Glu Glu Asn Val Glu Glu Asn Val Glu Glu Asn Asp Asp Glu Ser Val
   65 70 75 80
   Ala Ser Ser Val Glu Glu Ser Ile Ala Ser Ser Val Asp Glu Ser Ile
   85 90 95
   Asp Ser Ser Ile Glu Glu Asn Val Ala Pro Thr Val Glu Glu Ile Val
   100 105 110
   Ala Pro Thr Val Glu Glu Ile Val Ala Pro Ser Val Val Glu Ser Val
   115 120 125
   Ala Pro Ser Val Glu Glu Ser Val Glu Glu Asn Val Glu Glu Ser Val
   130 135 140
   Ala Glu Asn Val Glu Glu
   145 150
<210> 78
   <211> 397
   <212> PRT
   <213> Plasmodium falciparum
<400> 78
   Met Met Arg Lys Leu Ala Ile Leu Ser Val Ser Ser Phe Leu Phe Val
   1 5 10 15
   Glu Ala Leu Phe Gln Glu Tyr Gln Cys Tyr Gly Ser Ser Ser Asn Thr
   20 25 30
   Arg Val Leu Asn Glu Leu Asn Tyr Asp Asn Ala Gly Thr Asn Leu Tyr
   35 40 45
   Asn Glu Leu Glu Met Asn Tyr Tyr Gly Lys Gln Glu Asn Trp Tyr Ser
   50 55 60
   Leu Lys Lys Asn Ser Arg Ser Leu Gly Glu Asn Asp Asp Gly Asn Asn
   65 70 75 80
   Glu Asp Asn Glu Lys Leu Arg Lys Pro Lys His Lys Lys Leu Lys Gln
   85 90 95
   Pro Ala Asp Gly Asn Pro Asp Pro Asn Ala Asn Pro Asn Val Asp Pro
   100 105 110
   Asn Ala Asn Pro Asn Val Asp Pro Asn Ala Asn Pro Asn Val Asp Pro
   115 120 125
   Asn Ala Asn Pro Asn Ala Asn Pro Asn Ala Asn Pro Asn Ala Asn Pro
   130 135 140
   Asn Ala Asn Pro Asn Ala Asn Pro Asn Ala Asn Pro Asn Ala Asn Pro
   145 150 155 160
   Asn Ala Asn Pro Asn Ala Asn Pro Asn Ala Asn Pro Asn Ala Asn Pro
   165 170 175
   Asn Ala Asn Pro Asn Ala Asn Pro Asn Ala Asn Pro Asn Ala Asn Pro
   180 185 190
   Asn Ala Asn Pro Asn Val Asp Pro Asn Ala Asn Pro Asn Ala Asn Pro
   195 200 205
   Asn Ala Asn Pro Asn Ala Asn Pro Asn Ala Asn Pro Asn Ala Asn Pro
   210 215 220
   Asn Ala Asn Pro Asn Ala Asn Pro Asn Ala Asn Pro Asn Ala Asn Pro
   225 230 235 240
   Asn Ala Asn Pro Asn Ala Asn Pro Asn Ala Asn Pro Asn Ala Asn Pro
   245 250 255
   Asn Ala Asn Pro Asn Ala Asn Pro Asn Ala Asn Pro Asn Ala Asn Pro
   260 265 270
   Asn Lys Asn Asn Gln Gly Asn Gly Gln Gly His Asn Met Pro Asn Asp
   275 280 285
   Pro Asn Arg Asn Val Asp Glu Asn Ala Asn Ala Asn Ser Ala Val Lys
   290 295 300
   Asn Asn Asn Asn Glu Glu Pro Ser Asp Lys His Ile Lys Glu Tyr Leu
   305 310 315 320
   Asn Lys Ile Gln Asn Ser Leu Ser Thr Glu Trp Ser Pro Cys Ser Val
   325 330 335
   Thr Cys Gly Asn Gly Ile Gln Val Arg Ile Lys Pro Gly Ser Ala Asn
   340 345 350
   Lys Pro Lys Asp Glu Leu Asp Tyr Ala Asn Asp Ile Glu Lys Lys Ile
   355 360 365
   Cys Lys Met Glu Lys Cys Ser Ser Val Phe Asn Val Val Asn Ser Ser
   370 375 380
   Ile Gly Leu Ile Met Val Leu Ser Phe Leu Phe Leu Asn
   385 390 395
<210> 79
   <211> 574
   <212> PRT
   <213> Plasmodium falciparum
<400> 79
   Met Asn His Leu Gly Asn Val Lys Tyr Leu Val Ile Val Phe Leu Ile
   1 5 10 15
   Phe Phe Asp Leu Phe Leu Val Asn Gly Arg Asp Val Gln Asn Asn Ile
               20                  25                  30
   Val Asp Glu Ile Lys Tyr Arg Glu Glu Val Cys Asn Asp Glu Val Asp
           35                  40                  45
   Leu Tyr Leu Leu Met Asp Cys Ser Gly Ser Ile Arg Arg His Asn Trp
       50                  55                  60
   Val Asn His Ala Val Pro Leu Ala Met Lys Leu Ile Gln Gln Leu Asn
   65                  70                  75                  80
   Leu Asn Asp Asn Ala Ile His Leu Tyr Ala Ser Val Phe Ser Asn Asn
                   85                  90                  95
   Ala Arg Glu Ile Ile Arg Leu His Ser Asp Ala Ser Lys Asn Lys Glu
               100                 105                 110
   Lys Ala Leu Ile Ile Ile Lys Ser Leu Leu Ser Thr Asn Leu Pro Tyr
           115                 120                 125
   Gly Lys Thr Asn Leu Thr Asp Ala Leu Leu Gln Val Arg Lys His Leu
       130                 135                 140
   Asn Asp Arg Ile Asn Arg Glu Asn Ala Asn Gln Leu Val Val Ile Leu
   145                 150                 155                 160
   Thr Asp Gly Ile Pro Asp Ser Ile Gln Asp Ser Leu Lys Glu Ser Arg
                   165                 170                 175
   Lys Leu Ser Asp Arg Gly Val Lys Ile Ala Val Phe Gly Ile Gly Gln
               180                 185                 190
   Gly Ile Asn Val Ala Phe Asn Arg Phe Leu Val Gly Cys His Pro Ser
           195                 200                 205
   Asp Gly Lys Cys Asn Leu Tyr Ala Asp Ser Ala Trp Glu Asn Val Lys
       210                 215                 220
   Asn Val Ile Gly Pro Phe Met Lys Ala Val Cys Val Glu Val Glu Lys
   225                 230                 235                 240
   Thr Ala Ser Cys Gly Val Trp Asp Glu Trp Ser Pro Cys Ser Val Thr
                   245                 250                 255
   Cys Gly Lys Gly Thr Arg Ser Arg Lys Arg Glu Ile Leu His Glu Gly
               260                 265                 270
   Cys Thr Ser Glu Leu Gln Glu Gln Cys Glu Glu Glu Arg Cys Leu Pro
           275                 280                 285
   Lys Arg Glu Pro Leu Asp Val Pro Asp Glu Pro Glu Asp Asp Gln Pro
       290                 295                 300
   Arg Pro Arg Gly Asp Asn Phe Ala Val Glu Lys Pro Asn Glu Asn Ile
   305                 310                 315                 320
   Ile Asp Asn Asn Pro Gln Glu Pro Ser Pro Asn Pro Glu Glu Gly Lys
                   325                 330                 335
   Gly Glu Asn Pro Asn Gly Phe Asp Leu Asp Glu Asn Pro Glu Asn Pro
               340                 345                 350
   Pro Asn Pro Pro Asn Pro Pro Asn Pro Pro Asn Pro Pro Asn Pro Pro
           355                 360                 365
   Asn Pro Asp Ile Pro Glu Gln Glu Pro Asn Ile Pro Glu Asp Ser Glu
       370                 375                 380
   Lys Glu Val Pro Ser Asp Val Pro Lys Asn Pro Glu Asp Asp Arg Glu
   385                 390                 395                 400
   Glu Asn Phe Asp Ile Pro Lys Lys Pro Glu Asn Lys His Asp Asn Gln
                   405                 410                 415
   Asn Asn Leu Pro Asn Asp Lys Ser Asp Arg Tyr Ile Pro Tyr Ser Pro
               420                 425                 430
   Leu Ser Pro Lys Val Leu Asp Asn Glu Arg Lys Gln Ser Asp Pro Gln
           435                 440                 445
   Ser Gln Asp Asn Asn Gly Asn Arg His Val Pro Asn Ser Glu Asp Arg
       450                 455                 460
   Glu Thr Arg Pro His Gly Arg Asn Asn Glu Asn Arg Ser Tyr Asn Arg
   465                 470                 475                 480
   Lys His Asn Asn Thr Pro Lys His Pro Glu Arg Glu Glu His Glu Lys
                   485                 490                 495
   Pro Asp Asn Asn Lys Lys Lys Ala Gly Ser Asp Asn Lys Tyr Lys Ile
               500                 505                 510
   Ala Gly Gly Ile Ala Gly Gly Leu Ala Leu Leu Ala Cys Ala Gly Leu
           515                 520                 525
   Ala Tyr Lys Phe Val Val Pro Gly Ala Ala Thr Pro Tyr Ala Gly Glu
       530                 535                 540
   Pro Ala Pro Phe Asp Glu Thr Leu Gly Glu Glu Asp Lys Asp Leu Asp
   545                 550                 555                 560
   Glu Pro Glu Gln Phe Arg Leu Pro Glu Glu Asn Glu Trp Asn
                   565                 570
<210> 80
   <211> 272
   <212> PRT
   <213> Plasmodium falciparum
<400> 80
   Met Trp Ile Val Lys Phe Leu Ile Val Val His Phe Phe Ile Ile Cys
   1               5                   10                  15
   Thr Ile Asn Phe Asp Lys Leu Tyr Ile Ser Tyr Ser Tyr Asn Ile Val
               20                  25                  30
   Pro Glu Asn Gly Arg Met Leu Asn Met Arg Ile Leu Gly Glu Glu Lys
           35                  40                  45
   Pro Asn Val Asp Gly Val Ser Thr Ser Asn Thr Pro Gly Gly Asn Glu
       50                  55                  60
   Ser Ser Ser Ala Ser Pro Asn Leu Ser Asp Ala Ala Glu Lys Lys Asp
   65                  70                  75                  80
   Glu Lys Glu Ala Ser Glu Gln Gly Glu Glu Ser His Lys Lys Glu Asn
                   85                  90                  95
   Ser Gln Glu Ser Ala Asn Gly Lys Asp Asp Val Lys Glu Glu Lys Lys
               100                 105                 110
   Thr Asn Glu Lys Lys Asp Asp Gly Lys Thr Asp Lys Val Gln Glu Lys
           115                 120                 125
   Val Leu Glu Lys Ser Pro Lys Glu Ser Gln Met Val Asp Asp Lys Lys
       130                 135                 140
   Lys Thr Glu Ala Ile Pro Lys Lys Val Val Gln Pro Ser Ser Ser Asn
   145                 150                 155                 160
   Ser Gly Gly His Val Gly Glu Glu Glu Asp His Asn Glu Gly Glu Gly
                   165                 170                 175
   Glu His Glu Glu Glu Glu Glu His Glu Glu Asp Asp Asp Asp Glu Asp
   180 185 190
   Asp Asp Thr Tyr Asn Lys Asp Asp Leu Glu Asp Glu Asp Leu Cys Lys
   195 200 205
   His Asn Asn Gly Gly Cys Gly Asp Asp Lys Leu Cys Glu Tyr Val Gly
   210 215 220
   Asn Arg Arg Val Lys Cys Lys Cys Lys Glu Gly Tyr Lys Leu Glu Gly
   225 230 235 240
   Ile Glu Cys Val Glu Leu Leu Ser Leu Ala Ser Ser Ser Leu Asn Leu
   245 250 255
   Ile Phe Asn Ser Phe Ile Thr Ile Phe Val Val Ile Leu Leu Ile Asn
   260 265 270
<210> 81
   <211> 560
   <212> PRT
   <213> Plasmodium falciparum
<400> 81
   Met Ser Arg Lys Leu Phe Ala Ser Ile Leu Ile Gly Ala Leu Leu Gly
   1 5 10 15
   Ile Gly Ala Pro Pro Ser Ala His Ala Gly Ala Asp Asp Val Val Asp
   20 25 30
   Ser Ser Lys Ser Phe Val Met Glu Asn Phe Ser Ser Tyr His Gly Thr
   35 40 45
   Lys Pro Gly Tyr Val Asp Ser Ile Gln Lys Gly Ile Gln Lys Pro Lys
   50 55 60
   Ser Gly Thr Gln Gly Asn Tyr Asp Asp Asp Trp Lys Gly Phe Tyr Ser
   65 70 75 80
   Thr Asp Asn Lys Tyr Asp Ala Ala Gly Tyr Ser Val Asp Asn Glu Asn
   85 90 95
   Pro Leu Ser Gly Lys Ala Gly Gly Val Val Lys Val Thr Tyr Pro Gly
   100 105 110
   Leu Thr Lys Val Leu Ala Leu Lys Val Asp Asn Ala Glu Thr Ile Lys
   115 120 125
   Lys Glu Leu Gly Leu Ser Leu Thr Glu Pro Leu Met Glu Gln Val Gly
       130                 135                 140
   Thr Glu Glu Phe Ile Lys Arg Phe Gly Asp Gly Ala Ser Arg Val Val
   145                 150                 155                 160
   Leu Ser Leu Pro Phe Ala Glu Gly Ser Ser Ser Val Glu Tyr Ile Asn
                   165                 170                 175
   Asn Trp Glu Gln Ala Lys Ala Leu Ser Val Glu Leu Glu Ile Asn Phe
               180                 185                 190
   Glu Thr Arg Gly Lys Arg Gly Gln Asp Ala Met Tyr Glu Tyr Met Ala
           195                 200                 205
   Gln Ala Cys Ala Gly Asn Arg Val Arg Arg Ser Val Gly Ser Ser Leu
       210                 215                 220
   Ser Cys Ile Asn Leu Asp Trp Asp Val Ile Arg Asp Lys Thr Lys Thr
   225                 230                 235                 240
   Lys Ile Glu Ser Leu Lys Glu His Gly Pro Ile Lys Asn Lys Met Ser
                   245                 250                 255
   Glu Ser Pro Asn Lys Thr Val Ser Glu Glu Lys Ala Lys Gln Tyr Leu
               260                 265                 270
   Glu Glu Phe His Gln Thr Ala Leu Glu His Pro Glu Leu Ser Glu Leu
           275                 280                 285
   Lys Thr Val Thr Gly Thr Asn Pro Val Phe Ala Gly Ala Asn Tyr Ala
       290                 295                 300
   Ala Trp Ala Val Asn Val Ala Gln Val Ile Asp Ser Glu Thr Ala Asp
   305                 310                 315                 320
   Asn Leu Glu Lys Thr Thr Ala Ala Leu Ser Ile Leu Pro Gly Ile Gly
                   325                 330                 335
   Ser Val Met Gly Ile Ala Asp Gly Ala Val His His Asn Thr Glu Glu
               340                 345                 350
   Ile Val Ala Gln Ser Ile Ala Leu Ser Ser Leu Met Val Ala Gln Ala
           355                 360                 365
   Ile Pro Leu Val Gly Glu Leu Val Asp Ile Gly Phe Ala Ala Tyr Asn
   370 375 380
   Phe Val Glu Ser Ile Ile Asn Leu Phe Gln Val Val His Asn Ser Tyr
   385 390 395 400
   Asn Arg Pro Ala Tyr Ser Pro Gly His Lys Thr Gln Pro Phe Leu His
   405 410 415
   Asp Gly Tyr Ala Val Ser Trp Asn Thr Val Glu Asp Ser Ile Ile Arg
   420 425 430
   Thr Gly Phe Gln Gly Glu Ser Gly His Asp Ile Lys Ile Thr Ala Glu
   435 440 445
   Asn Thr Pro Leu Pro Ile Ala Gly Val Leu Leu Pro Thr Ile Pro Gly
   450 455 460
   Lys Leu Asp Val Asn Lys Ser Lys Thr His Ile Ser Val Asn Gly Arg
   465 470 475 480
   Lys Ile Arg Met Arg Cys Arg Ala Ile Asp Gly Asp Val Thr Phe Cys
   485 490 495
   Arg Pro Lys Ser Pro Val Tyr Val Gly Asn Gly Val His Ala Asn Leu
   500 505 510
   His Val Ala Phe His Arg Ser Ser Ser Glu Lys Ile His Ser Asn Glu
   515 520 525
   Ile Ser Ser Asp Ser Ile Gly Val Leu Gly Tyr Gln Lys Thr Val Asp
   530 535 540
   His Thr Lys Val Asn Ser Lys Leu Ser Leu Phe Phe Glu Ile Lys Ser
   545 550 555 560
<210> 82
   <211> 535
   <212> PRT
   <213> Plasmodium falciparum
<400> 82
   Gly Ala Asp Asp Val Val Asp Ser Ser Lys Ser Phe Val Met Glu Asn
   1               5                   10                  15
   Phe Ser Ser Tyr His Gly Thr Lys Pro Gly Tyr Val Asp Ser Ile Gln
               20                  25                  30
   Lys Gly Ile Gln Lys Pro Lys Ser Gly Thr Gln Gly Asn Tyr Asp Asp
   35 40 45
   Asp Trp Lys Glu Phe Tyr Ser Thr Asp Asn Lys Tyr Asp Ala Ala Gly
   50 55 60
   Tyr Ser Val Asp Asn Glu Asn Pro Leu Ser Gly Lys Ala Gly Gly Val
   65 70 75 80
   Val Lys Val Thr Tyr Pro Gly Leu Thr Lys Val Leu Ala Leu Lys Val
   85 90 95
   Asp Asn Ala Glu Thr Ile Lys Lys Glu Leu Gly Leu Ser Leu Thr Glu
   100 105 110
   Pro Leu Met Glu Gln Val Gly Thr Glu Glu Phe Ile Lys Arg Phe Gly
   115 120 125
   Asp Gly Ala Ser Arg Val Val Leu Ser Leu Pro Phe Ala Glu Gly Ser
   130 135 140
   Ser Ser Val Glu Tyr Ile Asn Asn Trp Glu Gln Ala Lys Ala Leu Ser
   145 150 155 160
   Val Glu Leu Glu Ile Asn Phe Glu Thr Arg Gly Lys Arg Gly Gln Asp
   165 170 175
   Ala Met Tyr Glu Tyr Met Ala Gln Ala Cys Ala Gly Asn Arg Val Arg
   180 185 190
   Arg Ser Val Gly Ser Ser Leu Ser Cys Ile Asn Leu Asp Trp Asp Val
   195 200 205
   Ile Arg Asp Lys Thr Lys Thr Lys Ile Glu Ser Leu Lys Glu His Gly
   210 215 220
   Pro Ile Lys Asn Lys Met Ser Glu Ser Pro Asn Lys Thr Val Ser Glu
   225 230 235 240
   Glu Lys Ala Lys Gln Tyr Leu Glu Glu Phe His Gln Thr Ala Leu Glu
   245 250 255
   His Pro Glu Leu Ser Glu Leu Lys Thr Val Thr Gly Thr Asn Pro Val
   260 265 270
   Phe Ala Gly Ala Asn Tyr Ala Ala Trp Ala Val Asn Val Ala Gln Val
   275 280 285
   Ile Asp Ser Glu Thr Ala Asp Asn Leu Glu Lys Thr Thr Ala Ala Leu
       290                 295                 300
   Ser Ile Leu Pro Gly Ile Gly Ser Val Met Gly Ile Ala Asp Gly Ala
   305                 310                 315                 320
   Val His His Asn Thr Glu Glu Ile Val Ala Gln Ser Ile Ala Leu Ser
                   325                 330                 335
   Ser Leu Met Val Ala Gln Ala Ile Pro Leu Val Gly Glu Leu Val Asp
               340                 345                 350
   Ile Gly Phe Ala Ala Tyr Asn Phe Val Glu Ser Ile Ile Asn Leu Phe
           355                 360                 365
   Gln Val Val His Asn Ser Tyr Asn Arg Pro Ala Tyr Ser Pro Gly His
       370                 375                 380
   Lys Thr Gln Pro Phe Leu His Asp Gly Tyr Ala Val Ser Trp Asn Thr
   385                 390                 395                 400
   Val Glu Asp Ser Ile Ile Arg Thr Gly Phe Gln Gly Glu Ser Gly His
                   405                 410                 415
   Asp Ile Lys Ile Thr Ala Glu Asn Thr Pro Leu Pro Ile Ala Gly Val
               420                 425                 430
   Leu Leu Pro Thr Ile Pro Gly Lys Leu Asp Val Asn Lys Ser Lys Thr
           435                 440                 445
   His Ile Ser Val Asn Gly Arg Lys Ile Arg Met Arg Cys Arg Ala Ile
       450                 455                 460
   Asp Gly Asp Val Thr Phe Cys Arg Pro Lys Ser Pro Val Tyr Val Gly
   465                 470                 475                 480
   Asn Gly Val His Ala Asn Leu His Val Ala Phe His Arg Ser Ser Ser
                   485                 490                 495
   Glu Lys Ile His Ser Asn Glu Ile Ser Ser Asp Ser Ile Gly Val Leu
               500                 505                 510
   Gly Tyr Gln Lys Thr Val Asp His Thr Lys Val Asn Ser Lys Leu Ser
           515                 520                 525
   Leu Phe Phe Glu Ile Lys Ser
       530                 535
<210> 83
   <211> 1315
   <212> PRT
   <213> Plasmodium falciparum
<400> 83
   Met Pro Ile Thr Ile Asn Asn Phe Arg Tyr Ser Asp Pro Val Asn Asn
   1               5                   10                  15
   Asp Thr Ile Ile Met Met Glu Pro Pro Tyr Cys Lys Gly Leu Asp Ile
               20                  25                  30
   Tyr Tyr Lys Ala Phe Lys Ile Thr Asp Arg Ile Trp Ile Val Pro Glu
           35                  40                  45
   Arg Tyr Glu Phe Gly Thr Lys Pro Glu Asp Phe Asn Pro Pro Ser Ser
       50                  55                  60
   Leu Ile Glu Gly Ala Ser Glu Tyr Tyr Asp Pro Asn Tyr Leu Arg Thr
   65                  70                  75                  80
   Asp Ser Asp Lys Asp Arg Phe Leu Gln Thr Met Val Lys Leu Phe Asn
                   85                  90                  95
   Arg Ile Lys Asn Asn Val Ala Gly Glu Ala Leu Leu Asp Lys Ile Ile
               100                 105                 110
   Asn Ala Ile Pro Tyr Leu Gly Asn Ser Tyr Ser Leu Leu Asp Lys Phe
           115                 120                 125
   Asp Thr Asn Ser Asn Ser Val Ser Phe Asn Leu Leu Glu Gln Asp Pro
       130                 135                 140
   Ser Gly Ala Thr Thr Lys Ser Ala Met Leu Thr Asn Leu Ile Ile Phe
   145                 150                 155                 160
   Gly Pro Gly Pro Val Leu Asn Lys Asn Glu Val Arg Gly Ile Val Leu
                   165                 170                 175
   Arg Val Asp Asn Lys Asn Tyr Phe Pro Cys Arg Asp Gly Phe Gly Ser
               180                 185                 190
   Ile Met Gln Met Ala Phe Cys Pro Glu Tyr Val Pro Thr Phe Asp Asn
           195                 200                 205
   Val Ile Glu Asn Ile Thr Ser Leu Thr Ile Gly Lys Ser Lys Tyr Phe
       210                 215                 220
   Gln Asp Pro Ala Leu Leu Leu Met His Glu Leu Ile His Val Leu His
   225                 230                 235                 240
   Gly Leu Tyr Gly Met Gln Val Ser Ser His Glu Ile Ile Pro Ser Lys
                   245                 250                 255
   Gln Glu Ile Tyr Met Gln His Thr Tyr Pro Ile Ser Ala Glu Glu Leu
               260                 265                 270
   Phe Thr Phe Gly Gly Gln Asp Ala Asn Leu Ile Ser Ile Asp Ile Lys
           275                 280                 285
   Asn Asp Leu Tyr Glu Lys Thr Leu Asn Asp Tyr Lys Ala Ile Ala Asn
       290                 295                 300
   Lys Leu Ser Gln Val Thr Ser Cys Asn Asp Pro Asn Ile Asp Ile Asp
   305                 310                 315                 320
   Ser Tyr Lys Gln Ile Tyr Gln Gln Lys Tyr Gln Phe Asp Lys Asp Ser
                   325                 330                 335
   Asn Gly Gln Tyr Ile Val Asn Glu Asp Lys Phe Gln Ile Leu Tyr Asn
               340                 345                 350
   Ser Ile Met Tyr Gly Phe Thr Glu Ile Glu Leu Gly Lys Lys Phe Asn
           355                 360                 365
   Ile Lys Thr Arg Leu Ser Tyr Phe Ser Met Asn His Asp Pro Val Lys
       370                 375                 380
   Ile Pro Asn Leu Leu Asp Asp Thr Ile Tyr Asn Asp Thr Glu Gly Phe
   385                 390                 395                 400
   Asn Ile Glu Ser Lys Asp Leu Lys Ser Glu Tyr Lys Gly Gln Asn Met
                   405                 410                 415
   Arg Val Asn Thr Asn Ala Phe Arg Asn Val Asp Gly Ser Gly Leu Val
               420                 425                 430
   Ser Lys Leu Ile Gly Leu Cys Lys Lys Ile Ile Pro Pro Thr Asn Ile
           435                 440                 445
   Arg Glu Asn Leu Tyr Asn Arg Thr Ala Ser Leu Thr Asp Leu Gly Gly
       450                 455                 460
   Glu Leu Cys Ile Lys Ile Lys Asn Glu Asp Leu Thr Phe Ile Ala Glu
   465                 470                 475                 480
   Lys Asn Ser Phe Ser Glu Glu Pro Phe Gln Asp Glu Ile Val Ser Tyr
                   485                 490                 495
   Asn Thr Lys Asn Lys Pro Leu Asn Phe Asn Tyr Ser Leu Asp Lys Ile
               500                 505                 510
   Ile Val Asp Tyr Asn Leu Gln Ser Lys Ile Thr Leu Pro Asn Asp Arg
           515                 520                 525
   Thr Thr Pro Val Thr Lys Gly Ile Pro Tyr Ala Pro Glu Tyr Lys Ser
       530                 535                 540
   Asn Ala Ala Ser Thr Ile Glu Ile His Asn Ile Asp Asp Asn Thr Ile
   545                 550                 555                 560
   Tyr Gln Tyr Leu Tyr Ala Gln Lys Ser Pro Thr Thr Leu Gln Arg Ile
                   565                 570                 575
   Thr Met Thr Asn Ser Val Asp Asp Ala Leu Ile Asn Ser Thr Lys Ile
               580                 585                 590
   Tyr Ser Tyr Phe Pro Ser Val Ile Ser Lys Val Asn Gln Gly Ala Gln
           595                 600                 605
   Gly Ile Leu Phe Leu Gln Trp Val Arg Asp Ile Ile Asp Asp Phe Thr
       610                 615                 620
   Asn Glu Ser Ser Gln Lys Thr Thr Ile Asp Lys Ile Ser Asp Val Ser
   625                 630                 635                 640
   Thr Ile Val Pro Tyr Ile Gly Pro Ala Leu Asn Ile Val Lys Gln Gly
                   645                 650                 655
   Tyr Glu Gly Asn Phe Ile Gly Ala Leu Glu Thr Thr Gly Val Val Leu
               660                 665                 670
   Leu Leu Glu Tyr Ile Pro Glu Ile Thr Leu Pro Val Ile Ala Ala Leu
           675                 680                 685
   Ser Ile Ala Glu Ser Ser Thr Gln Lys Glu Lys Ile Ile Lys Thr Ile
       690                 695                 700
   Asp Asn Phe Leu Glu Lys Arg Tyr Glu Lys Trp Ile Glu Val Tyr Lys
   705                 710                 715                 720
   Leu Val Lys Ala Lys Trp Leu Gly Thr Val Asn Thr Gln Phe Gln Lys
   725 730 735
   Arg Ser Tyr Gln Met Tyr Arg Ser Leu Glu Tyr Gln Val Asp Ala Ile
   740 745 750
   Lys Lys Ile Ile Asp Tyr Glu Tyr Lys Ile Tyr Ser Gly Pro Asp Lys
   755 760 765
   Glu Gln Ile Ala Asp Glu Ile Asn Asn Leu Lys Asn Lys Leu Glu Glu
   770 775 780
   Lys Ala Asn Lys Ala Met Ile Asn Ile Asn Ile Phe Met Arg Glu Ser
   785 790 795 800
   Ser Arg Ser Phe Leu Val Asn Gln Met Ile Asn Glu Ala Lys Lys Gln
   805 810 815
   Leu Leu Glu Phe Asp Thr Gln Ser Lys Asn Ile Leu Met Gln Tyr Ile
   820 825 830
   Lys Ala Asn Ser Lys Phe Ile Gly Ile Thr Glu Leu Lys Lys Leu Glu
   835 840 845
   Ser Lys Ile Asn Lys Val Phe Ser Thr Pro Ile Pro Phe Ser Tyr Ser
   850 855 860
   Lys Asn Leu Asp Cys Trp Val Asp Asn Glu Glu Asp Ile Asp Val Ile
   865 870 875 880
   Leu Lys Lys Ser Thr Ile Leu Asn Leu Asp Ile Asn Asn Asp Ile Ile
   885 890 895
   Ser Asp Ile Ser Gly Phe Asn Ser Ser Val Ile Thr Tyr Pro Asp Ala
   900 905 910
   Gln Leu Val Pro Gly Ile Asn Gly Lys Ala Ile His Leu Val Asn Asn
   915 920 925
   Glu Ser Ser Glu Val Ile Val His Lys Ala Met Asp Ile Glu Tyr Asn
   930 935 940
   Asp Met Phe Asn Asn Phe Thr Val Ser Phe Trp Leu Arg Val Pro Lys
   945 950 955 960
   Val Ser Ala Ser His Leu Glu Gln Tyr Gly Thr Asn Glu Tyr Ser Ile
   965 970 975
   Ile Ser Ser Met Lys Lys His Ser Leu Ser Ile Gly Ser Gly Trp Ser
               980                 985                 990
   Val Ser Leu Lys Gly Asn Asn Leu  Ile Trp Thr Leu Lys  Asp Ser Ala
           995                 1000                 1005
   Gly Glu  Val Arg Gln Ile Thr  Phe Arg Asp Leu Pro  Asp Lys Phe
       1010                 1015                 1020
   Asn Ala  Tyr Leu Ala Asn Lys  Trp Val Phe Ile Thr  Ile Thr Asn
       1025                 1030                 1035
   Asp Arg  Leu Ser Ser Ala Asn  Leu Tyr Ile Asn Gly  Val Leu Met
       1040                 1045                 1050
   Gly Ser  Ala Glu Ile Thr Gly  Leu Gly Ala Ile Arg  Glu Asp Asn
       1055                 1060                 1065
   Asn Ile  Thr Leu Lys Leu Asp  Arg Cys Asn Asn Asn  Asn Gln Tyr
       1070                 1075                 1080
   Val Ser  Ile Asp Lys Phe Arg  Ile Phe Cys Lys Ala  Leu Asn Pro
       1085                 1090                 1095
   Lys Glu  Ile Glu Lys Leu Tyr  Thr Ser Tyr Leu Ser  Ile Thr Phe
       1100                 1105                 1110
   Leu Arg  Asp Phe Trp Gly Asn  Pro Leu Arg Tyr Asp  Thr Glu Tyr
       1115                 1120                 1125
   Tyr Leu  Ile Pro Val Ala Ser  Ser Ser Lys Asp Val  Gln Leu Lys
       1130                 1135                 1140
   Asn Ile  Thr Asp Tyr Met Tyr  Leu Thr Asn Ala Pro  Ser Tyr Thr
       1145                 1150                 1155
   Asn Gly  Lys Leu Asn Ile Tyr  Tyr Arg Arg Leu Tyr  Asn Gly Leu
       1160                 1165                 1170
   Lys Phe  Ile Ile Lys Arg Tyr  Thr Pro Asn Asn Glu  Ile Asp Ser
       1175                 1180                 1185
   Phe Val  Lys Ser Gly Asp Phe  Ile Lys Leu Tyr Val  Ser Tyr Asn
       1190                 1195                 1200
   Asn Asn  Glu His Ile Val Gly  Tyr Pro Lys Asp Gly  Asn Ala Phe
       1205                 1210                 1215
   Asn Asn Leu Asp Arg Ile Leu Arg Val Gly Tyr Asn Ala Pro Gly
   1220 1225 1230
   Ile Pro Leu Tyr Lys Lys Met Glu Ala Val Lys Leu Arg Asp Leu
   1235 1240 1245
   Lys Thr Tyr Ser Val Gln Leu Lys Leu Tyr Asp Asp Lys Asn Ala
   1250 1255 1260
   Ser Leu Gly Leu Val Gly Thr His Asn Gly Gln Ile Gly Asn Asp
   1265 1270 1275
   Pro Asn Arg Asp Ile Leu Ile Ala Ser Asn Trp Tyr Phe Asn His
   1280 1285 1290
   Leu Lys Asp Lys Ile Leu Gly Cys Asp Trp Tyr Phe Val Pro Thr
   1295 1300 1305
   Asp Glu Gly Trp Thr Asn Asp
   1310 1315
<210> 84
   <211> 364
   <212> PRT
   <213> Plasmodium falciparum
<400> 84
   Met Lys Leu Lys Thr Leu Ala Leu Ser Leu Leu Ala Ala Gly Val Leu
   1 5 10 15
   Ala Gly Cys Ser Ser His Ser Ser Asn Met Ala Asn Thr Gln Met Lys
   20 25 30
   Ser Asp Lys Ile Ile Ile Ala His Arg Gly Ala Ser Gly Tyr Leu Pro
   35 40 45
   Glu His Thr Leu Glu Ser Lys Ala Leu Ala Phe Ala Gln Gln Ala Asp
   50 55 60
   Tyr Leu Glu Gln Asp Leu Ala Met Thr Lys Asp Gly Arg Leu Val Val
   65 70 75 80
   Ile His Asp His Phe Leu Asp Gly Leu Thr Asp Val Ala Lys Lys Phe
   85 90 95
   Pro His Arg His Arg Lys Asp Gly Arg Tyr Tyr Val Ile Asp Phe Thr
   100 105 110
   Leu Lys Glu Ile Gln Ser Leu Glu Met Thr Glu Asn Phe Glu Thr Lys
           115                 120                 125
   Asp Gly Lys Gln Ala Gln Val Tyr Pro Asn Arg Phe Pro Leu Trp Lys
       130                 135                 140
   Ser His Phe Arg Ile His Thr Phe Glu Asp Glu Ile Glu Phe Ile Gln
   145                 150                 155                 160
   Gly Leu Glu Lys Ser Thr Gly Lys Lys Val Gly Ile Tyr Pro Glu Ile
                   165                 170                 175
   Lys Ala Pro Trp Phe His His Gln Asn Gly Lys Asp Ile Ala Ala Glu
               180                 185                 190
   Thr Leu Lys Val Leu Lys Lys Tyr Gly Tyr Asp Lys Lys Thr Asp Met
           195                 200                 205
   Val Tyr Leu Gln Thr Phe Asp Phe Asn Glu Leu Lys Arg Ile Lys Thr
       210                 215                 220
   Glu Leu Leu Pro Gln Met Gly Met Asp Leu Lys Leu Val Gln Leu Ile
   225                 230                 235                 240
   Ala Tyr Thr Asp Trp Lys Glu Thr Gln Glu Lys Asp Pro Lys Gly Tyr
                   245                 250                 255
   Trp Val Asn Tyr Asn Tyr Asp Trp Met Phe Lys Pro Gly Ala Met Ala
               260                 265                 270
   Glu Val Val Lys Tyr Ala Asp Gly Val Gly Pro Gly Trp Tyr Met Leu
           275                 280                 285
   Val Asn Lys Glu Glu Ser Lys Pro Asp Asn Ile Val Tyr Thr Pro Leu
       290                 295                 300
   Val Lys Glu Leu Ala Gln Tyr Asn Val Glu Val His Pro Tyr Thr Val
   305                 310                 315                 320
   Arg Lys Asp Ala Leu Pro Ala Phe Phe Thr Asp Val Asn Gln Met Tyr
                   325                 330                 335
   Asp Ala Leu Leu Asn Lys Ser Gly Ala Thr Gly Val Phe Thr Asp Phe
               340                 345                 350
   Pro Asp Thr Gly Val Glu Phe Leu Lys Gly Ile Lys
           355                 360
<210> 85
   <211> 757
   <212> PRT
   <213> Artificial Sequence
<220>
   <223> PP21 biofusion protein
<400> 85
   Met Lys Tyr Leu Leu Pro Thr Ala Ala Ala Gly Leu Leu Leu Leu Ala
   1               5                   10                  15
   Ala Gln Pro Ala Met Ala Met Gly Lys Thr Lys Glu Tyr Ala Glu Lys
               20                  25                  30
   Ala Lys Asn Ala Tyr Glu Lys Ala Lys Asn Ala Tyr Gln Lys Ala Asn
           35                  40                  45
   Gln Ala Val Leu Lys Ala Lys Glu Ala Ser Ser Tyr Asp Tyr Ile Leu
       50                  55                  60
   Gly Trp Glu Phe Gly Gly Gly Val Pro Glu His Lys Lys Glu Glu Asn
   65                  70                  75                  80
   Met Leu Ser His Leu Tyr Val Ser Ser Lys Asp Lys Glu Asn Ile Ser
                   85                  90                  95
   Lys Glu Asn Asp Asp Val Leu Asp Glu Lys Glu Glu Glu Ala Glu Glu
               100                 105                 110
   Thr Glu Glu Glu Glu Leu Glu Gly Ala Asp Asp Val Val Asp Ser Ser
           115                 120                 125
   Lys Ser Phe Val Met Glu Asn Phe Ser Ser Tyr His Gly Thr Lys Pro
       130                 135                 140
   Gly Tyr Val Asp Ser Ile Gln Lys Gly Ile Gln Lys Pro Lys Ser Gly
   145                 150                 155                 160
   Thr Gln Gly Asn Tyr Asp Asp Asp Trp Lys Glu Phe Tyr Ser Thr Asp
                   165                 170                 175
   Asn Lys Tyr Asp Ala Ala Gly Tyr Ser Val Asp Asn Glu Asn Pro Leu
               180                 185                 190
   Ser Gly Lys Ala Gly Gly Val Val Lys Val Thr Tyr Pro Gly Leu Thr
           195                 200                 205
   Lys Val Leu Ala Leu Lys Val Asp Asn Ala Glu Thr Ile Lys Lys Glu
       210                 215                 220
   Leu Gly Leu Ser Leu Thr Glu Pro Leu Met Glu Gln Val Gly Thr Glu
   225                 230                 235                 240
   Glu Phe Ile Lys Arg Phe Gly Asp Gly Ala Ser Arg Val Val Leu Ser
                   245                 250                 255
   Leu Pro Phe Ala Glu Gly Ser Ser Ser Val Glu Tyr Ile Asn Asn Trp
               260                 265                 270
   Glu Gln Ala Lys Ala Leu Ser Val Glu Leu Glu Ile Asn Phe Glu Thr
           275                 280                 285
   Arg Gly Lys Arg Gly Gln Asp Ala Met Tyr Glu Tyr Met Ala Gln Ala
       290                 295                 300
   Cys Ala Gly Asn Arg Val Arg Arg Ser Val Gly Ser Ser Leu Ser Cys
   305                 310                 315                 320
   Ile Asn Leu Asp Trp Asp Val Ile Arg Asp Lys Thr Lys Thr Lys Ile
                   325                 330                 335
   Glu Ser Leu Lys Glu His Gly Pro Ile Lys Asn Lys Met Ser Glu Ser
               340                 345                 350
   Pro Asn Lys Thr Val Ser Glu Glu Lys Ala Lys Gln Tyr Leu Glu Glu
           355                 360                 365
   Phe His Gln Thr Ala Leu Glu His Pro Glu Leu Ser Glu Leu Lys Thr
       370                 375                 380
   Val Thr Gly Thr Asn Pro Val Phe Ala Gly Ala Asn Tyr Ala Ala Trp
   385                 390                 395                 400
   Ala Val Asn Val Ala Gln Val Ile Asp Ser Glu Thr Ala Asp Asn Leu
                   405                 410                 415
   Glu Lys Thr Thr Ala Ala Leu Ser Ile Leu Pro Gly Ile Gly Ser Val
               420                 425                 430
   Met Gly Ile Ala Asp Gly Ala Val His His Asn Thr Glu Glu Ile Val
           435                 440                 445
   Ala Gln Ser Ile Ala Leu Ser Ser Leu Met Val Ala Gln Ala Ile Pro
       450                 455                 460
   Leu Val Gly Glu Leu Val Asp Ile Gly Phe Ala Ala Tyr Asn Phe Val
   465                 470                 475                 480
   Glu Ser Ile Ile Asn Leu Phe Gln Val Val His Asn Ser Tyr Asn Arg
                   485                 490                 495
   Pro Ala Tyr Ser Pro Gly His Lys Thr Gln Pro Phe Leu His Asp Gly
               500                 505                 510
   Tyr Ala Val Ser Trp Asn Thr Val Glu Asp Ser Ile Ile Arg Thr Gly
           515                 520                 525
   Phe Gln Gly Glu Ser Gly His Asp Ile Lys Ile Thr Ala Glu Asn Thr
       530                 535                 540
   Pro Leu Pro Ile Ala Gly Val Leu Leu Pro Thr Ile Pro Gly Lys Leu
   545                 550                 555                 560
   Asp Val Asn Lys Ser Lys Thr His Ile Ser Val Asn Gly Arg Lys Ile
                   565                 570                 575
   Arg Met Arg Cys Arg Ala Ile Asp Gly Asp Val Thr Phe Cys Arg Pro
               580                 585                 590
   Lys Ser Pro Val Tyr Val Gly Asn Gly Val His Ala Asn Leu His Val
           595                 600                 605
   Ala Phe His Arg Ser Ser Ser Glu Lys Ile His Ser Asn Glu Ile Ser
       610                 615                 620
   Ser Asp Ser Ile Gly Val Leu Gly Tyr Gln Lys Thr Val Asp His Thr
   625                 630                 635                 640
   Lys Val Asn Ser Lys Leu Ser Leu Phe Phe Glu Ile Lys Ser Lys Thr
                   645                 650                 655
   Lys Glu Tyr Ala Glu Lys Ala Lys Asn Ala Tyr Glu Lys Ala Lys Asn
               660                 665                 670
   Ala Tyr Gln Lys Ala Asn Gln Ala Val Leu Lys Ala Lys Glu Ala Ser
           675                 680                 685
   Ser Tyr Asp Tyr Ile Leu Gly Trp Glu Phe Gly Gly Gly Val Pro Glu
       690                 695                 700
   His Lys Lys Glu Glu Asn Met Leu Ser His Leu Tyr Val Ser Ser Lys
   705 710 715 720
   Asp Lys Glu Asn Ile Ser Lys Glu Asn Asp Asp Val Leu Asp Glu Lys
   725 730 735
   Glu Glu Glu Ala Glu Glu Thr Glu Glu Glu Glu Leu Glu Leu Glu His
   740 745 750
   His His His His His
   755
<210> 86
   <211> 727
   <212> PRT
   <213> Artificial Sequence
<220>
   <223> PP22 biofusion protein
<400> 86
   Met Lys Tyr Leu Leu Pro Thr Ala Ala Ala Gly Leu Leu Leu Leu Ala
   1               5                   10                  15
   Ala Gln Pro Ala Met Ala Met Gly Leu Asn Asn Asn Ile Leu Gly Trp
               20                  25                  30
   Glu Phe Gly Gly Gly Ala Pro Gln Asn Gly Ala Ala Glu Asp Lys Lys
           35                  40                  45
   Thr Glu Tyr Leu Leu Glu Gln Ile Lys Ile Pro Ser Trp Asp Arg Asn
       50                  55                  60
   Asn Ile Pro Asp Glu Asn Glu Gln Val Ile Glu Asp Pro Gln Glu Asp
   65                  70                  75                  80
   Asn Lys Asp Glu Asp Glu Asp Glu Glu Thr Glu Thr Glu Asn Leu Glu
                   85                  90                  95
   Thr Glu Asp Asp Asn Asn Glu Glu Gly Ala Asp Asp Val Val Asp Ser
               100                 105                 110
   Ser Lys Ser Phe Val Met Glu Asn Phe Ser Ser Tyr His Gly Thr Lys
           115                 120                 125
   Pro Gly Tyr Val Asp Ser Ile Gln Lys Gly Ile Gln Lys Pro Lys Ser
       130                 135                 140
   Gly Thr Gln Gly Asn Tyr Asp Asp Asp Trp Lys Glu Phe Tyr Ser Thr
   145                 150                 155                 160
   Asp Asn Lys Tyr Asp Ala Ala Gly Tyr Ser Val Asp Asn Glu Asn Pro
                   165                 170                 175
   Leu Ser Gly Lys Ala Gly Gly Val Val Lys Val Thr Tyr Pro Gly Leu
               180                 185                 190
   Thr Lys Val Leu Ala Leu Lys Val Asp Asn Ala Glu Thr Ile Lys Lys
           195                 200                 205
   Glu Leu Gly Leu Ser Leu Thr Glu Pro Leu Met Glu Gln Val Gly Thr
       210                 215                 220
   Glu Glu Phe Ile Lys Arg Phe Gly Asp Gly Ala Ser Arg Val Val Leu
   225                 230                 235                 240
   Ser Leu Pro Phe Ala Glu Gly Ser Ser Ser Val Glu Tyr Ile Asn Asn
                   245                 250                 255
   Trp Glu Gln Ala Lys Ala Leu Ser Val Glu Leu Glu Ile Asn Phe Glu
               260                 265                 270
   Thr Arg Gly Lys Arg Gly Gln Asp Ala Met Tyr Glu Tyr Met Ala Gln
           275                 280                 285
   Ala Cys Ala Gly Asn Arg Val Arg Arg Ser Val Gly Ser Ser Leu Ser
       290                 295                 300
   Cys Ile Asn Leu Asp Trp Asp Val Ile Arg Asp Lys Thr Lys Thr Lys
   305                 310                 315                 320
   Ile Glu Ser Leu Lys Glu His Gly Pro Ile Lys Asn Lys Met Ser Glu
                   325                 330                 335
   Ser Pro Asn Lys Thr Val Ser Glu Glu Lys Ala Lys Gln Tyr Leu Glu
               340                 345                 350
   Glu Phe His Gln Thr Ala Leu Glu His Pro Glu Leu Ser Glu Leu Lys
           355                 360                 365
   Thr Val Thr Gly Thr Asn Pro Val Phe Ala Gly Ala Asn Tyr Ala Ala
       370                 375                 380
   Trp Ala Val Asn Val Ala Gln Val Ile Asp Ser Glu Thr Ala Asp Asn
   385                 390                 395                 400
   Leu Glu Lys Thr Thr Ala Ala Leu Ser Ile Leu Pro Gly Ile Gly Ser
   405 410 415
   Val Met Gly Ile Ala Asp Gly Ala Val His His Asn Thr Glu Glu Ile
   420 425 430
   Val Ala Gln Ser Ile Ala Leu Ser Ser Leu Met Val Ala Gln Ala Ile
   435 440 445
   Pro Leu Val Gly Glu Leu Val Asp Ile Gly Phe Ala Ala Tyr Asn Phe
   450 455 460
   Val Glu Ser Ile Ile Asn Leu Phe Gln Val Val His Asn Ser Tyr Asn
   465 470 475 480
   Arg Pro Ala Tyr Ser Pro Gly His Lys Thr Gln Pro Phe Leu His Asp
   485 490 495
   Gly Tyr Ala Val Ser Trp Asn Thr Val Glu Asp Ser Ile Ile Arg Thr
   500 505 510
   Gly Phe Gln Gly Glu Ser Gly His Asp Ile Lys Ile Thr Ala Glu Asn
   515 520 525
   Thr Pro Leu Pro Ile Ala Gly Val Leu Leu Pro Thr Ile Pro Gly Lys
   530 535 540
   Leu Asp Val Asn Lys Ser Lys Thr His Ile Ser Val Asn Gly Arg Lys
   545 550 555 560
   Ile Arg Met Arg Cys Arg Ala Ile Asp Gly Asp Val Thr Phe Cys Arg
   565 570 575
   Pro Lys Ser Pro Val Tyr Val Gly Asn Gly Val His Ala Asn Leu His
   580 585 590
   Val Ala Phe His Arg Ser Ser Ser Glu Lys Ile His Ser Asn Glu Ile
   595 600 605
   Ser Ser Asp Ser Ile Gly Val Leu Gly Tyr Gln Lys Thr Val Asp His
   610 615 620
   Thr Lys Val Asn Ser Lys Leu Ser Leu Phe Phe Glu Ile Lys Ser Leu
   625 630 635 640
   Asn Asn Asn Ile Leu Gly Trp Glu Phe Gly Gly Gly Ala Pro Gln Asn
   645 650 655
   Gly Ala Ala Glu Asp Lys Lys Thr Glu Tyr Leu Leu Glu Gln Ile Lys
   660 665 670
   Ile Pro Ser Trp Asp Arg Asn Asn Ile Pro Asp Glu Asn Glu Gln Val
   675 680 685
   Ile Glu Asp Pro Gln Glu Asp Asn Lys Asp Glu Asp Glu Asp Glu Glu
   690 695 700
   Thr Glu Thr Glu Asn Leu Glu Thr Glu Asp Asp Asn Asn Glu Glu Leu
   705 710 715 720
   Glu His His His His His His
   725
<210> 87
   <211> 709
   <212> PRT
   <213> Artificial Sequence
<220>
   <223> PP23 biofusion protein
<400> 87
   Met Lys Tyr Leu Leu Pro Thr Ala Ala Ala Gly Leu Leu Leu Leu Ala
   1               5                   10                  15
   Ala Gln Pro Ala Met Ala Met Gly Leu Asn Asn Asn Ile Leu Gly Trp
               20                  25                  30
   Glu Phe Gly Gly Gly Ala Pro Gln Asn Gly Ala Ala Glu Asp Lys Lys
           35                  40                  45
   Thr Glu Tyr Leu Leu Glu Gln Ile Lys Ile Pro Ser Trp Asp Arg Asn
       50                  55                  60
   Asn Ile Pro Asp Glu Asn Glu Gln Val Ile Glu Asp Pro Gln Glu Asp
   65                  70                  75                  80
   Asn Lys Asp Glu Asp Glu Asp Glu Glu Thr Glu Thr Glu Asn Leu Glu
                   85                  90                  95
   Thr Glu Asp Asp Asn Asn Glu Glu Gly Ala Asp Asp Val Val Asp Ser
               100                 105                 110
   Ser Lys Ser Phe Val Met Glu Asn Phe Ser Ser Tyr His Gly Thr Lys
           115                 120                 125
   Pro Gly Tyr Val Asp Ser Ile Gln Lys Gly Ile Gln Lys Pro Lys Ser
   130 135 140
   Gly Thr Gln Gly Asn Tyr Asp Asp Asp Trp Lys Glu Phe Tyr Ser Thr
   145 150 155 160
   Asp Asn Lys Tyr Asp Ala Ala Gly Tyr Ser Val Asp Asn Glu Asn Pro
   165 170 175
   Leu Ser Gly Lys Ala Gly Gly Val Val Lys Val Thr Tyr Pro Gly Leu
   180 185 190
   Thr Lys Val Leu Ala Leu Lys Val Asp Asn Ala Glu Thr Ile Lys Lys
   195 200 205
   Glu Leu Gly Leu Ser Leu Thr Glu Pro Leu Met Glu Gln Val Gly Thr
   210 215 220
   Glu Glu Phe Ile Lys Arg Phe Gly Asp Gly Ala Ser Arg Val Val Leu
   225 230 235 240
   Ser Leu Pro Phe Ala Glu Gly Ser Ser Ser Val Glu Tyr Ile Asn Asn
   245 250 255
   Trp Glu Gln Ala Lys Ala Leu Ser Val Glu Leu Glu Ile Asn Phe Glu
   260 265 270
   Thr Arg Gly Lys Arg Gly Gln Asp Ala Met Tyr Glu Tyr Met Ala Gln
   275 280 285
   Ala Cys Ala Gly Asn Arg Val Arg Arg Ser Val Gly Ser Ser Leu Ser
   290 295 300
   Cys Ile Asn Leu Asp Trp Asp Val Ile Arg Asp Lys Thr Lys Thr Lys
   305 310 315 320
   Ile Glu Ser Leu Lys Glu His Gly Pro Ile Lys Asn Lys Met Ser Glu
   325 330 335
   Ser Pro Asn Lys Thr Val Ser Glu Glu Lys Ala Lys Gln Tyr Leu Glu
   340 345 350
   Glu Phe His Gln Thr Ala Leu Glu His Pro Glu Leu Ser Glu Leu Lys
   355 360 365
   Thr Val Thr Gly Thr Asn Pro Val Phe Ala Gly Ala Asn Tyr Ala Ala
   370 375 380
   Trp Ala Val Asn Val Ala Gln Val Ile Asp Ser Glu Thr Ala Asp Asn
   385                 390                 395                 400
   Leu Glu Lys Thr Thr Ala Ala Leu Ser Ile Leu Pro Gly Ile Gly Ser
                   405                 410                 415
   Val Met Gly Ile Ala Asp Gly Ala Val His His Asn Thr Glu Glu Ile
               420                 425                 430
   Val Ala Gln Ser Ile Ala Leu Ser Ser Leu Met Val Ala Gln Ala Ile
           435                 440                 445
   Pro Leu Val Gly Glu Leu Val Asp Ile Gly Phe Ala Ala Tyr Asn Phe
       450                 455                 460
   Val Glu Ser Ile Ile Asn Leu Phe Gln Val Val His Asn Ser Tyr Asn
   465                 470                 475                 480
   Arg Pro Ala Tyr Ser Pro Gly His Lys Thr Gln Pro Phe Leu His Asp
                   485                 490                 495
   Gly Tyr Ala Val Ser Trp Asn Thr Val Glu Asp Ser Ile Ile Arg Thr
               500                 505                 510
   Gly Phe Gln Gly Glu Ser Gly His Asp Ile Lys Ile Thr Ala Glu Asn
           515                 520                 525
   Thr Pro Leu Pro Ile Ala Gly Val Leu Leu Pro Thr Ile Pro Gly Lys
       530                 535                 540
   Leu Asp Val Asn Lys Ser Lys Thr His Ile Ser Val Asn Gly Arg Lys
   545                 550                 555                 560
   Ile Arg Met Arg Cys Arg Ala Ile Asp Gly Asp Val Thr Phe Cys Arg
                   565                 570                 575
   Pro Lys Ser Pro Val Tyr Val Gly Asn Gly Val His Ala Asn Leu His
               580                 585                 590
   Val Ala Phe His Arg Ser Ser Ser Glu Lys Ile His Ser Asn Glu Ile
           595                 600                 605
   Ser Ser Asp Ser Ile Gly Val Leu Gly Tyr Gln Lys Thr Val Asp His
       610                 615                 620
   Thr Lys Val Asn Ser Lys Leu Ser Leu Phe Phe Glu Ile Lys Ser Ser
   625                 630                 635                 640
   Asn Glu Lys Gly Arg Pro Pro Thr Tyr Ser Pro Ile Leu Asp Asp Gly
   645 650 655
   Ile Glu Phe Ser Gly Gly Leu Tyr Phe Asn Glu Lys Lys Ser Thr Glu
   660 665 670
   Glu Asn Lys Gln Lys Asn Val Leu Glu Ser Val Asn Leu Thr Ser Trp
   675 680 685
   Asp Lys Glu Asp Ile Val Lys Glu Asn Glu Asp Val Lys Leu Glu His
   690 695 700
   His His His His His
   705
<210> 88
   <211> 867
   <212> PRT
   <213> Artificial Sequence
<220>
   <223> PP25 biofusion protein
<400> 88
   Met Lys Tyr Leu Leu Pro Thr Ala Ala Ala Gly Leu Leu Leu Leu Ala
   1 5 10 15
   Ala Gln Pro Ala Met Ala Met Gly Ser Asp Glu Leu Phe Asn Glu Leu
   20 25 30
   Leu Asn Ser Val Asp Val Asn Gly Glu Val Lys Glu Asn Ile Leu Glu
   35 40 45
   Glu Ser Gln Val Asn Asp Asp Ile Phe Asn Ser Leu Val Lys Ser Val
   50 55 60
   Gln Gln Glu Gln Gln His Asn Val Glu Glu Lys Val Glu Glu Ser Val
   65 70 75 80
   Glu Glu Asn Asp Glu Glu Ser Val Glu Glu Asn Val Glu Glu Asn Val
   85 90 95
   Glu Glu Asn Asp Asp Glu Ser Val Ala Ser Ser Val Glu Glu Ser Ile
   100 105 110
   Ala Ser Ser Val Asp Glu Ser Ile Asp Ser Ser Ile Glu Glu Asn Val
   115 120 125
   Ala Pro Thr Val Glu Glu Ile Val Ala Pro Thr Val Glu Glu Ile Val
       130                 135                 140
   Ala Pro Ser Val Val Glu Ser Val Ala Pro Ser Val Glu Glu Ser Val
   145                 150                 155                 160
   Glu Glu Asn Val Glu Glu Ser Val Ala Glu Asn Val Glu Glu Gly Ala
                   165                 170                 175
   Asp Asp Val Val Asp Ser Ser Lys Ser Phe Val Met Glu Asn Phe Ser
               180                 185                 190
   Ser Tyr His Gly Thr Lys Pro Gly Tyr Val Asp Ser Ile Gln Lys Gly
           195                 200                 205
   Ile Gln Lys Pro Lys Ser Gly Thr Gln Gly Asn Tyr Asp Asp Asp Trp
       210                 215                 220
   Lys Glu Phe Tyr Ser Thr Asp Asn Lys Tyr Asp Ala Ala Gly Tyr Ser
   225                 230                 235                 240
   Val Asp Asn Glu Asn Pro Leu Ser Gly Lys Ala Gly Gly Val Val Lys
                   245                 250                 255
   Val Thr Tyr Pro Gly Leu Thr Lys Val Leu Ala Leu Lys Val Asp Asn
               260                 265                 270
   Ala Glu Thr Ile Lys Lys Glu Leu Gly Leu Ser Leu Thr Glu Pro Leu
           275                 280                 285
   Met Glu Gln Val Gly Thr Glu Glu Phe Ile Lys Arg Phe Gly Asp Gly
       290                 295                 300
   Ala Ser Arg Val Val Leu Ser Leu Pro Phe Ala Glu Gly Ser Ser Ser
   305                 310                 315                 320
   Val Glu Tyr Ile Asn Asn Trp Glu Gln Ala Lys Ala Leu Ser Val Glu
                   325                 330                 335
   Leu Glu Ile Asn Phe Glu Thr Arg Gly Lys Arg Gly Gln Asp Ala Met
               340                 345                 350
   Tyr Glu Tyr Met Ala Gln Ala Cys Ala Gly Asn Arg Val Arg Arg Ser
           355                 360                 365
   Val Gly Ser Ser Leu Ser Cys Ile Asn Leu Asp Trp Asp Val Ile Arg
       370                 375                 380
   Asp Lys Thr Lys Thr Lys Ile Glu Ser Leu Lys Glu His Gly Pro Ile
   385 390 395 400
   Lys Asn Lys Met Ser Glu Ser Pro Asn Lys Thr Val Ser Glu Glu Lys
   405 410 415
   Ala Lys Gln Tyr Leu Glu Glu Phe His Gln Thr Ala Leu Glu His Pro
   420 425 430
   Glu Leu Ser Glu Leu Lys Thr Val Thr Gly Thr Asn Pro Val Phe Ala
   435 440 445
   Gly Ala Asn Tyr Ala Ala Trp Ala Val Asn Val Ala Gln Val Ile Asp
   450 455 460
   Ser Glu Thr Ala Asp Asn Leu Glu Lys Thr Thr Ala Ala Leu Ser Ile
   465 470 475 480
   Leu Pro Gly Ile Gly Ser Val Met Gly Ile Ala Asp Gly Ala Val His
   485 490 495
   His Asn Thr Glu Glu Ile Val Ala Gln Ser Ile Ala Leu Ser Ser Leu
   500 505 510
   Met Val Ala Gln Ala Ile Pro Leu Val Gly Glu Leu Val Asp Ile Gly
   515 520 525
   Phe Ala Ala Tyr Asn Phe Val Glu Ser Ile Ile Asn Leu Phe Gln Val
   530 535 540
   Val His Asn Ser Tyr Asn Arg Pro Ala Tyr Ser Pro Gly His Lys Thr
   545 550 555 560
   Gln Pro Phe Leu His Asp Gly Tyr Ala Val Ser Trp Asn Thr Val Glu
   565 570 575
   Asp Ser Ile Ile Arg Thr Gly Phe Gln Gly Glu Ser Gly His Asp Ile
   580 585 590
   Lys Ile Thr Ala Glu Asn Thr Pro Leu Pro Ile Ala Gly Val Leu Leu
   595 600 605
   Pro Thr Ile Pro Gly Lys Leu Asp Val Asn Lys Ser Lys Thr His Ile
   610 615 620
   Ser Val Asn Gly Arg Lys Ile Arg Met Arg Cys Arg Ala Ile Asp Gly
   625 630 635 640
   Asp Val Thr Phe Cys Arg Pro Lys Ser Pro Val Tyr Val Gly Asn Gly
   645 650 655
   Val His Ala Asn Leu His Val Ala Phe His Arg Ser Ser Ser Glu Lys
   660 665 670
   Ile His Ser Asn Glu Ile Ser Ser Asp Ser Ile Gly Val Leu Gly Tyr
   675 680 685
   Gln Lys Thr Val Asp His Thr Lys Val Asn Ser Lys Leu Ser Leu Phe
   690 695 700
   Phe Glu Ile Lys Ser Ser Asp Glu Leu Phe Asn Glu Leu Leu Asn Ser
   705 710 715 720
   Val Asp Val Asn Gly Glu Val Lys Glu Asn Ile Leu Glu Glu Ser Gln
   725 730 735
   Val Asn Asp Asp Ile Phe Asn Ser Leu Val Lys Ser Val Gln Gln Glu
   740 745 750
   Gln Gln His Asn Val Glu Glu Lys Val Glu Glu Ser Val Glu Glu Asn
   755 760 765
   Asp Glu Glu Ser Val Glu Glu Asn Val Glu Glu Asn Val Glu Glu Asn
   770 775 780
   Asp Asp Glu Ser Val Ala Ser Ser Val Glu Glu Ser Ile Ala Ser Ser
   785 790 795 800
   Val Asp Glu Ser Ile Asp Ser Ser Ile Glu Glu Asn Val Ala Pro Thr
   805 810 815
   Val Glu Glu Ile Val Ala Pro Thr Val Glu Glu Ile Val Ala Pro Ser
   820 825 830
   Val Val Glu Ser Val Ala Pro Ser Val Glu Glu Ser Val Glu Glu Asn
   835 840 845
   Val Glu Glu Ser Val Ala Glu Asn Val Glu Glu Leu Glu His His His
   850 855 860
   His His His
   865
<210> 89
   <211> 807
   <212> PRT
   <213> Plasmodium falciparum
<400> 89
   Ile Pro Glu Glu Gln Ile Glu Glu Val Ile Gln Glu Glu Ile Ile Glu
   1               5                   10                  15
   Gln Val Val Pro Glu Glu Leu Ile Glu Glu Val Val Pro Glu Glu Ile
               20                  25                  30
   Ile Glu Glu Val Ile Pro Glu Glu Ile Val Glu Glu Val Ile Tyr Glu
           35                  40                  45
   Glu Val Ile Pro Glu Glu Leu Val Glu Glu Val Ile Ala Glu Lys Leu
       50                  55                  60
   Val Lys Glu Ile Val Pro Glu Gln Val Arg Glu Glu Val Thr Leu Glu
   65                  70                  75                  80
   Glu Ile Val Glu Glu Met Ile Pro Glu Glu Phe Val Glu Glu Val Ala
                   85                  90                  95
   Pro Glu Val Glu Ile Glu Glu Ile Ile Pro Glu Glu Leu Ile Glu Glu
               100                 105                 110
   Val Ile Pro Glu Val Leu Val Glu Glu Ala Val Pro Glu Glu Leu Ile
           115                 120                 125
   Glu Lys Val Ile Pro Gly Ala Asp Asp Val Val Asp Ser Ser Lys Ser
       130                 135                 140
   Phe Val Met Glu Asn Phe Ser Ser Tyr His Gly Thr Lys Pro Gly Tyr
   145                 150                 155                 160
   Val Asp Ser Ile Gln Lys Gly Ile Gln Lys Pro Lys Ser Gly Thr Gln
                   165                 170                 175
   Gly Asn Tyr Asp Asp Asp Trp Lys Glu Phe Tyr Ser Thr Asp Asn Lys
               180                 185                 190
   Tyr Asp Ala Ala Gly Tyr Ser Val Asp Asn Glu Asn Pro Leu Ser Gly
           195                 200                 205
   Lys Ala Gly Gly Val Val Lys Val Thr Tyr Pro Gly Leu Thr Lys Val
       210                 215                 220
   Leu Ala Leu Lys Val Asp Asn Ala Glu Thr Ile Lys Lys Glu Leu Gly
   225                 230                 235                 240
   Leu Ser Leu Thr Glu Pro Leu Met Glu Gln Val Gly Thr Glu Glu Phe
                   245                 250                 255
   Ile Lys Arg Phe Gly Asp Gly Ala Ser Arg Val Val Leu Ser Leu Pro
               260                 265                 270
   Phe Ala Glu Gly Ser Ser Ser Val Glu Tyr Ile Asn Asn Trp Glu Gln
           275                 280                 285
   Ala Lys Ala Leu Ser Val Glu Leu Glu Ile Asn Phe Glu Thr Arg Gly
       290                 295                 300
   Lys Arg Gly Gln Asp Ala Met Tyr Glu Tyr Met Ala Gln Ala Cys Ala
   305                 310                 315                 320
   Gly Asn Arg Val Arg Arg Ser Val Gly Ser Ser Leu Ser Cys Ile Asn
                   325                 330                 335
   Leu Asp Trp Asp Val Ile Arg Asp Lys Thr Lys Thr Lys Ile Glu Ser
               340                 345                 350
   Leu Lys Glu His Gly Pro Ile Lys Asn Lys Met Ser Glu Ser Pro Asn
           355                 360                 365
   Lys Thr Val Ser Glu Glu Lys Ala Lys Gln Tyr Leu Glu Glu Phe His
       370                 375                 380
   Gln Thr Ala Leu Glu His Pro Glu Leu Ser Glu Leu Lys Thr Val Thr
   385                 390                 395                 400
   Gly Thr Asn Pro Val Phe Ala Gly Ala Asn Tyr Ala Ala Trp Ala Val
                   405                 410                 415
   Asn Val Ala Gln Val Ile Asp Ser Glu Thr Ala Asp Asn Leu Glu Lys
               420                 425                 430
   Thr Thr Ala Ala Leu Ser Ile Leu Pro Gly Ile Gly Ser Val Met Gly
           435                 440                 445
   Ile Ala Asp Gly Ala Val His His Asn Thr Glu Glu Ile Val Ala Gln
       450                 455                 460
   Ser Ile Ala Leu Ser Ser Leu Met Val Ala Gln Ala Ile Pro Leu Val
   465                 470                 475                 480
   Gly Glu Leu Val Asp Ile Gly Phe Ala Ala Tyr Asn Phe Val Glu Ser
                   485                 490                 495
   Ile Ile Asn Leu Phe Gln Val Val His Asn Ser Tyr Asn Arg Pro Ala
               500                 505                 510
   Tyr Ser Pro Gly His Lys Thr Gln Pro Phe Leu His Asp Gly Tyr Ala
           515                 520                 525
   Val Ser Trp Asn Thr Val Glu Asp Ser Ile Ile Arg Thr Gly Phe Gln
       530                 535                 540
   Gly Glu Ser Gly His Asp Ile Lys Ile Thr Ala Glu Asn Thr Pro Leu
   545                 550                 555                 560
   Pro Ile Ala Gly Val Leu Leu Pro Thr Ile Pro Gly Lys Leu Asp Val
                   565                 570                 575
   Asn Lys Ser Lys Thr His Ile Ser Val Asn Gly Arg Lys Ile Arg Met
               580                 585                 590
   Arg Cys Arg Ala Ile Asp Gly Asp Val Thr Phe Cys Arg Pro Lys Ser
           595                 600                 605
   Pro Val Tyr Val Gly Asn Gly Val His Ala Asn Leu His Val Ala Phe
       610                 615                 620
   His Arg Ser Ser Ser Glu Lys Ile His Ser Asn Glu Ile Ser Ser Asp
   625                 630                 635                 640
   Ser Ile Gly Val Leu Gly Tyr Gln Lys Thr Val Asp His Thr Lys Val
                   645                 650                 655
   Asn Ser Lys Leu Ser Leu Phe Phe Glu Ile Lys Ser Ile Pro Glu Glu
               660                 665                 670
   Gln Ile Glu Glu Val Ile Gln Glu Glu Ile Ile Glu Gln Val Val Pro
           675                 680                 685
   Glu Glu Leu Ile Glu Glu Val Val Pro Glu Glu Ile Ile Glu Glu Val
       690                 695                 700
   Ile Pro Glu Glu Ile Val Glu Glu Val Ile Tyr Glu Glu Val Ile Pro
   705                 710                 715                 720
   Glu Glu Leu Val Glu Glu Val Ile Ala Glu Lys Leu Val Lys Glu Ile
                   725                 730                 735
   Val Pro Glu Gln Val Arg Glu Glu Val Thr Leu Glu Glu Ile Val Glu
               740                 745                 750
   Glu Met Ile Pro Glu Glu Phe Val Glu Glu Val Ala Pro Glu Val Glu
           755                 760                 765
   Ile Glu Glu Ile Ile Pro Glu Glu Leu Ile Glu Glu Val Ile Pro Glu
       770                 775                 780
   Val Leu Val Glu Glu Ala Val Pro Glu Glu Leu Ile Glu Lys Val Ile
   785                 790                 795                 800
   Pro His His His His His His
                   805